# EUROPEAN PATENT APPLICATION

(11) **EP 4 843 485 A2**
(43) Date of publication of application: **25.11.2026**
(21) Application number: 26211678.3
(22) Date of filing: 03.03.2022
(51) Int. Cl.: A61B 5/00

(54) **RESUSCITATIVE CARE SYSTEM FOR CONTEXT SENSITIVE GUIDANCE**

(30) Priority: 11.03.2021 US 202163159735 P
(62) Document number(s) of the earlier application(s) in accordance with Art. 76 EPC: 22711843.7 / 4 305 638
(71) Applicant: ZOLL Medical Corporation, Chelmsford, MA 01824 (US)
(72) Inventor: BECK, George, Salem, 01970 (US); FREEMAN, Gary A., Waltham, 02453 (US); GIACOMETTI, Paolo, North Grafton, 01536 (US)
(74) Representative: Potter Clarkson

(57) **Abstract**

A context sensitive guidance (CSG) system for providing clinical interventions to a patient in an emergency medical event includes a CSG engine, patient interface devices configured to generate signals indicative of patient physiologic data, and a display device configured to provide a CSG user interface and medical device(s) configured to couple to the patient interface devices, the CSG engine and the display device, receive the signals, generate the physiologic data from the signals, and send the physiologic data to the CSG engine and the display device, wherein the CSG engine is configured to receive and evaluate the physiologic data, identify a clinical intervention, and send an output based on the clinical intervention to the medical device(s) configured to perform at least one operation in response to the sent output.

## Description

### CROSS-REFERENCE TO RELATED APPLICATIONS

This application claims the benefit under 35 U.S.C. §119(e) of U.S. Provisional Application No. 63/159,735 filed March 11, 2021. All subject matter set forth in the above referenced application is hereby incorporated by reference in its entirety into the present application as if fully set forth herein.

### BACKGROUND

In a pre-hospital and/or acute care treatment setting, medical responders often have difficulty in accurately determining the most effective medical interventions for a patient. In these settings, split second decisions about interventions for emergency conditions such as respiratory distress, cardiac arrest, and/or trauma are often required based on a minimal amount of information about a patient.

Respiratory distress (RD) is a common patient complaint. By some accounts, RD is a chief complaint in approximately 12% of prehospital EMS calls. During an emergency response based on a 911 call for a victim of respiratory distress, medical responders may be encountering the patient for the first time without knowledge of medical history and may have to perform immediate interventions to prevent the patient from dying before the patient can be transported to a hospital or another setting providing advanced care and diagnostics. A similar situation may occur in a battlefield triage, a hospital emergency room, or at a scene of a mass casualty event. In such settings, even well-trained paramedics, nurses, or physicians often have difficulty in identifying the most effective immediate interventions.

On its own, RD may be associated with a nonspecific finding where the root cause or causes can come from the respiratory, cardiac, endocrine or other systems. Determining the etiology of RD may be complex, often leaving care providers to provide interventions in the pre-hospital environment without a diagnosis of etiology.

To alleviate these difficulties, rescuers can benefit from tools that guide decision-making. Information about physiologic data, interventions delivered to the patient, and the patient's health history and status may be collected by sensors and from various databases. Such information may be integrated and analyzed in an automated fashion to provide lifesaving guidance for effective and immediate medical interventions.

### SUMMARY

The foregoing general description of the illustrative implementations and the following detailed description thereof are merely exemplary aspects of the teachings of this disclosure and are not restrictive.

An example of a context sensitive guidance (CSG) system for providing clinical interventions to a patient in an emergency medical event includes a CSG engine comprising hardware logic and/or software logic, a plurality of patient interface devices configured to generate sensor signals indicative of physiologic data for the patient, and a display device configured to provide a CSG user interface (UI), and at least one medical device configured to couple to the plurality of patient interface devices, communicatively couple to the at CSG engine and the display device, receive the sensor signals from the plurality of patient interface devices, generate the physiologic data from the sensor signals, and send the physiologic data to the CSG engine and the display device, wherein the CSG engine is configured to receive an input from the at least one medical device including the physiologic data, evaluate the physiologic data, identify at least one clinical intervention for the patient based on the evaluation, and send an output to the at least one medical device based on the identified clinical intervention, wherein the at least one medical device may be configured to perform at least one operation in response to the output from the CSG engine.

Implementations of such a system may include one or more of the following features. The at least one clinical intervention may include a high flow nasal cannula. The at least one clinical intervention may include one of CPAP or bilevel ventilation. The at least one clinical intervention may include a pharmacological intervention. The output from the CSG engine may include instructions to perform the identified clinical intervention, and the at least one operation performed by the at least one medical device may include the identified clinical intervention. The output from the CSG engine may include at least one CSG event marker associated with the identified clinical intervention, and the at least one operation performed by the at least one medical device may include a recordation of the at least one CSG event marker at a data storage region of the at least one medical device in response to receiving the output from the CSG engine. The physiologic data may be first physiologic data and the CSG engine may be configured to receive second physiologic data from the at least one medical device, update the at least one clinical intervention for the patient based on the second physiologic data, and send instructions to the at least one medical device based on the updated clinical intervention. The CSG engine may be configured to generate an etiology estimate for at least one patient presentation based on the first and second physiologic data, request targeted patient data based on the etiology estimate, receive the targeted patient data, convert the etiology estimate to an etiology determination based on the targeted patient data, and provide the etiology determination to one or more of the at least one medical device and the CSG UI. The CSG engine may be configured to identify at least one pharmacological intervention based on the etiology determination, provide a prompt for user confirmation of the at least one pharmacological intervention at the CSG UI, and send instructions to the at least one medical device based on the at least one pharmacological intervention. The instructions based on the at least one pharmacological intervention may include instructions may include a CSG event marker for the at least one pharmacological intervention. The instructions based on the at least one pharmacological intervention may include instructions may include an instruction to provide the at least one pharmacological intervention. The CSG system may include a mobile device, wherein the display device may be disposed at the mobile device. The mobile device may include the CSG engine. The mobile device may be communicatively coupled to a cloud server including the CSG engine. The mobile device may include the CSG engine. The at least one medical device may be configured to communicatively couple to the mobile device via a wireless communication link. The wireless communication link may be at least one of a Wi-Fi link and a Bluetooth link. The wireless communication link may be a preconfigured pairing between the at least one medical device and the mobile device. The CSG engine may be configured to receive an input from the mobile device including an assessment of patient breathing, evaluate the assessment of patient breathing for a respiratory distress presentation or a non-respiratory distress presentation, and if the assessment of patient breathing indicates the respiratory distress presentation, then implement a respiratory distress (RD) CSG engine, else implement a non-respiratory distress CSG engine. The CSG engine may include a RD CSG engine. The RD CSG engine may include a respiration/ventilation (R/V) CSG engine and a hemodynamic CSG engine. The at least one medical device may include a patient monitor/defibrillator and a ventilation system. The RD CSG engine may be configured to receive input from the patient monitor/defibrillator and the ventilation system. The plurality of patient interface devices may be respiratory gas sensors configured to couple to the patient monitor/defibrillator and lung mechanics sensors configured to couple to the ventilation system, and the input from the patient monitor/defibrillator and the ventilation system may include respiration/ventilation data. The respiratory gas sensors may be a pulse oximetry (SpO2) sensor and an end-tidal carbon dioxide (EtCO2) sensor, the lung mechanics sensors may be an airway pressure sensor and a pneumotachometer, and the respiration/ventilation data may include SpO2 data, EtCO2 data, lung resistance data, and lung elastance data. The RD CSG engine may be configured to receive the SpO2 data and the EtCO2 data, evaluate the SpO2 data and the EtCO2 data, identify the at least one clinical intervention for the patient based on the evaluation, wherein the at least one clinical intervention may include oxygen support for the patient from the ventilation system, send an output to the ventilation system including an instruction for the ventilation system to provide oxygen support. The oxygen support may include supplemental oxygen therapy or supplemental oxygen with one of continuous positive airway pressure (CPAP) therapy or bilevel positive airway pressure. The output to the ventilation system may include a CSG event marker for the oxygen support provided by the ventilation system. The RD CSG engine is configured to evaluate an abnormal life threatening (ALT) hemodynamic condition flag, and if the ALT hemodynamic condition flag is set, then divert from the R/V CSG engine to the hemodynamic CSG engine, else continue to implement the R/V CSG engine. The RD CSG engine may be configured to provide a ventilation instruction for oxygen support at the CSG UI. The RD CSG engine is configured to receive the lung resistance data and the lung elastance data, update the oxygen support for the patient based on the lung resistance data and the lung elastance data, and send an instruction to the ventilation system for the ventilation system to provide the updated oxygen support. The RD CSG engine is configured to generate an etiology estimate for a respiratory distress presentation of the patient based on the SpO2 data, the EtCO2 data, the lung resistance data, and the lung elastance data, request targeted data from the patient monitor/defibrillator and the ventilation system, and convert the etiology estimate to an etiology determination based on the targeted data. The targeted data may include one or more of spirometry data, exhaled gas analysis data, and hemodynamic data. The RD CSG engine is configured to provide a prompt at the CSG UI for user entry of patient medical history information, receive the patient medical history information, and convert the etiology estimate to the etiology determination based on the patient medical history information. The RD CSG engine is configured to request patient medical history information from a stored medical history file, receive the patient medical history information, and convert the etiology estimate to the etiology determination based on the patient medical history information. The stored medical history file may include a medical records file stored in a remote medical records database. The RD CSG engine may be configured to communicatively couple to the remote medical records database via a network. The stored medical history file may include a patient charting file. The etiology determination may include an obstructive respiratory disease, and the RD CSG engine may be configured to identify at least one pharmacological intervention for the obstructive respiratory disease, provide a prompt for user confirmation of the at least one pharmacological intervention for the obstructive respiratory disease, and provide an output to the at least one medical device based on the at least one pharmacological intervention. The at least one pharmacological intervention may include a bronchodilator, and the output to the at least one medical device may include an instruction to the ventilation system to provide the bronchodilator. The output to the at least one medical device may include a CSG event marker including a time, date, and dose of the bronchodilator. The etiology determination may include a restrictive respiratory disease, and the RD CSG engine may be configured to identify at least one pharmacological intervention for the restrictive respiratory disease, provide a prompt for user confirmation of the at least one pharmacological intervention for the restrictive respiratory disease, and provide an output to the at least one medical device based on the at least one pharmacological intervention. The at least one pharmacological intervention may include a diuretic, and the output to the at least one medical device may include a CSG event marker including a time, date, and dose of the diuretic. The plurality of patient interface devices may be hemodynamic sensors configured to couple to the patient monitor/defibrillator, and the portion of the input from the patient monitor/defibrillator may include hemodynamic data. The hemodynamic sensors may be a non-invasive blood pressure (NIBP) sensor and an electrode assembly configured to gather electrocardiogram (ECG) data and heart rate data, and provide electrotherapy to the patient. The hemodynamic CSG engine may be configured to receive the hemodynamic data, identify a presence or an absence of an abnormal life threatening (ALT) patient condition based on the hemodynamic data, in the presence of the ALT patient condition, set an ALT hemodynamic condition flag, provide an alert at the CSG UI indicative of the presence of the ALT patient condition, send a CSG event marker of the presence of the ALT patient condition to the at least one medical device, and in the absence of the ALT patient condition, provide an update at the CSG UI indicative of the absence of the ALT patient condition, send a CSG event marker of the absence of the ALT patient condition to the at least one medical device. The hemodynamic CSG engine may be configured to query the patient monitor/defibrillator for a device status, and provide the device status at the CSG UI. The hemodynamic CSG engine may be configured to provide the hemodynamic data at the CSG UI. The CSG engine may include a non-respiratory distress CSG engine including at least one of a trauma CSG engine, a sepsis CSG engine, a traumatic brain injury CSG engine, a cardiac arrest CSG engine, and a critical care monitoring CSG engine. The display device may include a touchscreen. The display device may be disposed at the at least one medical device. The at least one medical device may be configured to concurrently provide the CSG UI and an operational interface at the display device. The CSG engine may be disposed at the at least one medical device. The at least one medical device may include a patient monitor/defibrillator, a ventilation system, or a ventilation system coupled to a patient monitor/defibrillator. The CSG engine may include an RD CSG engine configured to receive the physiologic data from the at least one medical device, control a display in real-time of the physiologic data at the CSG UI, evaluate the physiologic data for a change in at least one physiologic parameter represented in the physiologic data, wherein the change indicates a degradation in a medical state of the patient, select at least one clinical intervention based on the degradation, provide instructions for the at least one clinical intervention, identify a subset of the physiologic data as critical physiologic data based on the degradation and the at least one clinical intervention, and modify the display of the physiologic data to emphasize the critical physiologic data. The physiologic data may include ECG, EtCO2, SpO2, heart rate, body temperature, and non-invasive blood pressure, the degradation in the medical state may include acute respiratory failure (ARF), and the critical physiologic data may include EtCO2 and SpO2. The RD CSG engine may be configured to provide a user notification of the degradation in the medical state of the patient, receive a user request for context sensitive guidance, and modify the display of the physiologic data by replacing a display of ECG data, EtCO2 data, SpO2 data, heart rate data, body temperature data, and non-invasive blood pressure data at the display device with a display of instructions for the at least one clinical intervention and the EtCO2 and SpO2 data at the CSG UI that excludes the ECG data, the heart rate data, the body temperature data, and the non-invasive blood pressure data. The RD CSG engine may be configured to provide the user notification of the degradation at at least one of a device view window, a trends view window, or a working view window at the display device. The RD CSG engine may be configured to establish a communicative coupling with the at least one medical device, identify the at least one medical device based on the first communicative coupling, and provide a connected devices window at the display device that indicates the identification of the at least one medical device. The RD CSG engine may be configured to select the at least one clinical intervention based on the identification of the at least one medical device. The RD CSG engine may be configured to provide the instructions for the at least one clinical intervention to the at least one medical device. The RD CSG engine may be configured to provide the instructions for the at least one clinical intervention at the CSG UI. The instructions may include instructions for caregiver use of the identified at least one medical device. The identified at least one medical device may be a ventilation system and the instructions may include at least one of ventilation system operation instructions and ventilation system assembly instructions. The RD CSG engine may be configured to provide the CSG UI window as a selectable window with at least one other selectable window at the display device, the at least one other selectable window including a working view window. The RD CSG engine may be configured to receive caregiver input via one or more of a touch screen entry or a voice command. The instructions may include drug delivery instructions. The drug delivery instructions may include a drug delivery confirmation control and a dose interval timer. The RD CSG engine may be configured to provide guidance selection controls at the CSG UI in conjunction with the instructions for the at least one clinical intervention. The guidance selection controls enable a caregiver to select a level of detail of the instructions. The guidance selection controls may include at least one of a continue instructions control, an exit instructions control, a proceed to a next step control, an increase a detail level for guidance control, and a return to a previous instruction control, and a mute or unmute audible UI output control. The RD CSG engine may be configured to receive a caregiver confirmation at the display device in response to the instructions for the at least one clinical intervention. The caregiver confirmation may include one or more of a medication administration confirmation, a clinical intervention step confirmation, and a medical device connection confirmation. The RD CSG engine may provide a signal indicative of an event marker to the at least one medical device in response to the caregiver confirmation.

An example of a context sensitive guidance (CSG) system for providing resuscitative care to a patient during a medical event includes a CSG engine comprising hardware logic and/or software logic, a medical device system communicatively coupled to the CSG engine and a mobile computing device, the medical device system including a plurality of patient interface devices configured to generate sensor signals indicative of physiologic data for the patient during the medical event, a medical device screen for presenting the physiologic data, and at least one medical device processor operably coupled with the plurality of patient interface devices and the medical device screen, the at least one medical device processor configured to receive the sensor signals corresponding to the patient, generate the physiologic data based on the sensor signals, display the physiologic data at the medical device screen in a first display format, and transmit the physiologic data to the CSG engine and the mobile device, and the mobile computing device including a mobile device display screen configured to provide a user interface, and a mobile device processor operably coupled to the mobile device display screen and configured to receive the physiologic data from the medical device system, and cause display of the physiologic data at the user interface as a real time view of the physiologic data displayed at the medical device screen in a second display format that provides a visual reproduction of the first display format, wherein the CSG engine is configured to receive the physiologic data from the medical device system, receive user input at the user interface, evaluate the physiologic data and the user input, identify a clinical intervention for the patient based on the evaluation, generate output for the user interface based on at least one of the clinical intervention and the evaluation, and send an output to the at least one medical device based on the identified clinical intervention, wherein the at least one medical device may be configured to perform at least one operation in response to the output from the CSG engine.

Implementations of such a system may include one or more of the following features. The visual reproduction of the first display format at the second display format may include a replication of the first display format. The visual reproduction of the first display format at the second display format may include a rendering in which one or more visual aspects of the physiologic data presented in the second display format are adjusted relative to the first display format. The mobile device processor may be configured to cause the user interface at the mobile device to provide a plurality of view windows including a device view window configured to provide the real time view of the physiologic data in the second display format, capture a user selection of a particular view window, and provide the particular view window based on the user selection. At least one of the plurality of view windows may be a scrollable interface configured to provide more information than may be displayed in the device view window. The device view window may include an ECG waveform, a pulse oximetry waveform, invasive blood pressure (IBP) and/or a CO2 waveform. The device view window may include current values for at least one of peripheral capillary oxygen saturation (SpO2¬), carbon monoxide saturation (SpCO), methemoglobin (SpMet), total hemoglobin (SpHB), blood oxygen content (SpOC), pleth variability index (PVI), perfusion index (PI), end-tidal carbon dioxide (ETCO2), non-invasive blood pressure, invasive blood pressure value, heart rate (HR), respiration rate, fraction of inspired oxygen (FiO2), and temperature. The plurality of view windows may include one or more of a trend window and a working window. The mobile device processor may be configured to generate trend data from the physiologic data, the trend data including physiologic data values over time, and cause the user interface to provide the trend data in the trend window. The trend data may include at least one of SpO2, ETCO2, systolic blood pressure, diastolic blood pressure, mean arterial pressure, or heart rate values over time. The trend window may include display of the trend data in a graphical format, a tabular format, or a combination thereof. The working window may include caregiver feedback data. The caregiver feedback data may include one or more of chest compression feedback, ventilation feedback, and CPR performance summary data. The working window may include one or more customized display sections for a respective caregiver role and the caregiver feedback data may be customized feedback based on the respective caregiver role. The plurality of view windows may include a CSG window configured to capture the user input for the CSG engine and provide the output for the user interface from the CSG engine. The output for the user interface may include a prompt for a user entry by a caregiver. The user input may include information entered by the caregiver at the CSG window in response to the prompt for data entry. The prompt for user entry may include a prompt for patient assessment data. The patient assessment data may include respiratory distress presentation data. The prompt for user entry may include a prompt to confirm the respiratory distress presentation data and/or a prompt to save the respiratory distress presentation data in a case file for the medical event. The prompt for user entry may include a prompt for a CSG event marker. The prompt for the CSG event marker may include a prompt for user entry of the CSG event marker. The prompt for the CSG event marker may include a prompt for user confirmation in response to automatic recordation of the CSG event marker by the CSG engine. The prompt for user entry may include a prompt for a summary of previously entered CSG event markers. The CSG event marker may include a pharmacological intervention marker, a ventilation intervention marker, a hemodynamic intervention marker, an intervention equipment marker, an etiology marker, or a physiologic data evaluation marker. The CSG event marker may include a customizable event marker for manually entering an event marker name. The at least one processor may be configured to, responsive to receiving the user entry of the CSG event marker, cause display of the CSG event marker at the CSG window. The CSG engine may be configured to transmit the CSG event marker to the medical device system in response to the user entry with an instruction to record the CSG event marker at the medical device system. The prompt for user entry may include a prompt for patient medical history data. The prompt for patient medical history data may include a prompt to request import medical history information from one or more of an EMS electronic patient care record, a hospital electronic health record, and a health information exchange. The CSG engine may be configured to import the medical history information in response to the import request. The prompt for user entry may include a prompt for user confirmation of an automatic import of medical history from one or more of an EMS electronic patient care record, a hospital electronic health record, and a health information exchange. The prompt for user entry may include a prompt to perform the clinical intervention. The prompt to perform the clinical intervention may include a prompt for user confirmation. The clinical intervention may include a pharmacological intervention, a ventilation intervention or a hemodynamic intervention. The prompt for the clinical intervention may include an intervention metric. The prompt for user entry may include a prompt for user confirmation of an automatic intervention instruction to the medical device system. The CSG window may be configured to provide the physiologic data from the medical device system and the evaluation of the physiologic data from the CSG engine. The physiologic data may include at least respiratory gas data and lung mechanics data. The physiologic data may include hemodynamics data. The CSG window may be configured to provide a medical device clinical intervention status. The output for the user interface may include at least one alarm. The user input may include a confirmation of the at least one alarm. The output for the user interface may include a user notification of at least one medical device instruction. The at least one medical device instruction may be a medical device instruction automatically generated by the CSG engine. The user input may include a confirmation of the medical device instruction automatically generated by the CSG engine. The at least one instruction may be a medical device instruction manually entered at the CSG window. The at least one instruction may be an instruction to perform the clinical intervention. The at least one instruction may be an instruction to record a CSG event marker. The at least one instruction may be an instruction to monitor the patient. The output for the user interface may include etiology information. The etiology information may include an etiology automatically determined by the CSG engine. The user input may include a confirmation of the automatically determined etiology. The etiology information may include etiology information manually entered at the CSG window. The output to the at least one medical device may include instructions to perform the identified clinical intervention. The at least one operation performed by the medical device system may include the identified clinical intervention. The physiologic data may be first physiologic data, and the CSG engine may be configured to receive second physiologic data from the medical device system, update the clinical intervention for the patient based on the second physiologic data, and send instructions to the medical device system based on the updated clinical intervention. The CSG engine may be configured to generate an etiology estimate for at least one patient presentation based on the first and second physiologic data, request targeted patient data based on the etiology estimate, receive the targeted patient data, convert the etiology estimate to an etiology determination based on the targeted patient data, and provide the etiology determination to one or more of the medical device system and the user interface. The mobile device may include the CSG engine. The mobile device may be communicatively coupled to a cloud server including the CSG engine. The mobile device may include a CSG application serviced by the CSG engine. The CSG engine may include a respiratory distress CSG engine. The respiratory distress CSG engine may include a respiration/ventilation CSG engine and a hemodynamic CSG engine. The medical device system may include a patient monitor/defibrillator and a ventilation system, and the RD CSG engine may be configured to execute the respiration/ventilation CSG engine based on medical device input from the patient monitor/defibrillator and the ventilation system, and execute the hemodynamic CSG engine based on medical device input from the patient monitor/defibrillator. The medical device system may be communicatively coupled to the mobile computing device via a wireless communication link. The wireless communication link may be at least one of: a Wi-Fi link, or a Bluetooth link. The wireless communication link may be a preconfigured pairing between the medical device system and the mobile device. The mobile device display screen may be a touchscreen.

An example of a method of providing context sensitive guidance (CSG) for resuscitative care of a patient during a medical event includes receiving an input including physiologic data from at least one medical device, evaluating the physiologic data from the at least one medical device, identifying a clinical intervention for the patient based on the evaluation, providing a CSG user output based on at least one of the physiologic data and the clinical intervention at a CSG user interface (UI) provided at a mobile device communicatively coupled to the at least one medical device, and sending an output to the at least one medical device based on the identified clinical intervention.
Implementations of such a method may include one or more of the following features. The output to the at least one medical device may include an instruction to perform the identified clinical intervention. The at least one operation performed by the at least one medical device may include the identified clinical intervention. The method may include automatically generating the instruction to perform the identified clinical intervention, and sending the automatically generated instruction as the output to the at least one medical device. The method may include providing a prompt to confirm the automatically generated instruction at a CSG user interface, and sending the automatically generated instruction in response to the user confirmation. The method may include providing a prompt to enter the instruction to perform the identified clinical intervention at a CSG user interface provided by the mobile device communicatively coupled to the at least one medical device, and sending the instruction in response to the user entry. The output to the at least one medical device may include at least one CSG event marker associated with the identified clinical intervention, and the at least one operation performed by the at least one medical device may include a recordation of the at least one CSG event marker at a data storage region of the at least one medical device in response to receiving the output from the CSG engine. The method may include automatically generating the at least one CSG event marker based on the identified clinical intervention, and sending the automatically generated CSG event marker to the at least one medical device. The method may include providing a prompt for a user to confirm the automatically generated CSG event marker at a CSG user interface, and sending the automatically generated instruction in response to the user confirmation. The method may include providing a prompt for a user to enter the at least one CSG event marker based on the identified clinical intervention at a CSG user interface provided by the mobile device communicatively coupled to the at least one medical device, and sending the at least one CSG event marker in response to the user entry. The prompt to enter the at least one CSG event marker may include a user selection menu of a plurality of CSG event markers. The physiologic data may be first physiologic data, and the method may include receiving second physiologic data from the at least one medical device, updating the clinical intervention for the patient based on the second physiologic data, and sending instructions to the at least one medical device based on the updated clinical intervention. The method may include generating an etiology estimate for at least one patient presentation based on the first and second physiologic data, requesting targeted patient data based on the etiology estimate, receiving the targeted patient data, converting the etiology estimate to an etiology determination based on the targeted patient data, and providing the etiology determination to one or more of the at least one medical device and the CSG UI. The method may include identifying at least one pharmacological intervention based on the etiology determination, providing a prompt for user confirmation of the at least one pharmacological intervention at the CSG UI, and sending an output to the at least one medical device based on the at least one pharmacological intervention. The output based on the at least one pharmacological intervention may include a CSG event marker for the at least one pharmacological intervention. The output based on the at least one pharmacological intervention may include an instruction to provide the at least one pharmacological intervention. The physiologic data may include SpO2 data and EtCO2 data, the clinical intervention may include oxygen support for the patient from a ventilation system, and the output to the at least one medical device may include an instruction for the ventilation system to provide oxygen support. The oxygen support may include supplemental oxygen therapy or supplemental oxygen with one of continuous positive airway pressure (CPAP) therapy or bilevel positive airway pressure. The method may include receiving lung resistance data and lung elastance data, updating the oxygen support for the patient based on the lung resistance data and the lung elastance data, and sending an instruction to the ventilation system for the ventilation system to provide the updated oxygen support. The method may include generating an etiology estimate for a respiratory distress presentation of the patient based on the SpO2 data, the EtCO2 data, the lung resistance data, and the lung elastance data, requesting targeted data from a patient monitor/defibrillator and the ventilation system, and convert the etiology estimate to an etiology determination based on the targeted data. The targeted data may include one or more of spirometry data, exhaled gas analysis data, and hemodynamic data. The method may include providing a prompt at the CSG UI for user entry of patient medical history information, receiving the patient medical history information, and converting the etiology estimate to the etiology determination based on the patient medical history information. The method may include requesting patient medical history information from a stored medical history file, receiving the patient medical history information, and converting the etiology estimate to the etiology determination based on the patient medical history information. The etiology determination may include an obstructive respiratory disease, and the method may include identifying at least one pharmacological intervention for the obstructive respiratory disease, providing a prompt for user confirmation of the at least one pharmacological intervention for the obstructive respiratory disease, and providing an output to the at least one medical device based on the at least one pharmacological intervention. The at least one pharmacological intervention may include a bronchodilator, and the output to the at least one medical device may include an instruction to the ventilation system to provide the bronchodilator. The output to the at least one medical device may include a CSG event marker including a time, date, and dose of the bronchodilator. The etiology determination may include a restrictive respiratory disease, and the method may include identifying at least one pharmacological intervention for the restrictive respiratory disease, providing a prompt for user confirmation of the at least one pharmacological intervention for the restrictive respiratory disease, and providing an output to the at least one medical device based on the at least one pharmacological intervention. The at least one pharmacological intervention may include a diuretic, and the output to the at least one medical device may include a CSG event marker including a time, date, and dose of the diuretic. The physiologic data may include hemodynamic data, and the method may include receiving the hemodynamic data, identifying a presence or an absence of an abnormal life threatening (ALT) patient condition based on the hemodynamic data, in the presence of the ALT patient condition, setting an ALT hemodynamic condition flag, providing an alert at the CSG UI indicative of the presence of the ALT patient condition, sending a CSG event marker of the presence of the ALT patient condition to the at least one medical device, and in the absence of the ALT patient condition, providing an update at the CSG UI indicative of the absence of the ALT patient condition, and sending a CSG event marker of the presence of the ALT patient condition to the at least one medical device. The method may include providing a plurality of view windows at a CSG user interface including a device view window configured to provide a real time view of the physiologic data as a visual reproduction of a display format for the at least one medical device, capturing a user selection of a particular view window, and providing the particular view window based on the user selection. Providing the visual reproduction may include replicating the display format of the at least one medical device. Providing the visual reproduction may include rendering a display of the physiologic data in which one or more visual aspects of the physiologic data are different than the display format of the at least one medical device. At least one of the plurality of view windows may be a scrollable interface configured to provide more information than may be displayed in the device view window. The plurality of view windows may include a CSG window configured to capture CSG user input and provide the CSG user output. The method may include prompting for a user entry by a caregiver, and the CSG user input may include information entered by the caregiver at the CSG window in response to the prompt for data entry. The method may include prompting for patient assessment data. The patient assessment data may include respiratory distress presentation data. The method may include one or more of prompting a user to confirm the respiratory distress presentation data and prompting the user to save the respiratory distress presentation data in a case file for the medical event. The method may include prompting a user for a CSG event marker. The method may include prompting the user for entry of the CSG event marker. The method may include prompting the user for confirmation in response to an automatic recordation of the CSG event marker. The method may include prompting for a summary of previously entered CSG event markers. The method may include causing display of the CSG event marker at the CSG window. The method may include transmitting the CSG event marker to the at least one medical device in response to the user entry with an instruction to record the CSG event marker at the at least one medical device. The method may include prompting for patient medical history data. The method may include prompting a user to request import medical history information from one or more of an EMS electronic patient care record, a hospital electronic health record, and a health information exchange. The method may include importing the medical history information in response to the import request. The method may include prompting for user confirmation of an automatic import of medical history from one or more of an EMS electronic patient care record, a hospital electronic health record, and a health information exchange. The method may include prompting a user to perform the clinical intervention. The method may include prompting for user confirmation of the clinical intervention. The clinical intervention may include a pharmacological intervention, a ventilation intervention or a hemodynamic intervention. The prompt for the clinical intervention may include an intervention metric. The method may include prompting for user confirmation of an automatic intervention instruction to the at least one medical device. The method may include providing the physiologic data at the CSG window. The method may include generating at least one alarm based on the physiologic data. The method may include prompting a user for a confirmation of the at least one alarm. The method may include prompting a user for at least one instruction for the at least one medical device. The at least one instruction may be an automatically generated medical device instruction and including prompting the user for confirmation of the automatically generated medical device instruction. The method may include prompting the user to manually enter at least one medical device instruction at the CSG window. The method may include providing automatically determined etiology information. The method may include prompting a user for a confirmation of the automatically determined etiology information. The method may include prompting a user to manually enter etiology information at the CSG window. The method may include receiving the input including the physiologic data at a CSG engine including an RD CSG engine, evaluating the physiologic data, by the RD CSG engine, for a change in at least one physiologic parameter represented in the physiologic data, wherein the change indicates a degradation in a medical state of the patient, identifying a subset of the physiologic data as critical physiologic data based on the degradation and the identified clinical intervention, and modifying a data display provided at the mobile device to emphasize the critical physiologic data at the CSG UI. The physiologic data may include ECG, EtCO2, SpO2, heart rate, body temperature, and non-invasive blood pressure, the degradation in the medical state may include acute respiratory failure (ARF), and the critical physiologic data may include EtCO2 and SpO2. Modifying the CSG UI provided at the mobile device may include displaying all of ECG data, EtCO2 data, SpO2 data, heart rate data, body temperature data, and non-invasive blood pressure data at the mobile device, providing a user notification of the degradation in the medical state of the patient, receiving a user request for context sensitive guidance, and providing the CSG UI including instructions for the clinical intervention and a display of the EtCO2 and SpO2 data, wherein the CSG UI excludes the ECG data, the heart rate data, the body temperature data, and the non-invasive blood pressure data. The method may include providing the user notification of the degradation at at least one of a device view window, a trends view window, or a working view window. The method may include establishing a communicative coupling with the at least one medical device, identifying the at least one medical device based on the first communicative coupling, and providing a connected devices window at the mobile device that indicates the identification of the at least one medical device. The method may include authenticating the at least one medical device, and establishing the communicative coupling in response to the authentication. The method may include enabling patient data encryption configured to prevent access to patient data by unauthenticated devices. The at least one medical device may be an initial medical device, and the method may include establishing at least one additional communicative coupling with at least one additional medical device subsequent to the initial medical device, identifying the at least one additional medical device based on the at least one additional communicative coupling, and updating the connected devices window to include the identification of the at least one additional medical device. The method may include receiving the physiologic data from the initial medical device, receiving additional physiologic data from the at least one additional medical device, and displaying at least one of the additional physiologic data in real-time at the mobile device. The method may include providing instructions for the clinical intervention to the at least one medical device. The method may include providing one or more of operational mode instructions, operational setting instructions, physiologic closed-loop control instructions to the at least one medical device. The at least one medical device may include a ventilation system and the method may include generating the physiologic closed-loop control instructions based on oxygen concentration of a patient's blood, wherein the physiologic closed-loop control instructions adjust oxygen delivery during a delivery of a mechanical ventilation to the patient to maintain the oxygen concentration of the patient's blood at a desired level or range. The method may include providing instructions for the clinical intervention at the CSG UI, the instructions including instructions for caregiver use of the identified at least one medical device. The identified at least one medical device may be a ventilation system, and the method may include providing at least one of ventilation system operation instructions and ventilation system assembly instructions. The method may include providing the ventilation operation instructions as one or more of power-on instructions and mode selection instructions. The method may include providing the ventilation system assembly instructions as one or more of hose connection instructions, oxygen tank connection instructions, and mask connection instructions. The method may include providing the instructions for the clinical intervention as one or more of bronchodilator administration instructions and mask positioning instructions. The method may include providing the instructions for the clinical intervention as patient monitoring instructions. The method may include providing an indication of therapy targets for the critical physiologic data and a status indication for the at least one medical device. The therapy targets for the critical physiologic data may include therapy targets for SpO2 and EtCO2, and he method may include providing the status indication for the at least one medical device as an indication of closed loop control of a ventilator and a FiO2 value updated in real-time. The method may include providing the instruction for the clinical intervention as caregiver instructions at the CSG UI window. The method may include providing a list of instructions, and visually distinguishing between a current step in the list of instructions and one or more of subsequent and previous steps in the list of instructions. The method may include providing the caregiver instructions as alphanumeric instructions, graphic instructions, and a combination thereof. The method may include providing the graphic instructions as drawings of the at least one medical device configured to guide a caregiver through use of the at least one medical device. The method may include providing the caregiver instructions as a hospital transport instruction. The method may include providing the caregiver instructions as drug delivery instructions. The method may include providing the drug delivery instructions as a drug delivery confirmation control and a dose interval timer. The method may include providing guidance selection controls at the CSG UI window in conjunction with the instructions for the at least one clinical intervention. The method may include providing the guidance selection controls that enable a caregiver to select a level of detail of the provided instructions, and receiving a selected level of detail of the provided instructions. The guidance selection controls may include at least one of a continue instructions control, an exit instructions control, a proceed to a next step control, an increase a detail level for guidance control, and a return to a previous instruction control, and a mute or unmute audible UI output control. The method may include receiving a caregiver confirmation at the mobile device in response to the instructions for the at least one clinical intervention. The caregiver confirmation may include one or more of a medication administration confirmation, a clinical intervention step confirmation, and a medical device connection confirmation. The method may include providing a signal indicative of an event marker to the at least one medical device in response to the caregiver confirmation. The method may include receiving caregiver input via one or more of a touch screen entry or a voice command. The method may include controlling the CSG UI based on the voice command from a caregiver. The method may include providing audible output. The method may include responding to a caregiver instruction to mute or unmute the audible output by muting or unmuting the audible output. The method may include providing the CSG UI window as a selectable window with at least one other selectable window at the mobile device, the at least one other selectable window including a working view window. The method may include providing at least a portion of the physiologic data in one or more customized display sections of the working view window. Modifying the data display based on the degradation may include providing a user notification of the degradation at the working view window, wherein the user notification may include an identification of the degradation and the critical physiologic data. The method may include providing the user notification in an unoccupied portion of the working view window to emphasize the critical physiologic parameters. The method may include excluding from the user notification physiologic data other than the critical physiologic data to emphasize the critical physiologic data. The method may include providing the user notification visually and audibly to emphasize the critical physiologic data. The method may include providing at least one CSG control at the working view window configured to transition the mobile device display screen from the working view window to the CSG UI in response to caregiver activation of the at least one CSG control.
The method may include displaying at the working view window a banner after completion of the at least one clinical intervention, the banner including one or more of values and trend indicators for the critical physiologic data. The method may include providing the instructions for the at least one clinical intervention and the critical physiologic data at the CSG UI. The method may include providing one or more of a medication dosage timer and reminders for intervention steps at the CSG UI. The method may include providing instructions for caregiver use of the at least one medical device at the CSG UI. The method may include providing status updates for the at least one medical device at the CSG UI. The method may include excluding from display one or more physiologic parameters other than the critical physiologic data to emphasize the critical physiologic data at the CSG UI. The method may include transitioning from the CSG UI window to the working view window in response to caregiver activation of at least one working view control. The at least one other selectable window may include one or more of a device view window and a trend view window. The method may include providing a real time display at the device view window of at least a portion of the physiologic data in a visual reproduction of a display format provided on a respective medical device. The method may include providing user-customizable data trend information at the trend view window for the physiologic data.

An example of a context sensitive guidance (CSG) system for providing clinical interventions to a patient in an emergency medical event includes a CSG engine comprising hardware logic and/or software logic, a plurality of patient interface devices including a pulse oximetry sensor, a capnography sensor, an airway pressure sensor, and a pneumotachometer, and a medical device system including a patient monitor/defibrillator coupled to a ventilation system, the medical device system configured to receive signals from the plurality of patient interface devices, generate physiologic data including SpO2, EtCO2, lung elastance, and lung resistance data from the received signals, and send the physiologic data to the CSG engine wherein the CSG engine is configured to evaluate the SpO2 and the EtCO2 data, identify at least one ventilation intervention for the patient based on the evaluation of the SpO2 and the EtCO2 data, send a first instruction to the medical device system based on the at least one ventilation intervention, evaluate the lung elastance and the lung resistance data, update the at least one ventilation intervention based on the evaluation of the lung elastance and the lung resistance data, and send a second instruction to the medical device system based on the updated at least one ventilation intervention, wherein the medical device system may be configured to perform at least one operation in response to the first and second instructions.

An example of a method of using context sensitive guidance (CSG) system to provide clinical interventions to a patient in an emergency medical event includes receiving signals from a plurality of patient interface devices including a pulse oximetry sensor, a capnography sensor, an airway pressure sensor, and a pneumotachometer, generating physiologic data including SpO2, EtCO2, lung elastance, and lung resistance data from the received signals, and evaluating the SpO2 and the EtCO2 data, identifying at least one ventilation intervention for the patient based on the evaluation of the SpO2 and the EtCO2 data, sending a first instruction to at least one medical device based on the at least one ventilation intervention, the at least one medical device including a patient monitor/defibrillator coupled to a ventilation system, evaluating the lung elastance and lung resistance data, updating the at least one ventilation intervention based on the evaluation of the lung elastance and lung resistance data, and sending a second instruction to the at least one medical device based on the updated at least one ventilation intervention, wherein the first and second instructions are instructions for the at least one medical device to perform at least one operation.

An example of a respiratory distress (RD) context sensitive guidance (CSG) system for providing RD CSG during an emergency medical encounter includes at least one medical device configured to couple to a patient via one or more patient interface devices, and a mobile computing device configured to communicatively couple to the at least one medical device and including a mobile device display screen, a RD CSG engine comprising hardware logic and/or software logic and configured to receive a plurality of physiologic parameters from the at least one medical device, provide a user interface (UI) at the mobile device display screen, control a display in real-time of the plurality of physiologic parameters at the UI, detect a degradation in a medical state of the patient based on a change in at least one physiologic parameter of the plurality of physiologic parameters, select at least one clinical intervention based on the detected degradation, provide instructions for the at least one clinical intervention, identify a subset of the plurality of physiologic parameters as critical physiologic parameters based on the degradation and the at least one clinical intervention, and modify the display of the plurality of physiologic parameters at the UI to emphasize the critical physiologic parameters.

Implementations of such a system may include one or more of the following features. The at least one medical device may include two or more medical devices, each medical device configured to deliver a clinical intervention that is different from any other of the two or more medical devices. The two or more medical devices may include a patient monitor/defibrillator and a ventilation system. The one or more patient interface devices may include a pulse oximeter, a nasal cannula or mask coupled to a capnography sensor, a non-invasive blood pressure (NIBP) sensor, a temperature probe, and electrodes. The plurality of physiologic parameters may include ECG, EtCO2, SpO2, heart rate, body temperature, and non-invasive blood pressure, the degradation in the medical state may include respiratory distress, and the critical physiologic parameters may include EtCO2 and SpO2. The two or more medical devices may include at least one first aid kit. The UI may be configured to display the plurality of physiologic parameters for only one patient. The at least one medical device may be configured to provide acute care clinical interventions. The RD CSG engine may be configured to replace a display of the plurality of physiologic parameters at a first data view window at the UI with a display of a CSG UI window inclusive of only the critical physiologic parameters to modify the display. The first data view window at the UI may include one of a device view window, a trends view window, or a working view window. The RD CSG engine may be configured to provide a user notification at the UI to modify the display. The user notification may include a notification of acute respiratory failure. The RD CSG engine may be configured to provide values and/or trends of the critical physiologic parameters with the user notification. The mobile computing device may include a communications interface configured to establish a first communicative coupling with the at least one medical device, and identify the at least one medical device based on the first communicative coupling, and wherein the RD CSG engine may be configured to provide a connected devices window at the UI that indicates the identification of the at least one medical device. The communications interface may be configured to authenticate the at least one medical device, and establish the first communicative coupling in response to the authentication. The RD CSG engine may be configured to enable patient data encryption configured to prevent access to patient data by unauthenticated devices. The at least one medical device may be an initial medical device, the communications interface may be configured to establish a second communicative coupling with at least one additional medical device subsequent to the initial medical device, and identify the at least one additional medical device based on the second communicative coupling, and the RD CSG engine may be configured to update the connected devices window to include the identification of the at least one additional medical device. The RD CSG engine may be configured to receive the plurality of physiologic parameters from the initial medical device, receive one or more additional physiologic parameters from the at least one additional medical device, and modify the UI in real-time to include at least one of the one or more additional physiologic parameters. The initial medical device may include a patient monitor-defibrillator and the plurality of physiologic parameters may include ECG, EtCO2, SpO2, heart rate, body temperature, and non-invasive blood pressure. The at least one additional medical device may include a ventilation system, and the one or more additional physiologic parameters may include one or more of airway pressure (Paw), plateau pressure (Pplat), peak inspiratory pressure (PIP), patient oxygen saturation (SpO2), fraction of inspired oxygen (FiO2), positive end-expiratory pressure (PEEP), forced vital capacity (FVC), forced expiratory volume (FEV), peak expiratory flow rate (PEF or PEFR), respiratory resistance (Rrs), respiratory compliance (Crs), inspired and expired tidal volume, minute volume (Ve), end-tidal CO2 (EtCO2), and volume of exhaled carbon dioxide (VCO2). The at least one medical device is a medical device suite associated with a same single patient, the mobile computing device may be configured to communicatively couple with only one medical device suite, and the plurality of physiologic parameters provided at the mobile device display screen are all associated with the same single patient. The medical device suite, the mobile computing device, and the same single patient are disposed at a point of patient care in a local patient care environment, and wherein the communications interface may be configured to wirelessly communicatively couple the mobile computing device to the medical device suite via one or more of Bluetooth^{®} and Wi-Fi. The local patient care environment may include one of a site of an emergency event, a medical triage area, a military field care site, a medical transport vehicle, or a hospital emergency room. The communications interface may be configured to communicatively couple the mobile computing device to one or more additional computing devices in the local patient care environment. The communications interface may be configured to communicatively couple the mobile computing device to one or more remote computing devices outside of the local patient care environment via a long-range wireless network. The one or more remote computing devices may include one or more of an electronic patient care record service, a health information exchange, an electronic medical record service, a cloud server, and a computing device associated with a telemedicine provider. The RD CSG engine may be configured to select the at least one clinical intervention based at least in part on the identified at least one medical device, and provide the instructions for the at least one clinical intervention based on the identified at least one medical device. The RD CSG engine may be configured to provide the instructions for the at least one clinical intervention to the at least one medical device. The instructions may include one or more of operational mode instructions, operational setting instructions, physiologic closed-loop control instructions for the at least one medical device. The at least one medical device may include a ventilator, and the RD CSG engine may be configured to generate the physiologic closed-loop control instructions based on oxygen concentration of a patient's blood, wherein the physiologic closed-loop control instructions adjust oxygen delivery during a delivery of a mechanical ventilation to the patient to maintain the oxygen concentration of the patient's blood at a desired level or range. The instructions for the at least one clinical intervention include instructions provided at a CSG UI window for caregiver use of the identified at least one medical device. The identified at least one medical device is a ventilation system and the instructions may include at least one of ventilation system operation instructions and ventilation system assembly instructions. The ventilation system operation instructions may include one or more of power-on instructions and mode selection instructions. The ventilation system assembly instructions may include one or more of hose connection instructions, oxygen tank connection instructions, and mask connection instructions. The instructions for the at least one clinical intervention may include one or more of bronchodilator administration instructions and mask positioning instructions. The instructions for the at least one clinical intervention may include patient monitoring instructions. The patient monitoring instructions include an indication of therapy targets for the critical physiologic parameters and a status indication for the at least one medical device. The therapy targets for the critical physiologic parameters may include therapy targets for SpO2 and EtCO2, and the status indication for the at least one medical device may include indication of closed loop control of a ventilator and a FiO2 value updated in real-time. The RD CSG engine may be configured to provide the instruction for the at least one clinical intervention as caregiver instructions at a CSG UI window. The caregiver instructions may include a list of instructions, and wherein the CSG UI window may be configured to a visually distinguish between a current step in the list of instructions and one or more of subsequent and previous steps in the list of instructions. The CSG UI window may be configured to provide the caregiver instructions as alphanumeric instructions, graphic instructions, and a combination thereof. The graphic instructions may include drawings of the at least one medical device, where the drawings are configured to guide a caregiver through use of the at least one medical device. The caregiver instructions may include a hospital transport instruction. The caregiver instructions may include drug delivery instructions. The drug delivery instructions include a drug delivery confirmation control and a dose interval timer. The RD CSG engine may be configured to provide guidance selection controls at the CSG UI window in conjunction with the instructions for the at least one clinical intervention. The guidance selection controls enable a caregiver to select a level of detail of the provided instructions. The guidance selection controls may include at least one of a continue instructions control, an exit instructions control, a proceed to a next step control, an increase a detail level for guidance control, and a return to a previous instruction control, and a mute or unmute audible UI output control. The RD CSG engine may be configured to receive a caregiver confirmation at the mobile computing device in response to the instructions for the at least one clinical intervention. The caregiver confirmation may include one or more of a medication administration confirmation, a clinical intervention step confirmation, and a medical device connection confirmation. The RD CSG engine provides a signal indicative of an event marker to the at least one medical device in response to the caregiver confirmation. The RD CSG engine may be configured to receive caregiver input via one or more of a touch screen entry or a voice command. The UI may include a digital assistant configured to control the UI based on the voice command from a caregiver. The RD CSG engine may be configured to provide audible output. The RD CSG engine may be configured to respond to a caregiver instruction to mute or unmute the audible output. The RD CSG engine may be configured to provide a selectable CSG UI window with at least one other selectable window at the mobile device display screen, the at least one other selectable window comprising a working view window. The working view window may be configured to provide at least a portion of the plurality of physiologic parameters in one or more customized display sections. Modification of the display based on the detected degradation may include to provide a user notification of the detected degradation at the working view window, and the user notification may include an identification of the detected degradation and the critical physiologic parameters. The RD CSG engine may provide the user notification in an unoccupied portion of the working view window to emphasize the critical physiologic parameters. The user notification may exclude one or more physiologic parameters other than the critical physiologic parameters to emphasize the critical physiologic parameters. The UI may provide the user notification visually and audibly to emphasize the critical physiologic parameters. The working view window may include at least one CSG control configured to transition the mobile device display screen from the working view window to a CSG UI window in response to caregiver activation of the at least one CSG control. The working view window may be configured to display a banner after completion of the at least one clinical intervention, the banner comprising one or more of values and trend indicators for the critical physiologic parameters. The CSG UI window may be configured to provide the instructions for the at least one clinical intervention and the critical physiologic parameters. The CSG UI window may be configured to provide one or more of a medication dosage timer and reminders for intervention steps. The CSG UI window may be configured to provide instructions for caregiver use of the at least one medical device. The CSG UI window may be configured to provide status updates for the at least one medical device. The CSG UI window may exclude one or more physiologic parameters other than the critical physiologic parameters to emphasize the critical physiologic parameters. The CSG UI window may include at least one working view control configured to transition from the CSG UI window to the working view window in response to caregiver activation of the at least one working view control. The at least one other selectable window may include one or more of a device view window and a trend view window. The device view window may be configured to provide a real time display of at least a portion of the plurality of physiologic parameters in a visual reproduction of a display format provided on a respective medical device. The trend view window may be configured to provide user-customizable data trend information for the plurality of physiologic parameters.

### BRIEF DESCRIPTION OF THE DRAWINGS

The accompanying drawings, which are incorporated in and constitute a part of the specification, illustrate one or more embodiments and, together with the description, explain these embodiments. The accompanying drawings have not necessarily been drawn to scale. Any values and/or dimensions illustrated in the accompanying graphs and figures are for illustration purposes only and may or may not represent actual or preferred values or dimensions. Where applicable, some or all features may not be illustrated to assist in the description of underlying features.
FIG. 1 shows an example of a device configuration for context sensitive guidance.
FIG. 2 shows examples of context sensitive guidance system components.
FIG. 3A shows an example of a device configuration for context sensitive guidance inclusive of various remote computing devices.
FIGS. 3B shows an example of a physical location of a patient care environment.
FIG. 3C shows an example of a device configuration for context sensitive guidance hosted by a cloud server.
FIG. 3D shows an example of a device configuration for context sensitive guidance implemented at a medical device.
FIG. 3E shows an example of a device configuration for respiratory distress context sensitive guidance.
FIG. 3F shows an example of a medical device suite.
FIG. 3G shows an example of a combined operational and CSG user interface on a medical device.
FIG. 4 shows examples of CSG engines.
FIGS. 5A-5C shows an example of a method of providing context sensitive guidance.
FIG. 5D shows an example of a method of providing context sensitive guidance at a user interface.
FIG. 6 shows process stages of a CSG initialization engine and a RD CSG engine.
FIG. 7 shows process stages of an R/V CSG engine with continuation of the engine to FIGS. 8A, 8B 9, 10, and 11.
FIG. 8A shows three branches of the R/V CSG engine in continuation from FIG. 7.
FIG. 8B shows process stages of a monitoring loop of the R/V CSG engine.
FIG. 9 shows a first branch of the R/V CSG engine in continuation from FIG. 8A.
FIG. 10 shows a second branch of the R/V CSG engine in continuation from FIG. 8A.
FIG. 11 shows a third branch of the R/V CSG engine in continuation from FIG. 8A.
FIG. 12 schematically shows process stages of a hemodynamic CSG engine.
FIGS. 13A and 13B illustrate a mobile device configured to provide a device view user interface.
FIG. 13C shows an example of a combined ventilation system/patient monitor/defibrillator device view user interface at the mobile device.
FIGS. 14A and 14B show examples of a working view window at the mobile device.
FIG. 15 shows an example of a trend view window at the mobile device.
FIG. 16 shows an example a CSG user interface provided at the mobile device.
FIG. 17 illustrates an example of a patient assessment screen.
FIG. 18 illustrates an example of a patient medical history screen.
FIG. 19 illustrates an example of a CSG event marker screen.
FIG. 20 illustrates an example of an event summary screen.
FIGS. 21A, 21B, and 21C show examples of various clinical intervention screens.
FIGS. 22A and 22B show an example of a medical device data screen.
FIG. 23 shows examples of an etiology information screen.
FIG. 24 shows an example environment for implementing CSG at a customizable mobile device.
FIG. 25 illustrates an example method for configuring connection between a medical device and a mobile device.
FIG. 26 illustrates an example method for causing display of case information from a medical device at a mobile device during a medical event.
FIG. 27A illustrates an exemplary ventilation system.
FIG. 27B illustrates an example of the mechanical ventilation apparatus for a ventilation system.
FIG. 27C shows an example of user interface for a ventilation system.
FIG. 28 shows schematic examples of components of various devices discussed with regard to FIGS. 1-27C and 29-43.
FIG. 29 shows an example of a working view window at the mobile device.
FIGS. 30A and 30B show examples of a CSG user interface with context sensitive guidance engaged.
FIG. 30C shows a summary flowchart for an example of a sequence of UI display screens for RD CSG.
FIG. 31 shows an example of a CSG user interface with instructions for a clinical intervention.
FIG. 32A shows an example of a CSG user interface with instructions for use of a medical device.
FIG. 32B shows an example of a medication dosage timer.
FIG. 33 shows an example of a CSG user interface with instructions for use of a medical device.
FIG. 34 shows an example of a CSG user interface with instructions for use of a medical device.
FIG. 35 shows an example of a CSG user interface with instructions for use of a medical device.
FIG. 36 shows an example of a CSG user interface with instructions for use of a medical device.
FIG. 37 shows an example of a CSG user interface with instructions for use of a medical device.
FIG. 38 shows an example of a CSG user interface with instructions for use of a medical device.
FIG. 39 shows an example of a CSG user interface with instructions for use of a medical device.
FIG. 40 shows an example of a CSG user interface with patient monitoring instructions.
FIG. 41 shows an example of a working view window with patient monitoring instructions.
FIG. 42 shows an example of a working view window with patient monitoring instructions.
FIG. 43 shows an example of a CSG user interface with medical device operation instructions.

### DETAILED DESCRIPTION

The description set forth below in connection with the appended drawings is intended to be a description of various, illustrative embodiments or implementations of the disclosed subject matter. Specific features and functionalities are described in connection with each illustrative embodiment or implementation; however, it will be apparent to those skilled in the art that the disclosed embodiments and implementations may be practiced without each of those specific features and functionalities.

Reference throughout the specification to "one embodiment," "an embodiment," or "an implementation" means that a particular feature, structure, or characteristic described in connection with an embodiment or implementation is included in at least one embodiment or implementation of the subject matter disclosed. Thus, the appearance of the phrases "in one embodiment" or "in an embodiment" or "in an implementation" in various places throughout the specification is not necessarily referring to the same embodiment or implementation. Further, the particular features, structures or characteristics may be combined in any suitable manner in one or more embodiments or implementations. Further, it is intended that embodiments and implementations of the disclosed subject matter cover modifications and variations thereof.

It must be noted that, as used in the specification and the appended claims, the singular forms "a," "an," and "the" include plural referents unless the context expressly dictates otherwise. That is, unless expressly specified otherwise, as used herein the words "a," "an," "the," and the like carry the meaning of "one or more." Additionally, it is to be understood that terms such as "left," "right," "top," "bottom," "front," "rear," "side," "height," "length," "width," "upper," "lower," "interior," "exterior," "inner," "outer," and the like that may be used herein merely describe points of reference and do not necessarily limit embodiments of the present disclosure to any particular orientation or configuration. Furthermore, terms such as "first," "second," "third," etc., merely identify one of a number of portions, components, steps, operations, functions, and/or points of reference as disclosed herein, and likewise do not necessarily limit embodiments of the present disclosure to any particular configuration or orientation.

Furthermore, the terms "approximately," "about," "proximate," "minor variation," and similar terms generally refer to ranges that include the identified value within a margin of 20%, 10% or preferably 5% in certain embodiments, and any values there between.

All of the functionalities described in connection with one embodiment or implementation are intended to be applicable to the additional embodiments and implementations described below except where expressly stated or where the feature or function is incompatible with the additional embodiments and implementations. For example, where a given feature or function is expressly described in connection with one embodiment or implementation but not expressly mentioned in connection with an alternative embodiment or implementation, it should be understood that that feature or function may be deployed, utilized or implemented in connection with the alternative embodiment or implementation unless the feature or function is incompatible with the alternative embodiment or implementation.

Emergent medical care focuses, at least initially, on timely management of a patient's presenting conditions. Such medical care occurs, for example, in response to a 911 call, in a military triage, or in an emergency room of a hospital. The goal of timely managing the patient's presenting conditions is to provide interventions that stabilize the patient and keep the patient alive long enough for the patient to receive treatments for diagnosed etiologies of the presenting conditions. As an example, a person under the care of emergency medical services may present with cardiac arrest. The emergent care may include defibrillation to restore a normal heart rhythm. This intervention for cardiac arrest most likely does not depend upon the specific etiology of the cardiac arrest and may stabilize and keep the patient alive long enough to receive treatments for the etiology of the cardiac arrest. For example, following a defibrillation intervention, treatments for the etiology of cardiac arrest, such as surgery and/or medications may vary depending on the etiology or diagnosis. For example, cardiac arrest may be consistent with various diagnoses such as coronary artery disease, valvular heart disease, or a congenital heart defect.

Guidance for a caregiver and/or a medical device is likely to improve the efficacy and efficiency of interventions provided during emergent care. Further and more substantial improvements in efficacy and efficiency are realized by tailoring the guidance to the physiologic status of the patient. Monitoring, analyzing, and evaluating physiologic data relevant to the patient presentation provides adaptable and data-driven guidance that is sensitive to the particular context of the patient's physiologic status. The guidance also determines, monitors, and updates the parameters of these interventions based on the physiologic data from the patient. Thus, while the guidance may enable decisions regarding which interventions to provide but it is not limited to such decisions. Further, in some cases, the physiologic data that is monitored, analyzed, and evaluated may provide indications of etiology and may enable interventions and treatments directed at that etiology.

In an implementation, a context sensitive guidance (CSG) engine for respiratory distress (RD) may minimize or alleviate caregiver distraction and confusion. The CSG engine may provide visual and/or audible user notifications to alert the caregiver that some condition of the patient requires additional and immediate attention. In an implementation, these notifications may be limited to only actionable information. For example, the user notifications may exclude physiologic parameters other than critical physiologic parameters. The critical physiological parameters may include those that the CSG engine identifies as requiring a caregiver's immediate attention as high priority indicators of a downturn and likely life threatening change in a patient's condition. Such selective notification may be beneficial so as to avoid alarm fatigue where users may exhibit more of a tendency to ignore alarms and/or alerts when excessively triggered. Thus, unlike the information provided at the display screen of a typical medical device or patient monitor, the CSG engine may curate the information provided to the caregiver. The large amount of data that may be provided at a medical device display screen, such as a patient monitor screen, shows an overall state of a patient. These data displays may not provide caregiver guidance. Rather, the caregiver has to sort, analyze, and evaluate this large amount of data to determine if clinical interventions are necessary. As is commonly the case for emergency medical services, particularly in the pre-hospital context, the caregiver may have multiple patients and other distractions (e.g., family members, moving the patient, care activities, environmental distractions, etc.). Thus, in contrast to the medical device screen information, the CSG engine does not merely provide information for the caregiver to analyze, monitor, and draw conclusions from. Rather, the CSG engine selects particular information that the caregiver needs to or should be aware of at a particular moment and selects and instructs the caregiver on immediate interventions. The CSG engine may downregulate or filter the large quantity of data available from a typical medical device to specific factors and steps that are immediately necessary to increase the odds of patient survival. This real-time context sensitive guidance from the CSG engine provides the caregiver with an understanding of the purpose and urgency of those interventions. In addition to providing caregiver information, the CSG engine may also provide context sensitive guidance to medical devices. This guidance may include physiologic closed-loop control. Thus, the CSG engine plays an active role in patient care by performing a number of tasks including, for example, recognizing critical medical conditions, determining appropriate intervention responses, coordinating the provision of those responses between the medical devices and the caregivers, setting priorities for those responses, confirming completion of those responses, and monitoring the results of those responses to ensure that the medical response has had the intended effect on the patient. The caregiver and the medical devices are tools at the disposal of the CSG engine to effectuate effective patient care.

In an implementation, the CSG engine may also provide varying and user-selectable levels of detail in regard to guidance. This may insure that a caregiver skill level and experience does not limit the provision of the determined care and, similarly, that unnecessary details do not slow or otherwise hamper that care. Human caregivers have a limited cognitive bandwidth and in emergency RD treatment situations are often inundated with information. The ability to off-load to the CSG engine the intake, the analysis, and the determination of the most efficient and effective response to a medical condition that may kill a patient in a matter of minutes may dramatically improve the care provided to the patient. Further, the CSG engine may relieve the caregiver of tracking their progress through a care sequence and of keeping track of possible missed or delayed steps.

As the caregiver and medical devices generate and collect patient data, the CSG engine monitors that information. An audible or visual guided user interface (GUI) that includes that patient data may provide the overall collection of data in the foreground while the CSG engine monitors in the background. In response to a detection of a degradation in the medical state of the patient, the CSG may move to the foreground, either automatically or by providing the caregiver with an option to obtain guidance. Thus any monitoring GUI (e.g., on a medical device, on a first-aid kit, on a computing device such as a tablet, on another mobile device such as a smartwatch, smartphone, heads-up AR and/or VR display, etc., and/or as provided by an audible digital assistant) may provide overall patient data (e.g., the working view, device view, trend view screens described herein) and then transition to the CSG GUI.

Aspects of the present disclosure are directed to systems and methods for generating context sensitive guidance (CSG) for medical diagnostics and the delivery of medical interventions by medical treatment and diagnostic devices. Such systems and methods for CSG additionally provide a guided user interface (GUI) for aspects of the context sensitive guidance directed at and interactive with a provider of medical care. The GUI may be implemented at a mobile device communicatively coupled via a wireless communication link to one or more medical treatment and/or diagnostic devices (e.g., a patient monitor, a patient monitor/defibrillator, a ventilation system 280, a respiratory distress management system, and/or a drug delivery device) used for medical interventions during an emergency medical event. The mobile device may be a mobile computing device such as a tablet, a personal display/digital assistant device, a phone, a laptop computer, a notebook computer, a wearable mobile device (e.g., a heads up display, an augmented reality device, a virtual reality device, a watch), or combinations thereof.

In an implementation, the mobile device may be a companion device for a single medical device or may be a companion device for a suite of medical devices. If the mobile device 105 is a companion device for a single medical device, the mobile device may be preconfigured before deployment in a medical event (e.g., a factory setting or software configuration setting) to communicate with a particular medical device. If the mobile device is a companion device for a suite of medical devices, the mobile device and the medical devices may be configured either via hardware and/or software to enable interoperable communication with one another. In an implementation, the companion device(s) may be preconfigured to be associated with the medical treatment and/or diagnostic device so as to streamline wireless communication pairing without having to undergo a time consuming inquiry and response negotiation for a secure connection to be established. In some embodiments, the companion device configured to operate with a medical treatment device can be used in an emergency medical services (EMS) environment by trained emergency responders at the scene of an emergency or during prehospital transport. It can also be used in EMS during the ground and air transport of patients between medical facilities. The companion device, in some examples, can also be used in a hospital emergency room, general medical-surgical and intermediate care floors, cardiac care unit, electrophysiology (EP) lab, operating rooms, and other similar areas of the hospital and/or for intra-hospital transport of patients.

In an example scenario, an emergency responder or other medical caregiver tasked with treating a patient or victim of an emergency event, may first observationally evaluate the presentation of the patient. For example, such an evaluation likely includes observation of patient responsiveness, any respiratory distress, mental confusion, visible wounds. Additionally, the caregiver may converse with a conscious patient or a family member or friend of an unconscious patient to determine a chief complaint, health history, or other information relevant to treatment. In initiating care, the caregiver may start with an Airway-Breathing-Circulation (ABC) evaluation. Based at least on the ABC evaluation, the caregiver may connect the victim to a ventilation device, a patient monitor, and/or a patient monitor/defibrillator to treat a patient presenting with abnormal respiratory and/or hemodynamic conditions. The caregiver may further step through an OPQRST assessment for any pain described and/or exhibited by the patient. "OPQRST" is a mnemonic for emergency care providers that reminds them to evaluate **O**nset, **P**rovocation/**P**alliation, **Q**uality, **R**adiation, **S**everity, and **T**ime associated with patient pain.

A patient presenting with respiratory distress (RD) generally requires a rapid and accurately targeted intervention. The caregiver may need to identify and implement the appropriate efficacious intervention within minutes in order to ensure that the patient stays alive and neurologically intact. Such an intervention includes actions that contribute directly to the care of the patient's presenting condition(s) and may determine the disposition of the patient. The intervention may or may not treat the etiology of the presenting condition(s). However, if the presenting condition(s) remain unchanged by an intervention, the patient may not live long enough for a treatment of the root cause.

For example, a patient may exhibit RD due to congestive heart failure (CHF). In this case, the RD has a cardiac etiology. An intervention to relieve the RD, such as providing oxygen to the patient to remedy hypoxemia, is necessary to keep the patient alive and neurologically intact until the cardiac etiology can be diagnosed and addressed with treatment. The treatment for the heart failure may be separate from the oxygen intervention. For example, this treatment may include angioplasty, high blood pressure medication, and/or arrhythmia medication.

As another example, the patient may exhibit RD due to pneumonia. In this case, the RD has a respiratory etiology. Here, an intervention to relieve the RD, such as providing oxygen, is necessary until the respiratory etiology can be diagnosed and addressed with a treatment. For example, the treatment may include administration of antibiotics for the pneumonia infection.

As a further example, a patient may be briefly exposed to an oxygen poor gaseous environment. For example, the patient may be in a car or home with a carbon monoxide leak and thus be exposed to low oxygen levels. In this case, an intervention of providing oxygen also treats the root cause of the RD. In this case, once the root cause of the patient's oxygen deficiency is diagnosed, the intervention will have functioned as a treatment.

Thus, as the above examples demonstrate, all treatments are interventions but not all interventions are treatments. A treatment includes actions by the caregiver that address both presenting conditions and the root cause, or etiology, of the presenting conditions. An intervention includes actions by the caregiver in response to particular presenting conditions. In some cases, such actions may only address presenting conditions and therefore may not be a treatment for the etiology of the conditions. In other cases, such actions may address presenting conditions along with the root cause of the conditions.

The context sensitive guidance (CSG) system, as described herein, provides care guidance in the context of presenting conditions of a patient before an accurate diagnosis is likely or in some cases before such diagnosis is even possible. The CSG system collects objective physiological and other medical data for the patient and identifies therapeutic interventions tailored to alleviate physiological conditions indicated by the data. The care guidance identifies and enables implementation of interventions directed at the initial presenting conditions which in some cases pose an acute and immediate risk to the patient's life. Once the patient is stabilized, the CSG system may provide further guidance in identifying a diagnosis, or specific root cause, and identifying and implementing treatments based on the diagnosis. Additionally, the CSG system may monitor the patient for indicators of a resumption of an acute and possibly life-threatening medical condition.

Based on an initial presentation of the patient and physiological data characterizing the initial presentation, a caregiver and/or medical device system implements a care plan that includes a set of interventions directed at the initial presentation. As care progresses, the caregiver and/or the medical device system accumulate data, for example, from physiological sensors, diagnostic tests and images, such as blood tests, x-rays, ultrasounds, etc., and observed or measured patient statuses before and after the various interventions administered to the patient. This data informs a differential diagnosis process. In the differential diagnosis process, etiologies of the impaired health are ruled out in a stepwise fashion with a resulting identification, or diagnosis, of one or more etiologies, or root causes. The goal of this process is a diagnosis, in other words, a definitive identification of one or more root causes of a patient's impaired health, and an identification of one or more treatments for the root cause based on the diagnosis. The CSG system protects the patient's health during the differential diagnosis process by identifying and implementing interventions necessitated by adverse medical conditions stemming from the root cause.

In some cases, a CSG system operational in a pre-hospital environment may identify both interventions and a diagnosis. In other cases, the CSG system may identify interventions but not the diagnosis. The diagnosis may not occur until after the patient is transported to a hospital and the results of more extensive tests and attempted treatments are analyzed. However, CSG enables the efficacious provision of critical care, without which, in many cases, a patient will not survive or remain neurologically intact long enough to reach a diagnosis stage.

Returning to the example of RD, the breathing portion of the ABC evaluation may indicate a breathing status ranging from a patient with no difficulty breathing, to a patient with slight to severe difficulty breathing, to a patient that is alive but not breathing on their own at all. For those patients breathing without difficulty, the caregiver will initially direct their attention to non-respiratory presenting conditions, such as trauma or sepsis.

For those patients with difficulty breathing or those not breathing at all, the caregiver will monitor for both proper respiratory and cardiac functions. RD may stem from a respiratory etiology, a cardiac etiology, or a combination root cause. However, regardless of the etiology, respiratory function and cardiac function are interrelated and presenting conditions of RD warrant cardiac monitoring and vice versa. Thus, the CSG system, as applied to a respiratory presentation of a patient, executes a respiration/ventilation (R/V) engine based on respiration and ventilation indicators for a patient in parallel with an engine based on hemodynamic indicators. Therefore, the caregiver of a victim of RD will connect the patient to the medical devices referred to above, namely the ventilation system and the patient monitor or patient monitor/defibrillator. These medical devices may monitor and measure respiratory health indicators, for example, respiratory gas exchange and/or lung mechanics, and cardiac health indicators, for example, blood pressure, heart rate, and an electrocardiogram (ECG). The respiratory gas exchange measurements may include, but are not limited to, pulse oximetry and capnography and the lung mechanics measurements may include, but are not limited to lung elastance and lung resistance.

A couple of examples illustrate the need for parallel operation of the R/V CSG engine and the hemodynamic CSG engine in implementing CSG for a presentation of RD. As a first example, a patient presenting with RD may be suffering from pneumonia. The patient requires interventions directed at the RD. However, pneumonia often raises a patient's heart rate. If this heart rate goes too high, the elevated heart rate may be an abnormal and life-threatening condition leaving the patient in immediate danger of death due to cardiac malfunction. Thus, while monitoring and implementing interventions for the patient's RD, the CSG system must monitor the hemodynamic indicators and implement cardiac interventions as needed.

As a second example, a patient presenting with RD may be suffering from CHF. The patient requires interventions directed at the RD. However, the cause of the CHF may be an arrhythmia that leaves the patient vulnerable to stroke. Therefore, as in the pneumonia example, while monitoring and implementing interventions for the patient's RD, the CSG system must monitor the hemodynamic indicators and implement cardiac interventions as needed.

In both of these examples, it is important to recognize that in pre-hospital care, for example in response to a 911 call, on a battlefield, or in another emergency treatment environment, the caregiver may, at least initially, only be aware of the presenting conditions and conditions revealed by the medical devices. Thus, underlying and perhaps chronic conditions may not be apparent or known by the caregiver. Thus, the CSG system for RD based on parallel monitoring of R/V and hemodynamics is crucial in treating RD presentations. The pneumonia infection or the chronic arrhythmia are likely initially unknown to the caregiver and it is only by monitoring both physiological systems that interventions may be properly applied to keep the patient alive long enough to reach a subsequent diagnosis of these etiologies. This stands in contrast to a non-emergency hospital setting or physician's office where the patient and the patient's health history are typically known and/or at least readily accessible to the caregiver.

The CSG system may reside on the mobile device described above, on a medical device, on a cloud server, or a combination thereof. The CSG system may be communicatively coupled to the medical devices and receive inputs from the medical devices that include physiological data collected from the patient by the medical devices along with data describing actions taken by the medical devices during care of the patient. The CSG system may also provide a GUI and receive inputs from the caregiver at the GUI regarding patient medical data and/or actions taken by the caregiver. Based on this received data, the CSG system may identify clinical interventions and update previously identified interventions and may generate etiology estimates and determinations. The CSG system may then instruct and/or control medical devices to implement the clinical interventions and/or provide patient record data and event markers to the medical devices. The CSG system may also request specific patient data from the medical devices. The CSG system may access medical records for the patient (e.g., an electronic patient care record (ePCR), i.e., a patient chart, generated during an encounter, an archived patient chart, a record in a health information exchange (HIE) or other regional data base, and/or a hospital electronic health record (EHR). Further, the CSG system may provide prompts and/or instructions for the caregiver at the GUI in order to implement the clinical interventions and guide patient care.

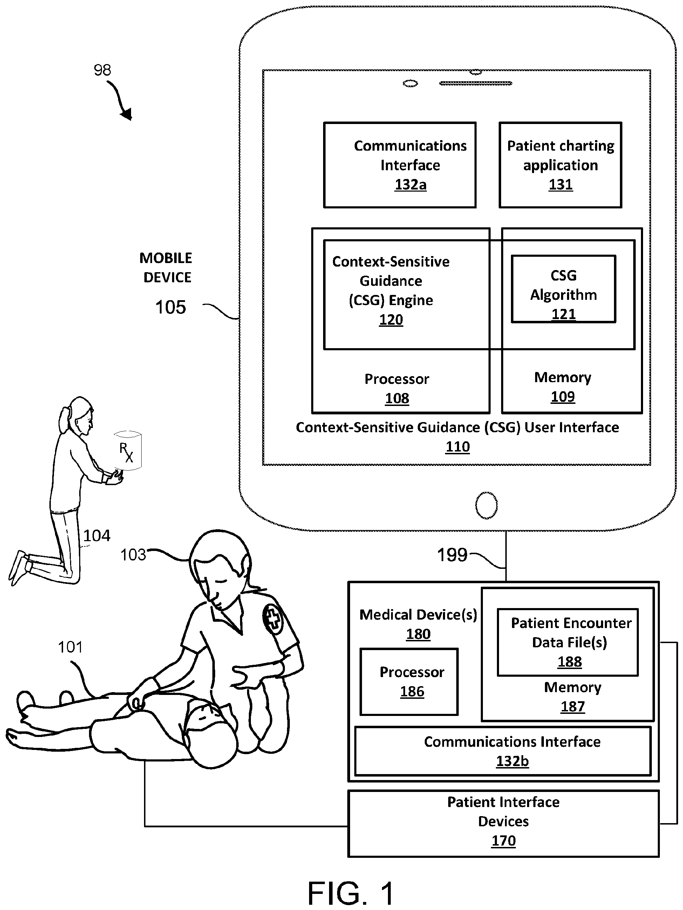

Referring to FIG. 1, an example of a device configuration for context sensitive guidance is shown. A quantity of each component in FIG. 1 is an example only and other quantities of each, or any, component could be used. As shown in FIG. 1, the patient care environment 98, a rescuer or caregiver 103 attends to a victim or patient 101 (the terms are used interchangeably here to indicate a person who is the subject of intended or actual medical treatment(s) and/or interventions), such as an individual who is in RD. The patient care environment 98 can be, for instance, at the scene of an accident or health emergency, in an ambulance, in an emergency room or hospital, or another type of emergency situation. The rescuer 103 may be, for instance, a civilian responder with limited or no training in lifesaving techniques; a first responder, such as an emergency medical technician (EMT), a paramedic, a police officer, a firefighter; or a medical professional, such as a physician or nurse. The rescuer 103 may be acting alone or may be acting with assistance from one or more other rescuers, such as a partner EMT 104.

In this illustration, the rescuers 103, 104 may deploy medical device(s) 180 coupled to patient interface devices 170. The medical device(s) 180 may be medical therapy delivery device(s) configured to gather physiological data from the patient via the patient interface devices 170, monitor this data, and delivery therapy to the patient (e.g., electrotherapy or respiratory therapy) based on the gathered and monitored data.

The patient interface devices 170 may further include other sensors for monitoring parameters indicative of the patient's health status, e.g., physical parameters such as the patient's heart rate, temperature, respiration rate, blood oxygen level, blood glucose level, or other parameters indicative of the patient's health status. Some sensors, such as heart rate or ECG sensors, can be included in electrode pads coupled to the patient monitor/defibrillator 285. Additionally, one or more sensors may monitor treatment(s) and/or intervention(s) delivered to the victim 101. For instance, a compression puck can be positioned beneath the hands of rescuer 103 as the rescuer 103 administers CPR by detecting a rate, depth, or duration of compressions delivered to the victim 101. Additionally, airflow sensor included in the ventilation system 280 may monitor volume and rate of ventilations administered to the victim 101 by rescuer 103, 104. Thus, some sensors can monitor both parameters indicative of the patient's health status and parameters indicative of the treatment and/or intervention delivered to the patient

The rescuers 103, 104 may use the at least one mobile device 105. The mobile device 105 may be a mobile device such as, for example but not limited to, a smartphone, a tablet, or a wearable device (e.g., watches or glasses). The rescuer(s) may also utilize additional computing devices, such as laptop computers or computing devices integrated into an ambulance, can be used to analyze health data about the patient or data indicative of treatments and/or interventions delivered to the patient or to communicate such data to a remote location (e.g., a dispatch center, an emergency room, or a remote server).

The mobile device 105 and the medical devices 180 may communicatively couple to one another via a local wireless communication channel 199 established between the devices. In an implementation, the devices 105 and/or 180 may also communicatively couple to other medical and/or computing devices at the patient care environment 98. The communication channel 199 enables data to be securely and accurately shared between two or more devices at the patient care environment 98. During deployment of the medical device(s) 180, these devices may store one or more patient encounter data files 188 that include records of data gathered during a patient case and of treatments and/or interventions by the medical device(s) along with records of device settings and operations during the patient case.

The mobile device 105 may include a processor 108 and a memory 109. The memory 109 (i.e., a non-transitory processor readable storage medium) may store a CSG algorithm 121 that includes instructions executable by the processor 108 to implement a CSG engine 120. The CSG engine 120 may include hardware logic and/or software logic provided by the processor 108 and/or the memory 109 to implement CSG at the mobile device 105 and/or at a medical device 180. The CSG engine 120 may implement a CSG user interface (UI) 110 at one or more input/output devices of the mobile device 105 including, for example, a display screen 106 and/or one or more audio and/or haptic devices. The display screen 106 may be a touchscreen.

Additionally, the mobile device 105 may include a patient charting application 131. The patient charting application 131 may generate an electronic patient care record (ePCR) as a contemporaneous record of caregiver observations, treatments and interventions provided, physiologic data for the patient, patient transport, transport destination, times and durations of patient care activities, emergency crew identification, patient demographic information, patient health insurance information, etc. The information entered into the ePCR may be entries from the caregiver captured by the mobile device (e.g., touchscreen or keyboard entries, voice entries), entries via one or more input devices (e.g., scanner, microphone, camera, etc.), and/or automated entries from communicatively coupled devices or databases. The application 131 may operate in parallel or in cooperation with the CSG engine 120. In an implementation, the CSG engine 120 may implement the patient charting application 131 and the CSG algorithm 121 through a combined user interface. In various implementations, the CSG engine 120 may provide a second output 225 to the patient charting application 131. The second output 225 from the CSG engine 120 may include information for recordation in the ePCR (e.g., event markers, physiologic data, data evaluations, intervention information, etc.).

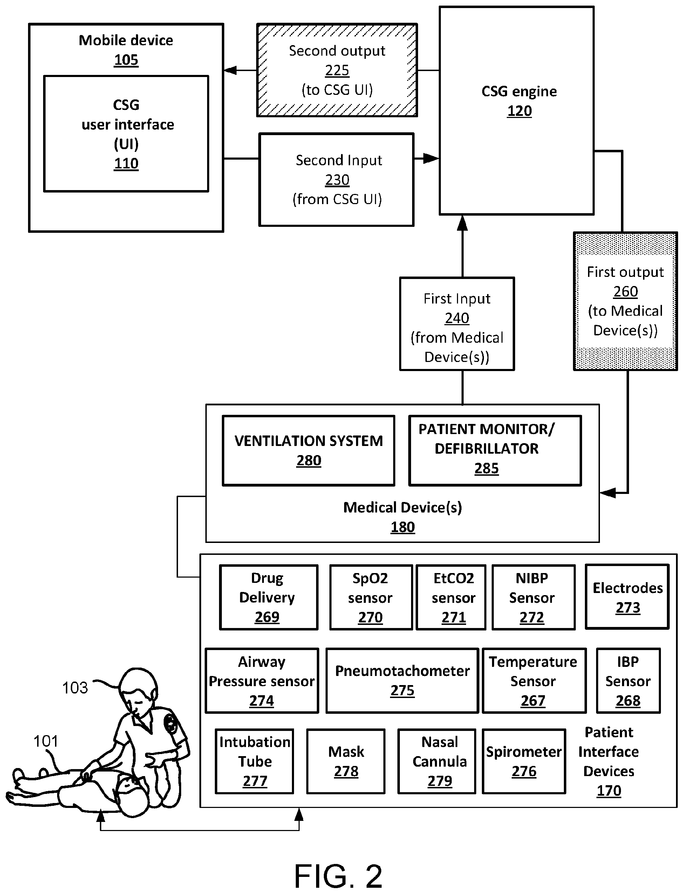

Referring to FIG. 2, examples of context sensitive guidance system components are shown. A quantity of each component in FIG. 2 is an example only and other quantities of each, or any, component could be used. As shown in FIG. 2, the medical devices 180 may include a patient monitor/defibrillator 285 and a ventilation system 280. The patient interface devices 170 may couple to the victim 101 and may include one or more sensors and/or intervention/treatment delivery devices. For example, the sensors may include one or more of a SpO2 sensor 270, an EtCO2 sensor 271, a non-invasive blood pressure (NIBP) sensor 272, an invasive blood pressure (IBP) sensor 268, a temperature sensor 267, an airway pressure sensor 274, a pneumotachometer 275, a spirometer 276, and electrodes 273. The intervention/treatment delivery devices may also include the electrodes 273 as electrodes may sense the heart rate and the electrical activity of the victim's heart and may also deliver electrotherapy (e.g., defibrillation shock and/or pacing) to the victim 101. The intervention/treatment delivery devices may include a mask 278 and a nasal cannula 279. The medical device(s) 180 and/or the patient interface devices 170 may include a drug delivery system 269.

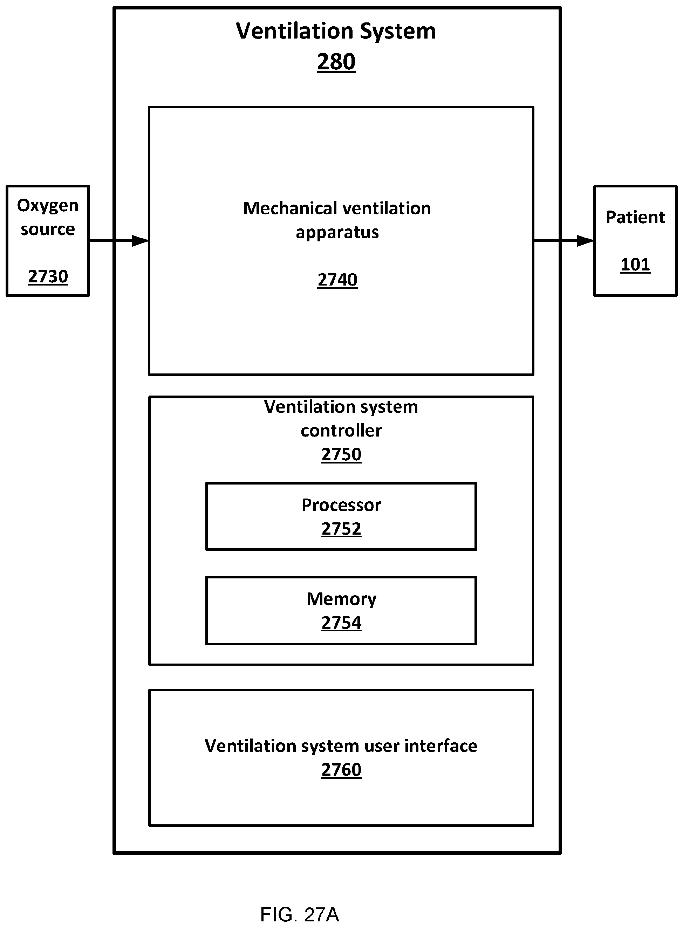

In an implementation, the ventilation system 280 and the patient monitor/defibrillator 285 may couple to one another communicatively and/or operationally. As such, these devices may share resources such as user interface, data management, external communication, processing of diagnostic/treatment algorithms, and/or processing and/or providing of various physiological signals. The ventilation system 280 (e.g., as described in more detail below with regard to FIGS. 27A-C) may rely on the patient monitor/defibrillator 285 for some of the functions of the ventilation system 280 and thus reduce the total size and weight of the medical device(s) 180. In other words, duplicative functional components between the patient monitor/defibrillator 285 and the ventilation system 280 may be streamlined to a single functional component. For example, in some embodiments, the ventilation system 280 may have the capability to provide patient ventilation in a first control mode, such as basic ventilation, in a stand-alone capacity. However, when coupled with another appropriate device or system (e.g., the patient monitor/defibrillator 285 and/or the mobile device 105), the ventilation system 280 may provide ventilation in a second control mode that is controlled or partly controlled by or via another device, system or interface (e.g., the patient monitor/defibrillator 285 and/or the mobile device 105), which may include user interaction with the other device, system or interface. In some embodiments, the first and second control modes may differ.

As shown in FIG. 2, the CSG engine 120 may receive various inputs and generate and send various outputs. The CSG engine 120 may receive a first input 240 from the medical device(s) 180 such as, for example, physiologic data, medical device status information, and/or requests for information. Additionally, the CSG engine 120 may receive a second input 230 from the CSG guided user interface 110. The second input 230 may include user-entered information from the CSG UI 110, information automatically gathered at the mobile device 105, requests for information from the CSG engine 120 and/or the medical device(s) 180, etc. In an implementation, the CSG engine 120 may cause the CSG UI 110 to provide a prompt and the second input 230 may be a response to the prompt. The CSG engine 120 may provide a first output 260 to the medical device(s) 180. The first output 260 may include, for example, instructions for the medical device(s) 180 to perform a particular process or procedure, information for recordation and/or display at the medical device(s), instructions to record and/or display the information, requests for medical data, etc. In an implementation the instructions to perform a particular process or procedure may cause the medical device(s) to automatically perform the particular process or procedure. In such an implementation, the instructions may function as a control signal. Instructions sent to the medical device(s) as the first output 260 may include instructions to perform an intervention, parameters of the intervention, and/or instructions to record information about an intervention or other determination of the CSG engine 120 (e.g., instructions to record an event marker). Additionally, the CSG engine 120 may generate and send a second output 225 to the CSG UI 110 in the form, for example, of instructions or prompts for the rescuers 103, 104, information for recordation and/or display at the mobile device 105, etc.

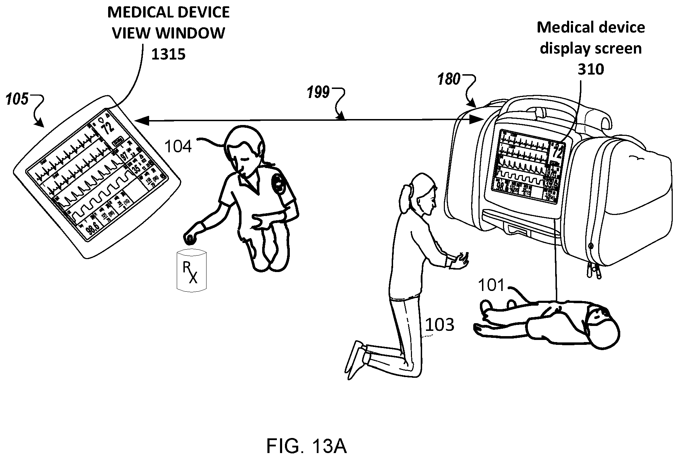

Aspects of the present disclosure are also directed to allowing a user, via inputs at a user interface screen of the mobile device 105, to supply inputs to the medical treatment device. During treatment of a critically ill patient, rescuers in the immediate vicinity of a patient (e.g., the caregiver 103 in FIG. 1 and FIG. 13A) are often consumed with tending to the medical needs of the patient, whether that includes administering electric shock or ventilation via the medical treatment device, administering chest compressions, administering ventilation, or treating wounds. Additionally, user input interfaces (e.g., keypads and other buttons for inputting information) that are local to the medical treatment device can be cumbersome to operate in time-critical situations. Instead, in some examples, users at a mobile device 105 can control one or more functional operations and/or provide one or more inputs at a user-friendly, convenient touchscreen at the mobile device 105 without interfering with patient treatment. In some scenarios, the caregiver providing direct care to the patient may also operate the mobile device 105. In other scenarios, a caregiver that is providing intermittent care and/or assistance to another caregiver and not providing direct care to the patient for some period of time may operate the mobile device 105. In some examples, mobile device 105 users can input patient information, record event markers, initiate 12-lead ECG analyses, or record a device snapshot. Therefore, allowing a user to provide instructions to activate one or more operations of the medical treatment device via the mobile device 105 provides enhanced technical flexibility that is not available when operating locally at the medical treatment device by allowing supervisors or other personnel at the scene of a medical event to observe, in real-time, how the medical event is progressing without having to hover over the treatment area, which may impede patient care.

In response to receiving user inputs at the mobile device 105 associated one of the control operations at the medical treatment device, the mobile device 105, in some implementations, transmits an instruction signal to cause the respective operation to occur at the medical treatment device. In some examples, instruction signals sent from the mobile device 105 to the medical treatment device can instruct the medical treatment device to update patient information, treatment information, or diagnostic information for the medical event. In response to receiving the respective signal, the medical treatment device performs the respective operation associated with the instruction signal, which may include storing provided information (e.g., transmitting patient information for updating at the medical treatment device) or recording an event marker (e.g., transmitting a treatment/event marker for the medical treatment device to record in the patient care record) or initiating a snapshot (e.g., transmitting an instruction signal for the medical treatment device to initiate a snapshot of ECG associated with the time of the instruction input) or activating an analysis feature (e.g., instruction signal for the medical treatment device to perform a 12-lead analysis) at the medical treatment device. In some embodiments, the instruction signals can also include control signals for causing the medical treatment device (a defibrillator) to initiate electrotherapy or apply another therapeutic treatment. When the medical treatment device is the ventilation system 280, the instruction signals can include control signals to administer the positive pressure ventilation to the patient. In some examples, upon initiation and/or completion of the respective operation, the medical treatment device transmits a notification signal to the mobile device 105. In response to receiving the notification signal from the medical treatment device, the mobile device 105 can cause display of a notification message at the mobile device 105 that the respective action is being performed; and then a subsequent notification signal from the medical treatment device for the mobile device 105 to display a notification message that he respective action has been performed. Thus, the systems and methods described herein provide a technical solution to the technical problem of enabling control of a medical treatment device by more than one user providing inputs at one or more mobile device 105 that are remote from the medical treatment device. For example, before the improvements described in the present disclosure were developed, personnel were limited to providing inputs or controlling operations directly at the medical treatment device interface. Because of this technical inconvenience, event markers, 12-lead analyses, and snapshots failed to be recorded at significant treatment points, reducing the effectiveness of event debriefs, personnel evaluations, and patient condition analyses. Therefore, the systems and methods described herein provide a solution to the clinical problem of providing patient care during critical medical events based on all available information in real-time (e.g., how treatment has been provided and how a patient is responding to all types of administered treatment). Further, the systems and methods described herein also solve the clinical problem of allowing supervising personnel to provide real-time treatment feedback to rescuers and others providing direct medical care, which creates a more effective clinical environment for both new and seasoned rescue teams and individuals.

In an implementation, the first output 260 to the medical device(s) 180 may include instructions for action at the medical device(s) 180. For example, the first output 260 may include, for example, an alarm instruction, an event marker instruction, a snapshot recording instruction, etc. As another example, the first output 260 may include a user selection of whether the patient is an adult, pediatric, or neonatal patient, which can be transmitted to the medical device(s) 180. In some examples, the patient type may determine alarm set points, waveform scale, defibrillation energy, and/or type of monitored case information.

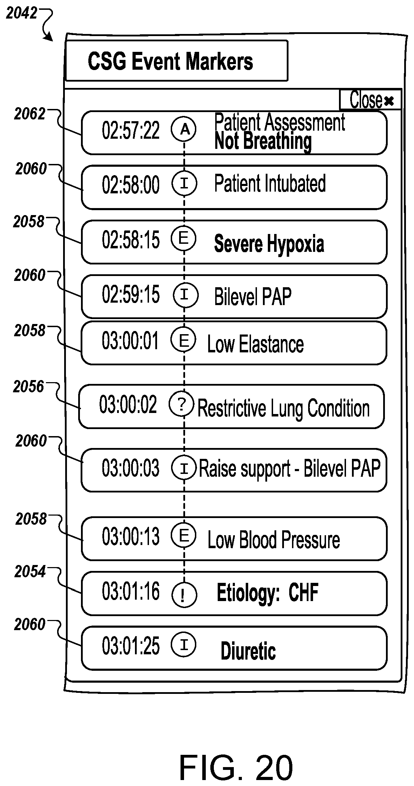

In an implementation, the first output 260 to the medical device(s) 180 may include patient information input to the CSG UI 110 and/or to another user interface at the mobile device 105. Such patient information input is described in more detail below with regard to FIG. 20. In some implementations, the second input 230 from the mobile device 105 may include a request to the medical device(s) 180 for any patient information already stored in memory 187 of the medical device(s) 180.

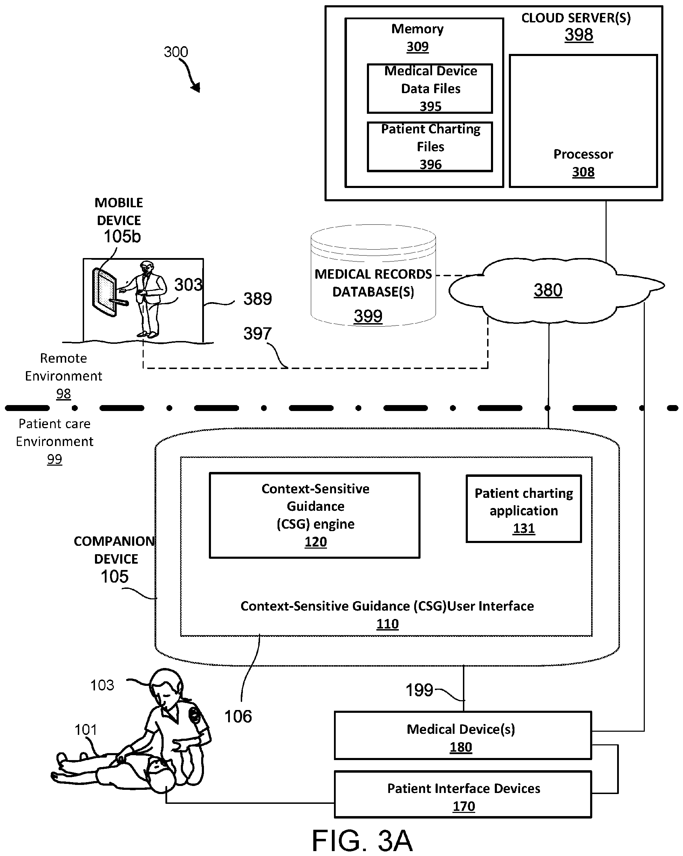

Referring to FIG. 3A, an example of a device configuration 300 for context sensitive guidance inclusive of various remote computing devices is shown. A quantity of each component in FIG. 3A is an example only and other quantities of each, or any, component could be used. In an implementation, at least one of at least one of the mobile device 105 and the medical device(s) 180 may communicatively couple to one or more remote devices via a network 380. The network 380 may be a computer network (e.g., the Internet), a cellular communications network, or a combination thereof. The remote devices may include cloud server(s) 398 and, optionally, a medical records database 399. In an implementation, the cloud server 398 may host the medical records database 399. The cloud server(s) 398 may include at least one processor 308 and a memory 309. The cloud server(s) 398 may additionally store medical device data files 395 uploaded to the cloud server(s) from the medical device(s) 180 and or from the medical device(s) 180 via the mobile device 105. The medical device data files 395 may be include files created by and stored at the medical device(s), i.e., the one or more patient encounter data files 188, and uploaded to the cloud server(s) 398 subsequent to a patient case. Further, the cloud server(s) may store patient charting files 396 uploaded to the cloud server from the mobile device 105.

Although illustrated in FIGS. 1, 2, and 3A-3D as a single mobile device 105 or 105a at the patient site, in various implementations, the patient care environment 98 may include one or more mobile devices 105 co-located at the patient site and communicatively coupled to the medical device(s) 180 via the communication link 199. In an implementation, at least one mobile device 105b may be located at one or more remote locations 389 from the patient site and accessed by at least one remotely located caregiver 303.

In some implementations, the wireless communication link 199 for connecting the medical device(s) 180 and the mobile device 105, may be a Wi-Fi network, a short-range wireless communication network or near field communication (NFC) network, local area network (LAN), wide area network (WAN), or the Internet. In other examples, the devices 180 and 105 can be configured to communicate over longer communications ranges such as over a cellular communication network. In some implementations, the medical devices 180 may function as a wireless access point to provide a direct wireless connection with the mobile device 105.

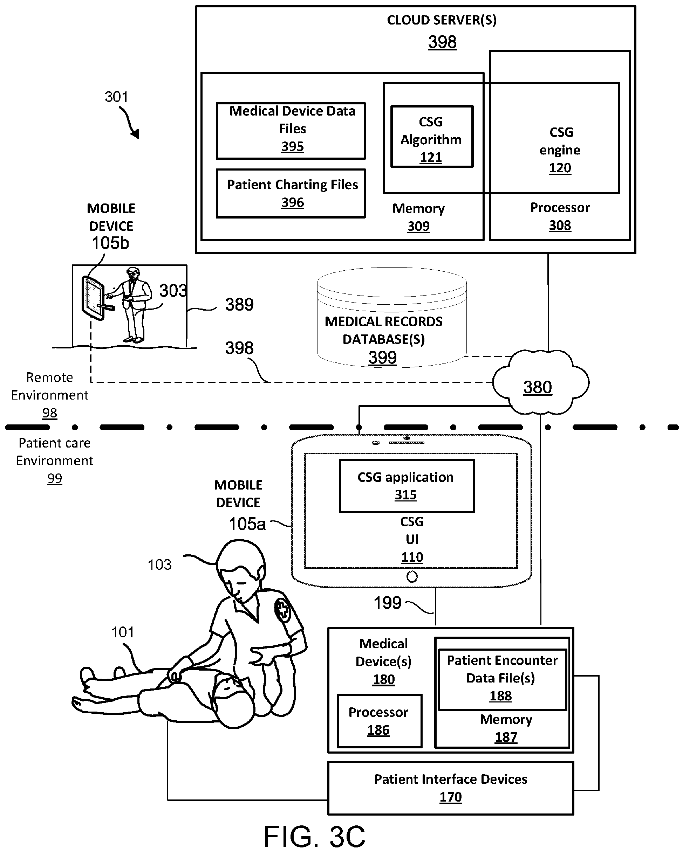

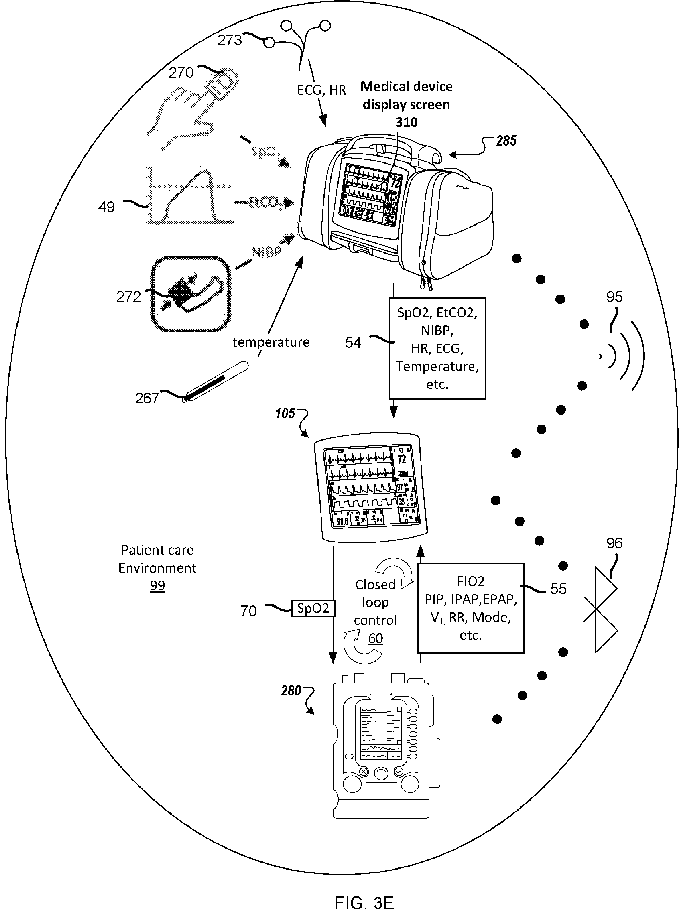

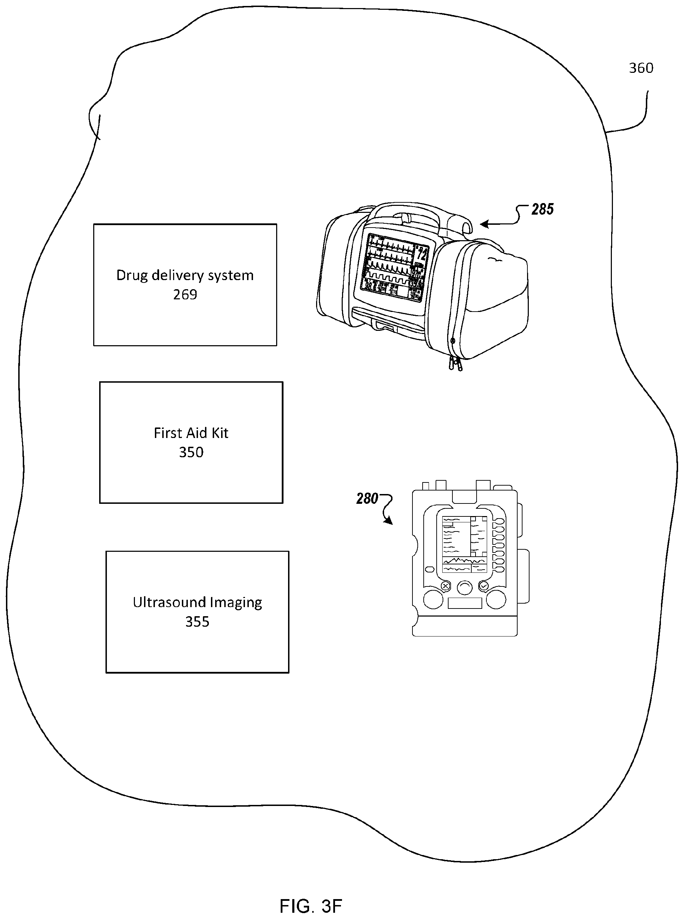

As shown in FIG. 3A, and similarly in FIGS. 3C-3G, the constellation of devices connected to and/or receiving information for a single patient, or victim, 101 form a patient care environment 98. Within the patient care environment 98, all of the medical device(s) 180 and patient interface devices 170 associated with the single patient form a medical device suite 360 (e.g., as shown in FIG. 3E and 3F, for example, the medical device suite 360 in this example includes the patient monitor/defibrillator 285, the ventilation system 280, and the interface devices 267, 271, 272, 270, and 273). The medical device suite 360 may be configured to provide acute care clinical interventions. In an implementation, the mobile computing device 105 is configured to communicatively couple with only one medical device suite 360, i.e., only with medical devices that are coupled to a single and same patient. Thus, all of the physiologic parameters and device information provided at the mobile device 105 are associated with that same single patient. As such, the data view windows at the mobile device provide data for one patient and the CSG UI is a dedicated UI for only one patient. The medical device suite 360 and the patient care environment 98 are disposed at a point of care for the patient 101.

In various implementations, some or all of the devices within the patient care environment 98 may be configured to additionally communicate with computing and/or communication devices in a remote environment 97 to enable, for example, telemedicine, health history, and data storage services. A long-range wireless network (e.g., a computer network, such as the Internet, and/or a cellular network) may enable the communicative coupling between one or more devices within the patient care environment 98 and the one or more devices in the remote environment. The one or more remote computing devices may include an electronic patient care record service, a health information exchange, an electronic medical record service, a cloud server, a computing device associated with a telemedicine provider, a computer aided dispatch service, a medical data exchange, or combinations thereof.

The patient care environment 98 is a patient-centric environment in which medical devices, computing devices, and caregivers are focused on the short-term care of the single patient. At any given time, the patient interface devices 170 are coupled to a single patient 101 treated by one or more caregivers 103. Thus the inputs and outputs to and from the CSG engine 120 are also for the single patient 101. The short-term care, which may be acute critical care, entails identifying one or more clinical presentations that require interventions to ensure that the patient stays alive and can be moved or transported to a longer term care environment.

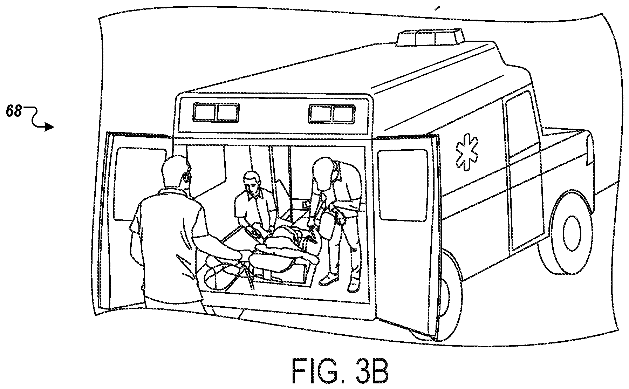

An emergency scene may include one or more patient care environments 98. Examples of an emergency scene may include, for example the site a medical transport vehicle scene 68 as shown, for example, in FIG. 3B. The emergency scene is not limited the scene 68 but rather includes any point of care location for a victim of respiratory distress or another emergency. Regardless of the specific physical location, but as illustrated by the scene 68, the emergency scene may be a chaotic and crowded environment in which acute critical care is necessary with a smaller choice of medical devices than might be available at a large hospital, for example. Each patient care environment within an emergency scene includes a single patient, the one or more caregivers attending to that patient, and the medical devices associated with that patient. In various situations, caregivers may attend to multiple patients and move between patient care environments. At an emergency scene there may be multiple caregivers and/or multiple patients crowded into a small area. The medical skill and experience of the responding caregivers may vary (for example, the fire rescue personnel or first responders may have less medical expertise than the ambulance crew in FIG. 3B. Additionally, the medical skill and experience may vary within a team of caregivers. Thus a CSG system, as described herein, that can adapt the guidance to available medical devices is critical to patient survival. Furthermore, there may not be access to remotely located caregivers and servers due to a lack of network connectivity. For example, a rural or military location, a parking garage, an interior location, or a moving vehicle may have little or no Internet or cellular network access or variable communication signal strength. Therefore, a CSG system, as described herein, that can adapt the guidance to the available caregiver skill levels is also critical to patient survival. Finally, because of the speed of care required to save a life and the environmental distractions that abound in an acute care scene, a CSG system, as described herein, that can focus the caregiver on the immediately necessary tasks and critical patient medical parameters is also critical to patient survival. Caregivers in these settings do not have the luxury of time to, for example, contemplate a patient's condition, request lengthy lab or imaging procedures, or comb through a patient's medical history. They must make accurate split second decisions that are aided by a CSG system designed for this environment as described herein.

Within the patient care environment 98, there may be one or more additional computing devices such as a smartphone, a laptop, a notebook, a heads-up device, a wearable device, a GPS or other navigation device, etc. The communications interfaces 132a and/or 132b may be configured to communicatively couple the mobile device 105 and/or one or more medical device(s) 180 to these local additional computing devices.

Referring to FIG. 3C, an example of a device configuration 301 for context sensitive guidance hosted by a cloud server is shown. A quantity of each component in FIG. 3C is an example only and other quantities of each, or any, component could be used. In an implementation, the memory 309 (i.e., a non-transitory processor readable storage medium) of the cloud server(s) 398 may store the instructions of the CSG algorithm 121. The CSG engine 120 may include hardware logic and/or software logic provided by the processor 308 and/or the memory 309 to implement CSG at the mobile device 105. In such an implementation, the cloud server 398 and the CSG engine 120 may service or host a CSG application 315 at the mobile device 105.

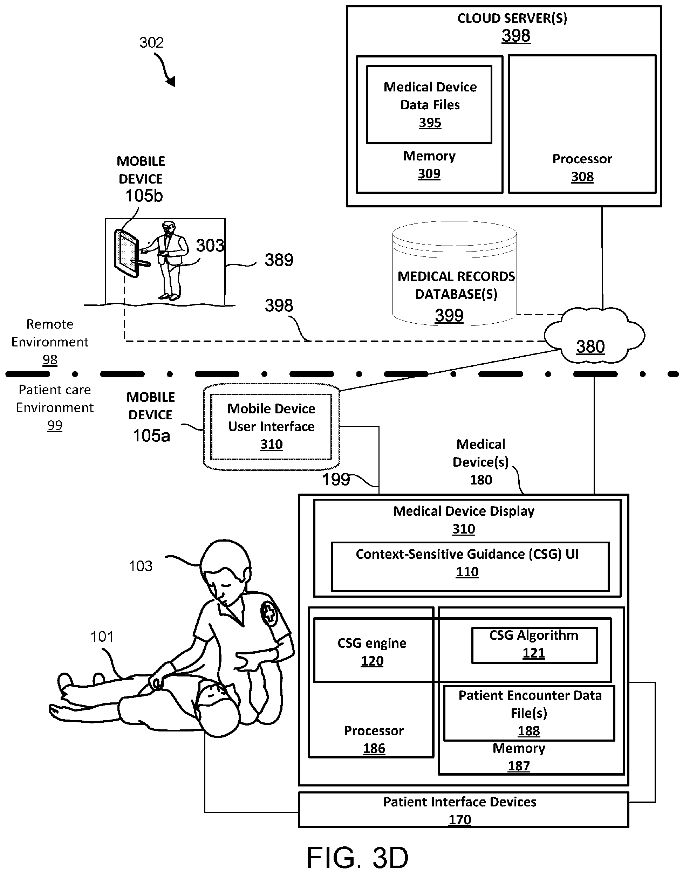

Referring to FIG. 3D, an example of a device configuration 302 for context sensitive guidance implemented at a medical device is shown. A quantity of each component in FIG. 3D is an example only and other quantities of each, or any, component could be used. In an implementation, the memory 187 (i.e., a non-transitory processor readable storage medium) of the medical device(s) 180 may include the CSG algorithm 121. The CSG engine 120 may include hardware logic and/or software logic provided by the processor 186 and/or the memory 187 to implement CSG at the medical device. A display 310 of the medical device(s) may provide the CSG user interface 110. For example, a display screen of a patient monitor/defibrillator 285 and/or a ventilation system 280 may provide the CSG user interface 110.

Referring to FIG. 3E, an example of a device configuration for respiratory distress context sensitive guidance is shown. A quantity of each component in FIG. 3E is an example only and other quantities of each, or any, component could be used. Within the patient care environment 98, the device configuration shown in FIG. 3E provides context sensitive guidance and appropriate clinical devices to address at least a presentation of respiratory distress (RD) in a patient. For example, the SpO2 sensor 270, the EtCO2 sensor 271 (e.g., as exemplified in FIG. 3E by EtCO2 data 49), the NIBP sensor 272, the temperature sensor 267, and the electrodes 273 may provide physiologic data 54 (e.g., SpO2 data, EtCO2 data, NIBP data, temperature data, ECG data, and heart rate data) to a medical device 180 like the patient monitor/defibrillator 285.

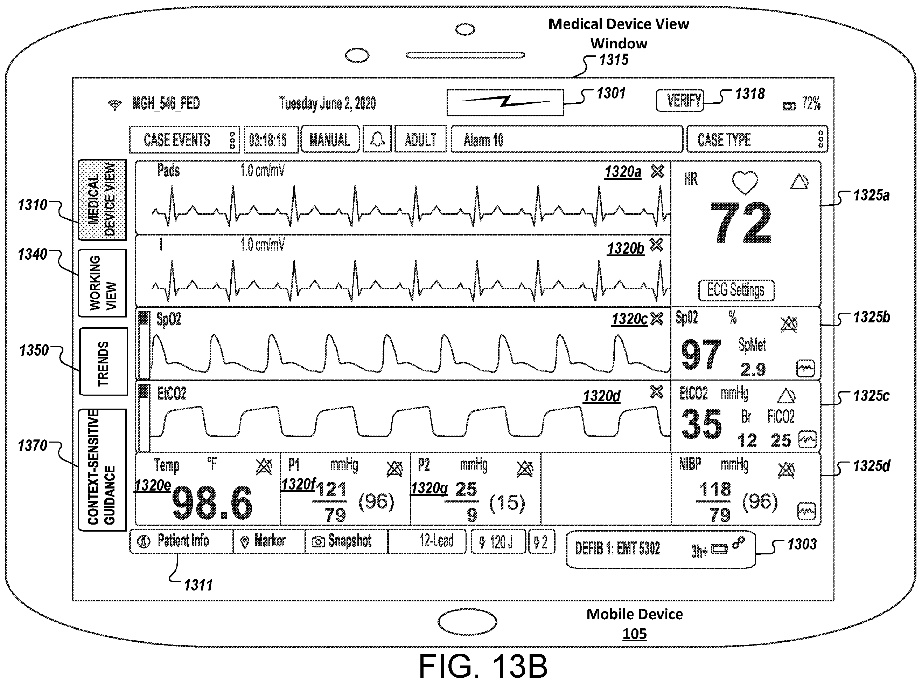

The monitor/defibrillator 285 may communicatively couple, for example via a Wi-Fi connection 95, as shown, or a Bluetooth connection 96 or another wireless communication coupling to the mobile device 105. The mobile device 105 and medical device(s) 180 may include communications interfaces 132a and 132b, respectively, that enable the communicative coupling. In turn, the patient monitor/defibrillator 285 may provide the physiologic data 54 in real-time at the medical device display screen 310 and also send the physiologic data 54 to the mobile device 105 for display at the mobile device 105 in various view formats as described below, for example, at least with regard to FIGS. 13B-16 and FIGS. 29-43. Those view formats may include a device view window that provides all of the data available at the medical device display screen 310 and in a similar format along with a CSG window 110 and optionally a trend view window 1515 and a working view window 1415. These various windows are described in further detail below.

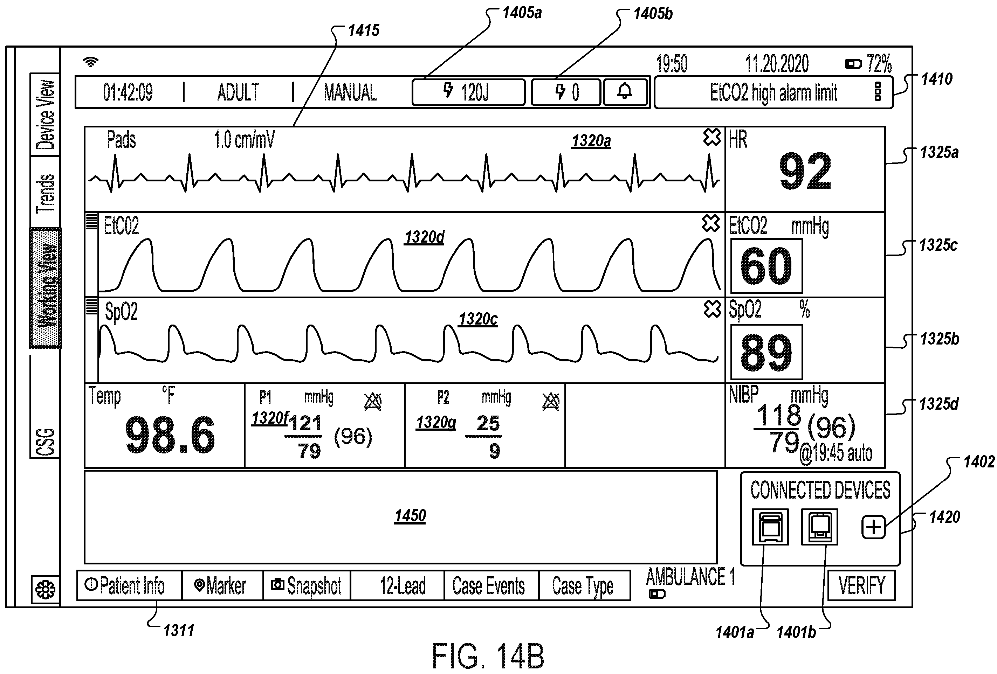

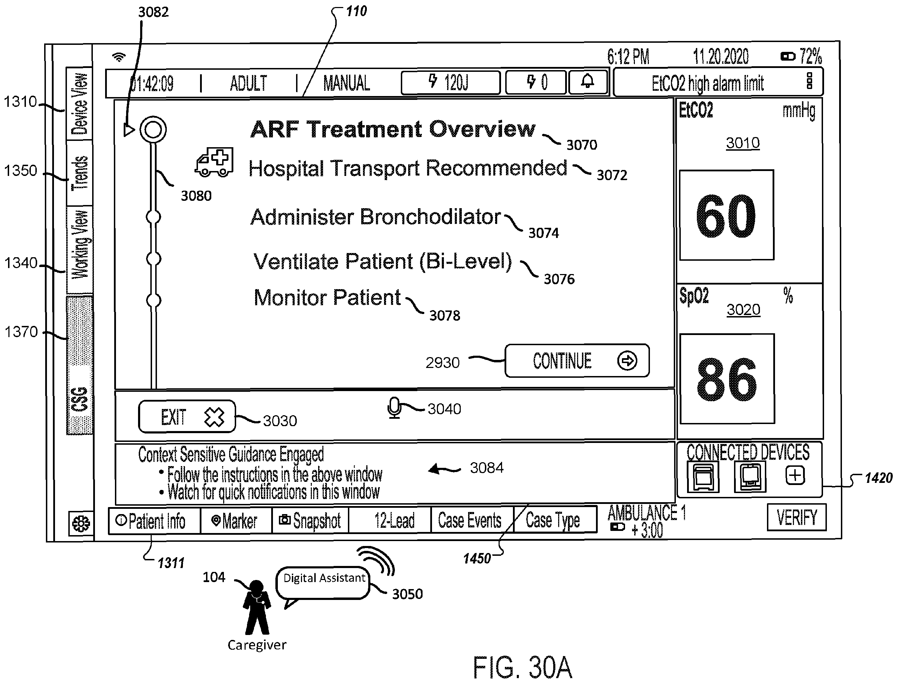

In an implementation, the mobile device 105 may identify the at least one medical device based on the communicative coupling. For example, the mobile device 105 may identify one or more of a type of the coupled medical device (e.g., a patient monitor/defibrillator 285, a patient monitor, a ventilation system 280, a first aid kit 350, an ultrasound imaging device 355, a drug delivery system 269, and other components of the medical suite 360 as shown for example in FIG. 3F), a capability of a coupled medical device (e.g., a therapy delivery capability, a monitoring capability, an imaging capability, a sensor attachment capability, a mode of operation, etc.), and/or a model number. In an implementation, one or more of the CSG window 110 and the working view window 1415 may include a connected devices window 1420 (as shown and discussed further in regard to FIGS. 14B and 30A). The connected devices window 1420 may identify the medical device(s) 180 coupled to the mobile device 105. In an implementation, the CSG engine 120 may modify or adjust a patient care protocol based on the available equipment.

In an implementation, the mobile device 105 may couple with a first or initial medical device, for example, at the outset of patient care. Subsequently, other devices (e.g., at least one additional medical device) may establish communications with the mobile device 105. The mobile device 105 may identify the subsequent devices based on the communicative coupling and the CSG processor 120 may update the connected devices window 1420 to include the identification of these subsequent devices as they are added to the system. In an implementation, the CSG engine 120 may control the CSG UI and display at the mobile device 105 to provide physiologic parameters from the initial medical device at the UI and display. When an additional medical device is coupled to the mobile device 105, the mobile device 105 may receive additional physiologic parameters from that additional device and, in response, may modify the CSG UI and other view windows at the mobile device (e.g., working, device, and/or trend views) in real-time to include one or more of the additional physiologic parameters. In an example, the initial medical device may be a patient monitor or a patient monitor/defibrillator (e.g., the patient monitor/defibrillator 285). The caregiver may initiate care of the victim 101 with this medical device to determine one or more presenting conditions of the victim 101. The patient monitor or patient monitor/defibrillator may provide, for example, one or more of ECG, EtCO2, SpO2, heart rate, body temperature, and non-invasive blood pressure (NIBP) data. Based at least in part on this data, the CSG system may identify a RD condition of the patient, possibly acute respiratory failure (ARF) and provide ventilation instructions to address the identified RD condition (e.g., the detected ARF); examples of such ventilation instructions are discussed in further detail herein. In such a scenario, the ventilation system 280 may be the additional medical device.

Based on the physiologic data and a CSG engine, the mobile device 105 may provide care instructions to a caregiver (e.g., at the CSG UI 110) and/or may provide instructions to the medical device 180 and/or one or more second medical devices such as the ventilation system 280. In an implementation, the instructions may be in the form of physiologic closed-loop control 60 of a medical device based on information sent and received from the medical device (e.g., SpO2 data 70 sent to the ventilation system 280 and ventilation system data 55 sent from the ventilations system 280). The ventilation system data 55 may include some or all of: ventilator system mode, airway pressure (Paw), plateau pressure (Pplat), peak inspiratory pressure (PIP), inspiratory positive airway pressure iPAP, expiratory positive airway pressure ePAP, tidal volume (VT), respiratory rate (RR), fraction of inspired oxygen (FiO2), positive end-expiratory pressure (PEEP), forced vital capacity (FVC), forced vital capacity at 1 second (FEV1), peak expiratory flow rate (PEF or PEFR), patient respiratory system resistance Rrs, patient respiratory system compliance Crs, inspired and expired tidal volume, Ve, end-tidal CO2 (EtCO2), volume of exhaled carbon dioxide (VCO2), and breaths per minute (BPM). In an implementation, a spirometer may provide one or more of forced vital capacity (FVC), forced vital capacity at 1 second (FEV1), and peak expiratory flow rate (PEF or PEFR). The spirometer may be separate from or a component of the ventilation system.

As part of the communicative coupling of the medical device(s) 180 to the mobile device 105, the communications interface 132a may authenticate the medical device(s) 180 and establish the communicative coupling in response to and based on the authentication. The CSG processor 120 and/or the communications interfaces (132a and/or 132b) may enable data encryption of some or all of the data exchanged between the mobile device 105 and the medical device(s) 180. In an implementation, the data encryption may separately shield patient data, in particular any patient identifying data (PID), to prevent or limit access to the PID by unauthenticated devices and/or users of the mobile device 105 and/or the medical device(s) 180 that lack authorization to access PID. In some cases, encryption may enable access to de-identified physiologic data.

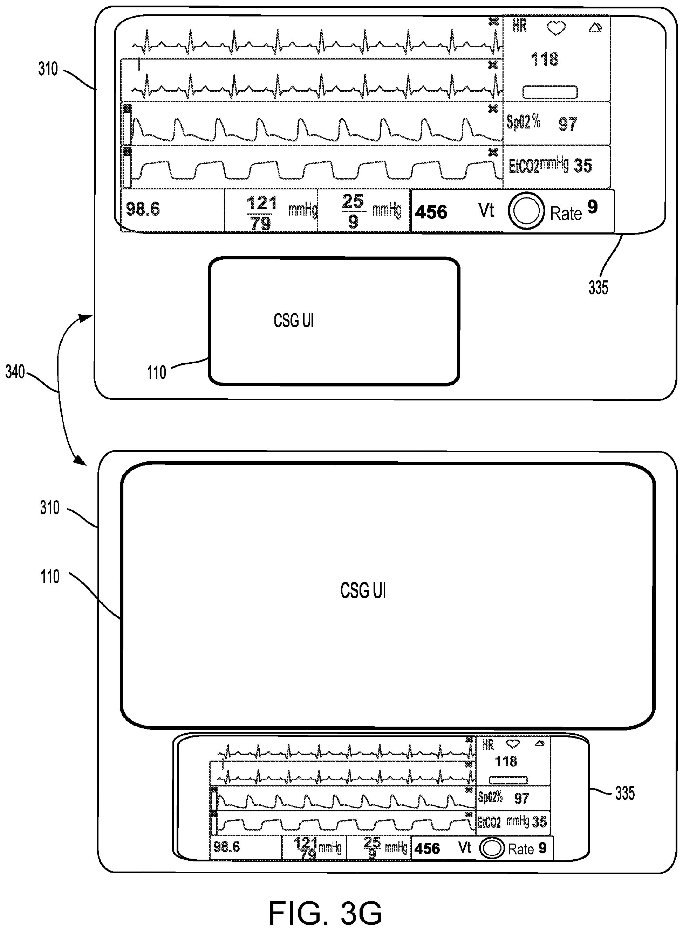

Referring to FIG. 3G, an example of a combined operational and CSG user interface on a medical device is shown. In an implementation, the processor 186 may control the display screen 310 to selectively display the operational interface 335. For example, the display screen 310 may provide an operational interface 335, the CSG user interface 110, or a combined display as shown in FIG. 3G. In an implementation, the medical device(s) 180 may enable a user to toggle 340 between a primary operational interface 335 and a primary CSG user interface 110. The operational interface 335 may provide patient data received by the medical device(s) 180 from the patient interface device(s) 170. The operational interface 335 may provide the patient data in real-time as the signals are received and processed by the processor 186 of the medical device(s) 180.

The processor 186 may be configured to implement a particular display mode to selectively display the operational interface 335 and the CSG user interface 110. The processor may select the implemented display mode from available display modes for the display screen 310. For example, the available display modes may include an operational interface only mode, a CSG user interface only mode, or a combined mode as illustrated in FIG. 3G.

In the combined operational/CSG mode, the processor 186 may control the display screen 310 to simultaneously display the operational interface 335 and the CSG user interface 110.

In the simultaneous display, the display screen may provide the operational interface 335 in a first portion of the display screen 310 and may provide the CSG user interface 110 in a second and different portion of the display screen 310. The first portion and the second portion may be the same size or may be different sizes. For example, as shown in FIG. 3G, the CSG user interface 110 may occupy a smaller area on the display screen 310 than the operational interface 335. This configuration may be a default state for the medical device(s) 180. The processor 186 may enable the display mode to toggle 340 to a mode where, the operational interface 335 may occupy the smaller area on the display screen 310 than the CSG user interface 110.

The operational interface 335 and the CSG user interface 110 may include labels in order to unambiguously visually distinguish between these two interfaces. This may prevent the user of the medical device(s) 180 from confusing the two interfaces. The labels may include different colors, different color schemes, different shapes, and/or distinguishing text and/or graphics that identify the interface as the operational interface 335 or the CSG user interface 110.

The medical device(s) 180 may simultaneously display the operational and CSG user interfaces in various combined mode display configurations. The combined mode display configuration may determine the relative sizes of the interfaces (i.e., which interface occupies the smaller area on the display screen 310 and which interface occupies the larger area on the display screen 310). For example, the simultaneous display may include a side-by-side configuration on one display screen without overlap between the two interfaces. As another example, one of the first portion and the second portion of the display screen 310 may be an inset window that overlaps the other of the first portion and second portion. The interface in the inset window may occupy a smaller area on the display screen than the interface that is not in the inset window. In various implementations, the inset window may occupy an area approximately 10%, 20%, 30% or up to 50% of the total display area. As a further example, the medical device(s) 180 may include multiple displays and the operational interface 335 and the CSG user interface 110 may occupy different displays. These configurations of the combined mode are examples only and not limiting of the disclosure.

In an implementation, the user of the medical device(s) 180 may provide user input that determines the selected display mode and/or the combined mode display configuration. For example, the user may select the display mode via a soft-key and/or other input device for the medical device(s) 180. In an implementation, the medical device(s) 180 may include a mechanical and/or electronic mode selection switch in the form of a button, a knob, a toggle switch, a touchscreen button, a soft-key (i.e. a mechanical switch whose function changes based on adjacent text on the display screen), etc. that may capture a user selection of the display mode. Selecting the display mode may include changing from one display mode to another and/or toggling between display modes in response to the user input. In an implementation, the display screen 310 may provide a display a display mode menu. In a further implementation, the medical device(s) 180 may capture the user selection via a microphone (e.g. a verbal mode selection) and/or a haptic input device (e.g., a tactile input mode selection).

In an implementation, the display screen 310 may be a touchscreen configured to capture the user input that determines the selected display mode and/or the combined mode display configuration. In an implementation, the touchscreen may be a pressure sensitive touchscreen and the gestures may include a push gesture to exert pressure on the display screen 310. The percentage of the total area of the display screen 310 occupied by the CSG user interface 110 may increase or decrease based on the pressure exerted on the pressure sensitive touchscreen by the user. For example, the CSG user interface 110 may occupy a first area on the display screen 310 that corresponds to a smaller percentage of the total area of the display screen 310 than a second area on the display screen 310 occupied by the operational interface 335. When the pressure sensitive touchscreen detects a pressure that exceeds a threshold in the first area of the display screen (e.g., as occupied by the CSG user interface 110), the first area may expand to occupy a relatively larger percentage of the total area of the display screen 310. If the detected pressure drops below the threshold, the first area may shrink in size in response to the detected drop in pressure. In some implementations, the pressure threshold may be, for example, 0.1, 0.2, 0.5, 1, 2, or 5 pounds of force. In some implementations, there may be two pressure thresholds. A first pressure threshold may determine an expansion of the area of the CSG user interface 110, and a second pressure threshold may determine a reduction of the area of the CSG user interface 110. Similarly, in some implementations, the operational interface 335 may occupy an area on the display screen 310 that is a smaller percentage of the total area of the display screen 310 than the CSG user interface 110. Measured pressure at the pressure sensitive touchscreen and/or one or more predetermined thresholds may control the expansion and reduction of the area of the operational interface 335 in a manner similar to the above-described control of the area of the CSG user interface 110.

In various implementations, the user may select the display mode via various touchscreen selections. For example, the user may tap a touchscreen icon to provide a touchscreen signal that causes the inset window to switch from the CSG user interface 110 to the operational interface 335. As another example, the user may select the display mode via selection from a touchscreen menu, or may scroll to the desired selection via a control interface such as a knob, dial or button. As another example, the user may tap or push on the CSG user interface to display this interface on the larger area of the display screen and move the operational interface to the inset window so that the CSG user interface intuitively moves to a desired location as indicated by the user touch commands. As a further example, the user may provide a touchscreen gesture to enlarge a desired interface. For instance, the user may provide a two-finger gesture to the CSG user interface to switch the display mode from the combined mode to the CSG user interface only mode. The user may provide the two-finger gesture to change the interface that is displayed in the inset window and/or to switch from the combined mode to the operational interface only mode. As yet another example, the user may drag and drop a desired interface to a desired area on the display screen. For example, the user may drag the operational interface 335 to the inset window and in this manner switch the relative sizes of the operational and CSG user interfaces.

In some implementations, the area of the CSG user interface 110 may be approximately 10%, 20%, 30% or up to 50% of the area of the operational interface 335. A double-tap and/or another gestural interaction with the touchscreen and/or exerted pressure of a pressure sensitive screen, in the area of the CSG user interface 110, may cause the CSG user interface 110 area to enlarge to up to 90% of the operational interface 335 area. Subsequent to this enlargement, touching anywhere on the operational interface 335 area of the touchscreen causes the CSG user interface 110 area to decrease back to its previous size.

As an alternative to or in addition to the user input, in an implementation, the processor 186 may detect a state of the medical device(s) 180. Based on the detected state of the medical device, the processor 186 may automatically determine and implement a processor-selected display mode. In an implementation, the processor 186 may override a user selection to implement the processor-selected display mode and/or configuration. The processor 186 may automatically switch the display configuration from the user-selected display mode and/or configuration to a processor-selected display mode and/or configuration. As an example, depending on the state of the medical device, it may be crucial for the caregiver to view the operational interface. In such a case, for example, if the display screen 310 is in the CSG user interface only mode or in a combined mode with a relatively larger CSG user interface displayed, the processor 186 may automatically change the implemented display mode to make the operational interface 335 available to the user of the medical device(s) 180 (e.g., switch to the operational interface only mode or the combined mode with a relatively larger operational interface 335).

In an implementation, the processor 186 may be configured to detect that the medical device is in a therapy delivery state. In the therapy delivery state, the medical device(s) 180 may currently or imminently be providing therapy to the patient based on a detected status of one or more patient interface device(s) 170 and/or an alarm state of the medical device(s) 180 and/or a data analysis mode of the medical device(s) presaging therapy delivery (e.g., ECG analysis prior to defibrillation). As alarm examples, a heart rate alarm triggered by a detected heart arrhythmia may indicate that cardiac therapy delivery may be imminent. Similarly, a blood oxygen level alarm may indicate that ventilation therapy delivery may be imminent.

In an implementation, the processor 186 may be configured to detect that the medical device is in a patient monitoring state. The patient monitoring state may correspond to the medical device being coupled to the patient via sensors only without therapy delivery components. For example, the medical device may be coupled to the patient via twelve-lead cardiac sensing electrodes but not with defibrillation electrodes.

In an implementation, the processor 186 may be configured to detect that the medical device is in a caregiver guidance state. For example, the medical device may be in the process providing compression and/or ventilation feedback during cardiopulmonary resuscitation CPR, etc. In the caregiver guidance state, the processor 186 may control the display such that caregiver feedback provided at the operational interface is provided in an operational interface only mode or in a combined mode with a relatively larger operational interface.

In an implementation, the processor 186 may limit the display modes available for user selection based on the state of the medical device. The processor 186 may disallow selection of one or more of the modes such that the processor may not implement the one or more of the modes at the display screen 310. For example, the processor 186 may limit the available display modes in response to a detection that the medical device(s) 180 is coupled to the patient via the therapy delivery component(s) 161. In this case, the processor 186 may disallow and/or disable selection of the CSG user interface only mode. In this way, the processor 186 may prevent the user of the medical device(s) 180 from putting the medical device(s) 180 into the CSG user interface only mode during delivery of critical care. As another example, the display screen 310 may be in the combined mode with the operational interface 335 in the smaller area of the display screen 310. In response to the detection of the therapy delivery components being coupled to the patient, the processor 186 may automatically switch the configuration to put the CSG user interface 110 into the smaller area of the display screen 310. As a further example, the display screen 310 may be in the combined mode with the CSG user interface 110 already in the smaller area of the display screen. In response to the detection of the therapy delivery components being coupled to the patient, the processor 186 may disable a user option to modify the display configuration to put the operational interface 335 into the smaller area of the display screen. In yet other examples, the processor 186 may allow or disallow selection of one or more of the display modes based on a type and/or skill level of a user (e.g., BLS, ALS, documenter, physician, etc.) and/or the processor 186 may allow or disallow selection of one or more of the display modes based on a clinical condition of the patient.

In an implementation, rather than automatically changing the display mode and/or configuration, the processor 186 may provide a display mode instruction for the user. For example, based on the detected medical device state, the processor 186 may control the display screen 310 and/or another output device, such as a microphone, to provide a user instruction to switch and/or maintain the display mode and/or configuration. The instruction for the user may be a message to switch to a particular mode or may be a message that the user should not select a particular mode.

In an implementation, the processor 186 may automatically determine, limit, or provide an instruction for the display mode and/or configuration based on a detected medical event. For example, the medical event may include a defibrillation shock, an arrhythmia, return of spontaneous circulation (ROSC) and/or another measured and/or observed physiological condition detected by the medical device(s) 180. The medical device(s) 180 may detect the medical event based on an automated assessment of the sensor signals and/or based on caregiver input. For example, in the case of a detection of certain heart rhythms, such as ventricular fibrillation or tachycardia, the medical device(s) 180 may instruct the user not to switch to the CSG user interface only mode.

In an implementation, the processor 186 may request a confirmation of a display mode change from the user. The confirmation may be part of a processor-controlled or a user-requested display mode change. In a further implementation, the processor 186 may generate an alarm indicating an occurrence of or an impending occurrence of the display mode and/or configuration change. In another implementation, the processor 186 may generate the alarm and request the confirmation.

In an implementation, the software, firmware, and/or hardware associated with the medical device(s) 180 may include a user and/or manufacturer configurable lockout setting that may prevent or limit display mode and/or configuration changes. The lockout setting may depend on the type of medical device and/or the status of the medical device. For example, an AED designed for use by caregivers who may have little or no medical training may include a lock-out setting that prevents the AED from being used in the CSG user interface only mode and/or from being used in a combined mode with the CSG user interface in the larger area of the display screen. As another example, a defibrillator may include a lock-out setting that prevents the device from being used in the CSG user interface only mode and/or from being used in a combined mode with the CSG user interface in the larger area of the display screen once the defibrillation electrodes are removed from a package and/or attached to the patient.

The CSG user interface 110 may leverage various display appearances to differentiate between this interface and the operational interface 335. The appearance of the CSG user interface 110 as an interface secondary to the operational interface 335 may remind the user to pay attention to the operational interface 335 to ensure that data review on the CSG user interface 110 enhances the delivery of care to the patient without detriment. In an implementation, the display screen 310 may display the CSG user interface 110 with a grayed-out boundary as compared to the operational interface 335 in order to distinguish between them. Additionally or alternatively, the display screen hosting the CSG user interface 110 may provide the CSG user interface 110 with a background color and/or pattern that is different from the operational interface 335 in order to distinguish between these interfaces. In various implementations, the CSG user interface 110 may exhibit graphical elements such as shadowing, parallax, and/or other three-dimensional rendering such as shading, highlights, reflections, etc. These graphical elements may cause the CSG user interface 110 to appear either nearer or farther from the viewer than the operational interface 335. The appearance of the CSG user interface 110 as farther from the viewer than the operational interface 335 may serve to indicate to the user that the operational interface 335 is a primary functional interface.

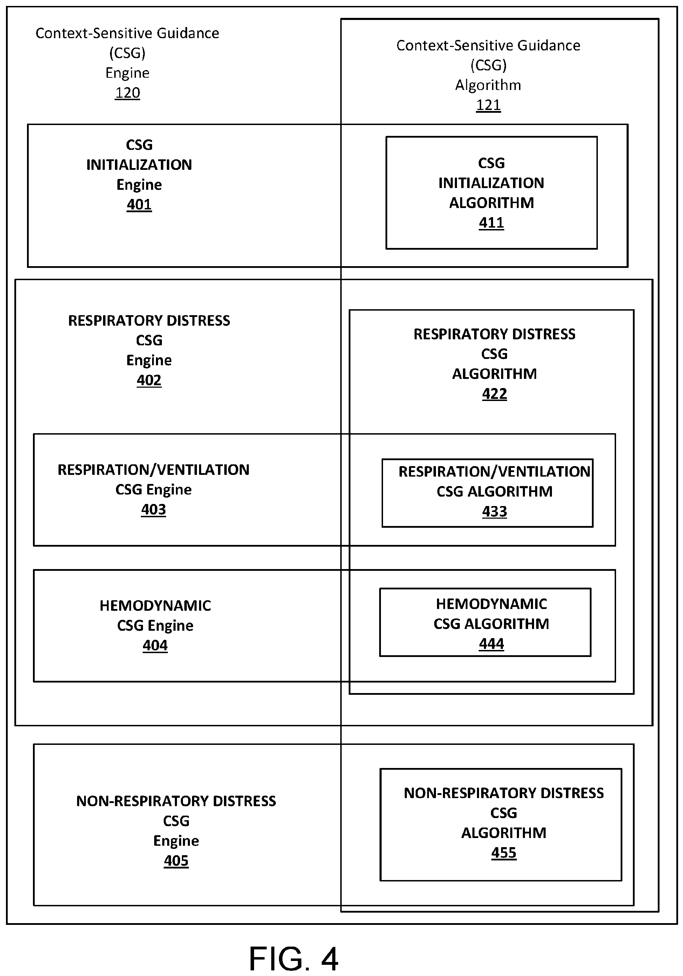

Referring to FIG. 4, examples of CSG algorithm and corresponding engines are shown schematically. One or more of the mobile device 105, the medical device 180, and the cloud server(s) 398 may include the CSG engine 120. The CSG algorithm 121 may include instructions stored in a non-transitory medium and executable by the processor 108, 186, or 308. The CSG algorithm 121 includes a CSG initialization algorithm 411, an RD CSG algorithm 422, and a non-RD CSG algorithm 455. The CSG engine 120 includes a CSG initialization engine 401, an RD CSG engine 402, and a non-RD CSG engine 405. Each engine includes the hardware logic and/or software logic to execute a corresponding algorithm.

The example of the CSG engine 120 as described herein is divided based on a patient's presentation with or without RD. However, this is an example only and in other examples, the CSG engine may be organized around one or more other clinical conditions (e.g., patient presentation with or without cardiac distress, emergent vs. non-emergent conditions, patient presentation with or without trauma, etc.). In practice, a patient often presents with multiple clinical conditions and therefore multiple engines run concurrently while managing the interactions with the user of the engine s and communicatively coupled devices to guide, monitor, and control patient care.

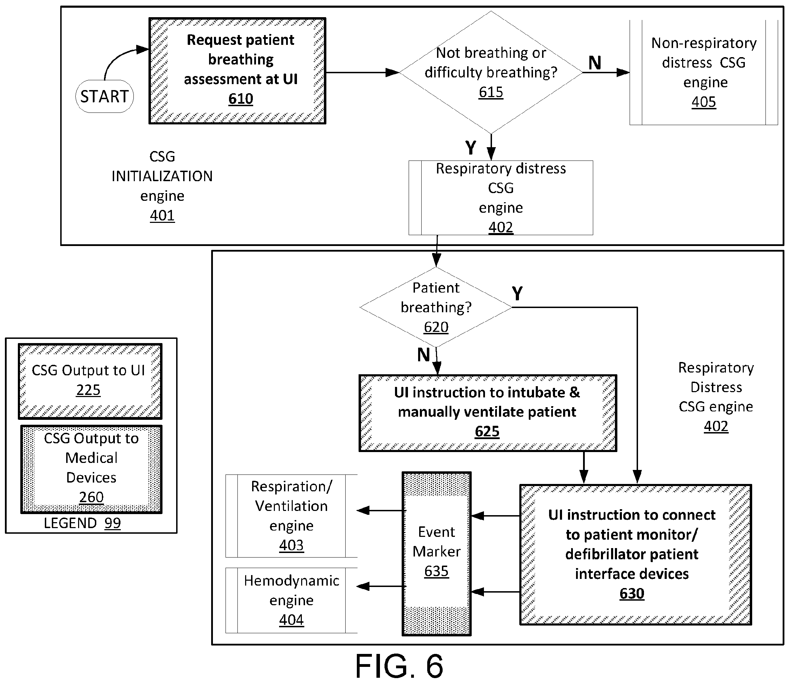

The CSG initialization engine 401 is discussed in more detail with regard to FIG. 6. The CSG initialization engine 401 utilizes a caregiver assessment of a medical care recipient to initiate care and initiate the appropriate CSG engine based on the caregiver assessment.

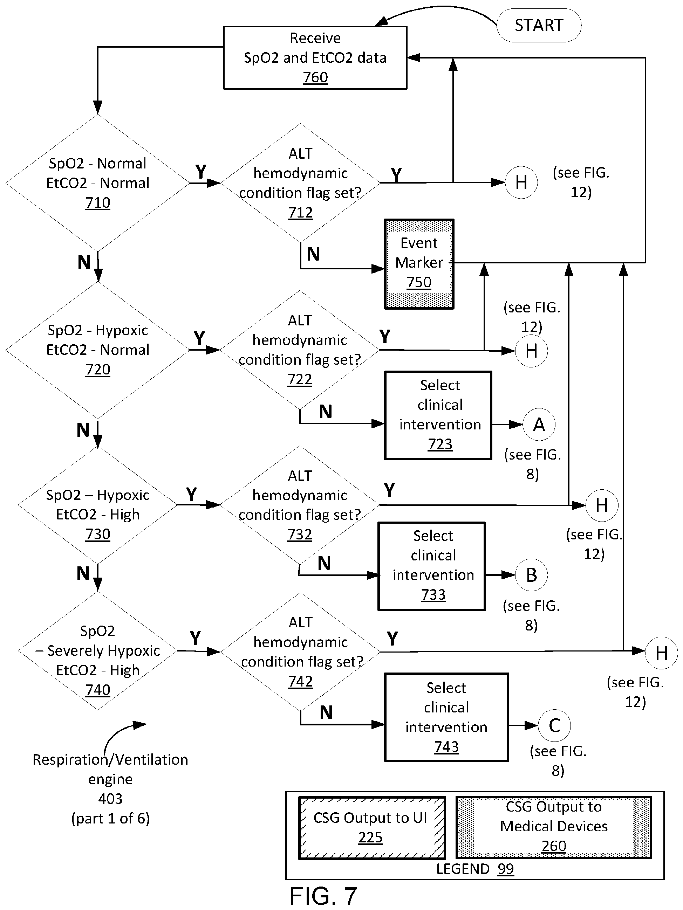

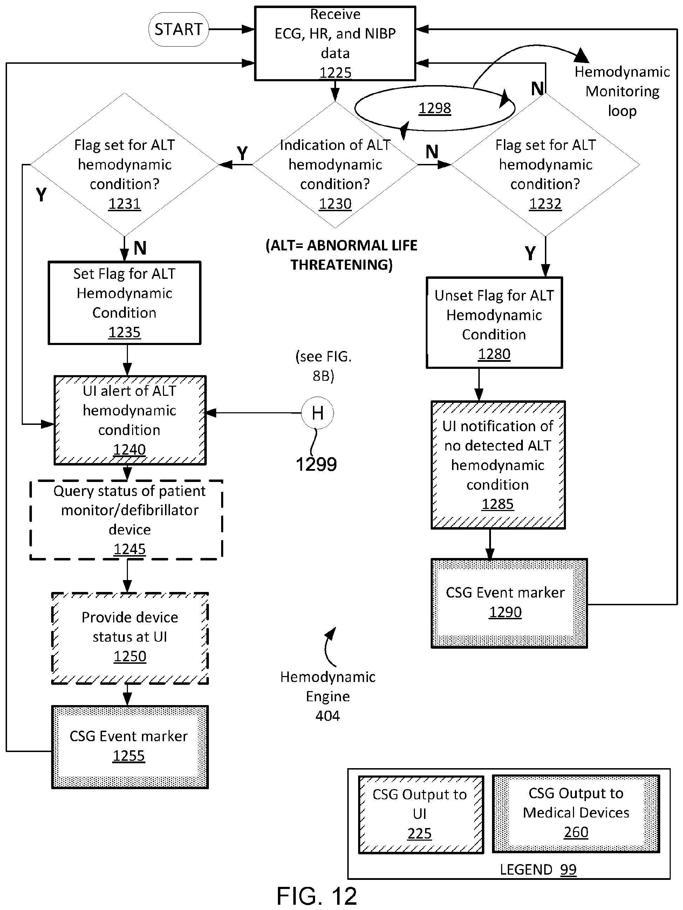

In the presence of an RD presentation, the CSG initialization engine 401 initiates the RD CSG engine 402. The RD CSG engine 402 includes an R/V CSG engine 403, and a hemodynamic CSG engine 404. The R/V CSG engine 403 is discussed in more detail with regard to FIGS. 7-11 and the hemodynamic CSG engine 404 is discussed in more detail with regard to FIG. 12. The R/V CSG engine 403 guides, monitors, and/or controls patient respiration and ventilation care as provided by the medical device(s) 180 and the caregiver 103. The hemodynamic CSG engine 404 guides, monitors, and/or controls patient hemodynamic care as provided by the medical device(s) 180 and the caregiver 103.

In the absence of an RD presentation, the CSG initialization algorithm 411 initiates the non-RD CSG algorithm, executable by the non-RD CSG engine 405. In various implementations, the non-RD CSG engine 405 may perform functions similar to those described herein for the RD CSG engine 402. However, rather than performing these functions in the context of a respiratory presentation, the non-RD CSG algorithm performs these functions in the context of a non-RD presentation such as, for example, but not limited to, cardiac presentation, trauma presentation, sepsis presentation, etc. In these cases, the medical device(s) 180 may include the ventilation system 280, the patient monitor/defibrillator 285, and/or other medical devices as appropriate for the particular patient presentation. In an implementation, the RD CSG engine 402 and the non-RD CSG engine 405 may run in parallel for a patient with multiple presentations.

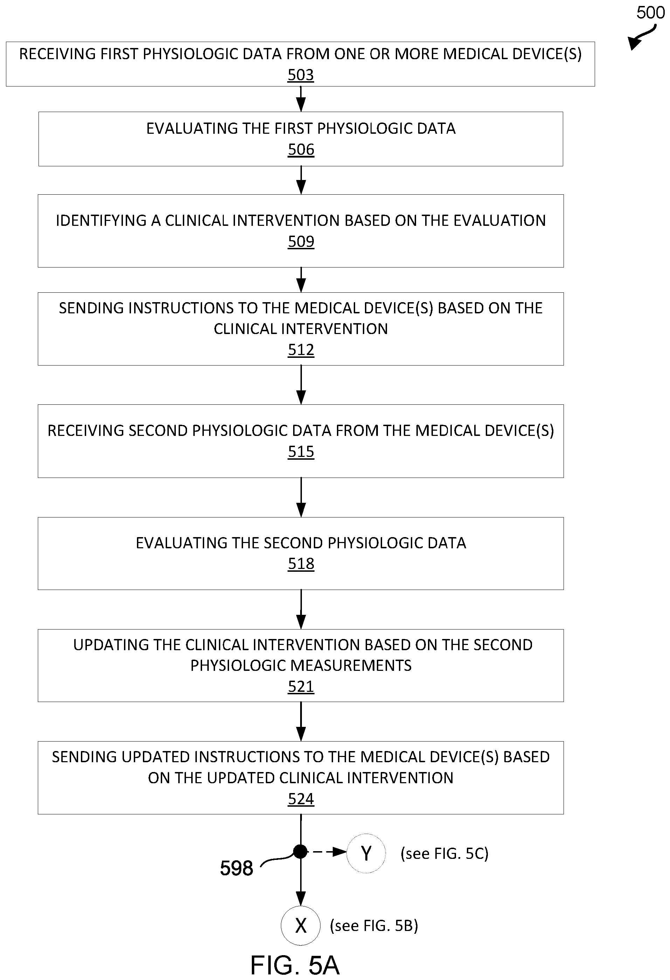

Referring to FIGS. 5A-5C, an example of a method 500 of providing context sensitive guidance is shown. The method 500 is, however, an example only and not limiting. The method 500 can be altered, e.g., by having stages added, removed, rearranged, combined, and/or performed concurrently. The CSG engine 120 may perform the method 500. FIGS. 7-12 exemplify an implementation of the method 500 as applied by the CSG engine 120 to the RD presentation. However, in other exemplary implementations, the CSG engine 120 may apply the method 500 to another patient presentation such as, for example, but not limited to, cardiac presentation, trauma presentation, sepsis presentation, neurologic presentation (e.g. stroke, drug overdose) etc. In these non-RD presentations, the specific physiological parameters, interventions, and medical device(s) may include one or more of the physiological parameters, interventions, and/or medical device(s) referred to in FIGS. 7-12 and/or may include physiological parameters, interventions, and/or medical device(s) that are different from those referred to in FIGS. 7-12.

At the stage 503, the method includes receiving first physiologic data from one or more medical device(s). For example, the CSG engine 120 at the mobile device 105 or the cloud server(s) 398 may receive the first physiologic data from the medical device(s) 180. In an implementation, the caregiver 103 may couple the medical device(s) 180 to the patient or victim 101 via the patient interface devices 170 to initiate patient care. The patient interface devices 170 may collect and/or measure one or more physiologic parameters for the victim 101. The medical device(s) 240 may provide the first physiologic data as first input 240 to the CSG engine 120.

In an implementation, the stage 503 may also include receiving a portion of the first physiologic data from the CSG UI 110. The caregiver 103, 104 may provide user input to the CSG UI 110 that includes physiologic data such as, for example, a caregiver observation and/or patient information measured by the caregiver without use of the medical device(s) 180. The CSG UI 110 may provide the user input as second input 230 to the CSG engine 120.

At the stage 506, the method includes evaluating the first physiologic data. For example, the CSG engine 120 at the mobile device 105 or the cloud server(s) 398 may evaluate the first physiologic data. In an implementation, the CSG engine 120 may evaluate all or a portion of the first physiologic data and may focus the evaluation on one or more physiologic parameters that determine an initial intervention. The initial intervention may be a same intervention regardless of the etiology of the patient's presentation. For example, physiologic parameters that indicate abnormal oxygen levels, cardiac arrhythmia, or significant blood loss indicate initial life-saving interventions. In the case of abnormal oxygen levels, the etiology could be, for example, exposure to a gas leak or asthma. Although these are different etiologies that may require different treatments later in the care of the patient, the initial intervention of supplemental oxygen may be the same.

In various implementations, the CSG engine 120 may evaluate the first physiologic data according one of a variety of analyses. For example, evaluation of the first physiologic data may include comparison of one or more physiologic parameters to a target range, a target minimum value, and/or a target maximum value for the parameter. Each parameter may correspond to a respective target range, minimum value, and/or maximum value. The CSG engine 120 may flag any of the one or more parameters that are outside of a target range, below a minimum value, and/or above a maximum value.

At the stage 509, the method includes identifying a clinical intervention based on the evaluation. For example, the CSG engine 120 may identify the clinical intervention.

The CSG engine 120 may determine or select at least one clinical intervention based at least in part on the received physiologic data and the evaluation of that data. In some implementations, the CSG engine 120 may receive and evaluate medical history data and/or data entered by the user at the CSG UI 110 based on caregiver observations and evaluations. The CSG engine 120 may select the at least one clinical intervention from a range of interventions according to a protocol (e.g., clinical practice guidelines and/or standards of care) that may include, for example, among others: pharmacologic, supplemental O2, mechanical respiratory or ventilation support, additional monitoring, or logistical instructions relative to the management of the patient. The CSG engine 120 may include one or more pre-determined clinical interventions. Each clinical intervention may correspond to particular values of one or more physiologic parameters. The CSG engine 120 may access protocols (e.g., the clinical practice guidelines and/or standards of care) stored, for example, in the memory 109 and/or the memory 309. These protocols may be pre-determined by a user of the CSG system and/or by an administrator or medical director of an emergency agency or other care provider service. In an implementation, the protocols may be customizable by the user, administrator, and/or the medical director. In an implementation, the CSG engine 120 may allow customization of the protocol at the time of care. In an implementation, remote caregiver 303 may select, provide, create, and/or customize or otherwise edit the pre-determined protocols.

At the stage 512, the method includes sending instructions to the medical device(s) based on the clinical intervention. The instructions may include implementation instructions for the clinical intervention and/or event marker(s) for the clinical intervention. For example, the CSG engine 120 at the mobile device 105 or the cloud server(s) 398 may send the instructions to the medical device(s) 180 as first output 260.

The implementation instructions may include control signals that cause the medical device(s) 180 to initiate or implement a particular intervention and/or to set intervention delivery or operational parameters for the medical device(s) 180. In an implementation, the medical device(s) 180 and/or the CSG UI 110 may request a user confirmation. The medical device(s) may initiate or implement a particular intervention and/or change or set delivery or operational parameters in response to receiving the user confirmation.

The instructions may include instructions for the medical device(s) 180 to record one or more event markers relevant to the evaluation of the first physiologic data and/or to the identified clinical intervention. In an implementation, the instructions may include the event marker. The medical device(s) 180 may record the one or more event markers in response to receiving the event markers and/or the event marker instructions from the CSG engine 120. The medical device(s) 180 may record the one or more event markers in the patient encounter data file(s) 188 in the memory 187. In an implementation, the medical device(s) may transmit the patient encounter data file(s) 188 to the cloud server(s) 398 during or after the patient encounter for storage as the medical device data files 395 in the memory 309. Alternatively, in an implementation, the medical device(s) 180 may transmit the one or more event markers to the cloud server(s) 398 for recordation in the medical device data files 395.

In an implementation, the method may include sending instructions to the CSG UI 110 as second output 225. The instructions sent to the CSG UI 110 may include a prompt for the caregiver to perform a particular intervention or other caregiving activity, a prompt for the caregiver to provide a confirmation or other information prior to the intervention, a prompt for the caregiver to adjust or set particular parameters at the medical device(s) 180, a prompt for the caregiver to couple or de-couple a particular patient interface device 170 to or from the patient, an alarm for the caregiver, and/or a message that informs the caregiver of actions to be taken or already taken by the medical device(s) 180. The instructions sent to the CSG UI 110 may also include coaching or other instructional feedback to ensure proper performance of a caregiving task by the caregiver.

Sending instructions to the CSG UI 110 may include sending instructions to one or more mobile devices. For example, in an implementation, the CSG engine 120 may send instructions and/or other second output 225 to one or more mobile devices 105 at the scene of the patient. As another example, in an implementation, the CSG engine 120 may send instructions and/or other second output 225 to one or more mobile devices 105a at the patient scene and to one or more mobile devices 105b located remotely from the patient scene. In an implementation, a remote caregiver 303 may view the CSG interface 110 at the remote mobile device 105b. The remote caregiver 303 may provide user input to the CSG interface 110 at the remote mobile device 105b and the remote mobile device 105b may transmit this user input to the mobile device 105a, the medical device(s) 180, and/or the cloud server(s) 398 via the network 380.

At the stage 515, the method includes receiving second physiologic data from the medical device(s). For example, the CSG engine 120 at the mobile device 105 or the cloud server(s) 398 may receive the second physiologic data from the medical device(s) 180. The second physiologic data may be the same type of data as the first physiologic data, a different type of data, or a combination thereof.

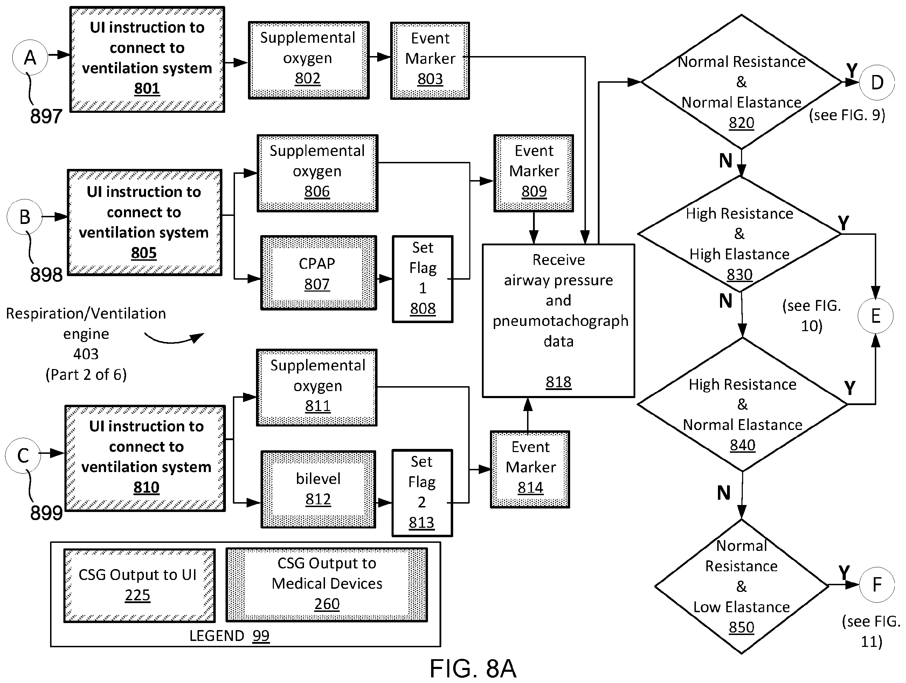

For example, the first physiologic data may include a pulse oximetry (SpO2) measurement or waveform and an end-tidal carbon dioxide (EtCO2) measurement or waveform, as shown in the example discussed in FIG. 7. The second physiologic data may include lung mechanics data (e.g., resistance and elastance), as shown in the example discussed in FIG. 8A, which is a different type of data than the first physiologic data. Alternatively, the second physiologic data may include updated measurements of the same type of data as the first physiologic data, for example, updated SpO2 and/or EtCO2 data. As a further alternative, the second physiologic data may include updated measurement of the first physiologic data combined with a different type of data. For example, the second physiologic data may include updated SpO2 and/or EtCO2 data combined with lung mechanics data (e.g., resistance and/or elastance data).

As another example, the first physiologic data may include blood pressure and heart rate data. The second physiologic data may include updated blood pressure and heart rate data. Alternatively, the second physiologic data may include a new type of data, for example, ECG data. As a further alternative, the second physiologic data may include a combination of updated blood pressure and/or updated heart rate data with the new type data, for example, the ECG data.

As discussed above, the instructions to the CSG UI 110 may include an instruction for the caregiver to couple or de-couple a particular patient interface device 170 to or from the patient. In an implementation, the patient interface device 170 that the caregiver couples to the patient in response to this instruction may be the source of the second physiologic data. For example, a caregiver may initially couple the patient to the patient monitor/defibrillator 285 as the source of the first physiologic data (e.g., the SpO2 and the EtCO2 data) and subsequently couple the patient to the ventilation system 280 as the source of the second physiologic data (e.g., the lung mechanics data).

In another example, the CSG UI 110 may include an instruction for the caregiver to couple an electroencephalograph (EEG) patient interface device 170 to the patient that is the source of the second physiologic data. In another example, the EEG may be configured to measure so-called depth of anesthesia via such methods as Bi-Spectral (BIS) index that may be used to distinguish between drug overdose and stroke. If the CSG engine determines that the more likely cause of the neurologic problem is drug overdose, the recommended therapeutic intervention will be delivering a naloxone dose.

At the stage 518, the method includes evaluating the second physiologic data. For example, the CSG engine 120 may evaluate the second physiologic data. In various implementations, the CSG engine 120 may evaluate the second physiologic data according one of the variety of analyses described above with regard to the first physiologic data.

At the stage 521, the method includes updating the clinical intervention based on the second physiologic measurements. For example, the CSG engine 120 may update the clinical intervention. The clinical intervention identified at the stage 509 may be a first clinical intervention and the updated clinical intervention from the stage 521 may be a second clinical intervention.

Updating the clinical intervention may include maintaining the first clinical intervention in progress from the stage 512. In this case, the update is a validation of the ongoing clinical intervention. For example, if the first clinical intervention at the stage 512 was a high flow nasal cannula (HFNC) at a particular flow rate and particular FiO2 (fraction of inspired oxygen), the update at the stage 521 may validate and continue such that the second intervention at the stage 521 is also HFNC at the same flow rate and FiO2.

Alternatively, updating the clinical intervention may include continuing to provide the same type of clinical intervention but modifying the parameters of that clinical intervention. For example, if the first clinical intervention at the stage 512 was a high flow nasal cannula (HFNC) at a particular flow rate and particular FiO2 (fraction of inspired oxygen), the update at the stage 521 may modify the first interventions such that the second intervention at the stage 521 is also HFNC but at a different flow rate and/or FiO2.

As a further alternative, updating the clinical intervention may include supplementing the first clinical intervention with the second intervention. For example, if the first clinical intervention at the stage 512 was a high flow nasal cannula (HFNC) at a particular flow rate and particular FiO2 (fraction of inspired oxygen), the update at the stage 521 may supplement the first intervention such that the second intervention is a combination of the first intervention (either maintained or modified) and one or more additional interventions. For example, the additional intervention may be CPAP provided with the HFNC or a pharmacological intervention provided with the HFNC or a combination of CPAP and a pharmacological intervention provided with the HFNC.

As yet another alternative, updating the clinical intervention may include replacing the first clinical intervention with one or more second interventions. For example, at the stage 521 the system may terminate HFNC and replace with CPAP.

As a further alternative, updating the clinical intervention may include initially implementing one or more interventions. For example, based on the first physiologic data, the caregiver and/or the medical device(s) 180 may not provide any intervention at the stage 509. However, based on the second physiologic data the caregiver and/or the medical device(s) 180 may provide one or more interventions as the initial interventions applied to the patient. Thus, the update in this scenario refers to a change from no interventions to one or more interventions.

The CSG engine 120 may update the at least one clinical intervention based on a range of intervention types and parameters according to a protocol. The CSG engine 120 may update the at least one clinical intervention according to implementations substantially similar to those described above with regard to the stage 509 (e.g., the identification of the clinical intervention based on the first physiologic data).

At the stage 524, the method includes sending updated instructions to the medical device(s) based on and indicative of the updated clinical intervention. The updated instructions may include instructions for the medical device(s) 180 to implement the clinical intervention and/or instructions to record event marker(s) for the clinical intervention. For example, the CSG engine 120 at the mobile device 105 or the cloud server(s) 398 may send the updated instructions to the medical device(s) 180.

In an implementation, the stages 515, 518, and 521 may occur substantially concurrently or in rapid succession to the stages 503, 506 and 509. If the medical device and sensors providing the first and second physiologic data are the same, the first clinical intervention may occur as rapid response by the caregiver to the patient presentation in initiating the intervention and the second clinical intervention may fine tune that response based on data collection by the medical devices and sensors. However, if the caregiver has to connect the patient to initial sensors to receive the first physiologic data and then additional sensors to receive the second physiologic data, the temporal proximity of these stages may depend on the speed at which the caregiver can connect the various sensors. Furthermore, the first physiologic data is not limited to a single type of data but may be one or more types of physiologic data collected based on the caregiver's initial impression. For example, as discussed below, if a patient exhibits respiratory distress (RD), the first physiologic data may include both SpO2 and EtCO2 data to evaluate the RD. The first physiologic data may also include heart rate, blood pressure, and other vital signs used to initially characterize or triage the patient condition. Similarly, the second physiologic data is not limited to a single type of data. This second physiologic data may be the same type or types of data as the first physiologic data but corresponding to a later time. Thus, the second physiologic data may reflect changes in the patient's condition in response to the first intervention according to the same metrics. Alternatively, the second physiologic data may add new types of data by the addition of sensors which may not only reflect changes in the patient's condition in response to the first intervention but may also refine the characterization of the patient's condition by adding additional metrics. For example, as discussed below, the first physiologic data may include the respiratory gas analysis (e.g., SpO2 and EtCO2) and then the second physiologic data may include lung mechanics data (e.g., lung elastance and resistance). The lung mechanics data may enable the caregiver and the medical devices to refine the intervention based on the more detailed understanding of the patient condition afforded by the addition of the second physiologic data.

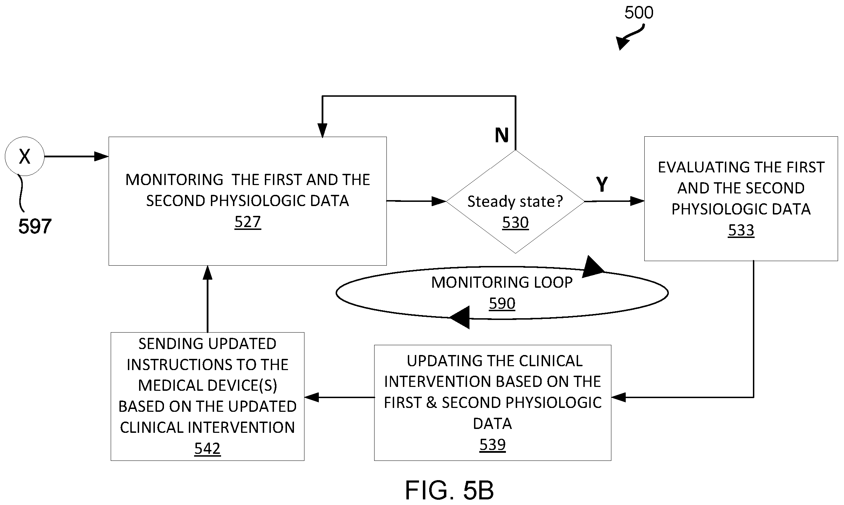

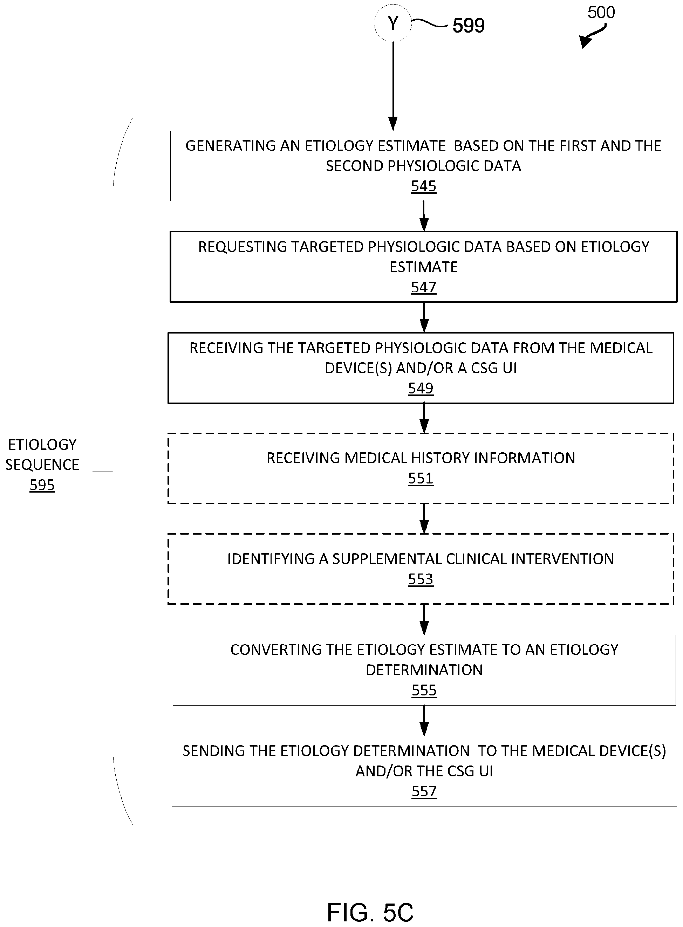

At the stage 524, the method 500 includes a node 598. At the node 598, the method follows the "X" connector to enter a monitoring loop 590 as shown in FIG. 5B. Additionally, at the node 598, the method 500 may optionally follow the "Y" connector to continue to the etiology sequence 595 shown in FIG. 5C. The monitoring loop 590 runs concurrently with the etiology sequence 595.

As shown in FIG. 5B, the method 500 enters the monitoring loop 590 at the X connection point 597. At this point in the method 500, the patient is connected to the medical devices via the patient interface devices necessary to provide the first and second physiologic data. For example, the patient may be connected to the patient monitor/defibrillator 285 via the SpO2 sensor 270 and the EtCO2 sensor 271 for the first physiologic data. Furthermore, the patient may also be connected to the ventilation system 280 via the airway pressure sensor 274 and the pneumotachometer 275 for the second physiologic data. The patient monitor/defibrillator may also be connected to the patient via the electrodes 273 to monitor heart rate, the NIBP sensor 272 to measure blood pressure, and/or one or more of the other patient interface devices 170 as determined by the patient presentation. Thus, at the stage 527, the CSG engine 120 is monitoring the data acquired by the medical device(s) 180 (e.g., the first physiologic data and the second physiologic data) from the patient via the patient interface devices 170 attached to the patient. Further, at the stage 527, the first and second physiologic data is substantially continuously provided to the CSG engine 120 (e.g., non-invasive blood pressure (NIBP) measurements are typically taken every 5 minutes, heart rate is typically recalculated every QRS and updated approximately every one to two seconds, heart rate is calculated over an average of 2-6 QRS intervals, pulse oximetry measurements are updated approximately every one to two seconds, capnography is updated upon every detected breath, etc.).

Once the patient is connected to the medical device(s) 180, the patient remains connected and the CSG engine 120 monitors the data, for example, according to the monitoring loop 590, until the patient's presenting condition is resolved and/or until the care of the patient changes hands (e.g., EMS arrives at a hospital and transfers the patient to the care of the hospital, the patient leaves the ICU, the patient is discharged from the hospital, a caregiver determines that an emergent condition is resolved and/or diagnosed, the patient dies, etc.).

The data monitoring at the stage 527 occurs after the implementation and update of the clinical intervention by the medical devices in response to instructions at the stages 512 and 524 in FIG. 5A. At least part of the purpose of the data monitoring at the stage 527 is to enable an evaluation at the stage 533 of the effect of previously applied interventions on the patient's physiologic systems. Additionally, the purpose of the data monitoring at the stage 527 is to enable an evaluation of a patient status at the stage 533 in the absence of any intervention changes or supplements to determine if a change or supplement is required to remedy a condition identified based on the evaluation.

In order to properly evaluate the monitored data at the stage 533, the monitoring stage 527 and the evaluating stage 533 are separated by a steady state inquiry 530. This steady state inquiry 530 accounts for possible lags between the application of an intervention and the manifestation of that intervention in the physiologic data. More specifically, if the caregivers and medical device(s) 180 apply the intervention to the patient at a time T₁, the manifestation this intervention in the monitored physiologic data may not be instantaneous and may appear at a time T₂ where (T₂-T₁) is approximately equal to the aforementioned lag, also referred to as a steady state delay. The duration of this steady state delay for any particular intervention and sensor data combination depends on a number of factors including, but not limited to, the type of intervention, the type of sensor used to collect data indicative of the effects of the intervention on the patient, the location of the sensor on the patient's body relative to the target location of the intervention, the type of physiological response to the intervention, the number and types of co-occurring physiological conditions and/or comorbidities in the patient that impact the efficacy of the intervention, the number and types of co-occurring interventions that impact the efficacy of the intervention, and the electronic connections between the sensor, medical device(s), and user interfaces.

For example, the CSG engine 120 may provide instructions for the clinical intervention of HFNC in response to a data evaluation of SpO2 indicative of hypoxia. The CSG engine 120 may monitor the SpO2 signal at the stage 527 and evaluate this signal at the stage 533. However, the impact of the HFNC on the SpO2 signal may be associated with a steady state delay between the time the HFNC is applied to the patient and the time the monitored SpO2 signal indicates the effects of the HFNC on the patient's lungs. This delay may be differ in duration between a finger oximetry probe and an earlobe oximetry probe. Further, the delay may depend on other factors affecting the oxygen saturation of the blood and the blood circulation for the particular patient (e.g., anesthesia, ambulation, blood pressure, weight, cardiac conditions, medications, etc.). The CSG engine 120 may apply an empirically derived steady state delay corresponding to an average lag time or an average lag time plus a predetermined number of standard deviations between the stages 527 and 533. For example, the duration of the steady state delay may be a value between 10-40 seconds or in some cases a value between 30-300 seconds. The CSG system may apply this delay any time data is monitored and evaluated after the application of an intervention. Thus, at the stage 530, the CSG engine 120 continues to monitor the physiologic data and waits for the duration of the pre-determined steady state delay until proceeding to the evaluation stage 533. If the system did not apply this steady state delay, then the system may adversely affect patient care by unnecessarily and/or incorrectly changing an intervention before evaluating data accurately indicative of the effect of the intervention.

At the stage 533, the CSG engine 120 evaluates the monitored first and second physiologic data in a manner similar to that described at the stages 506 and 518 as described above. Based on this evaluation, the CSG engine 120 updates the clinical intervention at the stage 539. As similarly described for the stage 521, updating the clinical intervention may include maintaining an ongoing intervention, modifying parameters of the ongoing intervention, supplementing the ongoing intervention, or replacing the ongoing intervention with one or more other and different interventions.

At the stage 536, the CSG engine 120 provides updated instructions to the medical device(s) based on the updated clinical intervention as similarly described for the stage 524. The CSG engine 120 then returns to the stage 527 to continue monitoring the physiologic data.

Optionally, in parallel with the monitoring loop 590, the method 500 may implement the etiology sequence 595 after the stage 524 and as shown in FIG. 5C. The method 500 may enter this sequence at the "Y" connection point 599 and proceed to the stage 555.

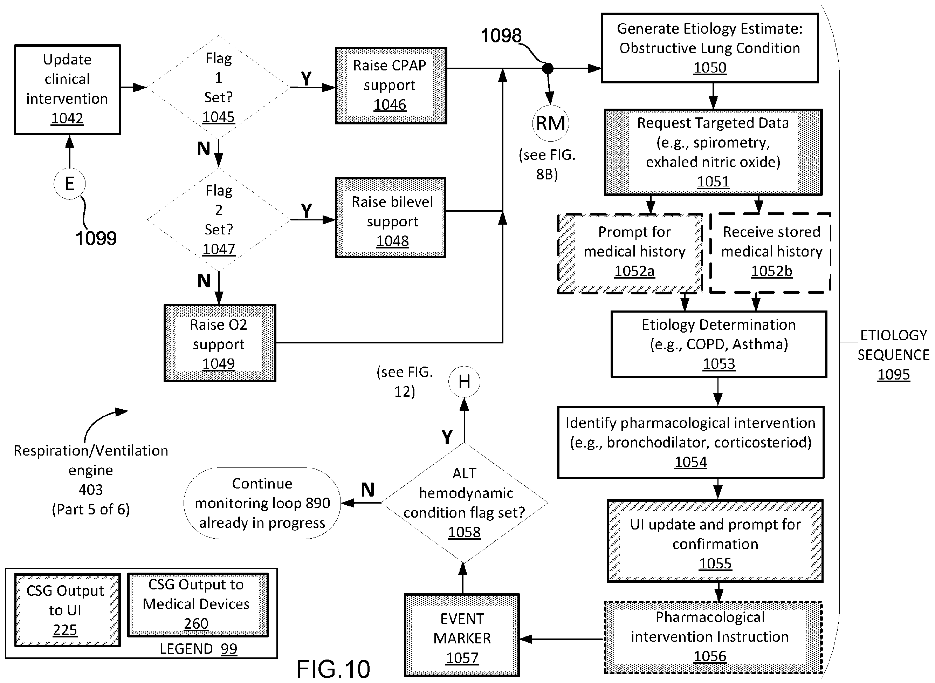

At the stage 555, the method includes generating an etiology estimate based on the first and the second physiologic data. For example, the CSG engine 120 may generate the etiology estimate. Based on the first and second physiologic data, the CSG engine 120 may initiate an etiology distinction engine with an etiology estimate. The etiology estimate indicates a broad etiology category clinically indicated by the first and second physiologic data and from which the etiology distinction engine may convert the etiology estimate to an etiology determination. For example, the first and second physiologic data for RD may indicate an etiology estimate of an obstructive lung condition, as discussed further with regard to FIG. 10. However, the obstructive lung condition is an estimate rather than a determination because the obstructive lung condition may originate from a variety of different underlying conditions such as, for example, chronic obstructive pulmonary disease (COPD) and asthma. In order to distinguish between these two etiologies, the CSG engine 120 may require additional information beyond the first and second physiologic data. However, the first and second physiologic data enable the CSG engine 120 to guide, monitor, and control interventions that provide therapy, potentially life-saving therapy, to the patient while the diagnostic process is ongoing. The benefit of the CSG engine 120 is that the interventions provided are context sensitive as they are tailored to the particular physiological context defined by at least the first and second physiologic data and the interaction between the CSG engine 120 and the medical device(s) 180.

At the stage 560, the method includes requesting targeted physiologic data based on the etiology estimate. For example, the CSG engine 120 may request the targeted physiologic data from the medical device(s) 180. The CSG engine 120 may identify and the CSG engine 120 may request one or more items of physiologic data that may differentiate between various etiology determinations within the scope of the broader etiology estimate. For example, an etiology estimate of an obstructive lung condition is indicative of both COPD and asthma. However, spirometry data and/or exhaled nitric oxide data may differentiate between COPD and asthma. These one or more items of physiologic data may individually differentiate between etiologies and/or may differentiate in the aggregate.

The CSG engine 120 may send the request for the identified targeted physiologic data to the medical device(s) 180 as first output 260 and/or may send the request to the mobile device 105 as second output 225 to the CSG UI 110.

The request to the medical device(s) 180 may include an instruction to send the data or may include an instruction to collect and send the data. In an implementation, the medical device(s) 180 may collect the targeted physiologic data in response to the request from the CSG engine 120. Thus, the request may trigger a collection the targeted physiologic data by the medical device(s) 180. Alternatively, the medical device(s) 180 may extract the targeted physiologic data from the patient encounter data file 188 stored at the medical device(s) 180. The extracted and stored data may have been collected by the medical device(s) 180 prior to the request from the CSG engine 120.

The request to the mobile device 105 may generate a prompt at the CSG UI 110 for the caregiver to enter or to collect and enter the targeted physiologic data. Thus the caregiver may change a setting on the medical device(s) 180 and/or the patient interface devices 170 already connected to the patient to collect this data. Alternatively, the caregiver may couple an additional medical device 180 or patient interface device 170 to the patient to collect the requested data. The CSG UI 110 may provide a user entry field to capture the requested data in response to the user entry of this data.

At the stage 549, the method includes receiving the targeted physiologic data from the medical device(s) 180 and/or from the mobile device 105. For example, the CSG engine 120 at the mobile device 105 or the cloud server(s) 398 may receive the targeted physiologic data from the medical device(s) 180 and/or from the CSG UI 110 at the mobile device 105. The medical device(s) 180 and/or the mobile device 105 may provide the targeted physiologic data automatically in response to the request from the CSG engine 120 or may request a user confirmation prior to providing the targeted physiologic data.

Optionally, at the stage 551, the method may include receiving medical history information for the patient. Additionally, in some implementations, the method 500 may include receiving medical history information at one or more of the stages 502 and 515. Accordingly, one or more of the stages 509 and 521 may include identifying and/or updating clinical interventions based on the medical history. Similarly, one or more of the stages 545 and/or 555 may include generating and/or converting the etiology estimate based on the medical history. For example, the medical history for the patient may include a chronic condition, such as asthma, COPD, heart disease, or diabetes. Such conditions inform the clinical interventions and may simplify the conversion of the etiology estimate to an etiology determination. As another example, the medical history may include medications or diseases, such as communicable blood borne diseases, that may also inform the clinical interventions and/or simplify the conversion of the etiology estimate to the etiology determination. In both of these examples, the medical history may provide information that contraindicates one or more clinical interventions or that indicates a need or potential need for additional clinical interventions beyond those indicated by the physiologic information gathered by the medical device(s).

For example, the CSG UI 110 may capture user input medical history for the patient. The CSG UI 110 may prompt the user and/or provide selectable input fields for the user to provide medical history. The user may obtain medical history from the patient and/or from an associate of the patient. For example, the user may interview the patient and/or the patient's associate to obtain medical history. The user or caregiver may also determine medical history by observation and/or evaluation of the patient's condition. For example, the user or caregiver may obtain information from a medic alert bracelet, from medication containers in the vicinity of the patient, from the scene of the patient's emergency (e.g., a chemical spill, a car accident, illegal drug paraphernalia, medical therapy equipment such as an oxygen tank or a wearable defibrillator, etc.). The user or caregiver may also observe symptoms of a chronic or historic condition by physical examination of the patient. The mobile device 105 may provide information provided at the CSG UI 110 as second input 230 from the UI to the CSG engine 120. The mobile device providing the information may be on scene with the patient (e.g., the mobile device 105a) and/or may be the remote mobile device 105b at the remote location 389. A remote caregiver 303 may have knowledge of and/or access to patient records and may provide medical history as input to the CSG UI 110 at the remote mobile device 105b.

As another example, the CSG engine 120 may receive medical history information from one or more medical records databases 399 via the network 380. The CSG engine 120 may send a query for the medical history information to the medical records databases 399. The CSG engine 120 may generate the query automatically during the process 500 and/or the CSG engine 120 may provide a user prompt and/or request user confirmation at the CSG UI 110 and generate the query in response to the prompt and/or the confirmation.

Optionally, in an implementation, the method 500 may include identifying one or more supplemental clinical interventions at the stage 553 based on the etiology determination and/or the medical history information. The CSG engine 120 may identify one or more clinical interventions, for example pharmacological interventions, specific to the etiology determination. For example, based on an etiology determination of COPD, the CSG engine 120 may identify administration of a bronchodilator as an additional clinical intervention, whereas, based on an etiology determination of asthma, the CSG engine 120 may identify administration of a corticosteroid as an additional clinical intervention.

At the stage 555, the method includes converting the etiology estimate to an etiology determination based on the targeted physiologic data and/or the medical history information. For example, the CSG engine 120 may convert the etiology estimate to the etiology determination. As discussed above, the targeted physiologic data may differentiate between various etiology determinations within the scope of the broader etiology estimate. Thus, in continuation of the example provided above with regard to the stage 560, spirometry data showing a reduced forced vital capacity (FVC) may indicate COPD whereas spirometry data showing nearly normal FVC may indicate asthma. In this manner, the spirometry data may differentiate between two possible etiologies for the obstructive lung condition indicated by the first and second physiologic data. As another example, the swelling of lung tissue associated with asthma may increase a level of exhaled nitric oxide. Thus, this increased level may indicate an etiology determination of asthma on its own or, because such increases may also occur with COPD, such an increase in aggregate with spirometry data showing near normal FVC may support the etiology determination of asthma. In an implementation, a combination of targeted data may enable the conversion of an etiology estimate to an etiology determination. For example, the targeted data may include a combination of spirometry data, s3-s4 heart sounds, and lung fluid retention data as measured by RF-based lung fluid measurement. Together, these data enable a distinction between CHF and COPD.

In implementation, the CSG algorithm may utilize medical history data along with targeted data to convert an etiology estimate to an etiology determination. For example, spirometry data alone may not distinguish between various etiologies for an obstructive lung disease but spirometry along with medical history may provide this distinction. As more specific examples, spirometry combined with a medical history indicating exposure to asbestos may enable an etiology determination of asbestosis or spirometry combined with a medical history indicating coal dust exposure may enable an etiology determination of black lung disease. As another example, a medical history indicating low blood pressure and cardiac disease combined with an etiology estimate inclusive of CHF may enable an etiology determination narrowed to CHF.

At the stage 557, the method includes sending the etiology determination to the medical device(s) and a CSG UI. For example, the mobile device 105 or the cloud server(s) 398 may send the etiology determination to the medical device(s) 180. The mobile device 105 may provide the etiology determination at the CSG UI 110 in response to an instruction from the CSG engine 120. In an implementation, the CSG engine 120 at the cloud server(s) 398 may service the CSG application 315 at the mobile device 105 and provide the etiology determination at the CSG UI 110 at the mobile device 105.

In an implementation, sending the etiology determination to the medical device(s) may include an instruction to record the etiology determination in the medical device data file for the patient encounter and/or may include an instruction to change and/or initiate a clinical intervention based on the etiology determination. For example, if the CSG engine 120 identifies a pharmacological intervention, the instruction may identify the medication along with a dose and administration instructions.

In an implementation, sending the etiology determination to the CSG UI 110 may include a prompt for a user confirmation of the etiology determination and/or an instruction to change and/or initiate a clinical intervention based on the etiology determination.

In an implementation, the CSG algorithm may also invoke non-linear evaluation methods at various stages of the method 500 described below (e.g., at the stages 506, 521, 533, and 536 to evaluate physiologic parameters and update the clinical intervention, at the stage 545 to evaluate physiologic data to generate an etiology estimate, at the stage 549 to identify a supplemental clinical intervention based on targeted data and, optionally, medical history). The non-linear evaluation of multiple physiologic parameters may function as a patient condition estimator where the estimated patient condition corresponds to a particular intervention. For example, an estimated patient condition of hypoxia corresponds to an intervention of delivering supplemental oxygen to the patient.

One example of a patient condition estimator is a Kalman filter. Generally, the Kalman filter models a linear system with Gaussian distribution, which may not be encountered in the physiologic setting. Thus at least one example of the patient condition estimator executes an extended Kalman filter (EKF) to solve the problem of non-Gaussian, nonlinear filtering. The EKF is based upon the principle of linearizing the measurements and evolution models using Taylor series expansions. The series approximations in the EKF process may, however, lead to poor representations of the nonlinear functions and probability distributions of interest. As a result, the EKF may diverge. Therefore, at least one example of the patient condition estimator executes an unscented Kalman filter (UKF) based on the hypothesis that it is easier to approximate a Gaussian distribution than it is to approximate arbitrary nonlinear functions. It has been shown that the UKF leads to more accurate results than the EKF and that it generates much better estimates of the covariance of the states (the EKF often seems to underestimate this quantity). The UKF, however, has a limitation in that it does not apply to general non-Gaussian distributions, as is often the case with ECG spectral distributions.

Another example of the patient condition estimator is a Sequential Monte Carlo process, also known as a particle filter. This example overcomes the non-Gaussian limitations and allows for a complete representation of the posterior distribution of the states, so that any statistical estimates, such as the mean, modes, kurtosis and variance, may be easily computed. Particle filters may, therefore, deal with any nonlinearities or distributions. Particle filters rely on importance sampling and, as a result, require the design of proposal distributions that can approximate the posterior distribution reasonably well. In general, it is hard to design such proposals. The most common strategy is to sample from the probabilistic model of the state evolution (transition prior). This strategy, however, may fail if the new measurements appear in the tail of the prior or if the likelihood is too peaked in comparison to the prior.

Some other example patient condition estimators include an estimator/predictor trajectory tracking technique known as the Unscented Particle Filter (UPF) and other non-linear estimators such as, for example, non-linear regression, neural networks, and convolutional neural networks.

In some embodiments, the CSG algorithm may be based, at least in part, on a Hidden Markov Model (HMM) with the sequence of patient condition and therapeutic intervention vectors as the input. A state transition matrix can be developed using HMM techniques known to those skilled in the art. In particular, the sequence of underlying patient states and recommended therapeutic interventions primitives, and condition and resultant intervention sentences is modeled as a hidden Markov model (HMM), defined as a variant of a finite state machine having a set of states, Q, an output alphabet, O, transition probabilities, A, output probabilities, B, and initial state probabilities, Π. The current state is not observable. Instead, each state produces an output with a certain probability (B). Usually the states, Q, and outputs, O, are understood, so an HMM is said to be a triple, λ = (A, B, Π). Each value of output alphabet, O, can be given a unique threshold and coefficient set. For instance, the HMM will have the transition probabilities stored in it, as a result either of training against a database or patient-specific training, indicating such understood "facts" (i.e. therapeutic intervention grammar). For instance, that it is a very low probability that a patient's blood pressure will decrease by more than 20mmHg if they were just given a dose of a vasopressor: there must been either an intervening the blood pressure reading may be lost in noise, or, alternatively, blood pressure may decrease with sepsis as a more likely estimate of the patient's underlying condition, in which case the optimal therapeutic intervention would be to continue vasopressor but also give broad-spectrum antibiotic for potential septic infection, and perform point of care blood analysis to do a measure of lactic acid.

Other techniques known in the field of HMM classification may be employed. For instance, discriminative training techniques that dispense with a purely statistical approach to HMM parameter estimation and instead optimize some classification-related measure of the training data. Some examples include maximum mutual information (MMI), minimum classification error (MCE) and minimum phone error (MPE) and use of the Viterbi algorithm to find the best path. Other techniques are to keep a set of good candidates instead of just keeping the best candidate, and to use a better scoring function (re scoring) to rate these good candidates. The set of candidates can be kept either as a list (the N-best list approach) or as a subset of the models (a lattice). Re-scoring may be done to minimize the Bayesian risk.

In some embodiments, so-called Deep Learning Networks may be employed. Deep networks may be employed for unsupervised or generative learning to capture high-order correlation of observed or visible data for pattern analysis or synthesis purposes when no information about target class labels is available. Additionally, deep networks may be used for supervised learning to directly provide discriminative power for pattern classification purposes, often by characterizing the posterior distributions of classes conditioned on the visible data. Further, hybrid deep networks may be employed where the goal is discrimination that is assisted with the outcomes of generative or unsupervised deep networks. Other classification methods include, for example, Deep Neural Networks (DNN) and Recurrent Neural Networks (RNN), Convolutional Neural Networks (CNN), Restricted Boltzman Machine (RBM), Deep Belief Network (DBN), Deep Boltzman Machine (DBM), etc.

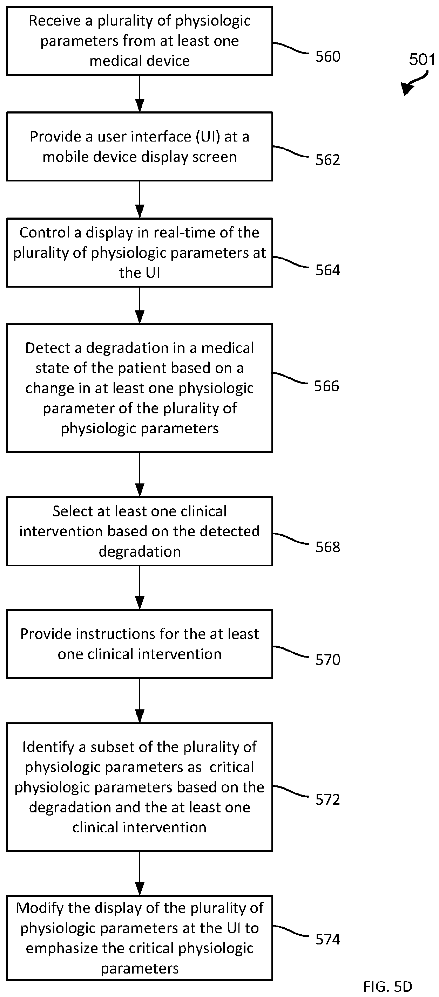

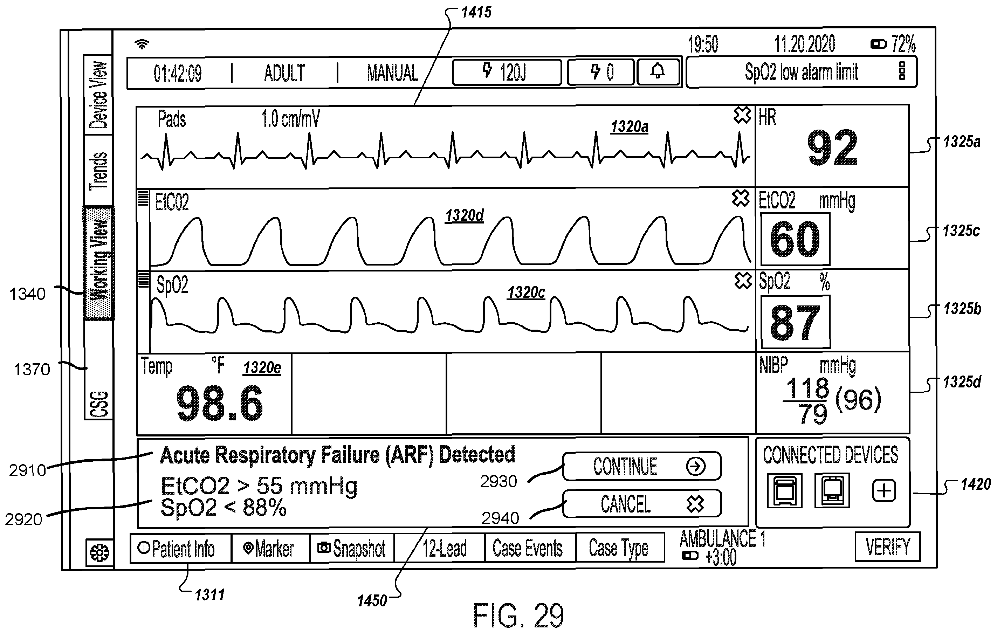

Referring to FIG. 5D, an example of a method of providing context sensitive guidance at a user interface (UI) is shown. The method 501 is, however, an example only and not limiting. The method 501 can be altered, e.g., by having stages added, removed, rearranged, combined, and/or performed concurrently. The CSG engine 120 may perform the method 501 and provide the CSG UI 110. FIGS. 29-43 exemplify an implementation of the method 501 as applied by the CSG engine 120 to the RD presentation. However, in other exemplary implementations, the CSG engine 120 may apply the method 501 to another patient presentation such as, for example, but not limited to, cardiac presentation, trauma presentation, sepsis presentation, neurologic presentation (e.g. stroke, drug overdose) etc. In these non-RD presentations, the specific physiological parameters, interventions, and medical device(s) may include one or more of the physiological parameters, interventions, and/or medical device(s) referred to in FIGS. 29-43 and/or may include physiological parameters, interventions, and/or medical device(s) that are different from those referred to in FIGS. 29-43.

At the stage 560, the method 501 includes receiving a plurality of physiologic parameters from at least one medical device. For example, the CSG engine 120 at the mobile device 105 or the cloud server(s) 398 may receive the plurality of physiologic parameters for the victim 101 from the medical device(s) 180. In an implementation, the stage 560 may also include receiving a portion of the plurality of physiologic parameters via caregiver input to the CSG UI 110. The medical device(s) 180 are configured to couple to the victim 101 via the one or more patient interface devices 170. Additionally, the medical device(s) 180 are configured to communicatively couple to a mobile device 105. The mobile device 105 includes one or more output devices such as the mobile device display screen 106 configured to provide the CSG UI 110.

In an implementation, the medical device(s) 180 may be two or more medical devices. At least two of the two or more medical devices or each of the two or more medical devices may be configured to provide a different clinical intervention and/or monitor different parameters than at least one or all of the other medical devices. For example, there may be two medical devices as shown in FIG. 3E and one may be a patient monitor/defibrillator 285 and the other may be a ventilation system 280. The patient interface devices 180 may include at least a pulse oximeter 270, a nasal cannula 279 or mask 278 coupled to a capnography sensor 271 to provide capnography data 49, a non-invasive blood pressure (NIBP) sensor 272, a temperature probe 267, and electrodes 273. In an implementation, the two medical devices may include a patient monitor without defibrillation capabilities.

At the stage 562, the method 501 includes providing a user interface (UI) at a mobile device. For example, the CSG engine 120 may implement the CSG UI 110 at one or more input/output devices of the mobile device 105 including, for example, a display screen 106 and/or one or more audio and/or haptic devices. The CSG UI 110 may be combined with and/or function as a patient charting interface. The CSG UI 110 may be combined with other data view windows as described below, for example, at least with regard to FIGS. 13B-16 and FIGS. 29-43. Those view formats may include one or more device view windows (e.g., windows 1315 and 1316) that provide all of the data available at the medical device display screen 310 and in a similar format along with a CSG UI window 110 and optionally a trend view window 1515 and a working view window 1415.

At the stage 564, the method 501 includes controlling a display in real-time of the plurality of physiologic parameters at the mobile device user interface. For example, the CSG engine 120 may select which of the plurality of physiologic parameters is provided at the UI 110 in a working view window 1415 and/or a CSG UI window 110 at the mobile device display screen 106. Additionally, the CSG engine 120 may execute the instructions to determine an appearance of the selected physiologic parameters.

At the stage 566, the method 501 includes detecting a degradation in a medical state of the patient based on a change in at least one physiologic parameter of the plurality of physiologic parameters. As a RD example, the plurality of physiologic parameters may include at least EtCO2 and SpO2. In an implementation, the plurality of physiologic parameters may include one or more of ECG, heart rate, body temperature, and non-invasive blood pressure in addition to the EtCO2 and SpO2. The CSG engine 120 may receive these parameters from the medical device 180 and monitor all of these parameters for a change in at least one of these parameters that indicates a degradation of the medical state of the victim 101.

The degradation in the medical state of the victim 101 may be a change in medical state for which a clinical intervention is recommended or required to prevent the change from causing death or other irreversible, or potentially irreversible, damage to the health of the victim 101. Furthermore, the recommended or required clinical intervention may need to be provided within seconds or within 1-3 minutes in order to be efficacious. Detection of this degradation by the CSG system, along with subsequent guidance from this system, may be critical. The CSG system may supplement or replace the need for the caregivers to continuously evaluate the myriad of physiologic parameters provided on a medical device. Furthermore, even if the medical device provides alarms that indicate a potentially dangerous change in a parameter, the medical device still relies on the caregiver to notice and respond to the alarm. In many environments, including those shown in FIGS. 3B-3D, the caregivers are providing care in chaotic, noisy, and crowded environment in which the patient status may be changing rapidly and in which there may be many other factors competing for the caregiver's attention. Furthermore, the skill level of a caregiver at a scene may or may not match the optimum skill level for the required intervention. Thus, a CSG system that can do more than a mere a medical device alarm, for example, by identifying the meaning of the change in patient state and providing guidance may significantly improve the quality and efficacy of care.

In an implementation, the change that indicates degradation of the victim's medical state may be that the value of one or more parameters drop below or rise above a threshold and/or move out of a range. For example, the CSG engine 120 may execute the R/V CSG engine 403 to identify SpO2 > 94% as normal and EtCO2 between 35 mm Hg and 45 mm Hg as normal. If the SpO2 drops to a value between 85% and 94%, this change would represent a degradation of the victim's medical state from normal to hypoxic. If the SpO2 further drops to below 85% then this change would represent a degradation of the victim's medical state from hypoxic to severely hypoxic.

In an implementation, the CSG engine 102 may detect the degradation based on a concurrent changes in multiple parameters. For example, in one case, the patient may be slightly hypoxic, for example with a SpO2 of 93%. If the system detects a normal EtCO2 (e.g., 35 mm Hg < EtCO2 < 45 mm Hg), the system may not identify the change in SpO2 as indicative of a degradation. However, if the SpO2 of 93% occurs concurrently with an abnormally high EtCO2 (e.g., EtCO2 > 45 mm Hg), then the system may detect the degradation in the medical state based on both of these parameters. In an implementation, the CSG engine 102 can evaluate all of the physiological data that it receives all at the same time. The CSG system may detect the degradation based on changes in first parameters that the CSG system predicts are indicative of imminent changes in second parameters.

At the stage, 568, the method 501 includes selecting at least one clinical intervention based on the detected degradation. For example, the CSG engine 120 may select that at least one clinical intervention based on a stored clinical treatment protocol. In an implementation, the stored clinical treatment protocol may include a choice of interventions that depends on what medical equipment is available and/or the skill level of the caregiver. In an implementation, the CSG engine may prioritize the possible interventions based on a standard of care, local medical guidance/procedures, and/or temporal or geographic data that could affect the time that it would take a point-of-care crew to move a patient to a health facility that may provide more definitive or long-term treatments rather than emergency interventions. The CSG engine 120 may identify connected and available medical devices based on a communicative coupling between the mobile device 105 and the connected and available medical devices 180. Additionally or alternatively, the CSG engine 120 may identify available medical equipment based on inventory data provided and/or confirmed by the caregiver.

As one example related to RD, the degradation in the medical state may be that the patient has entered a state of acute respiratory failure (ARF). For example, ARF may be indicated if EtCO2 > 55 mmHg and SpO2 < 88%. The CSG engine 102 may select one or more clinical interventions appropriate for the detected degradation state. For example, as shown below in FIGS. 8A, 9, 10, and 11, the CSG engine 102 may select supplemental oxygen or supplemental oxygen combined with CPAP or bilevel and may additionally select a particular pharmacological intervention. Although the CSG system has selected a clinical intervention, the CSG system will continue to monitor the physiologic parameters for further changes in these parameters. These further changes may indicate an improvement in the patient's medical state, a further degradation of the patient's medical state, a stable patient medical state, and/or the introduction of a new condition. For example, although the physiologic parameters related to RD may stay stable, the CSG system may detect changes in cardiac parameters (e.g., as monitored by a hemodynamic algorithm as shown in FIG. 12 and discussed below) or in trauma parameters (e.g., as monitored by a non-respiratory distress CSG engine 405). Therefore, while the caregiver attends to the RD condition, the system can continue to monitor other physiologic systems and conditions and provide instructions and alerts as needed. This enables the clinician to focus on fewer tasks and data than without the CSG system thereby improving the quality and efficacy of care.

At the stage 570, the method 501 includes providing instructions for the at least one clinical intervention. For example, the CSG engine 120 may provide instructions based on the coupled or inventoried medical equipment as described in conjunction with the stage 568. In an implementation, the CSG engine 120 may provide the instructions for the at least one clinical intervention to one or more medical devices communicatively coupled to the mobile device 105. These instructions may be part of a closed loop control for a medical device. For example, as shown in FIG. 3E, the CSG engine 120 may utilize data from a first medical device, e.g., the patient monitor/defibrillator 285, to implement physiologic closed-loop control of a second medical device, e.g., the ventilation system 280. The CSG engine 120 may generate the physiologic closed-loop control instructions based on an oxygen concentration of a patient's blood as measured by a pulse oximetry probe 270 that is coupled to the patient monitor/defibrillator 285. In this example, the physiologic closed-loop control instructions may adjust an oxygen delivery to the patient during the delivery of a mechanical ventilation of the patient in order to maintain the oxygenation of the patient's blood at a desired level and/or within a desired range.

The term closed loop control, as used herein, may refer to control of one or more ventilation related or patient related parameters, such as with relatively little or no required user action, participation or intervention, and can include reference to, but is not limited to reference to, fully automated or fully automatically regulated control. Closed loop control may include, for example, device facilitated or algorithmically facilitated tracking, control and adjustment of one or more parameters, which may or may not include user involvement or participation. Where user involvement or participation is included, it may include, for example, confirming a suggested or recommended ventilation setting change or configuration, deciding on implementing a course of action, selecting one of several suggested courses of action, responding to a presented alert or alarm, or other decisions, choices or actions. User involvement or participation could also include, for example, setting or changing a parameter, where a closed loop control algorithm proceeds from there, initially according to the user-set or user-changed parameter setting. In various embodiments, if there is user involvement, it may be, for example, among other things, in whole or in part user-initiated, or in whole or in part prompted, suggested, recommended or required.

In some embodiments, closed loop control may be utilized but may be subject to manual adjustment or override by the user. For example, in some embodiments, although FiO2 and PEEP may be algorithmically and automatically controlled, a user may be able to intervene and manually change the FiO2 and/or PEEP setting. In some embodiments, following any manual adjustments, closed loop control of FiO2 and/or PEEP may resume from that point, at least until any further manual adjustments are made.

Various embodiments as described herein may apply to, for example, out-of-hospital or pre-hospital patient care (although not limited to such care). Some aspects of embodiments described herein take into account practical factors relating to such care contexts. For example, in pre-hospital patient care, the care provider may be less trained than a hospital-based care provider. Furthermore, no support or less support from other care providers and/or hospital systems may be available. Also, pre-hospital care may involve less patient and equipment physical stability, and may involve movement or transport. Additionally, pre-hospital patient care may extend for long periods of time, sometimes extending to many hours, a day or several days. During this time, the resulting physical and mental stress, and overall exhaustion, of the patient and the care provider can be significant factors to consider in determining optimal ventilator operation, procedures and algorithms. As such, in some embodiments, these pre-hospital conditions are taken into account, such as in connection with determination of parameter values, ranges and thresholds, frequency of change of parameters, overall simplicity, and balances between parameter stability and the need for adjustment. In some embodiments, such balances and optimization approaches may be thought of as providing or favoring a form of conceptual "guardrails" in view of the particular concerns and elevated potential risks that tend to accompany pre-hospital care contexts and situations.

In some embodiments, overall, optimization in view of pre-hospital care related factors can favor increased simplicity, increased stability and a generally more limited or conservative approach with regard to ventilation parameter value determinations and adjustments. Such approaches may include one or several of the following. Some embodiments may include frequent user checks and confirmations, user warnings, or user alerts. Some embodiments may include determined and displayed context-sensitive guidance provided to the user, which may provide additional user support, given pre-hospital circumstances, in which a user with limited training may be providing care under stressful and distracting circumstances. For example, some embodiments provide optimized approaches including greater overall stability, less frequent changes in parameters, and more or greater change in conditions required for changes in parameter values. Furthermore, some embodiments include use of more conservative parameter values, or more conservative high or low limits, such as with regard to parameters that may present a potentially high degree of patient risk. These may include, for example, PEEP, PIP, driving or plateau pressure, Vt and Ve. For example, aspects that may reflect such optimization may include PEEP change eligibility rules, which can limit and specify conditions required for PEEP changes or potential PEEP changes, and/or can be used in determining a maximum PEEP. Aspects reflecting such optimization may further include PEEP selection rules, which can specify a change in FiO2 required to indicate a PEEP change in view of a current PEEP level. However, in some circumstances, pre-hospital considerations may warrant optimizations or balances in favor of rapid or increased changes, such as, for example, increasing FiO2 to 100% upon detection of a defined patient desaturation condition.

In various embodiments, FiO2 may be continuously adjusted based on measured patient SpO2, which is used as an indicator of patient oxygen saturation. Generally, a lower SpO2, or decreasing SpO2 (as may be determined, for example, based on a single or current SpO2 measurement, or several SpO2 measurements over a recent period of time) may tend to indicate a "sicker" patient, or patient with more impaired lung or respiratory system function, who is in need of more oxygenation support. In some embodiments, generally, when a patient's SpO2 is below a target level (such as a pre-determined SpO2 value), and potentially also based at least in part on how much below, and/or decreasing, FiO2 may algorithmically tend to be increased in order to increase support of a patient's oxygenation. When SpO2 is above the target level, and potentially also based at least in part on how much above, FiO2 may algorithmically tend to be decreased, since the patient may not be in need of as much oxygenation support. As such, FiO2 may be increased or decreased based at least in part on the need of the patient as indicated at least in part by SpO2 and/or, for example, some other indication(s), such as one or more other non-invasively sensed, measured or determined indications of patient oxygen saturation. Algorithmically determined factors, such as derivative gain and proportional gain factors, may affect a direction (increase or decrease) and amount of FiO2 adjustment.

In some embodiments, one or more previously measured SpO2 values may be taken into account algorithmically in determining an FiO2 adjustment. In particular, in some embodiments, proportional gain and derivative gain may take into account one or more previously measured SpO2 values. Generally, in some embodiments, derivative gain may take into account an effective rate of decrease in SpO2 for some period of time up to and including the time of the currently measured SpO2 (such as may include use of a moving average). Derivative gain may tend to increase the FiO2 adjustment when SpO2 is decreasing, such as in a manner that may be associated with, or proportional to, a determined rate of SpO2 decrease. However, in some embodiments, derivative gain may not operate to tend to decrease FiO2 when SpO2 is increasing, and may in this regard be asymmetric in operation. Furthermore, in some embodiments, proportional gain may take into account a determined magnitude of the difference between the current (or current and recent) SpO2 and a target SpO2, where a larger difference may lead to a greater adjustment.

In some embodiments, PEEP is adjusted based at least in part on FiO2, but also based at least in part on the current PEEP. Since FiO2 may be adjusted based at least part on patient SpO2, and since SpO2 may be associated with how "sick," compromised or functionally impaired the patient's lungs or respiratory system are, conceptually, FiO2 may to some degree represent or effectively operate as a surrogate or indicator of how "sick" the patient's lungs or respiratory system are. For example, in some instances, a relatively low and/or decreasing SpO2 may indicate substantial and/or increasing degree of functional lung impairment, and may result in a relatively high FiO2. This high FiO2 may, under appropriate circumstances, warrant and lead to an increase in PEEP (as may be determined with reference to, for example, PEEP change eligibility rules and PEEP selection rules), where PEEP may help essentially open alveoli and support respiratory function. Conversely, in some instances, a relatively high and/or increasing SpO2 may indicate less substantial and/or decreasing lung impairment (i.e., generally, the patient may be breathing "better" on their own), and may lead to a relatively low FiO2, which may, in some instances, warrant and lead to a decrease in PEEP (as may be determined with reference to, for example, PEEP change eligibility rules and PEEP selection rules).

PEEP can support a patient by, for example, increasing alveolar pressure and volume, which can essentially distend and prevent the collapse of alveoli, improving oxygenation. However, too high a PEEP can cause risk to a patient. For example, too high a PEEP can lead to an increased thoracic pressure, which in turn can lead to a decrease in patient blood pressure by inhibiting or otherwise limiting venous blood return, causing low blood pressure related risk to the patient. Also, since it can take some time for an increased PEEP to have full effect on alveoli, the full effect of an increased PEEP may take some time, such as minutes, to fully emerge. That can be a reason to avoid increasing PEEP too rapidly, since there may be a possibility of essentially "overshooting the mark" in terms of PEEP increase rapidity and creating patient risk. Furthermore, overly high or over-frequent changing of the PEEP can create other risks to the patient, such as risk of barotrauma, damaging or rupturing alveoli, pneumothorax, pulmonary interstitial emphysema, pneumomediastimum, fibrogenesis, inflammation or a damaging immune response. As such, while it can be important to increase PEEP where warranted, in can also be important to avoid increasing PEEP too much or too frequently. In some embodiments, algorithms including PEEP change eligibility rules and PEEP selection rules provide an optimized approach to PEEP control, balancing assessed need for PEEP adjustment with need for avoiding too high a PEEP and avoiding over-frequent changes in PEEP. A more conservative or slower approach to decreasing PEEP may be warranted, relative to increasing PEEP, since decreasing PEEP is not undertaken in order to address a need of the patient for increased support.

In some embodiments, various parameters in addition to FiO2 and PEEP may be continuously adjusted or adjusted using closed loop control. These may include, for example, adjustments that may be based at least in part on EtCO2, which may indicate or suggest normocapnia, hypercapnia or hypocapnia. For example, in some embodiments, parameters including Vt, Ve and PIP may be continuously adjusted.

In an implementation, the instructions provided by the CSG engine 120 to the one or more medical devices may be instructions for a mode of operation, an operational setting, an instruction to send data to the mobile device 105, an instruction to request data at the medical device, an instruction to request a caregiver confirmation, an instruction to store data, and/or an instruction to record an event marker in an event data file.

As one example related to RD, the instructions for the medical device and/or the caregiver may include instructions to provide supplemental oxygen or supplemental oxygen combined with one of CPAP or bilevel and/or instructions to maintain or change a previously provided intervention (e.g., maintain, increase, or decrease a level of supplemental oxygen or add CPAP or bilevel) and/or instructions to provide a pharmacologic intervention.

In an implementation, the instructions for the at least one clinical intervention may be instructions provided at the CSG UI 110. These instructions may instruct a caregiver on the use of an item of medical equipment. The item of medical equipment may be in an inventory accessible to and/or created by the CSG engine 120 and/or communicatively coupled to the mobile device 105. In an example, the at least one medical device may be a ventilation system and the instructions may guide the caregiver through assembly and/or operation of the ventilation system.

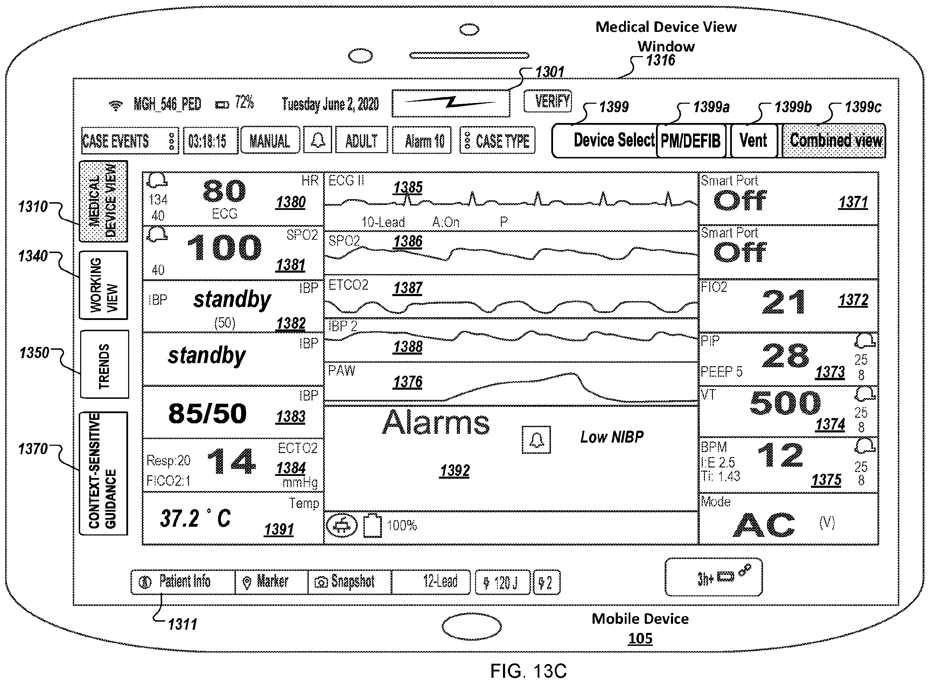

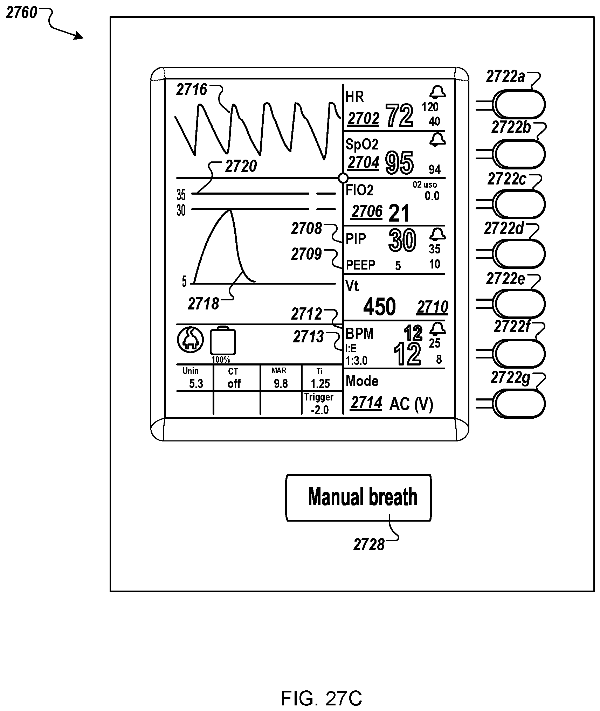

At the stage 572, the method 501 includes identifying a subset of the plurality of physiologic parameters as critical physiologic parameters based on the degradation and the at least one clinical intervention. The CSG engine 120 may identify one or more parameters that are critical to patient survival and/or specifically responsive to the clinical intervention. This identification may be based on a pre-determined designation of critical parameters that correspond to a clinical intervention. Alternatively or additionally, this identification may be based on a machine learning algorithm designed to train a model to predict critical parameters based on the patient's medical state and the applied clinical intervention. In one example, the clinical intervention may be ventilation and the critical parameters may be SpO2 and EtCO2. In general, the modification of the display allows the CSG engine 120 to provide guidance to the caregivers by focusing their attention on these critical parameters without requiring the caregivers to determine, from amongst the many parameters provided during patient care (e.g., the variety of parameters shown in FIG. 13B, 13C, and 27C that are displayed both on a medical device and on the mobile device 105) which of these parameters are most worthy of their attention because these parameters will best represent the prevention of a patient dying and the efficacy of an applied intervention. Such a determination by a caregiver is difficult and prone to error particularly in a dynamically changing and often noisy and chaotic emergency environment where fast actions and decisions are crucial to patient survival. Furthermore, the skill level and clinical experience of an emergency caregiver covers a wide range and guidance from a CSG system enables more uniform and effective care.

At the stage 574, the method 501 includes modifying the display of the plurality of physiologic parameters at the mobile device display screen to emphasize the critical physiologic parameters. For example, the CSG engine 120 may control the mobile device display screen 106 to change one or more graphical elements at the display that emphasize the critical physiologic parameters. Specific examples of these changes are provided below. In general, the modification of the display allows the CSG engine 120 to provide guidance to the caregivers by focusing their attention on these critical parameters free from the distraction of the many other parameters and display features. Additionally, instead of having to watch several medical devices, which may be difficult to access in a crowded environment and which either require a single caregiver to constantly divert attention between devices or require several caregivers assigned to each device without one person seeing the whole picture, the mobile device 105 with a modified display provides a one-stop shop with unambiguously displayed priority parameters. Emphasizing the critical parameters may include, without limitation, reducing the number of displayed parameters, changing display feature color, changing display feature sizes, providing user notification banners, accompanying visual changes with sounds, and combinations thereof.

Referring to FIG. 6, process stages of a CSG initialization algorithm and a RD CSG algorithm are shown schematically. The algorithms 411 and 422, however, are examples only and not limiting. The algorithms 411 and 422 can be altered, e.g., by having stages added, removed, rearranged, combined, and/or performed concurrently. The process stages shown in FIG. 6 illustrate an application of the CSG engine 120 to an RD presentation.

The legend 99, shown in FIG. 6 as well as in FIGS. 7-12, provides a graphical distinction between various outputs from the CSG engine 120. The legend refers back to FIG. 2 which illustrates second output 225 to the UI and first output 260 to the medical device(s) 180. The second output 225 is indicated by a diagonal slash pattern and the first output 260 is indicated by a dot pattern. These two types of outputs manifest themselves in various process stages of the CSG engine 120 as illustrated in FIGS. 6-12. The pattern (i.e., either diagonal slash or dot) indicated at various process stages indicates that the particular stage is a specific instance of either the second output 225 or the first output 260.

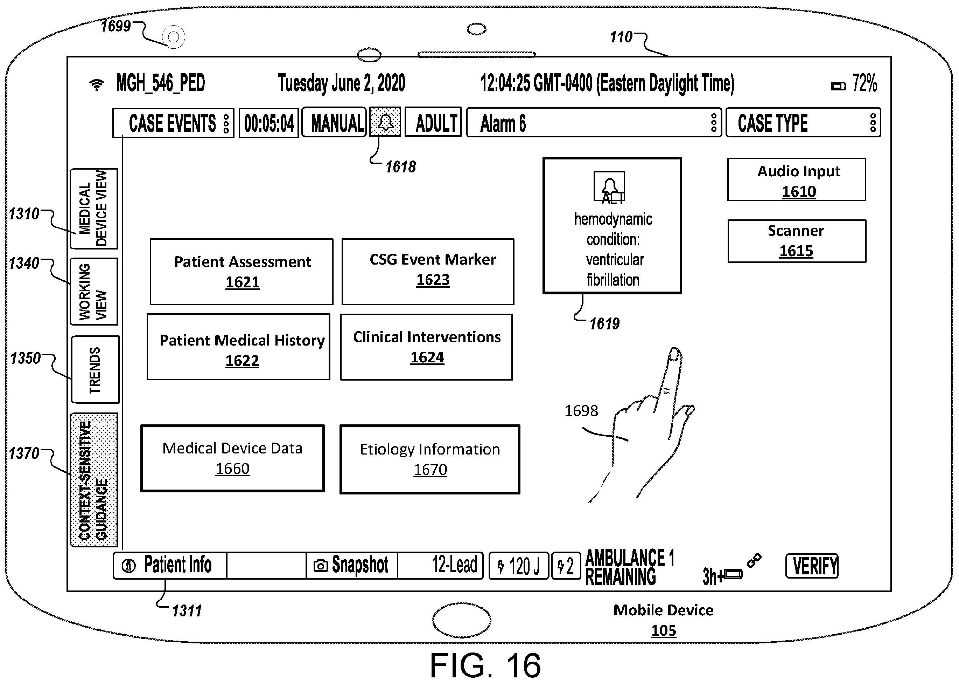

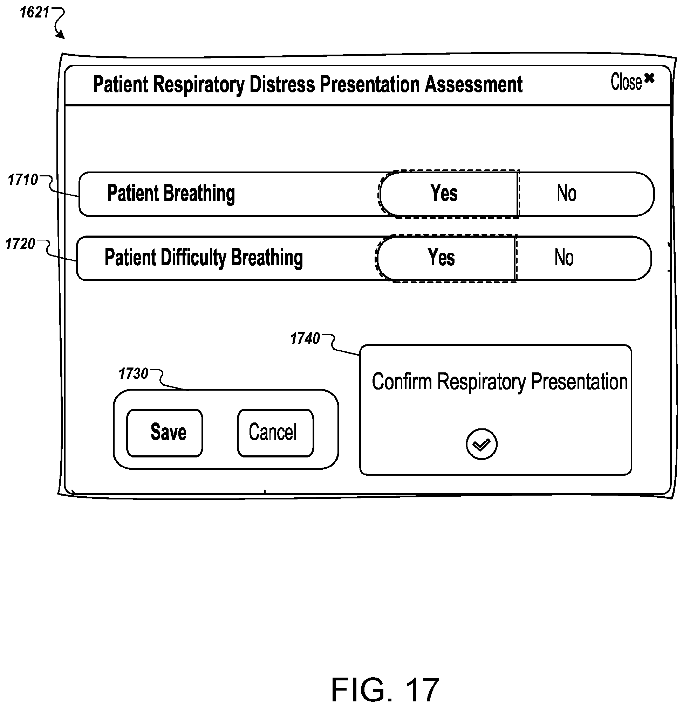

In an implementation, the CSG initialization algorithm 411 executes steps to identify a victim of RD and initiate the RD CSG engine 402. In order to identify that a victim presents with RD, at the stage 610, the CSG initialization algorithm may provide a prompt or reminder at the CSG UI 110 for the caregiver to perform a breathing assessment. As discussed further below with regard to FIGS. 16 and 17, the CSG UI 110 may provide a user entry screen 1621 for the breathing assessment data. At the stage 615, the algorithm 411 decides between an RD presentation or a non-RD presentation based on whether the patient is either not breathing or having difficulty breathing. If either of these conditions are true, the CSG initialization algorithm 411 initiates the RD CSG engine 402. If neither of these conditions are true then there is no clinical manifestation of RD and the CSG initialization algorithm 411 does not initiate the RD CSG engine 402 and may initiate a non-RD CSG engine 405 for another presentation such as trauma, sepsis, drug overdose, etc. For example, in some cases, a person suffering from an asthma attack may be in RD without any other concurrent medical conditions. Likewise, a person suffering from a gunshot wound may present with trauma but without any RD.

In some cases, the CSG initialization algorithm may initiate both the RD CSG engine 402 in parallel with the non-RD CSG engine 405. In these cases, a patient may present with RD concurrently with another condition. For example, a person suffering from a gunshot wound to the chest may present with RD and trauma. In this case, the RD and non-RD presentation algorithms may execute in parallel.

At the stage 620, the RD CSG engine 402 may distinguish between patients that are not breathing and those having difficulty breathing. For the patient that is not breathing, the engine 402 may provide second output 225 to the CSG UI 110 at the stage 625 that includes an instruction at the CSG UI 110 for the caregiver to intubate and manually ventilate the patient. For example, the caregiver 103 may couple the patient 101 to the intubation tube 277.

In an implementation, the CSG UI 110 may provide assistance or guidance relating to endotracheal placement or esophageal placement for the intubation tube 277. For example, the ventilation system 280 may detect and confirm appropriate tube placement, and may prompt, alert or provide caregiver feedback via the CSG UI 110. In implementations, the ventilation system 280 may detect tube leaks, obstructions, and/or dislodgements and provide a user alert via the CSG UI 110. Further, the ventilation system 280 may detect successful ET tube reattachment and provide confirmation at the CSG UI 110.

At the stage 630, the second output 225 may include an instruction or reminder at the CSG UI 110 for the caregiver to connect the patient to the patient monitor/defibrillator 285 via the patient interface devices 170. For example, the caregiver 103 may couple the patient 101 to at least the SpO2 sensor 270 and the EtCO2 sensor 271. For the patient that is breathing, the engine 402 may provide second output 225 to the CSG UI 110 at the stage 630 that includes an instruction at the CSG UI 110 for the caregiver to connect the patient to the patient monitor/defibrillator 285 via the patient interface devices 170. For example, the caregiver 103 may couple the patient 101 to at least the SpO2 sensor 270 and the EtCO2 sensor 271.

At the stage 635, the engine 402 may also provide first output 260 to the medical device(s) to that includes an event marker for either case (i.e., not breathing with intubation and manual ventilation or breathing with connection to the patient monitor/defibrillator). The medical device(s) 180 may store this event marker in a data file for the patient encounter.

Following the generation of the CSG outputs 225 and 260 at the stages 625, 630, and 635, the RD CSG engine 402 initiates both of the R/V CSG engine 403 and the hemodynamic CSG engine 404. As discussed above, these algorithms run in parallel due to the interrelated nature of respiration, ventilation, and hemodynamics as applied to cardiovascular interventions.

Because the algorithms 433 and 444 run in parallel, the UI instruction at the stage 630 may include an instruction or reminder for the caregiver 103 to couple the patient 101 to the NIBP sensor 272 and the electrodes 273. These sensors enable the patient monitor/defibrillator 285 to provide input to the hemodynamic algorithm 444 described in detail below with regard to FIG. 12 and referenced in regard to FIGS. 7-11.

Referring to FIGS. 7-11, process stages of an R/V algorithm are shown schematically. The engine 403, however, is an example only and not limiting. The engine 403 can be altered, e.g., by having stages added, removed, rearranged, combined, and/or performed concurrently. The process stages shown in FIGS. 7-11 illustrate an application of the R/V CSG engine 403 to an RD presentation. The legend 99, shown in FIGS. 7-11, as well as in FIGS. 6 and 12, provides a graphical distinction between the second output 225 to the UI and first output 260 to the medical device(s) 180, as discussed in more detail in regard to FIGS. 2 and 6.

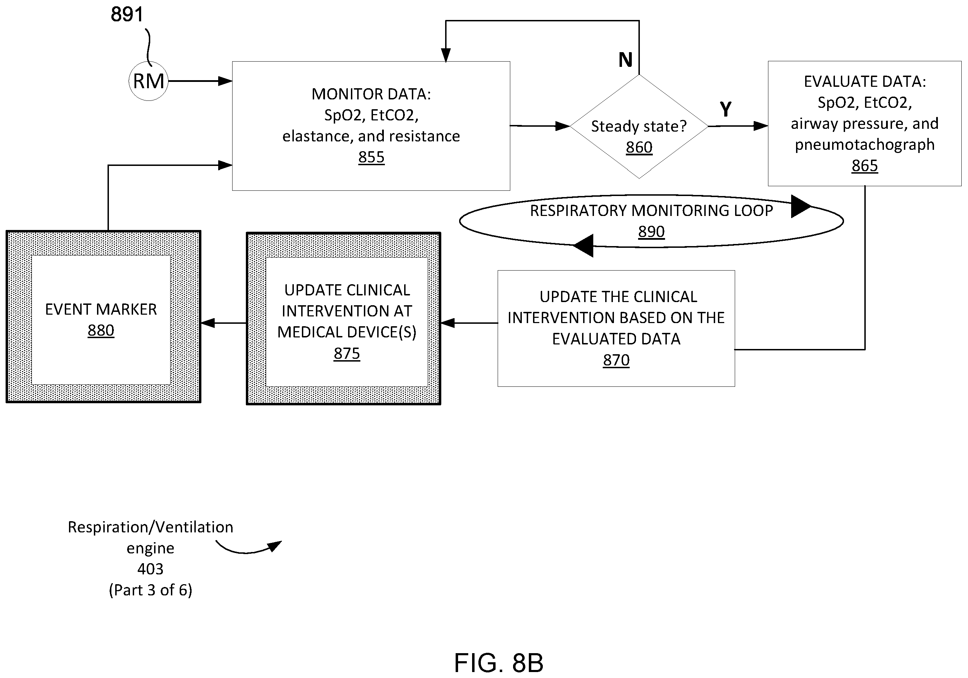

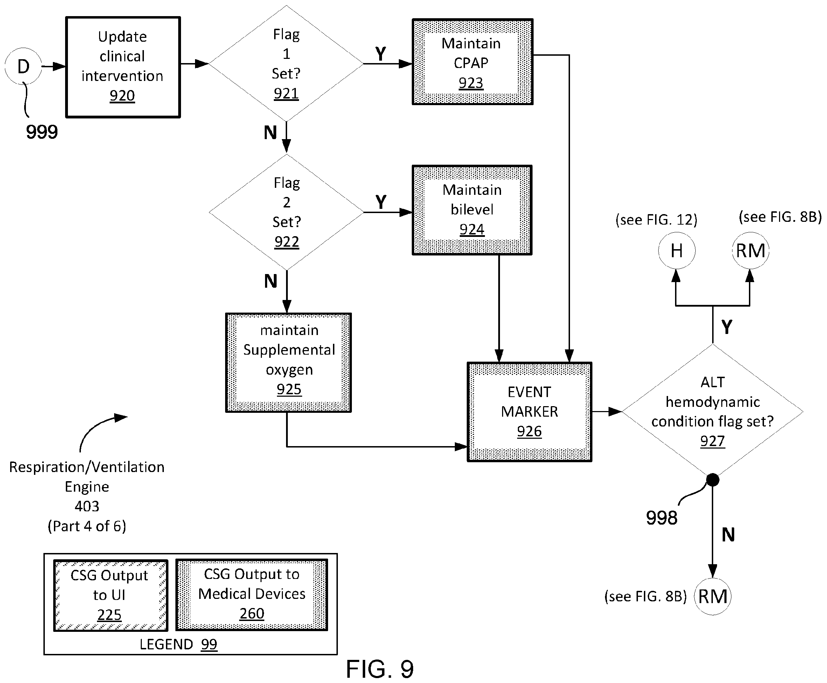

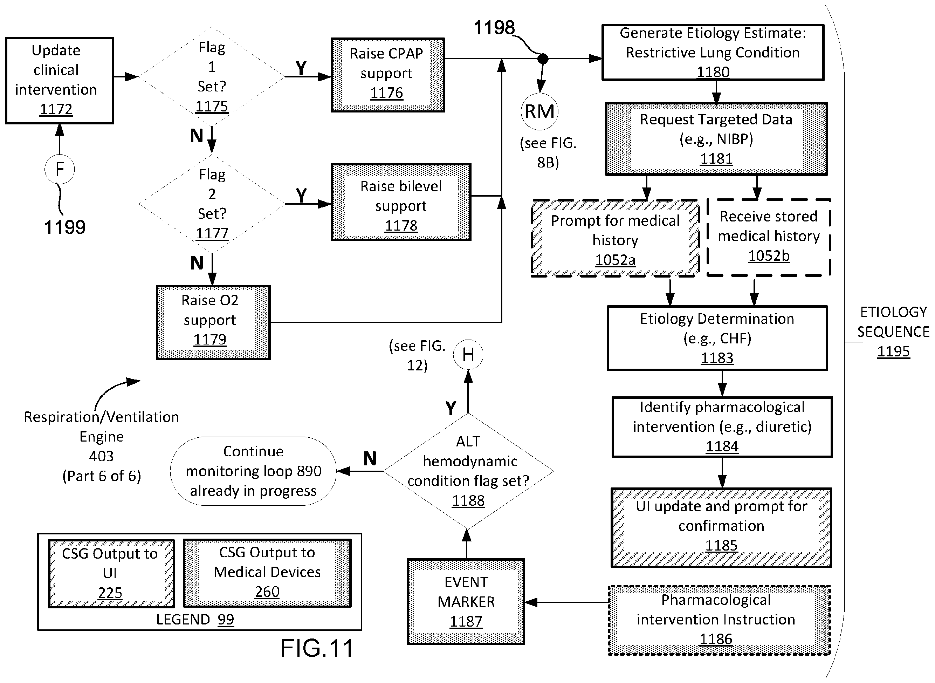

The schematic illustration of the R/V CSG engine 403 begins in FIG. 7. The "start" indicated in FIG. 7 indicates an entry point from FIG. 6 upon initialization of the R/V CSG engine 403 by the algorithm 422. The portion of the R/V CSG engine 403 in FIG. 7 continues to the "A" connection point 897, the "B" connection point 898, or the "C" connection point 899 in FIG. 8A. From FIG. 8A, the R/V CSG engine 403 continues to one of the "D" connection point 999 in FIG. 9, the "E" connection point 1099 in FIG. 10, or the "F" connection point 1199 in FIG. 11. FIGS. 9-11 continue to the "RM" connection point 891 in FIG. 8B from the nodes 998, 1098, and 1198. Additionally, FIGS. 7-11 include links to the "H" connection point 1299 in FIG. 12.

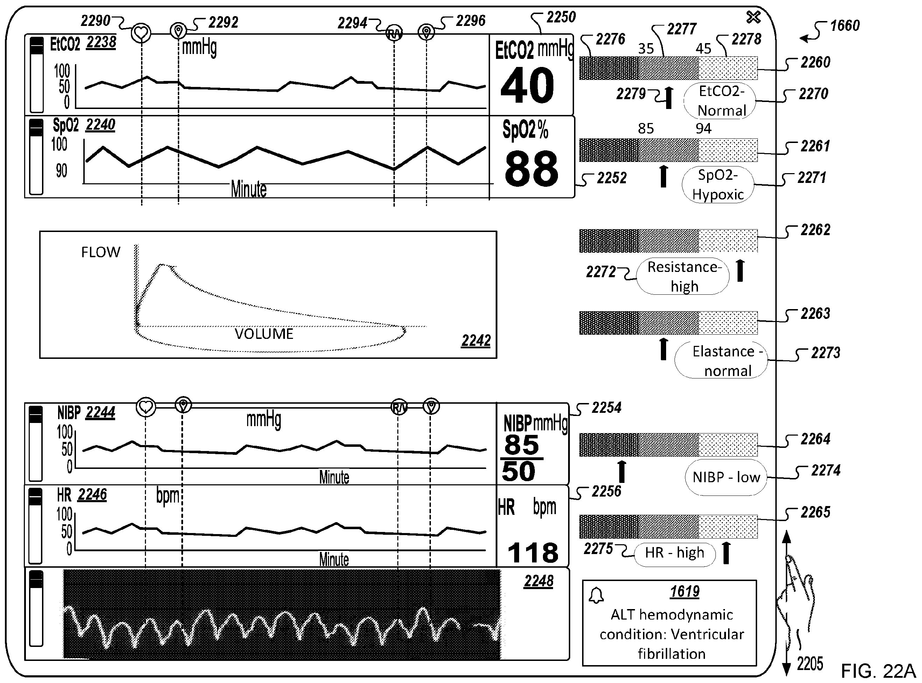

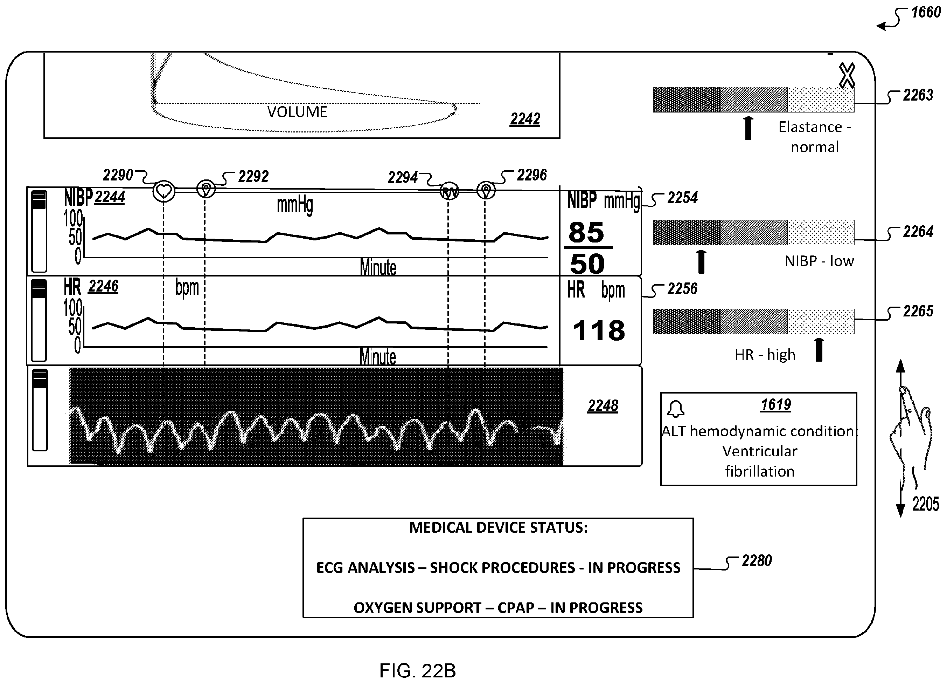

Upon initialization by the algorithm 422 (as shown in FIG. 6), the R/V CSG engine 403 receives respiration data, e.g., SpO2 and EtCO2 data, from the patient monitor/defibrillator 285 at the stage 760. The R/V CSG engine 403 receives this data as first input 240 from the medical device(s) 180. The process stage 760 provides an exemplary implementation of the stage 503 of the method 500 shown in FIG. 5A. In an implementation, as described in more detail with regard to FIGS. 16, 22A, and 22B, the stage 760 includes providing the received data at the CSG UI 110.

Upon receipt, the R/V CSG engine 403 evaluates the respiration data at the stages 710, 720, 730, and 740 for one of four conditions. The process stages 710, 720, 730, and 740 provide an exemplary implementation of the stage 506 of the method 500 shown in FIG. 5A. At the stage 710, the R/V CSG engine 403 identifies normal SpO2 (e.g., SpO2 > 94%) and normal EtCO2 (e.g., 35 mm Hg < EtCO2 < 45 mm Hg). At the stage 720, the R/V CSG engine 403 identifies an SpO2 value that indicates that the patient is hypoxic (e.g., 85% < SpO2 < 94%) and normal EtCO2 (e.g., 35 mm Hg < EtCO2 < 45 mm Hg). At the stage 730, the R/V CSG engine 403 identifies an SpO2 value that indicates that the patient is hypoxic (e.g., 85% < SpO2 < 94%) and an abnormally high EtCO2 (e.g., EtCO2 > 45 mm Hg). At the stage 740, the R/V CSG engine 403 identifies an SpO2 value that indicates that the patient is severely hypoxic (e.g., SpO2 < 85%) and an abnormally high EtCO2 (e.g., EtCO2 > 45 mm Hg).

Following each of the stages 710, 720, 730, and 740, the R/V CSG engine 403 checks the status of an ALT hemodynamic condition flag, e.g., at the stages 712, 722, 732, and 742. At the stages 712, 722, 732, and 742, if the ALT hemodynamic condition flag is set, then the CSG engine 120 proceeds to the hemodynamic algorithm 444 shown in FIG. 12 as indicated by the "H" connection points in FIG. 7. Concurrently with the hemodynamic algorithm 444, the R/V CSG engine 403 continues to monitor the SpO2 and EtCO2 data until the ALT hemodynamic condition flag is unset.

Although the ALT hemodynamic condition decision points are shown at particular points within the R/V CSG engine 403, these stages are examples only. In various implementations, the R/V CSG engine 403 may include ALT hemodynamic condition decision points at other points within the R/V CSG engine 403 in addition to or as alternatives to those shown in FIGS. 7, 9, 10, and 11. As discussed above, the CSG engine 120 initiates both of the hemodynamic algorithm 444 and the R/V CSG engine 403 at the stage 635 in FIG. 6 and these algorithms run in parallel. Therefore, the R/V CSG engine 403 may include the additional and/or alternative ALT hemodynamic condition decision points as a check on the status of the hemodynamic algorithm 444 running in parallel.

The ALT hemodynamic condition flag indicates that the hemodynamic algorithm 444 has identified the presence of an ALT hemodynamic patient condition. The hemodynamic algorithm 444 runs in parallel with the R/V CSG engine 403 and is illustrated schematically in FIG. 12. As discussed further in regard to FIG. 12, the ALT hemodynamic condition is a hemodynamic condition that poses an immediate threat to patient survival, such as, for example, cardiac arrest. Thus, in addition to any R/V intervention, the caregiver and CSG engine 120 must address and provide interventions for the ALT hemodynamic condition or else the patient is likely to die. Further, the intervention for the ALT hemodynamic condition may be of a higher priority with regard to patient survival than the R/V intervention. Thus, the interventions for the ALT hemodynamic condition may be the primary patient care required to keep the patient alive long enough to be able to provide clinical interventions for respiratory distress. Depending on the clinical situation, there may be multiple etiologies of respiratory distress and some respiratory distress interventions may be secondary to the ALT hemodynamic condition and some may be in conjunction with the ALT hemodynamic condition. In various situations, an RD intervention provided in conjunction with the ALT hemodynamic condition may alleviate multiple etiologies.

As one example, a patient may present with RD. The patient may have asthma, however, at the outset, the caregiver may not be aware of the asthma etiology and may merely be aware of an evaluation of respiratory gases that indicate that the patient is hypoxic. Additionally, the same patient may go into cardiac arrest, which may or may not be related to the asthma. For example, the patient may have cardiac disease in addition to asthma. However, similarly to the asthma, at the outset, the caregiver may not be aware of the cardiac disease etiology and may merely be aware of an ECG waveform that indicates that the patient is in ventricular fibrillation. In the course of providing an intervention for the cardiac arrest, the caregiver may initiate cardiopulmonary resuscitation (CPR) which includes respiratory support. Although triggered by the cardiac arrest, this respiratory support during may treat the hypoxia. Thus, as the R/V CSG engine 403 loops through the respiratory gas analyses, these may show an improvement in the hypoxic condition of the patient. Once the patient is stabilized in regard to the cardiac arrest, then the caregiver may resume evaluation and interventions directed at the RD.

In the absence of the ALT hemodynamic condition at the stage 712, given the evaluation of the respiration data as normal at the stage 710, the R/V algorithm proceeds to the stages 750 and 755 to provide first output 260 to the medical device(s) 180. The absence of the ALT hemodynamic condition flag indicates that the hemodynamic algorithm 444 has not identified an ALT hemodynamic patient condition. At the stage 750, the first output 260 includes an event marker and an instruction for the medical device(s) to record the event marker in the patient encounter data file 188. This event marker indicates the normal respiration data evaluation from the stage 710.

In the absence of the ALT hemodynamic condition flag at the stage 722, based on the evaluation of the respiration data at the stage 720, the R/V CSG engine 403 selects a clinical intervention at the stage 723 and proceeds to the connection point A 897 in FIG. 8A. In the absence of the ALT hemodynamic condition at the stage 732, based on the evaluation of the respiration data at the stage 730, the R/V CSG engine 403 selects a clinical intervention at the stage 733 and proceeds to the connection point B 898 in FIG. 8A. In the absence of the ALT hemodynamic condition at the stage 742, based on the evaluation of the respiration data at the stage 740, the R/V CSG engine 403 selects a clinical intervention at the stage 743 and proceeds to the connection point B 899 in FIG. 8A.

The process stages 723, 733, and 743 provide exemplary implementations of the stage 509 of the method 500 shown in FIG. 5A. At these stages, the R/V CSG engine 403 determines or selects at least one clinical intervention based at least in part on the received respiratory data and the evaluation of that data. In some implementations, the R/V CSG engine 403 may receive and evaluate medical history data and/or data entered by the user at the CSG UI 110 based on caregiver observations and evaluations. As similarly discussed in regard to the stage 509, the R/V CSG engine 403 may select the at least one clinical intervention from a range of interventions according to an RD protocol (e.g., clinical practice guidelines and/or standards of care) that may include, for example, among others: pharmacologic, supplemental O2, mechanical respiratory or ventilation support, additional monitoring, or logistical instructions relative to the management of the patient.

Turning to FIG. 8A, three branches of the R/V CSG engine 403 in continuation from FIG. 7 are shown. All three of these branches include a UI instruction to connect the patient to the ventilation system 280 (e.g., the stages 801, 805, 810) and an instruction to the medical device(s) to provide supplemental oxygen (e.g., the stages 802, 806, 811). The process stages 802, 806, and 811, along with the process stages 750 and 755 described above and the process stages 803, 807, 809, 812, and 814 described below, provide exemplary implementations of the stage 512 of the method 500 shown in FIG. 5A.

At the stages 801, 805, and 810 the R/V CSG engine 403 provides second output 225 to the CSG UI 110. The second output 225 at these stages includes the UI instruction as a user prompt or reminder to connect the patient to the ventilation system 280 via the patent interface devices 170, specifically the nasal cannula 279 or the mask 278.

Looking at the branch designated by the connection point A 897, the stage 801 provides second output 225 that includes a user prompt or reminder to connect the patient to the ventilation system 280 via either the mask 278 or the nasal cannula 279. In an implementation, the second output 225 may indicate one of the mask 278 or the nasal cannula 279 or may provide both options to the caregiver and request a confirmation input as to the device selected by the caregiver. At the stage 802, the R/V CSG engine 403 provides first output 260 to the ventilation system 280 to provide supplemental oxygen based on the respiration evaluation at the stage 720. Subsequently, at the stage 803, the R/V CSG engine 403 provides a first output 260 to the medical device(s) 180 that includes an event marker and an instruction for the medical device(s) to record the event marker in the patient encounter data file 188. This event marker indicates the respiration data evaluation from the stage 720, the supplemental oxygen intervention, and the selected oxygen delivery device, i.e., nasal cannula or mask.

Looking at the branch designated by the connection point B 898, the stage 805 provides second output 225 that includes a user prompt or reminder to connect the patient to the ventilation system 280 via the mask 278. In an implementation, the second output 225 may request a confirmation input from the caregiver for the mask connection. Based on the respiration evaluation at the stage 730, the R/V CSG engine 403 then provides first output 260 to the ventilation system 280 to provide supplemental oxygen at the stage 806 and to provide CPAP at the stage 807. Following the instruction to provide CPAP, the R/V algorithm sets a CPAP indication flag, e.g., flag 1, at the stage 808. The presence of absence of flag 1 will determine future instructions from the R/V CSG engine 403. Subsequently, at the stage 809, the R/V CSG engine 403 provides a first output 260 to the medical device(s) 180 that includes an event marker and an instruction for the medical device(s) to record the event marker in the patient encounter data file 188. This event marker indicates the respiration data evaluation from the stage 730 and the administration of supplemental oxygen and CPAP interventions via the mask.

Looking at the branch designated by the connection point C 899, the stage 810 provides second output 225 that includes a user prompt or reminder to connect the patient to the ventilation system 280 via the mask 278. In an implementation, the second output 225 may request a confirmation input from the caregiver for the mask connection. Based on the respiration evaluation at the stage 740, the R/V CSG engine 403 then provides first output 260 to the ventilation system 280 to provide supplemental oxygen at the stage 811 and to provide bilevel positive airway pressure support (referred to herein as "bilevel") at the stage 812. Following the instruction to provide bilevel, the R/V algorithm sets a bilevel indication flag, e.g., flag 2, at the stage 813. The presence of absence of flag 2 will determine future instructions from the R/V CSG engine 403. Subsequently, at the stage 814, the R/V CSG engine 403 provides a first output 260 to the medical device(s) 180 that includes an event marker and an instruction for the medical device(s) to record the event marker in the patient encounter data file 188. This event marker indicates the respiration data evaluation from the stage 740 and the administration of supplemental oxygen and bilevel interventions via the mask.

In an implementation, one or more of the UI instructions at the stages 801, 805, and 810 or the medical device instructions at the stages 802, 806, 807, 811, and 812 include operational parameters for the ventilation system 280. The operational parameters may include gas composition (e.g., oxygen concentration), gas flow rate, gas flow rate of increase, gas flow rate of decrease, gas pressure, and time. One or more of these parameters may define a ventilation mode, for example, CPAP mode, bilevel mode, or high flow nasal cannula (HFNC) mode, or other modes of invasive ventilation.

The UI instructions at the stages 801, 805, and 810 may include one or more values for these parameters and/or may specify the ventilation mode. In an implementation, the caregiver 103 may adjust these parameters or the mode using controls on the medical device(s) 180. For example, the medical device(s) 180 may include mechanical controls and/or software controls activated at a touchscreen interface and the caregiver may manipulate these controls to adjust the parameters and/or the mode. The UI instructions may include a prompt at the CSG UI 110 for confirmation by the caregiver of the settings as adjusted at the medical device(s) 180. The CSG UI may include patient respiratory parameter and status data, and prompt the user to initiate user-controlled therapy based respiratory or other sensor data, and/or may, to various degrees interactively guide the user in this regard.

The medical device instructions at the stages 802, 806, 807, 811, and 812 enable control of the ventilation system 280 by the CSG engine 120. In various implementations, the CSG engine 120 may operate with different levels of autonomy with regard to control of the ventilation system 280. For example, in some implementations, in some modes of operation, CSG engine 120 may control the ventilation system 280 in response to interactive user input at the CSG UI 110. The user may enter operational parameters or request changes in these parameters or in ventilation modes at the CSG UI 110 and the CSG engine 120 may control the ventilation system 280 in response to these user instructions. Alternatively, the CSG engine 120 may use physiologic closed loop control (PCLC) to autonomously or semi-autonomously control the ventilation system 280. In PCLC, the CSG algorithm may adjust and control respiration and ventilation interventions, using monitored patient data. Thus, the CSG engine 120 may control the medical device(s) 180 to implement desired parameters or modes without user input or with user input limited to confirmation of operational parameters identified and implemented by the CSG engine 120. The CSG engine 120 may request user permissions to control various operations and/or may request a user confirmation of the automatically implemented settings at the medical device(s). Further, the CSG engine 120 may enable a caregiver override or adjustment of the automatically implemented settings. In an implementation, the CSG engine 120 may control the medical device(s) 180 based on a combination of caregiver selections and automatic control by the CSG engine 120.

In some implementations, the first output 260 to the medical device(s) 180 may enable the PCLC of the medical device(s) 180 by the CSG engine 120. As such, the first output 260 may be configured to change, or automatically change, ventilation mode or parameters, such as automatically increasing or decreasing provided gas flow to the patient, or switching between modes such as CPAP, bilevel or HFNC modes, as appropriate or optimal based determined or measured evolving or changing patient physiological parameters as evaluated by the CSG engine 120. As an example, the first output 260 may cause or instruct the ventilation system 280 to automatically ramp baseline pressures to initiate CPAP while automatically adjusting parameters like trigger rise time and/or cycle criteria. As another example, the CSG engine 120 may monitor the SpO2 signal to automatically regulate O2 delivery to maintain a preselected SpO2 target. As a further example, the CSG engine 120 may monitor an effectiveness of any intervention based on patient condition as indicated by the physiologic data. For example, if the SpO2 and EtCO2 data indicate that a patient is decompensating, the CSG engine 120 may provide a second output 225 to alert the caregiver and recommend a use of bilevel ventilation. The CSG engine 120 may ramp the provided therapy to bilevel conditions either automatically or in response to a user confirmation at the CSG UI 110.

At the stage 818, the R/V CSG engine 403 receives lung mechanics data from the medical device(s) 180. The process stage 818 provides an exemplary implementation of the stage 515 of the method 500 shown in FIG. 5A. The R/V CSG engine 403 receives this data as first input 240 from the medical device(s) 180. In an implementation, as described in more detail with regard to FIGS. 16, 22A, and 22B, the stage 818 includes providing the received data at the CSG UI 110.

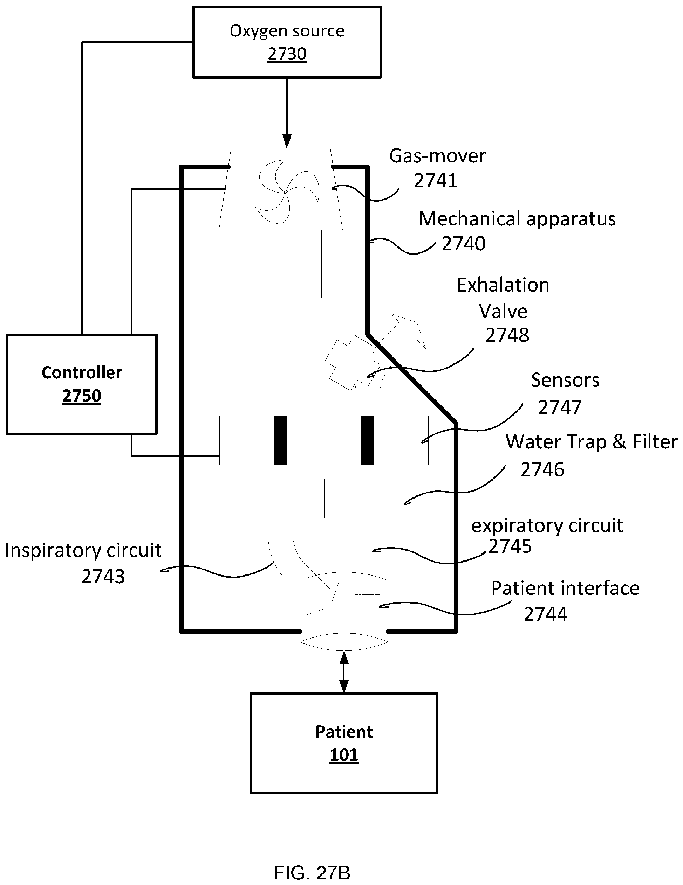

As described below with regard to FIG. 27B, the ventilation system 280 may provide oxygen to the patient from an oxygen source 2730 through an inspiratory circuit 2743 coupled to a patient interface 2744 (e.g., the mask 278 or nasal cannula 279). The patient interface 2744 is also coupled to an expiratory circuit 2745. The inspiratory and expiratory circuits may include sensors 2747 such as, for example, the airway pressure sensor 274 and the pneumotachometer 275, as shown in FIG. 2. The lung mechanics data received at the stage 818 may include lung resistance and lung elastance or compliance (elastance is the reciprocal of compliance) as calculated by the ventilation system 280 from data collected by the airway pressure sensor 274 and the pneumotachometer 275. The ventilation system 280 may provide the lung resistance and lung elastance or compliance data to the CSG engine 120 at the stage 818. The lung mechanics data received at the stage 818 enables the CSG engine 120 to differentiate between various categories of RD. Such differentiation may be inconclusive based solely on the respiration data received at the stage 760 and update the clinical interventions to refine the patient care based on this additional data.

Upon receipt, the R/V CSG engine 403 evaluates the lung mechanics data at the stages 820, 830, 840, and 850 for one of four conditions. The process stages 820, 830, 840, and 850 provide an exemplary implementation of the stage 518 of the method 500 shown in FIG. 5A. At the stage 820, the R/V CSG engine 403 identifies normal resistance (e.g., 2.7 - 15 cm H₂O/L/sec) and normal elastance (e.g., 7.1-13.2 cm H₂O/L) and proceeds to the connection point D 999 in FIG. 9. At the stage 830, the R/V CSG engine 403 identifies a high resistance (e.g., > 15 cm H₂O/L/sec) and high elastance (e.g., > 13.2 cm H₂O/L) and proceeds to the connection point E 1099 in FIG. 10. At the stage 840, the R/V CSG engine 403 identifies a high resistance (e.g., > 15 cm H₂O/L/sec) and a normal elastance (e.g., 7.1-13.2 cm H₂O/L) and proceeds to the connection point E 1099 in FIG. 10. At the stage 850, the R/V CSG engine 403 identifies a normal resistance (e.g., 2.7 - 15 cm H₂O/L/sec) and low elastance (e.g., < 7.1 cm H₂O/L) and proceeds to the connection point F 1199 in FIG. 11.

Referring to FIG. 8B, process stages of a monitoring loop of the R/V algorithm are shown. The R/V CSG engine 403 enters the respiratory monitoring loop 890 at the "RM" connection point 891. As described above in regard to FIG. 8A, the R/V algorithm trifurcates according to the evaluation of the airway pressure and pneumotachograph data. This evaluation corresponds to the stage 518 of the method 500 shown in FIG. 5A. In each of the resultant three pathways, described below in regard to FIGS. 9, 10, and 11 respectively, the R/V CSG engine 403 completes the stages 521 and 524 of the method 500 according to the particular data evaluation. As shown in FIGS. 9, 10, and 11, once the R/V CSG engine 403 provides a clinical intervention update to the medical device(s), each of the algorithmic pathways shown in FIGS. 9, 10, and 11 includes a node at which the algorithm enters the respiratory monitoring loop 890. The respiratory monitoring loop 890 is an example of the monitoring loop 590 in FIG. 5B. These nodes are examples of the node 598 in FIG. 5A and are shown as the node 998 in FIG. 9, the node 1098 in FIG. 10, and the node 1198 in FIG. 11. Additionally, at the nodes 1098 and 1198, the R/V CSG engine 403 continues to the etiology sequences 1095 and 1195 as examples of the etiology sequence 595 in FIG. 5C.

In addition to the R/V algorithm pathways to the respiratory monitoring loop 890 via the nodes 998, 1098, and 1198, the algorithmic pathway shown in FIG. 9 includes a decision point 927 regarding the existence of an abnormal life threatening (ALT) hemodynamic condition. As discussed in further detail below, at this decision point, the R/V CSG engine 403 branches to the hemodynamic algorithm 444 (e.g., as shown in FIG. 12) in the presence of the ALT hemodynamic condition. In conjunction with the hemodynamic algorithm 444, the R/V CSG engine 403 enters the respiratory monitoring loop 890. This is illustrated in FIG. 9 with a forked path to the connection points "H" and "RM" after the stage 927.

The respiratory monitoring loop 890 begins at the stage 855. The stage 855 is an example of the stage 527 in FIG. 5B and includes monitoring of the first and second physiologic data, namely, the SpO2 and EtCO2 data and the elastance and resistance data. At this point in the R/V algorithm, the patient is connected to the patient monitor/defibrillator 285 via the SpO2 sensor 270 and the EtCO2 sensor 271 for the SpO2 and EtCO2 data. The patient is also connected to the ventilation system 280 via the airway pressure sensor 274 and the pneumotachometer 275 for the elastance and resistance data. The medical device(s) 180 provide this data substantially continuously to the CSG engine 120. For example, pulse oximetry, airway pressure, and pneumotachograph measurements may be updated at least every one to two seconds and capnography may be updated upon every detected breath.

At the stage 860, the respiratory monitoring loop 890 provides a steady state delay, as similarly described in regard to the stage 530 in FIG. 5B. This steady state delay accounts for effects of the clinical interventions updated at the stage 920, 1042, or 1172 and/or supplemented at the stage 1056 or 1186 and/or updated during a previous iteration of the respiratory monitoring loop 890. Once the steady state delay has elapsed, the R/V CSG engine 403 proceeds to the stage 865 to evaluate the monitored data as an example of the stage 533 in FIG. 5B. In this example, the R/V CSG engine 403 evaluates the SpO2 and EtCO2 data for one of the four conditions described in regard to the stages 710, 720, 730, and 740 in FIG. 7. Additionally, the algorithm evaluates the airway pressure data and the pneumotachograph data for one of the four conditions described in regard to the stages 820, 830, 840, and 850 in FIG. 8A.

Based on the evaluation, the R/V CSG engine 403 updates the clinical intervention at the stage 870 as an example of the stage 539 in FIG. 5. As similarly described for the stage 539, updating the clinical intervention may include maintaining an ongoing intervention, modifying parameters of the ongoing intervention, supplementing the ongoing intervention, or replacing the ongoing intervention with one or more other and different interventions. The ongoing intervention may include supplemental oxygen or supplemental oxygen with CPAP or bilevel.

At the stages 875, the R/V CSG engine 403 may provide instructions to the medical device(s) 180 to implement the updated clinical intervention from the stage 870. At the stage 880, the R/V CSG engine 403 may provide instructions to the medical device(s) to record an event marker indicating the updated clinical intervention. The stages 875 and 880 are examples of the stage 542 in FIG. 5. Following the stage 880, the R/V CSG engine 403 returns to the stage 855 to monitor data and re-start the respiratory monitoring loop 890.

Turning to FIG. 9, a first branch of the R/V CSG engine 403 in continuation from FIG. 8A is shown. At the stage 920, the R/V CSG engine 403 updates the at least one clinical intervention in progress (e.g., as selected at the stage 723, 733, or 743). The process stage 920 provides an exemplary implementation of the stage 521 of the method 500 shown in FIG. 5A. As described in more detail with regard to the stage 521, updating the clinical intervention may include changing, modifying, supplementing, or replacing the clinical intervention in progress. The R/V CSG engine 403 may update the at least one clinical intervention based at least in part on the evaluation of the respiratory data combined with the evaluation of the lung mechanics data at the stage 820. In some implementations, at the stage 920, the R/V CSG engine 403 may receive and evaluate additional medical history data and/or data entered by the user at the CSG UI 110 based on caregiver observations and evaluations.

In a manner similar to the selection of the clinical intervention at the stage 723, 733, or 743, the R/V CSG engine 403 may update the at least one clinical intervention at the stage 920 according to an RD protocol (e.g., clinical practice guidelines and/or standards of care) that may include, for example, among others: pharmacologic, supplemental O2, mechanical respiratory or ventilation support, additional monitoring, or logistical instructions relative to the management of the patient. In the example of FIG. 9, at the stage 920, the update to the clinical intervention may include a maintenance of any ongoing supplemental oxygen, CPAP, or bilevel support according to previously determined operational parameters.

Following the update, the R/V CSG engine 403 proceeds to the stages 921 and 922. At the stages 921 and 922, the R/V CSG engine 403 checks the status of flag 1 and of flag 2, respectively. If the R/V CSG engine 403 determines that flag 1 is set, this indicates that CPAP therapy is already in progress. In this case, at the stage 923, the R/V CSG engine 403 provides first output 260 to the ventilation system 280 to cause or instruct implement the updated clinical intervention with an instruction to continue, or maintain, the CPAP intervention in progress. If the R/V algorithm determines that flag 2 is set, this indicates that bilevel therapy is already in progress. In this case, at the stage 922, the R/V CSG engine 403 provides first output 260 to the ventilation system 280 to implement the updated clinical intervention with an instruction to continue, or maintain, the bilevel intervention in progress. If neither flag is set, at the stage 925, the R/V CSG engine 403 provides first output 260 to the ventilation system 280 to implement the updated clinical intervention with an instruction to continue the supplemental oxygen intervention. At the stage 926, the R/V CSG engine 403 provides a first output 260 to the medical device(s) 180 that includes an event marker and an instruction for the medical device(s) to record the event marker in the patient encounter data file 188. This event marker indicates the lung mechanics evaluation from the stage 820 and the maintenance of supplemental oxygen, CPAP, or bilevel. The process stages 923, 924, 925, and 926 provide exemplary implementations of the stage 524 of the method 500 shown in FIG. 5A.

At the stage 927, if the ALT hemodynamic condition flag is set, then the CSG engine 120 proceeds to the hemodynamic CSG engine 404 shown in FIG. 12 as indicated by the "H" connection point in FIG. 9. In conjunction with the hemodynamic CSG engine 404, the R/V CSG engine 403 enters the respiratory monitoring loop 890. This is illustrated in FIG. 9 with a forked path to the connection points "H" and "RM" after the stage 927. In the absence of the ALT hemodynamic condition flag at the stage 927, at the node 998 the R/V algorithm proceeds to the respiratory monitoring loop 890 at the "RM" connection point.

Turning to FIG. 10, a second branch of the R/V CSG engine 403 in continuation from FIG. 8A is shown. At the stage 1042, the R/V CSG engine 403 updates the at least one clinical intervention in progress (e.g., as selected at the stage 723, 733, or 743). The process stage 1042 provides an exemplary implementation of the stage 521 of the method 500 shown in FIG. 5A. As described in more detail with regard to the stage 521, updating the clinical intervention may include changing, modifying, supplementing, or replacing the clinical intervention in progress. The R/V CSG engine 403 may update the at least one clinical intervention based at least in part on the evaluation of the respiratory data combined with the evaluation of the lung mechanics data at the stage 830 or 840.

The CSG engine 120 may arrive at the stage 1042 upon an evaluation detecting either high resistance/high elastance at the stage 830 or high resistance/normal elastance at the stage 840. The difference in the elastance evaluations does not alter the intervention update at the stage 1042. However, this difference is relevant to the etiology determination at the stage 1053. In some implementations, at the stage 1042, the R/V CSG engine 403 may receive and evaluate additional medical history data and/or data entered by the user at the CSG UI 110 based on caregiver observations and evaluations.

In a manner similar to the selection of the clinical intervention at the stage 723, 733, or 743, the R/V CSG engine 403 may update the at least one clinical intervention at the stage 1042 according to an RD protocol (e.g., clinical practice guidelines and/or standards of care) that may include, for example, among others: pharmacologic, supplemental O2, mechanical respiratory or ventilation support, additional monitoring, or logistical instructions relative to the management of the patient. In the example of FIG. 10, at the stage 1042, the update to the clinical intervention may include raised supplemental oxygen, CPAP, or bilevel support relative to previously determined operational parameters.

Following the update, the R/V CSG engine 403 proceeds to the stages 1045 and 1047. At the stages 1045 and 1047, the R/V CSG engine 403 checks the status of flag 1 and of flag 2, respectively and proceeds to the stages 1046, 1048, and 1049. The process stages 1046, 1048, and 1049 provide exemplary implementations of the stage 524 of the method 500 shown in FIG. At the stages 1046, 1048, and 1049, the CSG engine 120 may control the medical device(s) 180 via instructions to the medical device(s) in a manner similar to that described above in regard to the medical device instructions at the stages 802, 806, 807, 811, and 812 The medical device instructions at the stages 1046, 1048, and 1049 enable control of the ventilation system 280 by the CSG engine 120. In various implementations, the CSG engine 120 may operate with different levels of autonomy with regard to control of the ventilation system 280 as similarly described above with regard to the stages 802, 806, 807, 811, and 812.

If the R/V CSG engine 403 determines that flag 1 is set, this indicates that CPAP therapy is already in progress. In this case, at the stage 1046, the R/V CSG engine 403 provides first output 260 to the ventilation system 280 to implement the updated clinical intervention with an instruction to raise support for the CPAP intervention in progress (e.g., increase the . expiratory positive airway pressure (ePAP)). If the R/V algorithm determines that flag 2 is set, this indicates that bilevel therapy is already in progress. In this case, at the stage 1048, the R/V CSG engine 403 provides first output 260 to the ventilation system 280 to implement the updated clinical intervention with an instruction to raise support for the bilevel intervention in progress (e.g., increase ePAP and inspiratory positive airway pressure (iPAP)). If neither flag is set, at the stage 1049, the R/V CSG engine 403 provides first output 260 to the ventilation system 280 to implement the updated clinical intervention with an instruction to raise support for the supplemental oxygen intervention in progress (e.g., increase the fraction of inspired oxygen (FiO2).

Following the stages 1046, 1048, and 1049, the R/V CSG engine 403 bifurcates at the node 1098 to the respiratory monitoring loop 890 in FIG. 8B and to the etiology sequence 1095. The node 1098 is an example of the node 598 in FIG. 5A and the etiology sequence 1095 is an example of the etiology sequence 595 in FIG. 5C.

The etiology sequence 1095 starts at the stage 1050. At the stage 1050, the R/V CSG engine 403 generates an etiology estimate based on the respiratory data and the lung mechanics data. The process stage 1050 exemplifies the stage 545 of the method 500 in FIG. 5C. In this example, the etiology estimate is of an obstructive lung condition. In an implementation, the R/V CSG engine 403 may send the etiology estimate to the CSG UI 110 as second output 225. The R/V CSG engine 403 may arrive at the stage 1050 with an evaluation of high resistance and either normal elastance (as evaluated at the stage 840) or high elastance (as evaluated at the stage 830). Both of asthma and COPD are obstructive lung conditions. However, normal elastance may support an etiology determination of asthma, whereas high elastance may support an etiology determination of COPD. Thus, the R/V CSG engine 403 may proceed to the stages 1051 and optionally 1052a and/or 1052b to obtain data to further distinguish between various etiologies of obstructive lung conditions and/or confirm the etiology indicated as possible or likely just based on the elastance data.

Given that an obstructive lung condition may stem from a variety of etiologies, at the stage 1051, the R/V CSG engine 403 may request and receive targeted data. The process stage 1051 exemplifies the stages 547 and 549 of the method 500 in FIG. 5C. The targeted data may be one or more types of patient data that may enable distinctions between the varieties of etiologies within the scope of an obstructive lung condition. In this example, the R/V CSG engine 403 may request one or more of spirometry data and exhaled nitric oxide data to enable a distinction between COPD and asthma which are both obstructive lung conditions. The R/V CSG engine 403 may request the targeted data with first output 260 to the medical device(s) 180 (e.g., an instruction to send or to collect and send the targeted data). The R/V CSG engine 403 may receive the targeted data from the medical device(s) as first input 240. Additionally or alternatively, the R/V CSG engine 403 may request and receive the targeted data from the mobile device 105. The R/V algorithm may request the data via second output 225 to the CSG UI 110 and receive the data as second input 230 from the CSG UI 110.

At the stage 1052a, the R/V CSG engine 403 may optionally provide a second output 225 to the CSG UI 110 to prompt the user to enter patient medical history information and/or caregiver evaluations or observations. Additionally or alternatively, the R/V CSG engine 403 may optionally receive stored medical history information for the patient at the stage 1052b from one or more of the memory 109, the memory 187, the memory 309, and the medical record database(s) 399. The process stages 1052a and 1052b exemplify the stages 547 and 549 of the method 500 in FIG. 5C.This stored medical history information may include previously stored medical history information for the patient accessed via a query from the CSG engine 120 to the storage location. In an implementation, the R/V CSG engine 403 may supplement the targeted data with the medical history information received at stage 1052a and/or 1052b to differentiate between the variety of etiologies within the scope of the etiology estimate. For example, the stored medical history information may indicate a history of a particular lung disease or condition within the scope of obstructive lung conditions (e.g., a history of asthma or COPD). As another example, the stored medical history information may indicate a lack of a pre-existing lung disease or condition. This may guide the R/V CSG engine 403 to evaluate the physiologic data for evidence of a circumstantial cause (e.g., an environmental cause such as smoke inhalation or a toxic gas leak). In an implementation, the R/V CSG engine 403 may request additional targeted data for this evaluation and/or may query the user for circumstantial information at the CSG UI 110.

At the stage 1053, the R/V CSG engine 403 may convert the etiology estimate to an etiology determination based on the targeted data and/or the medical history information. The process stage 1053 exemplifies the stage 555 of the method 500 in FIG. 5C. In an implementation, the etiology determination may be a diagnosis of the root cause of the presenting RD or may be an etiology subset of the etiology estimate from the stage 1050. In the latter case, the CSG engine 120 may not have enough information to arrive at a diagnosis.

In an implementation, the CSG engine 120 may determine a confidence factor for the etiology determination. The algorithm may determine this confidence factor based on the number and types of data provided in the targeted data and the medical history. For example, an obstructive lung condition combined with the spirometry data may indicate a moderate confidence of COPD (e.g., 50-80% depending on how far above the normal range of elastance measurements the elastance is for this particular patient). However, spirometry data indicative of COPD may increase this confidence level (e.g., from 50-80% to 65- 80%) and a medical history of COPD may further increase this confidence level (e.g., from 65%-80% to 85-99%).

In an implementation, the stage 1053 may include sending the etiology determination to the medical device(s) 180 as first output 260 and/or sending the etiology determination to the CSG UI 110 as second output 225. Such an implementation exemplifies the stage 557 of the method 500 in FIG. 5C. The R/V CSG engine 403 may send the etiology determination to the medical device(s) 180 with an instruction to store the etiology determination in the patient encounter data file 188 as an event marker. Additionally or alternatively, the R/V CSG engine 403 may send the etiology determination to the medical device(s) 180 with an instruction to provide the etiology determination at a user interface of the medical device (e.g., display the etiology determination at a screen and/or provide an audible announcement of the etiology determination). The R/V CSG engine 403 may send the etiology determination to the CSG UI 110 for display. Additionally, the R/V CSG engine 403 may request a user confirmation of the etiology determination at the mobile device 105 and/or at the medical device(s) 180.

The CSG engine 120 may determine the etiology at the stage 1053 based on one or more of the targeted data and the medical history data combined with the previously evaluated physiologic data. For example, normal elastance (as evaluated at the stage 840) may support an etiology determination of asthma, whereas high elastance (as evaluated at the stage 830) may support an etiology determination of COPD.

Based on the etiology determination, at the stage 1054, the R/V CSG engine 403 may supplement the clinical intervention. The R/V CSG engine 403 may further update the clinical intervention according to an RD protocol (e.g., clinical practice guidelines and/or standards of care) that may include, for example, among others: pharmacologic, supplemental O2, mechanical respiratory or ventilation support, additional monitoring, or logistical instructions relative to the management of the patient. For example, the R/V CSG engine 403 may identify a pharmacological intervention to supplement ongoing ventilation support. For example, based on an etiology determination of COPD or asthma, the R/V CSG engine 403 may identify the pharmacological intervention of a bronchodilator or corticosteroid. The R/V CSG engine 403 may further identify a dose and schedule for the pharmacologic intervention. Additionally, the R/V CSG engine 403 may identify and monitor particular physiologic indicators of the efficacy or adverse effects of the administered medication and adjust doses and schedules based on this physiologic indicator.

At the stages 1055 and 1056, the R/V CSG engine 403 may implement the further updated clinical intervention. As with other clinical interventions, the CSG engine 120 may implement the updated clinical intervention, e.g., the pharmacologic intervention, with different levels of autonomy. Via a second output 225 to the CSG UI 110, the CSG engine 120 may direct caregiver to administer the medication and/or may request permission from the caregiver for an automated medication administration. Optionally, the CSG engine 120 may automatically control the medical device(s) 180 to deliver the medication via a first output 260 to the medical device(s) 180. For the pharmacologic intervention, the level of autonomy may depend on the type of medication and the appropriate medication delivery system. As an example, the mask 278 may include an integrated nebulizer configured to deliver medications to the patient. As another example, the patient interface devices 170 may include an intravenous or other drug delivery device 269 that may be automatically controlled to deliver medication to the patient 101.

At the stage 1057, the R/V CSG engine 403 provides a first output 260 to the medical device(s) 180 that includes one or more event markers and an instruction for the medical device(s) to record the one or more event markers in the patient encounter data file 188. These event markers indicate one or more of the updated clinical intervention, the etiology estimate, the received targeted data, the etiology determination, and the pharmacological intervention.

At the stage 1058, if the ALT hemodynamic condition flag is set, then the CSG engine 120 proceeds to the hemodynamic CSG engine 404 shown in FIG. 12 as indicated by the "H" connection point in FIG. 10. In the absence of the ALT hemodynamic condition flag at the stage 1058, the R/V CSG engine 403 continues the respiratory monitoring loop 890 already in progress. This algorithm executes this loop at the node 1098 in parallel with the etiology sequence 1095.

Turning to FIG. 11, a third branch of the R/V CSG engine 403 in continuation from FIG. 8A is shown. At the stage 1042, the R/V CSG engine 403 updates the at least one clinical intervention in progress (e.g., as selected at the stage 723, 733, or 743). The process stage 1042 provides an exemplary implementation of the stage 521 of the method 500 shown in FIG. 5A. As described in more detail with regard to the stage 521, updating the clinical intervention may include changing, modifying, supplementing, or replacing the clinical intervention in progress. The R/V CSG engine 403 may update the at least one clinical intervention based at least in part on the evaluation of the respiratory data combined with the evaluation of the lung mechanics data at the stage 850. In some implementations, at the stage 1042, the R/V CSG engine 403 may receive and evaluate additional medical history data and/or data entered by the user at the CSG UI 110 based on caregiver observations and evaluations.

In a manner similar to the selection of the clinical intervention at the stage 723, 733, or 743, the R/V CSG engine 403 may update the at least one clinical intervention at the stage 1172 according to an RD protocol (e.g., clinical practice guidelines and/or standards of care) that may include, for example, among others: pharmacologic, supplemental O2, mechanical respiratory or ventilation support, additional monitoring, or logistical instructions relative to the management of the patient. In the example of FIG. 11, at the stage 1172, the update to the clinical intervention may include raised supplemental oxygen, CPAP, or bilevel support relative to previously determined operational parameters.

Following the update, the R/V CSG engine 403 proceeds to the stages 1175 and 1177. At the stages 1175 and 1177, the R/V CSG engine 403 checks the status of flag 1 and of flag 2, respectively and proceeds to the stages 1176, 1178, and 1179. The process stages 1176, 1178, and 1179 provide exemplary implementations of the stage 524 of the method 500 shown in FIG. At the stages 1176, 1178, and 1179, the CSG engine 120 may control the medical device(s) 180 via instructions to the medical device(s) in a manner similar to that described above in regard to the medical device instructions at the stages 802, 806, 807, 811, and 812 The medical device instructions at the stages 1176, 1178, and 1179 enable control of the ventilation system 280 by the CSG engine 120. In various implementations, the CSG engine 120 may operate with different levels of autonomy with regard to control of the ventilation system 280 as similarly described above with regard to the stages 802, 806, 807, 811, and 812.

If the R/V CSG engine 403 determines that flag 1 is set, this indicates that CPAP therapy is already in progress. In this case, at the stage 1176, the R/V CSG engine 403 provides first output 260 to the ventilation system 280 to implement the updated clinical intervention with an instruction to raise support for the CPAP intervention in progress (e.g., increase the . expiratory positive airway pressure (ePAP)). If the R/V algorithm determines that flag 2 is set, this indicates that bilevel therapy is already in progress. In this case, at the stage 1178, the R/V CSG engine 403 provides first output 260 to the ventilation system 280 to implement the updated clinical intervention with an instruction to raise support for the bilevel intervention in progress (e.g., increase ePAP and inspiratory positive airway pressure (iPAP)). If neither flag is set, at the stage 1179, the R/V CSG engine 403 provides first output 260 to the ventilation system 280 to implement the updated clinical intervention with an instruction to raise support for the supplemental oxygen intervention in progress (e.g., increase the fraction of inspired oxygen (FiO2).

Following the stages 1176, 1178, and 1179, the R/V CSG engine 403 bifurcates at the node 1198 to the respiratory monitoring loop 890 in FIG. 8B and to the etiology sequence 1195. The node 1198 is an example of the node 598 in FIG. 5A and the etiology sequence 1195 is an example of the etiology sequence 595 in FIG. 5C.

The etiology sequence 1195 starts at the stage 1180. At the stage 1180, the R/V CSG engine 403 generates an etiology estimate based on the respiratory data and the lung mechanics data. The process stage 1180 exemplifies the stage 545 of the method 500 in FIG. 5C. In this example, the etiology estimate is of a restrictive lung condition. In an implementation, the R/V CSG engine 403 may send the etiology estimate to the CSG UI 110 as second output 225.

Given that a restrictive lung condition may stem from a variety of etiologies, at the stage 1181, the R/V CSG engine 403 may request and receive targeted data. The process stage 1181 exemplifies the stages 547 and 549 of the method 500 in FIG. 5C. The targeted data may be one or more types of patient data that may enable distinctions between the varieties of etiologies within the scope of an obstructive lung condition. In this example, the R/V CSG engine 403 may request NIBP to enable a distinction between congestive heart failure (CHF) and other restrictive lung conditions such as those caused by pregnancy. The R/V CSG engine 403 may request the targeted data with first output 260 to the medical device(s) 180 (e.g., the first output 260 may include an instruction to send or to collect and send the targeted data). The R/V CSG engine 403 may receive the targeted data from the medical device(s) as first input 240. Additionally or alternatively, the R/V CSG engine 403 may request and receive the targeted data from the mobile device 105. The R/V CSG engine 403 may request the data via second output 225 to the CSG UI 110 and receive the data as second input 230 from the CSG UI 110.

At the stage 1182a, the R/V CSG engine 403 may optionally provide a second output 225 to the CSG UI 110 to prompt the user to enter patient medical history information and/or caregiver evaluations or observations. Additionally or alternatively, the R/V CSG engine 403 may optionally receive stored medical history information for the patient at the stage 1182b from one or more of the memory 109, the memory 187, the memory 309, and the medical record database(s) 399. The process stages 1052a and 1052b exemplify the stages 547 and 549 of the method 500 in FIG. 5C.This stored medical history information may include previously stored medical history information for the patient accessed via a query from the CSG engine 120 to the storage location. In an implementation, the R/V CSG engine 403 may supplement the targeted data with the medical history information received at stage 1182a and/or 1182b to differentiate between the varieties of etiologies within the scope of the etiology estimate. For example, the stored medical history information may indicate a history of a condition within the scope of restrictive lung condition (e.g., a history of cardiac disease). As another example, the stored medical history information may indicate a lack of a pre-existing lung disease or condition. This may guide the R/V CSG engine 403 to evaluate the physiologic data for evidence of a circumstantial or environmental cause of the restrictive lung condition. For example, circumstantial or environmental causes of restrictive lung conditions may include exposure to asbestos, silica, coal dust, cigarettes, cigars, or other smoking or vaping products. In an implementation, the R/V algorithm may request additional targeted data for this evaluation and/or may query the user for circumstantial information at the CSG UI 110.

At the stage 1183, the R/V CSG engine 403 may convert the etiology estimate to an etiology determination based on the targeted data and/or the medical history information. The process stage 1183 exemplifies the stage 555 of the method 500 in FIG. 5C. In an implementation, the etiology determination may be a diagnosis of the root cause of the presenting RD or may be an etiology subset of the etiology estimate from the stage 1180. In the latter case, the CSG engine 120 may not have enough information to arrive at a diagnosis. In an implementation, the CSG engine 120 may determine a confidence factor for the etiology determination.

In an implementation, the stage 1183 may include sending the etiology determination to the medical device(s) 180 as first output 260 and/or sending the etiology determination to the CSG UI 110 as second output 225. Such an implementation exemplifies the stage 557 of the method 500 in FIG. 5C. The R/V CSG engine 403 may send the etiology determination to the medical device(s) 180 with an instruction to store the etiology determination in the patient encounter data file 188 as an event marker. Additionally or alternatively, the R/V CSG engine 403 may send the etiology determination to the medical device(s) 180 with an instruction to provide the etiology determination at a user interface of the medical device (e.g., display the etiology determination at a screen and/or provide an audible announcement of the etiology determination). The R/V CSG engine 403 may send the etiology determination to the CSG UI 110 for display. Additionally, the R/V CSG engine 403 may request a user confirmation of the etiology determination at the mobile device 105 and/or at the medical device(s) 180.

Based on the etiology determination, at the stage 1184, the R/V CSG engine 403 may further update the clinical intervention. The R/V CSG engine 403 may further update the clinical intervention according to an RD protocol (e.g., clinical practice guidelines and/or standards of care) that may include, for example, among others: pharmacologic, supplemental O2, mechanical respiratory or ventilation support, additional monitoring, or logistical instructions relative to the management of the patient. For example, the R/V CSG engine 403 may identify a pharmacological intervention to supplement ongoing ventilation support. For example, based on an etiology determination of CHF, the R/V CSG engine 403 may identify the pharmacological intervention of a diuretic. The R/V CSG engine 403 may further identify a dose and schedule for the pharmacologic intervention. Additionally, the R/V CSG engine 403 may identify and monitor particular physiologic indicators of the efficacy or adverse effects of the administered medication and adjust doses and schedules based on this physiologic indicator.

At the stages 1185 and 1186, the R/V CSG engine 403 may implement the further updated clinical intervention. As with other clinical interventions, the CSG engine 120 may implement the updated clinical intervention, e.g., the pharmacologic intervention, with different levels of autonomy. Via a second output 225 to the CSG UI 110, the CSG engine 120 may direct caregiver to administer the medication and/or may request permission from the caregiver for an automated medication administration. Optionally, the CSG engine 120 may automatically control the medical device(s) 180 to deliver the medication via a first output 260 to the medical device(s) 180. For the pharmacologic intervention, the level of autonomy may depend on the type of medication and the appropriate medication delivery system. As an example, the mask 278 may include an integrated nebulizer configured to deliver medications to the patient. As another example, the patient interface devices 170 may include an intravenous or other drug delivery device 269 that may be automatically controlled to deliver medication to the patient 101.

At the stage 1187, the R/V CSG engine 403 provides a first output 260 to the medical device(s) 180 that includes one or more event markers and an instruction for the medical device(s) to record the one or more event markers in the patient encounter data file 188. These event markers indicate one or more of the updated clinical intervention, the etiology estimate, the received targeted data, the etiology determination, and the pharmacological intervention.

At the stage 1188, if the ALT hemodynamic condition flag is set, then the CSG engine 120 proceeds to the hemodynamic CSG engine 404 shown in FIG. 12 as indicated by the "H" connection point in FIG. 11. In the absence of the ALT hemodynamic condition flag at the stage 1118, the R/V CSG engine 403 continues the respiratory monitoring loop 890 already in progress. This algorithm executes this loop at the node 1198 in parallel with the etiology sequence 1195.

Referring to FIG. 12, process stages of a hemodynamic algorithm are shown schematically. The engine 404, however, is an example only and not limiting. The engine 404 can be altered, e.g., by having stages added, removed, rearranged, combined, and/or performed concurrently. The process stages shown in FIG. 12 illustrate an application of the hemodynamic CSG engine 404 to an RD presentation. The legend 99, shown in FIG. 12, as well as in FIGS. 6-11, provides a graphical distinction between the second output 225 to the UI and first output 260 to the medical device(s) 180, as discussed in more detail in regard to FIGS. 2 and 6. The "H" connection point 1299 in FIG. 12 indicates an entry point from the process steps in FIGS. 7-11 and these figures include links to the "H" connection point 1299. The "start" indicated in FIG. 12 indicates an entry point from FIG. 6 upon initialization of the hemodynamic CSG engine 404 by the engine 402.

Upon initialization by the engine 402 (as shown in FIG. 6), at the stage 1225, the hemodynamic CSG engine 404 receives hemodynamic data, e.g., electrocardiogram (ECG) data, heart rate (HR) data, and non-invasive blood pressure (NIBP) data, from the patient monitor/defibrillator 285 at the stage 1225. The hemodynamic CSG engine 404 receives this data as first input 240 from the medical device(s) 180. In an implementation, as described in more detail with regard to FIGS. 16, 22A, and 22B, the stage 1225 includes providing the received data at the CSG UI 110. As explained below, the hemodynamic CSG engine 404 continues to receive the hemodynamic data as collected by the patient monitor/defibrillator 285 and to evaluate the hemodynamic data over the duration of the execution of the CSG algorithm.

Upon receipt, the hemodynamic CSG engine 404 evaluates the hemodynamic data at the stage 1230. Specifically, the hemodynamic CSG engine 404 evaluates the hemodynamic data for indications of an ALT hemodynamic patient condition. For example, an ECG may indicate an arrhythmia associated with ventricular fibrillation or other indication of cardiac arrest. As another example, an elevated HR (e.g., HR > 120 bpm) and an ECG indicative of tachycardia may indicate an imminent stroke. As a further example, a low HR (e.g., HR < 60 bpm) and a low NIBP (e.g., NIBP < 90/60) may indicate a potentially fatal drug overdose.

If any or all of the hemodynamic data indicate an ALT hemodynamic condition, the hemodynamic CSG engine 404 proceeds to the stage 1231 to check the ALT hemodynamic condition flag (ALT condition flag) status. If the ALT condition flag is not already set based on a previous iteration of the hemodynamic algorithm, then the engine 404 proceeds to the stage 1235 to set the flag and then continues to the stage 1240. If the ALT condition flag is already set based on a previous iteration of the hemodynamic algorithm, then the engine 404 proceeds to the stage 1240.

If none of the hemodynamic data indicate the ALT hemodynamic condition at the stage 1230, the hemodynamic CSG engine 404 proceeds to the stage 1232 to check the ALT condition flag status. If the ALT condition flag is not already set based on a previous iteration of the hemodynamic algorithm, then the engine 404 returns to the stage 1225 to update or refresh the hemodynamic data. If the ALT condition flag is already set based on a previous iteration of the hemodynamic algorithm, then, as discussed further below, the engine 404 proceeds to the stage 1280.

Over the course of the patient encounter, as long as the execution of the CSG engine 120 continues, the hemodynamic CSG engine 404 substantially continually executes to monitor and evaluate the status of the hemodynamic data via the hemodynamic monitoring loop 1298. Going around this loop, the engine 404 receives data at stage 1225, evaluates for the ALT hemodynamic condition at stage 1230, looks for the ALT hemodynamic condition flag at stage 1232, and, in the absence of this flag, returns to the stage 1225 to refresh the data. When the data from the stage 1225 indicates the ALT hemodynamic condition, the engine 404 exits the hemodynamic monitoring loop 1298 to the left side of FIG. 12 to remain in an alert status state until the data no longer indicates the ALT hemodynamic condition. When the data from the from the stage 1225 does not indicate the ALT hemodynamic condition but a flag remains set from a previous indication of the ALT hemodynamic condition, the engine 404 exits the hemodynamic monitoring loop 1298 to the right side of FIG. 12 to unset the flag and refresh the data.

Other elements of the CSG engine 120 running in parallel with the hemodynamic CSG engine 404, such as, for example, the R/V CSG engine 403, may periodically check the status of the ALT condition flag. If this flag is set, the other elements may divert their process flow in response so that the CSG engine 120 can address the ALT hemodynamic condition. For example, at the stages 722, 732, and 742 in FIG. 7 and at stages 927, 1058, and 1188 in FIGS. 9, 10, and 11 respectively, the R/V CSG engine 403 checks the ALT condition flag status as controlled by the concurrently executing hemodynamic CSG engine 404. If the ALT condition flag status is "set" at any of these stages, the R/V CSG engine 403 maintains any ongoing interventions and diverts to the stage 1240 in FIG. 12. The R/V CSG engine 403 may maintain any implemented interventions during execution of the hemodynamic CSG engine 404. In an implementation, the CSG engine 120 may prioritize UI elements and/or medical device operations relevant to monitoring of and clinical interventions for the ALT hemodynamic condition indicated at the stage 1230.

Returning to the stage 1240, based on the indication of the ALT hemodynamic condition at the stage 1230, the hemodynamic CSG engine 404 may provide a second output 225 that includes a caregiver alert at the CSG UI 110 that indicates the detected ALT hemodynamic condition.

Optionally, at the stage 1245 the hemodynamic CSG engine 404 may query the patient monitor/defibrillator for a patient status. In response to this query, the patient monitor/defibrillator 285 may provide a first input 240 with a device status for the patient monitor/defibrillator 285. For example, the device status may include an ECG analysis status or result and a defibrillation status, e.g., detection of a shockable rhythm, administration of a defibrillation shock, etc. As a further option, the CSG engine 120 may provide the information from the patient monitor/defibrillator 285 to the CSG UI 110 for display at the stage 1250.

At the stage 1255, the hemodynamic CSG engine 404 may provide an event marker to the medical device(s) 180 with an instruction to record the event marker in the patient encounter data file 188. The event marker indicates the interrupt to and status of any parallel CSG algorithms along with the detected ALT hemodynamic condition. Further, at the stage 1255, the hemodynamic CSG engine 404 returns to stage 1225 to receive updated hemodynamic data and re-iterate.

With no indication of the ALT hemodynamic condition at the stage 1230, the hemodynamic algorithm proceeds to the stage 1232. At the stage 1232, the hemodynamic CSG engine 404 checks the status of the ALT hemodynamic condition flag.

If the ALT condition flag has not been set in a previous iteration of the hemodynamic CSG engine 404, the engine 404 returns to the stage 1225 to update or refresh the hemodynamic data. The hemodynamic CSG engine 404 then re-iterates for monitoring and evaluation of the hemodynamic data.

If the ALT condition flag has been set in a previous iteration of the hemodynamic CSG engine 404, the engine 404 arrives at the stage 1232 because the previously detected hemodynamic condition is no longer detected in the hemodynamic data and the patient may not require an immediate intervention to address an ALT hemodynamic condition. In this case, the hemodynamic CSG engine 404 unsets the flag at the stage 1280.

At the stage 1285, the hemodynamic algorithm provides a second output 225 to the CSG UI 110. The second output 225 at stage 1285 includes a notification for display at the CSG UI 110 that the hemodynamic data no longer indicates the ALT hemodynamic condition that caused the flag to be set in a previous iteration of the hemodynamic CSG engine 404.

At the stage 1290, the hemodynamic CSG engine 404 may provide an event marker to the medical device(s) 180 with an instruction to record the event marker in the patient encounter data file 188. The event marker indicates that the hemodynamic data no longer indicates the ALT hemodynamic condition. Further, at the stage 1290, the hemodynamic CSG engine 404 returns to stage 1225 to receive updated hemodynamic data and re-iterate.

Referring to FIGS. 13A and 13B, a mobile device configured to provide a device view user interface is illustrated. As shown in FIG. 13A, a caregiver 103 may deploy a medical device 180, for example a patient monitor/defibrillator, to treat a victim 101. The medical device 180 may include a display screen 310 configured to display medical device data for the care of the patient. The display screen 310 may provide the operational interface 335 as discussed in regard to FIG. 3G. The medical device 180 may communicate with the mobile device 105. In an implementation, the device view window, or user interface, is configured to provide a real time display of at least a portion of the plurality of physiologic parameters in a visual reproduction of a display format provided on a respective medical device at the operational interface of that respective medical device.

The mobile device 105 may be configured to provide a device view window 1315 as shown, for example, in FIG. 13B. Another example of a device view window 1316 is shown in FIG. 13C as discussed below.

In addition to or as an alternative to any of the elements shown in FIG. 13B, the device view window 1315 may provide one or more elements of the user interface 2760 as shown in FIG. 27C. The device view may improve care from a team of caregivers and/or in a chaotic emergency environment where the screen of the medical device may not be readily accessible to all team members and/or may be inconveniently located for optimum viewing (e.g., under a gurney, on a moving gurney, under an ambulance bench, etc.). For example, a first caregiver 103 may attend to the victim 101 with assistance and therapy from the medical device, such as the patient monitor/defibrillator 285, and the medical device display screen 310. A second caregiver 104 may prepare medications, or a gurney, or attend to another task without a view of the medical device display screen 310. In some scenarios, the second caregiver 104 may supervise a larger care team and possibly other victims. The second caregiver 104 may view the device view window 1315 on the mobile device 105.

In some implementations, the mobile device can display sensor data in real-time from one or more physiological sensors connected to the medical treatment device. In some examples, the mobile device can display a visual reproduction of the information displayed at the medical treatment device in a first display. This first display view, referred to as a device view, allows additional medical team members to participate in patient treatment without having to be immediately proximate to the medical treatment device. For example, a rescue team supervisor (e.g., the caregiver 104 in FIG. 13B) who oversees and coordinates patient treatment during a critical patient care event (e.g., chest pain, cardiac arrest, traumatic brain injury (TBI), RD, or critical care monitoring) can view, in real-time, the same waveforms and patient data displayed at the medical treatment device. This allows supervisors or other medical team members to observe both rescuer treatment actions and patient data simultaneously so that the supervisors can provide recommendations to further enhance patient treatment and coordinate treatment by multiple rescuers. In some examples, the device view displays a visual reproduction of case information displayed at the medical treatment device.

The medical device data displayed in the device view of the mobile device 105 is a visual reproduction of that information displayed at a display interface of the medical device(s) 180 when one or more of the following visual elements of the display interface of the medical device(s) 180 is reproduced in the device view: the display layout, magnification of each data section, physiologic waveform selection, physiologic numeric readout selection, resolution, waveform duration, waveform size, text size, font, and display colors. In some examples, the visual reproduction of the display screen of the medical device(s) 180 at the mobile device 105 can exactly replicate at is displayed at the medical device(s) 180 or one or more of the visual elements may be altered from what is displayed at the medical device(s) 180. In one example, font and size of one or more items of displayed case information may be enlarged and/or highlighted to draw the eyes of users of the mobile device 105 to the respective case information. Waveforms or other data of a certain type may be magnified or color coded in a particular fashion. For example, ECG waveforms, such as in a 12-lead analysis, may be magnified similarly, or certain waveforms may be emphasized in resolution, color, or other manner of reproduction. Alternatively, in some embodiments, numerical values representing non-continuous physiological measurements (e.g., NIBP, SpO2, HR, and/or EtCO2) may be shown in a similar sized font or layout. In some cases, for example, a visual reproduction may be a replication of the information as presented on the display interface of the medical treatment device, or alternatively with slight variations thereof

In some examples, the device view UI screen 1315 includes one or more selectable inputs at the display interface that cause more, different, or additional information to be displayed, cause one or more actions to be taken at the medical device(s) 180, or provide additional user input interface screens that allow users to submit information that can be transmitted to the medical device(s) 180. For example, the device view 1315 can include one or more view selection tabs 1310, 1340, 1350, and 1370 that allow the user to toggle between different display views for the display at the mobile device 105. In one example, user selection of selection tab 1310 causes the device view UI screen 1315 to be displayed at the mobile device 105. The device view UI screen 1315 can display a number of waveforms and metrics indicative of a condition of a patient and/or a status of medical treatment being administered to the patient. For example, as shown in FIG. 13B, the device view window 1315 may display ECG waveforms 1320a and 1320b, a pulse oximetry waveform 1320c, and a capnography waveform 1320d along with temperature 1320e, invasive blood pressures 1320f and 1320g, heart rate 1325a, pulse oximetry (SpO2) discrete measurement 1325b, end-tidal carbon dioxide (EtCO2) discrete measurement 1325c, and non-invasive blood pressure (NIBP) 1325d.

In some examples, the visual reproduction may encompass an exact replication of the data displayed at the medical treatment device. In other examples, the visual reproduction may include data and formatting variations that can enhance viewing and comprehension of the case information by the mobile device user. In some examples, display layout, magnification of each data section, physiologic waveform selection, physiologic numeric readout selection, resolution, waveform duration, waveform size, text size, font, and/or display colors may vary from what is displayed at the medical treatment device(s).

In some implementations, the information displayed at the device view UI screen 1315 may vary from the information displayed at a display interface of the medical device(s) 180. In some examples, the differences between the interfaces can include differences in a type of information displayed, a display layout, or a display format. For example, an amount of magnification of each data section, resolution, size, and screen position may vary between the device view UI screen 1315 and the display interface of the medical device(s) 180. Additionally, relative waveform sizes and colors, fonts, and text size may vary between the device view 1315 at the mobile device 105. In one example, the device view UI screen 1315 may display numeric values of all physiological case information in a maximized, large-number format without waveforms. In some examples, one or more items of case information displayed at the device view UI screen 1315 may vary from the case information displayed at the display of the medical device(s) 180.

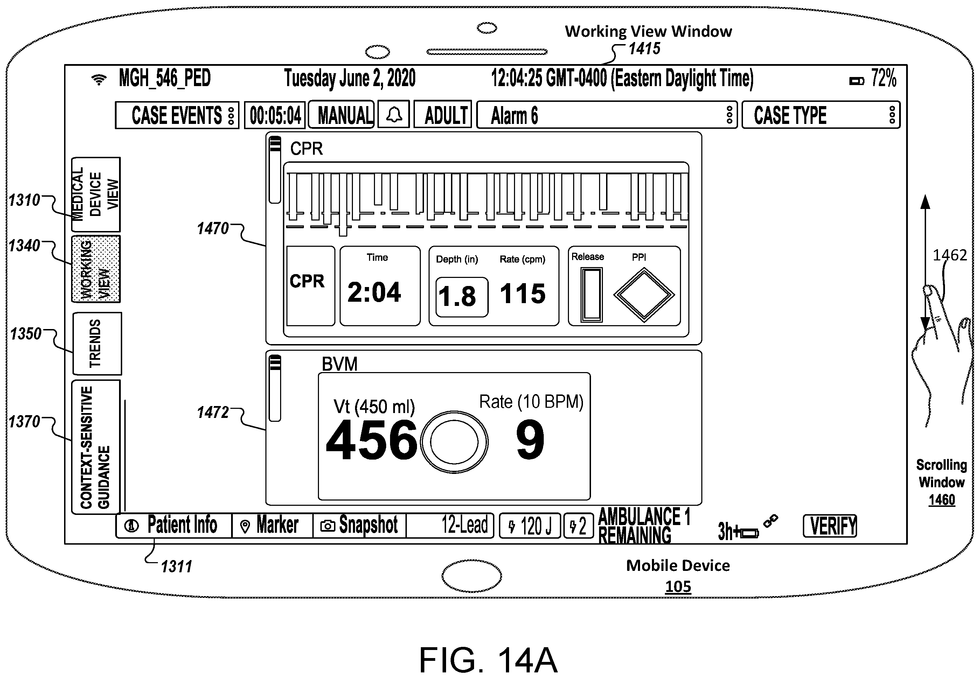

In an implementation, the device view window 1315 may include a patient information control 1311 (e.g., as shown in FIGS. 13B, 13C, 14A, 14B 15, 16, and 29-43). Activation of the control 1311 may enable the user to enter patient identification information at the mobile device 105.

Referring to FIG. 13C, an example of a combined ventilation system/patient monitor/defibrillator device view user interface at the mobile device is shown. In an implementation, one or more of the device view window 1315 and/or 1316, the working view window 1415 and the trend view window 1515 may include a device selection control 1399 (e.g., patient monitor/defibrillator selection control 1399a, ventilation system selection control 1399b, and combined device selection control 1399c). The control 1399 may enable the user to select data for display from a single medical device (e.g., the patient monitor/defibrillator 285 or the ventilation system 280) or select a combined display. In one example, case information for each type of medical treatment device is viewable at a respective separate display interface of the mobile device. In another example, case information for different medical treatment devices can be combined within the same display interface screen at the mobile device 105. In this way, the mobile device user may customize the case information displayed at the mobile device 105 as received from one or more of the medical device(s) 180.

The data provided in the example of the combined device view window shown in FIG.13F includes data gathered by the ventilation system 280 by the patient monitor/defibrillator 285. For example, ventilation system 280 may provide the port information 1371, the fraction of inspired oxygen (FiO2) data 1372, the peak inspiratory pressure (PIP) 1373, the tidal volume (VT) 1374, and the breaths per minute (BPM) 1375. Additionally, the ventilation system 280 may provide the airway pressure waveform (PAW) 1376. A device view window dedicated to data gathered by the patient monitor/defibrillator 285 may not include these values. The patient monitor/defibrillator 285 may provide the HR 1380, the SpO2 value 1381, the invasive blood pressure (IBP) 1382, the NIBP 1383, the EtCO2 1384, the ECG waveform 1385, the SpO2 waveform 1386, the EtCO2 waveform 1387, and the IBP waveform 1388.

The patient monitor/defibrillator 285 or the ventilation system 280 may provide the temperature data 1391. One or more of the patient monitor/defibrillator 285 and the ventilation system 280 may provide alarms 1392.

Referring to FIGS. 14A and 14B, examples of working view windows at the mobile device are shown. The working view window 1415 may be accessed via the working view tab 1340 on the mobile device 105 and may provide a presentation of the data available on the medical device display in a manner that differs from that of the medical device display. As an example, the working view window 1415 may provide a different collection of data, a different arrangement of data, instructions and options not available on the device display, etc. and their view may be customized to the user of the mobile device and/or customized based on the status of the patient. In an implementation, the tab 1340 may be referred to based on a device nomenclature. As examples, if the mobile device is a tablet, this tab may be labeled a "tablet view," if the mobile device is a smartphone, this tab may be labeled a "phone view," if the mobile device is a headset, this tab may be labeled a "headset view," etc. The working view windows 1415 may display different data than the device view window 1315 and/or 1316. The device view window 1315 and/or 1316 provides a view of all of the information on the medical device screen 310 at the mobile device 105 such that a rescuer may use the device view windows interchangeably with the medical device screen. Rescuers and other medical team members may also wish to view additional information than what is provided in the device view window 1315 and/or 1316. In some implementations, the mobile device may also display, in a second display view, referred to as the working view window 1415 that is separate from the device view window 1315 and/or 1316 and accessible to the user on the same mobile device 105 (e.g., via the selection tab 1340 or other user input selection), one or more patient data dashboards customized to preferences or treatment roles of a user of the mobile device. In some examples, the data dashboards display physiological sensor data such as ECG waveforms, pulse oximetry waveforms, and CO2 waveforms. In some examples, the pulse oximetry waveforms can include waveforms for one or more of peripheral capillary oxygen saturation (SpO2), carbon monoxide saturation (SpCO), methemoglobin (SpMet), total hemoglobin (SpHB), blood oxygen content (SpOC), pleth variability index (PVI), or perfusion index (PI). The working view window 1415, in some examples, can be a scrollable display window 1460, operable via a scrolling gesture 1462, that includes data dashboards displaying treatment-based sensor data associated with treatment devices or methods instead of or in addition to the information displayed within the device view. For example, a CPR data dashboard 1470 can display real-time CPR information, for example, including chest compression depth and chest compression rate. The displayed CPR information may provide real-time feedback to a rescuer delivering CPR chest compressions to a patient regarding whether the depth of each chest compression is within a target range and a target compression rate. In some cases, a rescue team member providing CPR chest compressions to a patient may select to have the chest compression dashboard displayed more prominently than other dashboards within the working view to more easily view feedback associated with administration of CPR chest compressions. Similarly, in some embodiments, a ventilation dashboard 1472 may display real-time ventilation information including, in some examples, ventilation rate, tidal volume, and minute volume. In some situations, a rescuer providing ventilation to a patient may select to have the ventilation dashboard displayed within the working view to more easily view feedback associated with providing patient ventilation. The mobile device 105 may enable user(s) to add or remove any dashboards to suit their viewing preferences. In some cases, the working view may be created and customized ad hoc during medical treatment by controls that allow the viewer of the mobile device 105 to format the medical device data as they wish.

As shown, for example in FIG. 14B, in an implementation, the working view window 1415 may include one or more medical device operation parameter indicators (e.g., defibrillation shock energy indicators 1405a and 1405b), a scrolling alarm indicator 1410, a notification banner window 1450, and the connected device window 1420 discussed above. In an implementation, the alarm indicator 1410 may indicate the one or more parameters that have crossed an alarm threshold. For example, in the case of ARF, EtCO2 may have exceeded an alarm threshold and SpO2 may have dropped below an alarm threshold. Thus, the indicator 1410 may alternate between displaying "EtCO2 high alarm limit" and "SpO2 low alarm limit. The working view window 1415 may further include the connected devices window 1420. The connection devices window 1420 may include one or more graphic images (e.g., 1401a and 1401b) that identify the medical device(s) 180 coupled to the mobile device 105. The CSG engine 120 may populate this window automatically based on detected connections. In an implementation, a user may tap or verbally invoke an add device control 1402 to provide identification information for a connected medical device. The notification banner window 1450 may provide notifications for the user. For example, notifications may include information regarding the monitored parameters, the clinical interventions, operations and/or assembly of medical devices, and user options for control of the working view 1415 and/or the CSG UI 110.

In addition to or as an alternative to any of the elements shown in FIGS. 14A and 14B, the working view windows 1415 may provide one or more elements of the user interface 2760 as shown in FIG. 27C. In an effort to reduce the size and weight of the medical treatment device(s) 180 , and in particular of the patient monitor/defibrillator 285, to enhance portability and usability, the size(s) of the information display screen(s) on the medical devices (e.g., the display screen 310 of the patient monitor/defibrillator 285 as shown in FIG. 13B is/are often reduced to the point where the display area is too small to concurrently display all medical device data, including the sensor information gathered from the patient as well as all the information gathered about diagnostics, treatment, device performance, caregiver performance, etc. In fact, in many cases while a patient monitor/defibrillator may only be a volume of less than 0.5 ft.³ and a display size of just 8.5 diagonal, the amount of information gathered and generated by the patient monitor/defibrillator at any one time would easily fill an entire display screen of 36" diagonal or more. This presents a dilemma for the caregiver using the medical device: the choice of what information to display is oftentimes difficult and displaying one particular parameter comes at the expense of losing visibility of another parameter. In addition, information that is not visible at a particular time during patient care may result in ineffective or harmful interventions and medical treatments for the patient. The working view window 1415 provides a page with free of the device display area limitations. The user may customize the information shown in the working view window 1415 and this window is a scrolling window 1460 thereby increasing the available display area. Additionally, the user may toggle between the device view window 1315 and/or 1316 and the working view window 1415.

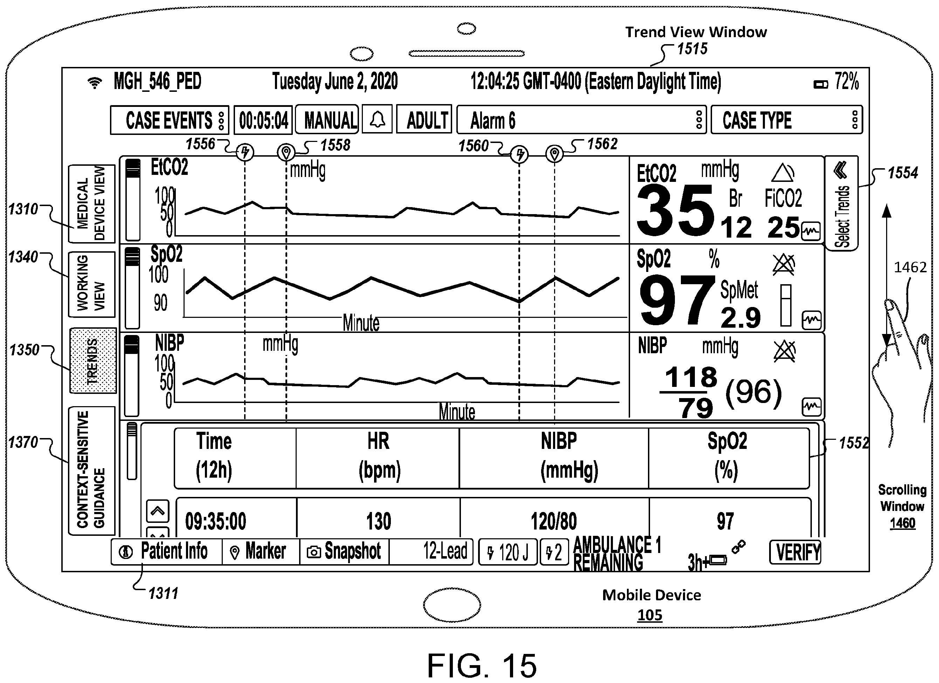

Referring to FIG. 15, an example of a trend view window at the mobile device is shown. In some implementations, the mobile device 105 may provide a trend view window 1515 in response to a user selection of the trends tab 1350. In addition to or as an alternative to any of the elements shown in FIG. 15, the trend view window 1515 may provide one or more elements of the user interface 2760 as shown in FIG. 27C. In some implementations, the mobile device 105 may allow the user to customize the trends that are displayed within the display interface. In an implementation, the trend view window is configured to provide user-customizable data trend information for one or more physiologic parameters received at the mobile device from one or more communicatively coupled medical devices. For example, upon selecting the trends tab 1350, the mobile device 105 may display a list of one or more trends for the user to select and/or de-select based upon trend viewing preferences. The trends view window 1515 may display physiological sensor value trends (e.g., SpO2 1540, EtCO2 1538, NIBP 1542) over time during the medical event. In some cases, the trend data can be displayed in a graphical or tabular format. The trends view window 1515 may be a scrolling window 1460 so that users can access a larger set of data with a scrolling gesture 1462. The trend view window 1515 may also include discrete data values. In one example, user can select for display waveforms and/or mean values for one or more of HR, pulse rate (PR), SpO2, NIBP, invasive blood pressure (systolic BP, diastolic BP, mean arterial pressure), EtCO2, respiratory rate/breathing rate (RR/BR), pleth variability index (PVI). Additionally, the user can select whether to display data table 1552. In some embodiments, the user can also select a time interval for recording trend data that is displayed within the trend view 1515, such as within data table 1552. In some implementations, inputs provided at the trend selection tab 1554 can also allow to select the predetermined time interval e.g., every 10 seconds, 20 seconds, 30 seconds, 1 minute, 3 minutes, 5 minutes) for recording and/or displaying trend data in the trends view UI screen 1515.

In an implementation, the trends view window 1515 may include event marker annotations 1556, 1558, 1560, 1562 overlaid on the trend plots. These annotations enable the user to readily discern an impact of a respective intervention or treatment on a patient's condition over the course of time. In an implementation, the medical device(s) 180 may automatically record event markers for various treatments, interventions, or other activities during patient care provided to the patient or caregivers may manually input event marker data at the medical device(s) 180. Event markers may indicate activities such as, for example, administration of a particular drug, placement of an IV, oxygen administration, detection of a shockable rhythm, application of electric shock to the patient, initiation of chest compressions, initiation of ventilations, etc.

In an implementation, caregivers using the mobile device 105 may manually input event marker data at the mobile device 105 that correspond to certain types of medical interventions (e.g., administration of oxygen or medication). In some examples, upon selection of the trends view selector 1350, the mobile device 105 may transmit a data request, alone or as part of a bulk data transfer request, for event marker data to display overlaid on trend graphs 1538, 1540, 1542. Upon selection of one of the event marker annotations 1556, 1558, 1560, 1562, the mobile device 105 may cause display of details about the selected event marker, such as amount of energy in an applied shock, amount of medication administered, display screen of defibrillator waveforms and/or ventilation data, and/or a snapshot view of data from the display interface at the medical device(s) 180 and/or the device view 1315 and/or 1316 at the time associated with the selected event marker.

In some implementations, multiple caregivers may provide care for the victim 101. For example, after viewing the working view and device view, the caregiver 104 (e.g., on scene but not directly administering care to the patient) and/or the remote caregiver 303 may deem a particular piece of medical device data to be relevant to the decision by the caregiver 103 (e.g., on scene and directly administering care to the patient). In some scenarios, the caregiver 103 view a display at the medical device(s) 180 but not a mobile device 105. In such a scenario, the caregiver 104 and/or the remote caregiver 303 may send an instruction from the mobile device 105 to the medical device(s) 180 to effectuate a change to its operation and/or a change to the data display at the medical device(s) 180. For example, the instruction may include a command to initiate a blood pressure reading via an oscillometric blood pressure cuff, if the supervisor notices that the time duration since the last blood pressure reading has been exceeded. As another example, a 12-lead may be initiated from the mobile device 105. As a further example, when treating a patient presenting with RD, the caregiver 103 may have made the choice to not include either the capnography waveform or EtCO2 values on a display screen of the medical device(s) 180. The caregiver 104 and/or 303 may see in the working view that the capnography waveform is indicative of an obstructive lung condition and toggles over to the device view and sees that the capnography information is not being displayed (upon which the caregiver 103 is basing their potentially flawed medical decisions). Based on this, the caregiver 104 and/or 303 may initiate an instruction from the mobile device 105 to the medical device(s) 180 to alter the display format and display the capnography information. Alternatively, the instruction may take the form of a request to alter the display format. The request may be in the form of a text request to the caregiver operating the medical treatment device (e.g. "Important patient info: please display capnograph").

Referring to FIGS. 16, an example a CSG user interface provided at the mobile device is shown. The design or layout of the CSG UI 110 and the constituent screens as illustrated herein are examples only and not limiting of the disclosure. The CSG UI 110 and the constituent screens include various interaction elements (e.g., entry fields, selectable menus, buttons, arrows, checkboxes, soft-keys, sliders, etc.) and associated displayed instructions to prompt the caregiver for a user entry of information... The mobile device 105 may provide the user-entered information as the second input 230 to the CSG engine 120. Additionally, the CSG UI 110 and the constituent screens provide information included in the second output 225. The second output 225 includes information from the CSG engine 120 provided to the mobile device 105 for display at the CSG UI 110. Additionally, the CSG UI 110 may be a touch-activated interface controlled by the user via one of more touchscreen gestures 1698 and may include a scrolling window as described in regard to other windows at the mobile device 105.

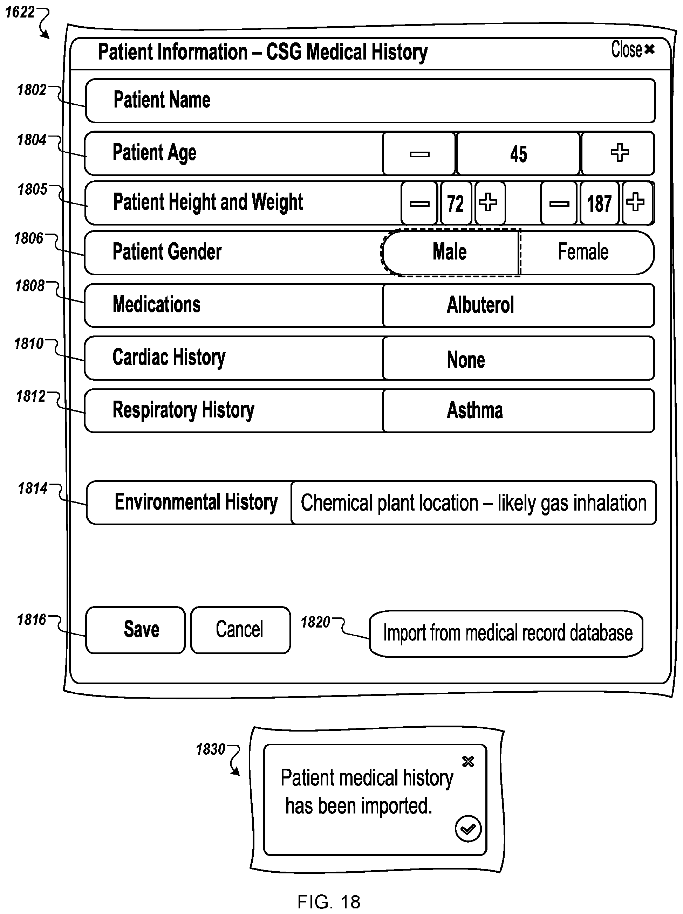

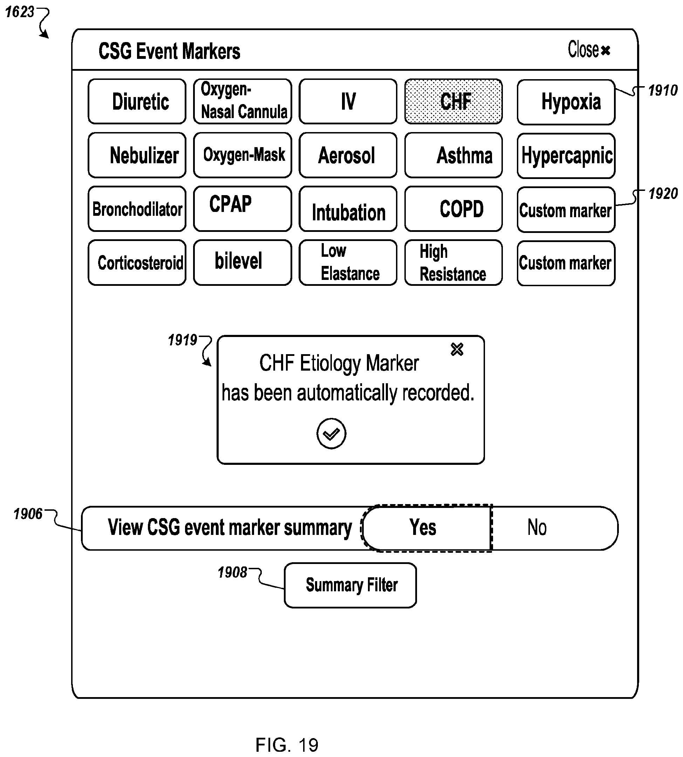

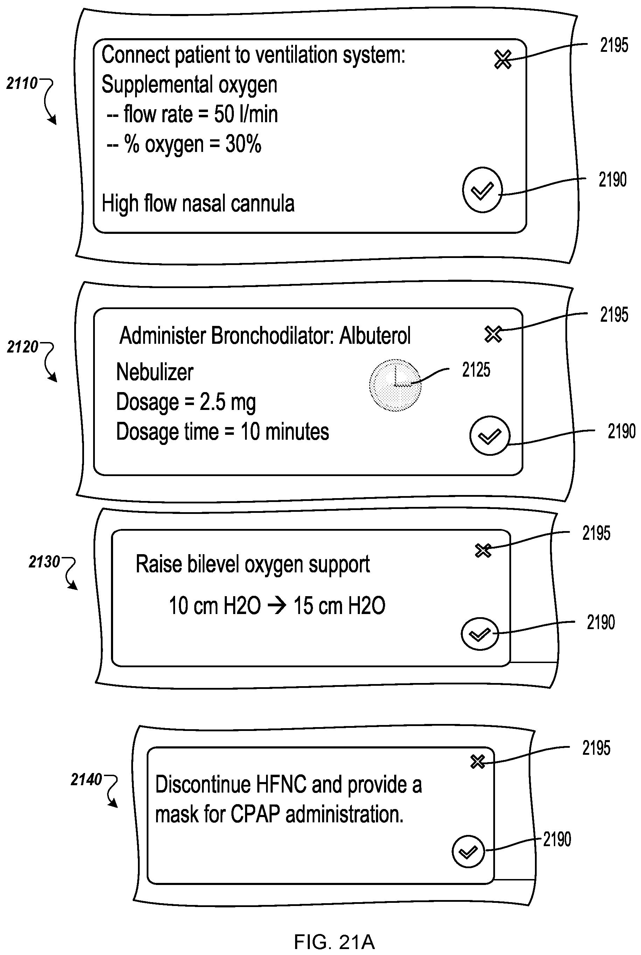

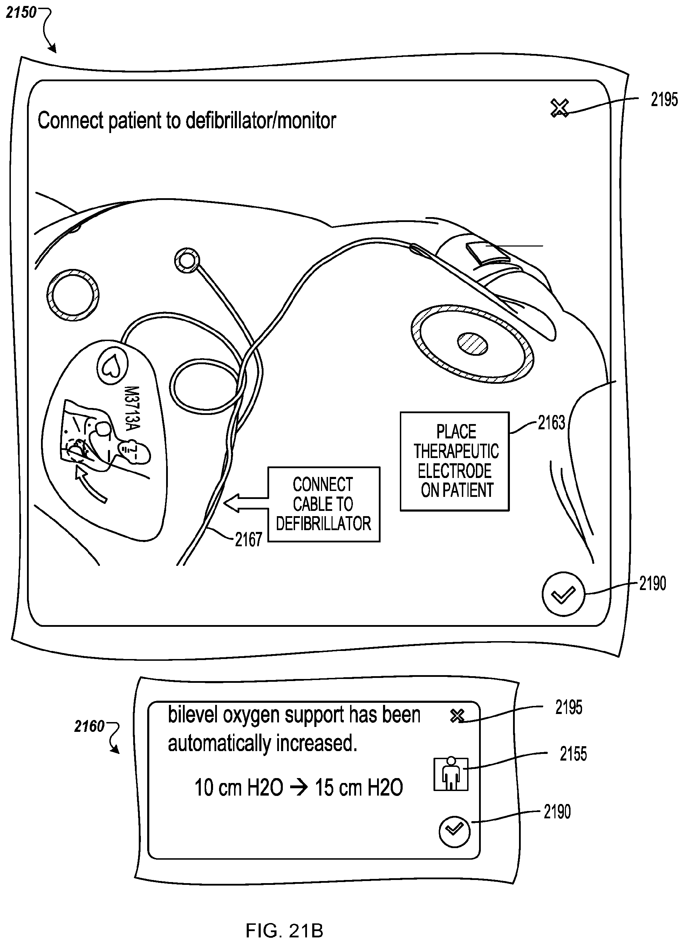

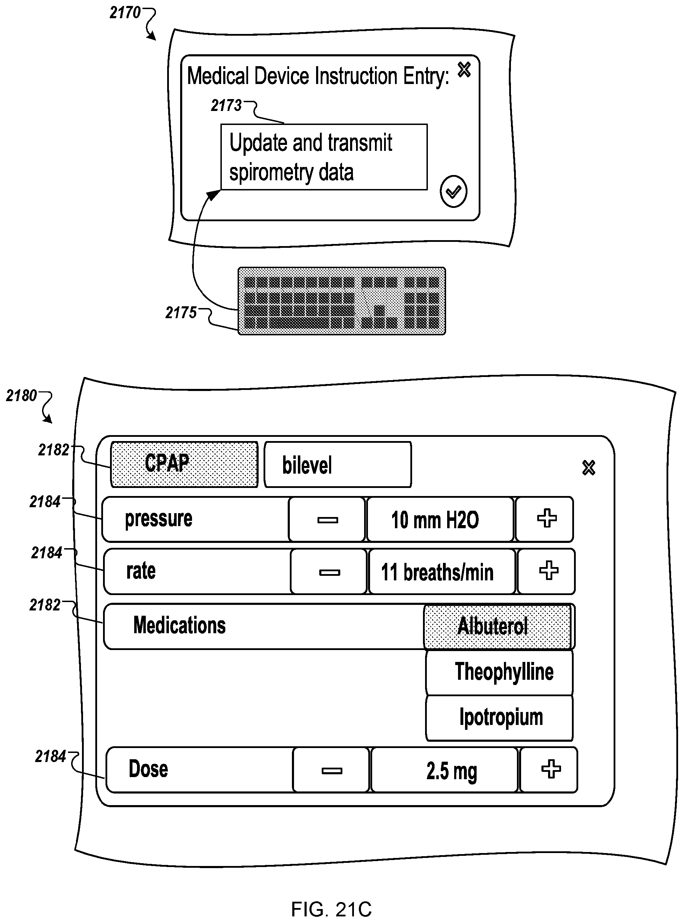

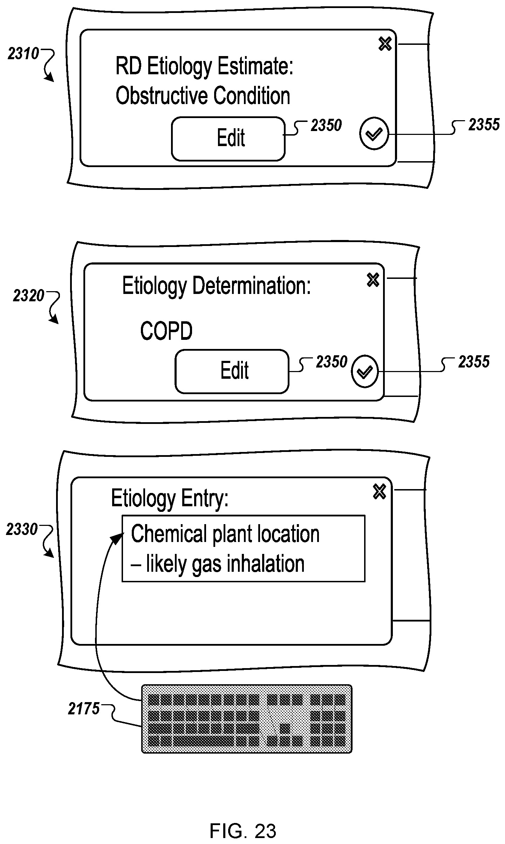

The user may access the CSG user interface 110 at the mobile device 105 via the context-sensitive guidance (CSG) tab 1370. The CSG user interface 110 may provide an audio input control 1610, a scanner control 1615, a patient assessment screen 1621 (e.g., as exemplified in FIG. 17), a patient medical history screen 1622 (e.g., as exemplified in FIG. 18), a CSG event marker screen 1623 (e.g., as exemplified in FIGS. 19 and 20), and a clinical interventions screen 1624 (e.g., as exemplified in FIGS. 21A, 21B, and 21C), a medical device data screen 1660 (e.g., as exemplified in FIGS. 22A and 22B), and an etiology information screen 1670 (e.g., as exemplified in FIG. 23).

The audio input control 1610 may enable voice input of information from the user, as detected by the audio sensor 1699, or microphone, at the mobile device 105. In lieu of or in addition to the menu or keyboard entries illustrated herein for user entry of information, the CSG UI 110 may capture voice information and convert this information to text entries. In some implementations, the voice information may include annotations for event marker data.

The scanner control 1615 may enable the mobile device 105 to capture information for the CSG UI 110 via a barcode scan, a QR code scan and/or a biometric scan. In one example, the user may activate the scanner control 1615 to scan a barcode of a medication being administered to the patient, and the mobile device 105 may convert the scanned identification code into medication type and/or dosage. In other examples, the user may activate the scanner control 1615 to scan demographic information, payment information, medical device identification codes, caregiver identification codes, patient and/or caregiver biometrics, etc.

The alarms control 1618 may enable the caregiver to view alarms at the CSG UI 110. In an implementation, an activation of the alarms control 1618 may enable display of alarms in an alarm window 1619. In an implementation, the CSG engine 120 activates the alarms control 1618 as a default operating condition. In an implementation, the CSG engine 120 may cause the CSG UI 110 to display the alarm window 1619 together with one or more of the other screens. In this manner, a caregiver may always have access to the alarm information during care of a patient.

Referring to FIG. 17, with further reference to FIG. 16, an example of a patient assessment screen is illustrated. The patient assessment screen 1621 provides an example of a RD presentation assessment. The caregiver may access the screen 1621 at the stage 610 of the CSG initialization algorithm 411 as shown in FIG. 6. The RD presentation assessment is an example only and not limiting of the disclosure. For example, in various implementations, the patient assessment screen 1621 may provide entry fields for information from caregiver assessments of one or more of airway, breathing, circulation, palliative or precipitating factors, quality of pain, region or radiation of pain, severity of pain, temporal nature of pain, mental status evaluation, circumstantial or environmental information, other visual and/or tactile evaluations of the victim by the caregiver, other caregiver assessments based on communication with the patient, etc.

In the RD example of FIG. 17, the assessment screen 1621 includes toggle switches 1710 for patient breathing and toggle switches 1720 for patient difficulty breathing. The toggle switches are examples only and not limiting of the disclosure. For example, the fields may accommodate typed entry, menu selection, and other user interaction elements or activation elements (e.g., buttons, arrows, checkboxes, sliders, etc.). The screen 1621 may further include an activation element 1730 to save or cancel user entries and an activation element 1740 to confirm user entries at the assessment screen 1621.

Referring to FIG. 18, with further reference to FIG. 16, an example of a patient medical history screen is illustrated. For example, the patient medical history entry screen 1622 at the CSG UI 110 in may include input fields for one or more of a patient name 1802 and/or patient identification code, a patient age 1804, a patient gender 1806, a patient height and/or weight 1805, and/or other patient medical and/or demographic information. When the medical devices 180 include a ventilation system 280, patient height, gender, and/or weight may determine ventilation volume and rate for administering oxygen and respiratory interventions to the patient. The patient medical information may include medications 1808, cardiac history 1810, respiratory history 1812, and/or environmental history 1814.

In an implementation, the patient medical history entry interface 1622 may include a save control 1816 and/or a medical record database import control 1820. The medical record database import control 1820 may be configured to import patient medical history data from a PCR file stored at the mobile device 105 and/or stored in one or more medical record databases 399. Additionally or alternatively, the medical record database import control 1820 may enable the import of patient medical history data from a hospital electronic health record (EHR) stored at the one or more medical record databases 399. In an implementation, the medical record databases 399 may include a health information exchange (HIE) and the medical record database import control 1820 may enable the import of patient medical history data from the HIE.

In an implementation, the screen 1622 may include a confirmation control 1830 to capture user confirmation of a medical history import. The screen 1622 may provide the confirmation control 1830 in response to a user request via the control 1820. Alternatively, the CSG engine 120 may automatically import patient medical history from another file on the mobile device (e.g., an electronic patient chart from the patient charting application 131), the medical device(s), and/or the medical database(s) 399. For example, the CSG engine 120 may automatically import medical history data at one of the stages 1052b or 1182b. The CSG UI 110 may provide the confirmation control 1830 to inform the user of the automatic import and capture a user confirmation to indicate that the user is aware of this import.

In some examples, a supervisor or other caregiver that is not actively hands on providing direct patient care may have more flexibility to attend to administrative items like inputting patient information, viewing information streaming in real time from the medical treatment device (e.g., defibrillator, patient monitor, the ventilation system 280) that may or may not be immediately provided on the screen of the medical treatment device, and/or documenting other aspects of the medical event. Additionally, inputting patient information at an input interface at the medical device(s) 180 may be cumbersome and tedious. Therefore, providing the patient information input interface at the mobile device frees up caregivers to better pay attention to their assigned tasks without getting tied up trying to tediously input patient information at the input interface of the medical treatment device.

Referring to FIG. 19, with further reference to FIG. 16, an example of a CSG event marker screen is illustrated. The CSG event marker screen 1623 enables a user selection of one or more event marker controls 1910 to input CSG event markers at the mobile device. Allowing caregivers to input event marker information at the mobile device 105 may free up other caregivers who are providing direct patient care (e.g., CPR, ventilation, or shocks) to focus on their respective tasks without having to stop to input event marker information. In some implementations, in response to selection of CSG event markers selector 1623, the mobile device 105 displays the event marker input UI screen 1623 that allows the user of the mobile device 105 to input CSG event marker information at the mobile device 105. In some examples, the medical device(s) 180 can automatically record event markers for events that the medical device(s) 180 can automatically detect, such as identification of a shockable ECG rhythm, administration of a defibrillation shock to the patient, and/or starting chest compressions and/or ventilations. However, other treatment measures that are not detectable by the medical device(s) 180 can have an impact on patient response indicated in the waveforms, metric values, and trends displayed at the medical device(s) 180 and in the display views at the mobile device 105. For example, as shown in trends view UI screen 1515 (FIG. 15), visual indications of event markers 1556, 1558, 1560, 1562 are overlaid on trend graphs 1538, 1540, 1542 to provide additional context regarding patient response to a treatment associated with the respective marker. Therefore, providing event markers indicating when certain interventions such as medications or therapeutic measures provided by the medical device(s) and/or the caregivers have been taken can provide context to caregivers monitoring patient response during a medical event and can also enhance post-event debriefing and analysis.

In one example, when an intervention is provided to the patient, the caregiver can select the event marker screen 1623 to select one or more event markers to indicate a type of provided intervention. For example, the intervention may be pharmacological (e.g., a diuretic, a nebulizer, a bronchodilator, a corticosteroid, etc.). In some examples, the caregiver can also provide amplifying information such as time of administration, dosage, type of drug delivery (e.g., IV, aerosol), etc. Additionally, the caregiver may provide markers for etiology estimates (e.g., estimates of CHF, asthma, COPD) and/or for a respiratory intervention such as a nasal cannula, a mask, or an intubation.

In an implementation, the CSG event markers may include markers for physiological evaluation such as hypoxia, hypercapnic, a lung elastance evaluation, and/or a lung resistance evaluation. Recordation of the physiological evaluations based upon which the CSG engine 120 determines appropriate interventions provide a record of the initial care of the patient which may enable subsequent diagnosis of an etiology and treatment. The interventions provided before and after these evaluations and the outcomes of these interventions may confirm or provide support for a particular diagnosis or treatment recommendation.

In some examples, in addition to the examples of preset event markers discussed above, the event marker input UI screen 1623 can also include one or more customizable event markers 1920 that allow mobile device users to add additional and customizable event markers to further enhance a caregiver's ability to add meaningful annotations to the case information displayed at the medical device(s) and/or the mobile device 105.

In some examples, upon detecting that CSG event marker information has been submitted at the event marker input UI screen 1623, then the mobile device 105 transmits an instruction signal (e.g., the CSG output to medical device(s) 260) to the medical device(s) 180 with the submitted event marker information and instructs the medical device(s) 180 to link and store the submitted event marker information with the respective case information in the patient encounter data file 188 stored at the medical device(s) 180 and/or the medical device data files 395 created at the medical device(s) 180 and sent to the cloud server(s) 398. Accordingly, the treatment record may be stored on the medical device(s) 180, without requiring storage thereof on the mobile device. Alternatively, in some cases, the instruction to log the event marker may be sent from the mobile device not only to the medical treatment device, but also a cloud server that stores the treatment record. Alternatively, the cloud server may poll the medical treatment device for the most current treatment record without requiring direct communication between the cloud server and the mobile device. It may be preferable for the medical treatment device to be operated only by the person immediately using it without interference, instead of having others move or otherwise engage with it. Further, as the code progresses, the caregiver 103 may want to actively look back within the existing case to see events of interest, such as lung mechanics and/or respiratory gas status or what the effect of certain vital signs were once medications were delivered.

In some aspects, once the event marker information has been saved, the medical device(s) 180 send a confirmation signal to the mobile device 105 indicating that the event marker information has been saved. In response to receiving the confirmation signal, in some implementations the mobile device 105 may display a confirmation message 1919 at the CSG UI 110 confirming that the submitted event marker information has been linked to the respective case information and updated at the medical device(s) 180. In some examples, the medical device(s) 180 may send confirmation signals to the mobile device 105 both when linking and saving of the event marker information has commenced and concluded. In such an example, separate confirmation messages may be displayed to confirm that a submitted event marker is being recorded (linked and saved) and that recording/saving of the submitted event marker has been completed. The confirmation message 1919 may be beneficial to put the caregiver at ease (during a stressful situation) that the event marker is indeed being recorded.

In an implementation, the event marker screen 1623 may include an event marker summary control 1906. The event marker summary control 1906 may enable the user to request an event marker summary screen, for example the screen 2042 discussed below with regard to FIG. 20). The CSG UI 110 may also provide an event filter selector 1908 that causes the CSG engine 120 to filter the events presented in the screen 2042 to only the types of events the mobile device user wishes to see. For example, filter types may include the categories of event markers exemplified in FIG. 19 and/or subsets of these categories (e.g., pharmacological treatments, respiratory treatments, hemodynamic treatments, manual patient assessments, automated patient assessments, respiratory etiologies, hemodynamic etiologies, respiratory physiological sensor evaluations, hemodynamic physiological sensor evaluations, and/or combinations thereof). The event markers may further include alarms and/or medical history information.

Referring to FIG. 20, with further reference to FIG. 16, an example of an event summary screen is illustrated. In response to receiving an event summary request, for example via a user selection of the event summary control 1695, the mobile device 105 may display the event summary screen 2732. The event summary screen 2732 that presents one or more time-stamped events that have occurred over the course of treating a patient. In some examples, the displayed event markers may include, for example, patient assessment 2062, a clinical intervention 2060, a physiologic data evaluation 2058, an etiology estimate 2056, and/or an etiology determination 2054. In some examples, the listed events may be color-coded based on event type.

Referring to FIGS. 21A, 21B, and 21C with further reference to FIG. 16, examples of various clinical intervention screens are shown. The clinical intervention screen 1624 may provide one or more of the example screens 2110, 2120, 2130, 2140, 2150, 2160.
The clinical intervention screen 1624 may provide intervention instructions generated by the CSG engine 120 (e.g., the screens 2110, 2120, 2130, 2140, and 2150), intervention notifications generated by the CSG engine 120 (e.g., the screen 2160) and/or prompt the user for entry of clinical intervention instructions (e.g., the screens 2170 and 2180). One or more of these screens may include a user confirmation control 2190 and/or a user cancellation control 2195. In an implementation, the CSG engine 120 may generate a CSG event marker for the clinical intervention in response to activation of the user confirmation control 2190 and/or in response to activation of the user cancellation control 2195.

In an implementation, activation of the user confirmation control 2190 may provide user confirmation that a particular intervention has been administered by the user. In this case, activation of the user cancellation control 2195 may either clear the screen or may reject the clinical intervention.

In an implementation, activation of the user confirmation control 2190 may enable the CSG engine 120 to automatically control the medical device(s) 180 to provide the intervention. In this case, activation of the user cancellation control 2195 may either clear the screen or may reject the clinical intervention.

In an implementation, activation of the user confirmation control 2190 may indicate a user acknowledgement of intervention automatically implemented by the CSG engine 120 at the medical device(s) without affirmative permission from the caregiver. In this case, activation of the user cancellation control 2195 may clear the screen.

As illustrated in the example screen 2110, the clinical intervention screen 1624 may specify equipment (e.g., high flow nasal cannula). Further, the intervention instructions may specify operational parameters for the equipment (e.g., flow rate, oxygen percentage).

As illustrated in the example screen 2120, the clinical intervention screen 1624 may specify a pharmacologic intervention (e.g., albuterol) and a delivery system (e.g., nebulizer). In an implementation, the clinical intervention screen 1624 may specify a dosage amount and a dosage delivery time. In an implementation, the clinical intervention screen 1624 may include a timer, e.g., the timer 2125. The timer 2125 may be a user controlled timer or a timer automatically controlled by the CSG UI 110. The timer 2125 may generate an alarm to remind a user to repeat, modify, or cease an intervention.

As illustrated in the example screens 2130 and 2140, the clinical intervention screen 1624 may specify a change to a clinical intervention to be implemented by the user. For example, as shown in screen 2130, the clinical intervention screen 1624 may specify a change in the operational parameters for a particular intervention. As another example, as shown in screen 2140, the clinical intervention screen 1624 may specify a change from a first intervention to a second and different intervention.

As illustrated in the example screen 2150, the clinical intervention screen 1624 may guide the caregiver in implementing the clinical intervention. For example, the clinical intervention screen 1624 may include guidance instructions 2163 and/or guidance graphics 2167 for the placement, insertion, and/or other activities involved in the clinical intervention.

As illustrated in the example screen 2160, the clinical intervention screen 1624 may provide notification of an intervention that has been automatically implemented by the CSG engine 120. In an implementation, the clinical intervention screen 1624 may include a manual override control 2155. Activation of the manual override control may initiate the clinical interventions control 1624 to enable control of the medical device(s) by the CSG engine 120 by user instructions provided at the CSG UI 110.

As illustrated in the example screen 2170, the clinical intervention screen 1624 may capture user input of a clinical intervention in user fillable field 2173. For example, the user may manipulate a keyboard 2175 or other data entry device to provide enter instructions into the user fillable field 2173. The CSG engine 120 may control the medical device(s) 180 to perform the clinical intervention based on the user instructions captured at the user fillable field 2173.

As illustrated in the example screen 2170, the clinical intervention screen 1624 may capture menu driven user input. For example, the user may select a clinical intervention from one or more clinical intervention menus 2182. Additionally, the user may select operational parameters for the clinical intervention from one or more operational parameter menus 2184. The CSG engine 120 may control the medical device(s) 180 to perform the clinical intervention based on the user instructions captured via the menu driven user input.

Referring to FIGS. 22A and 22B, with further reference to FIG. 16, an example of a medical device data screen is shown. The medical device data screen 1660 may be a scrollable screen. This is illustrated schematically in FIGS. 22A and 22B where FIG. 22B shows a portion of the screen 1660 below the portion shown in FIG. 22A. The medical device data screen 1660 may be a scrolling window so that users can access a larger set of data with a scrolling gesture 2205. In some implementations, the mobile device 105 may allow the user to customize the data displayed at the medical device data screen 1660. For example, upon selecting the medical device data tab 1660, the mobile device 105 may display a list of available medical device data for the user to select and/or de-select based upon viewing preferences. In some cases, the data screen 1660 may be created and customized ad hoc during medical treatment by controls that allow the viewer of the mobile device 105 to format the medical device data as they wish. In an implementation, the CSG engine 120 may pre-customize the data screen 1660 based on preferences or treatment roles of a user of the mobile device

In an implementation, the medical device data screen 1660 may display physiologic sensor data received from the medical device(s) 180 (e.g., at the stages 503, 515, and/or 547 of the method 500, at the stages 760, 818, 1051, and 1181 of the R/V CSG engine 403, and/or the stage 1225 of the hemodynamic CSG engine 404). The medical device data screen 1660 may display this data as trends and/or waveforms (e.g., EtCO2 2238, SpO2 2240, airway data 2242, NIBP 2244, heart rate 2246, and ECG 2248) over time during the patient encounter. In some cases, the medical device data screen 1660 may display the physiologic sensor data as discrete values in a numerical format (e.g., EtCO2 2250, SpO2 2252, NIBP 2254, and HR 2256) and/or in a graphical format (e.g., EtCO2 2260, SpO2 2261, lung resistance 2262, lung elastance 2263, NIBP 2264, and HR 2265).

In an implementation the display of the physiologic data may include one or more event markers (e.g., the markers 2290, 2292, 2294, and 2296) overlaid on the physiologic data and/or in another graphical or tabular format. In an implementation, caregivers may manually input event marker data at the mobile device 105 and on the data screen 1660 that correspond to certain types of medical interventions (e.g., administration of oxygen or medication). In some examples, upon selection of the medical device data screen 1660, the mobile device 105 may transmit a data request, alone or as part of a bulk data transfer request, for event marker data to display on the data screen 1660. Upon selection of one of the event marker annotations 2290, 2292, 2294, and 2296 the mobile device 105 may cause display of details about the selected event marker, such as amount of energy in an applied shock, amount of medication administered, display screen of defibrillator waveforms and/or ventilation data, and/or a snapshot view of data from the display interface at the medical device(s) 180 and/or the device view 1315 and/or 1316 at the time associated with the selected event marker
In an implementation, the medical device data screen 1660 may display the evaluations of the physiologic sensor data by the CSG engine 120 (e.g., at the stages 506 and 518 of the method 500, at the stages 710, 720, 730, 740, 820, 830, 840, and 850 of the R/V CSG engine 403, and/or the stage 1230 of the hemodynamic CSG engine 404). The data screen 1660 may provide these evaluations graphically. For example, ranges for the various indicators may be indicated as low, normal, or high with a graphical distinction as shown schematically with various crosshatch patterns (e.g., patterns 2276, 2277, and 2278), colors, amount of fill of a graphic symbol, etc. In an implementation, the data screen 1660 may provide the evaluation of the physiologic signal with an evaluation indictor 2279 shown, for example, as an arrow 2279 positioned relative to the graphically provided ranges. Alternatively or additionally, the medical device data screen 1660 may provide textual indications of the evaluation of the physiologic signal (e.g., the evaluation indications 2270, 2271, 2272, 2273, 2274, and 2275).

As shown in FIG. 22B, the medical device data screen 1660 may provide a medical device status window 2280. The CSG engine 120 may receive medical device status information as a first input 240 from the medical devices. The CSG engine 120 may receive this information automatically according to a predetermined schedule, in response to a detected clinical event, and/or in response to a query or instruction from the CSG engine 120 to the medical device(s) 180 (e.g., as a first output 260 to the medical device(s) 180).

Referring to FIG. 23, with further reference to FIG. 16, examples of an etiology information screen are shown. In the example screen 2310, the etiology information screen 1670 provides an etiology estimate (e.g., at the stage 545 of the method 500 and/or at the stages 1050 or 1180 of the R/V CSG engine 403). In the example screen 2320, the etiology information screen 1670 provides an etiology determination (e.g., at the stage 557 of the method 500 and/or at the stages 1053 or 1183 of the R/V CSG engine 403). One or both of the example screens 2310 and 2320 may include an edit control 2350 and/or a confirmation control 2355. The example screens 2310 and 2320 may provide an etiology estimate or etiology determination as generated by the CSG engine 120. The user of the mobile device 105 may confirm the etiology estimate or etiology determination via an activation of the confirmation control 2355. The user confirmation may merely acknowledge the etiology estimate or determination. The CSG engine 120 may operate in a same way regardless of the user confirmation. Alternatively, the CSG engine 120 may provide the etiology estimate or determination as a candidate subject to confirmation by the user. In the latter case, the user confirmation may modify the operation of the CSG engine 120. In this case, the screens 2310 and/or 2320 may include the edit control 2350. In an implementation, the CSG engine 120 may generate the etiology estimate or determination as a candidate and provide the edit control 2350 so that the user may reject the candidate and provide a user entered etiology estimate or determination. In an implementation, the CSG engine 120 may prompt the user for an etiology estimate or determination (e.g., at the example screen 2330). The example screen 2330 may provide a fillable field (e.g., configured to capture information entered by the user via a data entry mechanism, such as a keyboard 2175, or by a menu entry).

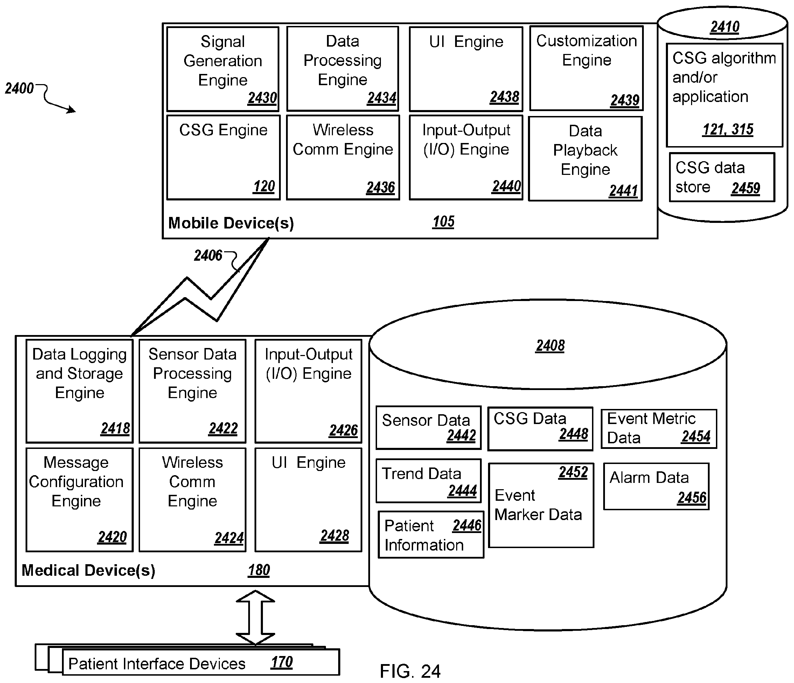

Referring to FIG. 24, an example environment for implementing CSG at a customizable mobile device is shown. This environment includes a medical treatment system with a set of devices for providing treatment and/or interventions to the patient during a medical event. The system may include the medical device(s) 180 and one or more mobile devices 105 communicatively coupled to the medical device(s) 180 via communication link 2406. The communication link 2406 may include the communication link 199 shown in FIG. 1 and/or the communication link 397 shown in FIG. 3A. The devices 105, 180, in the illustrated example, may be co-located at a patient care site. In other implementations, one or more mobile devices 105b may be located remotely from the patient care site as illustrated in FIGS. 3A-3D by the remote location 389 of a caregiver 303 with the mobile device 105b. The devices may be configured for data communication in a wired or wireless manner for transferring information between certain devices 105, 180 of the system during and/or subsequent to delivery of therapy and other interventions.

In some implementations, the wireless communication link 2406 for connecting the medical device(s) 180 and the mobile device 105, in some examples, may be a Wi-Fi network, other short-range wireless communication network or near field communication (NFC) network, local area network (LAN), wide area network (WAN), or the Internet. In other examples, the devices 105, 180 can be configured to communicate over longer communications ranges such as over a cellular communication network. In some implementations, the medical device(s) 180 may function as a wireless access point to provide a direct wireless connection with the mobile device 105. In other examples, the wireless communication link 2406 can be provided via Bluetooth personal area network.

In some embodiments, different devices 105, 180 may be configured to communicate with one another over different types of communication links 2406. In some implementations, the devices 105, 180 can be configured to transmit data via a short-range wireless communication transmitter, e.g. a Bluetooth beacon, to a receiver. In one example, a first mobile device 105b may communicate with the medical device(s) 180 via a Wi-Fi and/or cellular communication link from a remote location while a second mobile device 105a may communicate with the medical treatment device via a Bluetooth communication link. In some implementations, a mobile device 105 can connect to the medical device(s) 180 via the wireless communication link without having to physically access the medical device(s) 180. In some examples, transport layer security (TLS) is used at an application level to provide a secure (encrypted) connection between the devices 105, 180. As a second layer of protection, encrypted Wi-Fi or encrypted Bluetooth can be used at a physical layer.

In some implementations, when the wireless communication link 2406 is a cellular communication link, the functionality of the medical device(s) 180 can be extended to clinicians who are off-scene and/or performing remote telemedicine (e.g., the caregiver 303 at remote location 389 as shown in FIGS. 3A-3D). For example, when EMS are transporting a patient to the hospital in an ambulance, a medical team awaiting the arrival of the patient to the hospital can stream real-time case information at a mobile device 105 at the hospital via cellular link. In some examples, the wireless communication link 2406 can include combinations of multiple wireless communication networks based on proximity of the medical device(s) 180 to the mobile device 105 or mobile devices 105a, 105b.

In some implementations, each of the medical device(s) 180 and the mobile device 105 includes a respective wireless communication engine 2424, 2436 for enabling wireless communication between the devices 105, 180 via the wireless communication link 2406. For example, the wireless communication engine 2424 of the medical device(s) 180 can be configured to transmit messages generated by message configuration engine 2420 to the mobile device 105. Wireless communication engine 2436 of the mobile device 105 can be configured to transmit instruction signals generated by signal generation engine 2430. In some examples, such instruction signals may be for controlling one or more functional operations of the medical device(s) 180. In some examples, the wireless communication engines 2424, 2436 are configured to apply encryption protocols to outgoing and transmitted signals. Similarly, the wireless communication engines 2424, 2436 can decrypt incoming and received signals.

In certain embodiments, the wireless communication engine 2424 of the medical device(s) 180 can be configured to detect that a respective mobile device 105 is within communication range and in response, initiates one or more actions to connect to the mobile device 105 via the wireless communication link 2406. In some implementations, a mobile device 105 that is pairable with the medical device(s) 180 can be preconfigured as a companion device to automatically connect to the medical device(s) 180 via the wireless communication link 199 when within communication range, without having to discriminate between other devices that happen to be within range and/or negotiate a wireless communication connection. Further, rather than requiring a user to potentially spend significant amounts of time in manually configuring the system of each companion mobile device to connect to the medical device(s) 180 or accessing a screen to view and then select from possible device connections, companion mobile device(s) located at the emergency scene may be pre-configured to dynamically join and/or leave the secure network or pairing with the medical device(s) 180, for example, automatically and/or with one or more simple actions (e.g., switch actuation, pressing a button, near field communication connection, radio frequency, location/proximity recognition, gestural code, tap/bump recognition, motion-activated, sound/vibration, voice command/recognition, amongst others) and/or merely by being in close physical proximity to one another such as by a Bluetooth proximity connection. For example, upon selecting icon 1301 in a device view 1315 and/or 1316 of the companion device (e.g., as shown in FIGS. 13B and 13C), a device user can view all available pre-configured wireless communication links that are available for the companion mobile device to connect to the medical device(s) 180. The user can also view other available networks that have not been pre-configured for connection. In some examples, the companion mobile device may be pre-configured for pairing to other medical treatment devices, and those preconfigured networks can also be displayed upon selecting icon 1301. The connected devices window 1420 may provide functionality as similarly described for the wireless communication engine 2424 and/or the icon 1301.

Once such connection via the wireless communication link 2406 is made, despite the presence of numerous other devices located nearby, patient information (e.g., physiological data, patient history, rescue info) can be sent back and forth between the connected devices 105, 180 in a reliable and secure manner (e.g., according to HIPAA standards, 802.11i protocols) using any suitable type of communication. Companion device versions of the mobile device 105 that are correctly paired with their respective medical device(s) 180 can help avoid risk of erroneous patient information to be transmitted between medical devices, which could be detrimental to patient outcomes. In some embodiments, to maintain accurate and secure communications, the proximity-based interaction may invoke an authentication protocol, such as the use of encrypted keys, vector initialization, hash encryption, digital certificates, etc., ensuring no drops and/or leakage of data transfer between devices. Additionally, the wireless communication engine 2424 of the medical device(s) 180 can be configured to simultaneously cause transmission of real-time streaming data to multiple companion devices via separate wireless communication links 2406 for each companion device (e.g., the multiple mobile devices 105a, 105b).

In some implementations, a number of additional security-oriented design elements can also be implemented for the medical treatment system to ensure that data exchanged between the medical device(s) 180 and mobile device 105 remains secure. For example, the medical device(s) 180 and/or the mobile device 105 can use certificate-based authentication to ensure the authenticity of the respective paired device. In some examples, upon initial connection and setup, the devices 105, 180 can execute an association process to tie a particular mobile device 105 as a companion device to a single medical device(s) 180 so that only the companion device (and any other similarly paired companion devices) can interoperate with the medical device(s) 180. In some embodiments, any Representational State Transfer (REST) or WebSocket communications may require an authenticated connection to enable data exchange between the devices 105, 180. The medical device(s) 180, in some examples, prohibit connection to open Wi-Fi communication links and may only connect to manually defined (e.g., supervisor-defined) Wi-Fi networks. As an additional security measure, when the wireless communication link 2406 is a Bluetooth connection, the devices are paired during initial setup when initial connection settings are configured. Further, the data and computer architecture of the medical device(s) 180 can be designed for additional security, which can include separating communications and clinical control onto separate microprocessors.

In some examples, when more than one mobile device 105 or companion device is paired with the medical device(s) 180 , one of the mobile or companion devices may be designated in advance as the primary mobile or companion device. The primary mobile or companion device may be so designated by the medical device(s) 180 during device setup, pairing and provisioning by receiving and storing an encrypted token from the medical device(s) 180. The encrypted token may be sent with every instruction from the primary companion device to the medical device(s) 180.

In some implementations, the mobile device 105 can display status information for the medical device(s) 180 at one or more of the display views (FIGS. 13B, 14A, 14B, 15, 16, and 29-43) of the mobile device 105. Additionally or alternatively, the mobile device 105 may display status information at the connected devices window 1420. For example, as shown in FIGS. 13B and 13C device view 1315 and/or 1316 can include status bar 1303 that displays an amount of battery life, connection status, and a unique name for the medical device(s) 180. Additionally, in some examples, the device view 1315 and/or 1316 can include a paired device verification input selector 1318 (e.g., shown in the device view user interface of FIGS.13B and 13C) that allows the mobile device 105 to cause an indicator to flash at the medical device(s) 180 to confirm that the mobile device 105 is connected to the correct medical device(s) 180 at time of use. For example, one or more display views of the mobile device (e.g., device view UI screen 1315 and/or 1316 in FIGS. 13B and 13F) can include a paired device verification input selector 1318 that allows a mobile device user to verify to which medical device(s) 180 the mobile device 105 is connected. In some examples where multiple medical events are occurring at the same time, such as in a trauma unit of a hospital or on a scene of a mass casualty, there may be multiple rescue teams operating multiple medical treatment devices within close proximity of one another. In such situations, it may be easy to mix up mobile devices that are paired to respective medical treatment devices. Therefore, in some implementations, when the verification input selector 1318 is selected, the mobile device 105 may generate an instruction signal that causes the connected medical device(s) 180 to generate an indication of being paired with the companion device 105. In some implementations, upon receiving a verification signal from the mobile device 105, the medical device(s) 180 may output a visual and/or audible indication of being connected to the companion device. In some examples, the indication can be a flashing light and/or a tonal sound pulse.

In some implementations, the mobile device 105 includes a signal generation engine 2430 that generates instruction signals, data requests, and other data signals for transmitting to the medical device(s) 180. In some examples, in response to receiving user inputs (e.g., selections at a touchscreen of the mobile device 105) to view one or more items of case information at customized user interface screens on the mobile device 105, the signal generation engine 2430 can generate a data request message from transmitting to the medical device(s) 180 . In some examples, the data requests can be of one or more data requests type based on a type and amount of data being requested. For example, one type of data request includes a request for a single type of data from the medical treatment device (e.g., trends data) or for a relatively small number of pieces of data (e.g., ventilation data for generating a ventilation dashboard). Another type of data request can include requests for complex data groupings, such as all case information necessary to recreate a device view or requested case type view. Generating specific data requests of the medical device(s) 180 allows the mobile device to flexibly modify the set of data it has subscribed to at any moment. In some examples, data subscriptions for the mobile device 105 correspond to all of the data requested by the mobile device 105 for display at any given time. Requesting data from the medical treatment device as a set of data subscriptions provides mobile device 105 the ability to show different types of data on demand and minimizes bandwidth used by avoiding transmission of unnecessary data that the mobile device user does not wish to have displayed.

In certain embodiments, the signal generation engine 2430 can also generate instruction signals for causing one or more functional operations to occur at the medical device(s) 180. In some examples, the one or more functional operations can include linking and storing certain submitted data associated with the medical event (e.g., patient information or event marker data) and/or capturing or analyzing certain medical event data (e.g., generating 12-lead ECG analysis or lung mechanics data analysis). For example, the signal generation engine 2430 can generate a patient information signal in response to receiving submission of patient identification information via activation of a patient information input interface of the mobile device 105 by the patient information control 1311 (e.g., as shown in FIGS. 13B, 13C, 14A, 14B, 15, 16, and 29-43). The patient information signal can include the submitted patient information, and in response to receiving the signal, the medical device(s) 180 can link and store received patient information 2446 with sensor data 2442 and other case information for the patient within data repository 2408. In addition, in response to receiving one or more event marker inputs at the mobile device, the signal generation engine 2430 can generate an event marker signal, which can include submitted event marker data. In an implementation, activation of a confirmation control at the CSG UI (e.g., the confirmation control 3140 and/or 3275) may generate the event marker signal. In response to receiving the signal, the medical device(s) 180 stores the submitted event marker data 2452 in data repository 2408. In some examples, the signal generation engine 2430 can generate an instruction signal that causes the medical device(s) 180 to perform a particular data analysis, send specific data to the mobile device, and/or administer a particular therapy.

In some implementations, the medical device(s) may store alarm data 2456 in data repository 2408. The alarm data 2456 may include alarms provided by the medical device(s). The alarm data 2456 may indicate alarm information such as type, priority, time, etc. Types of alarms may include patient safety alarms (e.g., high/low airway pressure, high/low tidal volume, high/low breath rate/apnea, PEEP leak, insufficient flow, spontaneous breath-PIP high/low, spontaneous breath-VT high/low, patient inspiratory demand not met, auto-PEEP, patient disconnect, exhalation system failure/fault, calibration error, suspicious triggers, tubing compliance faults, SpO2 sensor off/low/error, heart rate high/low, etc.), use environment alarms (e.g., low battery, power faults, climatic environment faults, oxygen supply faults, gas intake faults, etc.), and/or self-check alarms (e.g., internal communication errors, pneumatic system failures, power system faults, pulse oximetry module faults, preventive maintenance alerts, etc.). The alarm priorities may be one of "high," "medium," and "low" or a priority according to another rating scheme (e.g., severity indicated as numbers or letters) based on the severity of the associated alarm. In some examples, the medical device(s) may categorize all patient safety alarms as high priority alarms.

The medical device(s) 180, in some implementations, can include a message configuration engine 2420 for generating messages for sending to the mobile device 105. In one example, in response to receiving a data request from the mobile device 105, the message configuration engine 2420 can package the requested data in one or more predetermined message configurations or formats for transmission. In some implementations, real-time or near real-time data (e.g., case information derived from patient interface devices 170) can be transmitted as streaming data in a JavaScript Object Notation (JSON) format sent over a WebSocket. Historical and bulk data transfers, in some examples, can be transmitted as Representational State Transfer (REST) data in JSON-formatted messages. Both types of message communications (streaming data and REST data) can occur over a transport layer security (TLS) connection, which can use a TCP/IP protocol. The TCP/IP protocol can be provided over Wi-Fi or Bluetooth physical media. When data transfer occurs in real-time or near real-time, case information is simultaneously displayed at the mobile device 105 and the medical device(s) 180 or an amount of latency for displaying the case information at the mobile device is less than a predetermined threshold.

In addition to configuring messages for sending real-time case information for display at the mobile device 105 in some implementations, the message configuration engine 2420 can generate one or more confirmation messages when an action is taken at the medical device(s) 180 in response to receiving an instruction signal from the mobile device 105. For example, in response to associating and storing submitted patient information provided by the mobile device 105 the message configuration engine 2420 can generate a message confirming that the submitted patient information has been linked to the respective case information within data repository 2408. The message configuration engine 2420 can also generate event marker recording confirmation messages confirming that recording of the submitted event marker at the medical device(s) 180 has commenced and/or completed. As another example, the message configuration engine 2420 can generate a message confirming an inspiratory gas mixture and/or a change in support level from oxygen to CPAP or bilevel or CPAP to bilevel.

In some implementations, the mobile device 105 may include a data playback engine 2441. The data playback engine 2441 can provide caregivers the ability to view and scroll through past case information at the mobile device 105. In some examples, while viewing past case information, the mobile device 105 can provide users the ability to jump to a current (live) view at any time during the medical event. In one example, the mobile device 105 may indicate on the display screen whether a live view is being displayed. In some embodiments, mobile device users can jump forward and backward in time in discrete time segments (e.g., 5, 10, 15, 20, 30 seconds) to view a patient's physiological condition and caregiver performance data at different points during the medical event. In one example, the mobile device display interface can provide a touch spot for each waveform that provides for playback of the respective waveform. Additionally, the mobile device 105 can play back past medical event data at different speeds (e.g., 0.5x, 1x, 2x) to gain a better perspective of how patient conditions and care have progressed. In some examples, the mobile device can display past medical data for a number of different monitored physiological sensor inputs (e.g., ECG, SpO2, CO2), vital sign data, and caregiver performance data. Additionally, even when non-real-time data is being displayed at the mobile device 105, the medical device(s) 180 continues to transmit real-time streaming case information to the mobile device 105. In one example, the mobile device 105 can also provide a lookback feature for limited time increments (e.g., 10, 20, 30 seconds) that allows the user to quickly view waveform data at the lookback increment. In example, the lookback feature can be activated via a touch spot on the respective waveform.

In some implementations, the medical device(s) 180 can include an input/output (I/O) engine 2426 that may gather information from a number of patient interface devices 170 built into the medical device(s) 180 and/or in communication with the medical device(s) 180. The I/O engine 2426 can also receive user inputs at a user input interface (e.g., keypad or touchscreen) on the medical device(s) 180. In some examples, raw sensor data from the patient interface devices 170 can be processed and analyzed by sensor data processing engine 2422, which generates a number of clinical metrics and trends regarding aspects of the treatment session (e.g., for use by clinical personnel) that can be output by the medical device(s) 180 (e.g., displayed at a screen of the medical device(s) 180 or printed as a report). The generated metrics and trends can be stored in data repository 2408 for the respective patient and/or medical event as metric data 2454, and trend data 2444, respectively. GUI engine 2428, in some embodiments, causes data processed and analyzed by the sensor data processing engine 2422 to be presented at a display interface screen on the medical device(s) 180.

The medical device(s) 180 may include a data logging and storage engine 2418. The engine 2418 may enable storage of the various types of medical device data shown in FIG. 24 in the data repository 2408. The engine 2418 may log the data as it is generated by or received at the medical device(s) 180, associated a time stamp with the data, and then store the logged data in the repository 2408.

In some examples, sensor data processing engine 2422 also processes raw sensor data into first sensor information that is provided to GUI engine 2428 for display at medical device(s) 180 and second sensor information that is provided to GUI engine 2438 for display at mobile device 105. In some examples, because the display interfaces at the medical device(s) 180 and mobile device 105 can be differently sized, the sensor processing engine 2422 can time-slice the sensor data differently based on the display device. In one example, the local display for the medical device(s) 180 receives small intervals of first sensor information more frequently (e.g., 8ms at a time) while the mobile device 105 receives larger intervals of second sensor information less frequently (e.g. 120ms at a time). This configuration of first and second sensor information can allow the mobile device 105 to display sensor data in real-time yet also improves data transmission efficiency because sending larger data messages is more efficient than sending smaller data messages. Additionally, even though the content of the first and second sensor information is the same, the first and second sensor data may be represented differently. For example, the first sensor information displayed at the medical treatment device may include binary data records while the second sensor information the mobile device 105 receives may be a JSON representation as a Websocket stream. Additionally, both the first and second sensor information signals can carry additional information per-sample bit-flags that represent specific detected conditions associated with the processed sensor data sample. Other additional information, such as medical device settings may be provided to both of the GUI engines 2428, 2438 as additional messages separate from the sensor data.

In some aspects, a data processing engine 2434 of the mobile device 105 can be configured to process sensor data 2442 and other case information received from the medical device(s) 180 in one or more received data messages. GUI engine 2438 can cause the processed medical event data to be displayed in one or more display sections of a device interface at the mobile device 105. An I/O engine 2440 can receive and process user inputs at a device interface, such as a touchscreen interface, where a user makes selections to customize the display of data at the mobile device 105 as well as to cause one or more functional operations to occur at the medical device(s) 180. GUI engine 2438, in some implementations, causes data processed and analyzed by the data processing engine 2434 and configured by customization engine 2439 to be presented at a display interface screen on the mobile device. Customization engine 2439, in some embodiments, can be configured to control and manage the configuration of display screens presented at the display interface of the mobile device 105 by the GUI engine 2438 based on a user role and/or data presentation preferences. Roles may include, for example, chest compression team member, ventilation team member, drug administrator, supervisor, documenter, remote clinicians, or non-clinicians (e.g., police and/or fire personnel as first responder(s)).

The mobile device may further include the CSG engine 120. A data store 2410 associated with the mobile device 105 may include a CSG data store 2459 and a CSG algorithm 121 and/or CSG application 315. The CSG data store 2459 may include CSG data provided to the mobile device 105 by the user and/or by the medical device(s) 180. The data storage region 2408 of the medical device(s) 180 may include CSG data 2448 received from the mobile device 105 and/or collected by or generated at the medical device(s) 180.

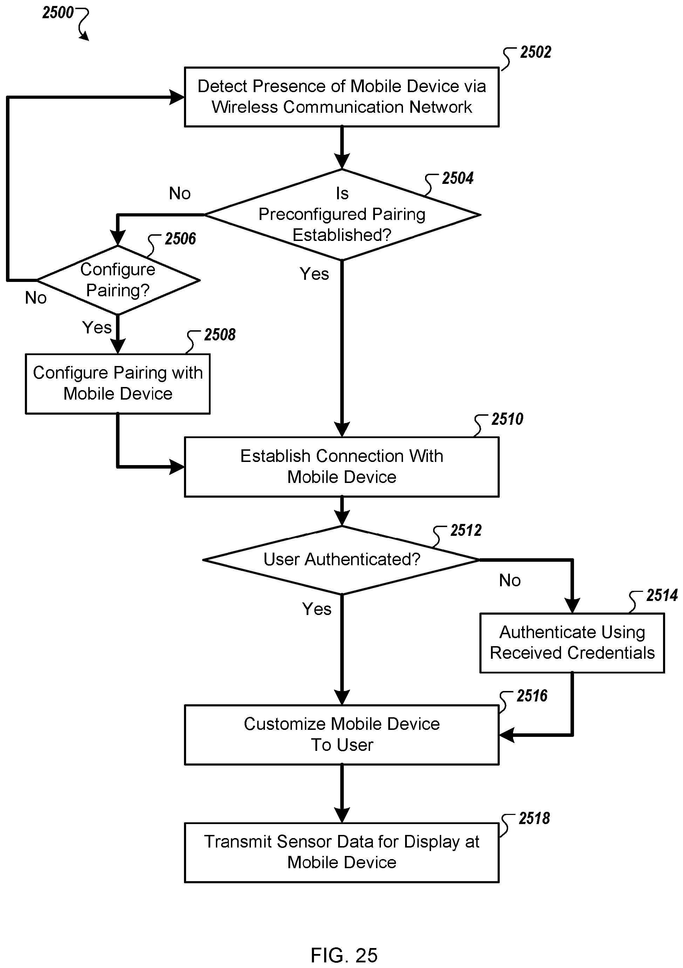

Referring to FIG. 25, an example method for configuring connection between a medical device and a mobile device is illustrated. In an implementation, the mobile device 105 may be a companion device. In response to the connection, the connected devices window 1420 may display an indication of each device that is communicatively coupled to the mobile device 105. In some examples, the method 2500 begins with medical device(s) 180 detecting the presence of the mobile device 105 due to the mobile device 105 being within communication range of the medical device(s) 180 (2502). If the medical device(s) 180 and the detected mobile device 105 already have a preconfigured pairing with each other established (2504), then in some examples, the medical device(s) 180 and the mobile device 105 establish a wireless connection (2506). In some embodiments, the previously paired medical device(s) 180 and the mobile device 105 may automatically connect to one another. In other examples, upon detection of the respective device, the medical device(s) 180 and/or the mobile device 105 may present a notification to a user at a display interface requesting user authorization for connection to the other device 105, 180. In response to presenting the notification, if one or both of the devices 105, 180 receives an input confirming the connection to the other device 105, 180, then the wireless communication link between the devices 105, 180 is established.

If a preconfigured pairing between the two devices 105, 180 has not been established, then in some aspects, a determination may be made whether to establish a wireless connection between the devices 105, 180 (2506). In some examples, only devices 105, 180 that have been through an initial setup and pairing process can wirelessly connect to one another. In another example, a supervisor or system administrator may be authorized to configure a pairing between the medical device(s) 180 and the mobile device (2508) prior to initial connection between the devices 105, 180 (2510). In one example, this initial pairing can include one or more types of wireless communication links such as Wi-Fi, Bluetooth, or Zigbee connections. Thus the mobile device 105 may be a companion device based on a configuration prior to use or a point-of-use configuration.

In certain embodiments, if a credentialed user has been authenticated at the mobile device (2512), then in some examples, the mobile device 105 may customize one or more mobile device display views to user preferences or a treatment role of the user (2516) based on role data, as stored or provided at the mobile device at time of use. In some examples, if less than a threshold amount of time has passed between uses of the mobile device 105, the mobile device 105 may automatically authenticate a previous user to use the mobile device 105. Otherwise, if more than the threshold period of time has passed and/or a new user is logging in to use the mobile device, then in some implementations, the mobile device 105 may authenticate the user with received credentials, such as username/password inputs, at least one biometric input provided at a biometric input interface (e.g., fingerprint, iris recognition, facial recognition), and/or a badge scan via a scanning sensor on the mobile device (e.g., RFID scan or a computer-readable code such as a QR code) (2514). In some examples, upon authentication, the user may provide one or more inputs confirming or modifying a treatment role of the user in the associated medical event. Based on the indicated treatment role, the mobile device 105 may further customize the display views at the mobile device 105 to the indicated role of the user. In some embodiments, based on the customized display interface screens being presented at the mobile device as well as user inputs received at the display interface, the medical device(s) 180 may transmit requested sensor data for display at the mobile device in the customized format (2518). In some implementations, in response to establishment of the connection between the mobile device 105 and medical device(s) 180 and/or authentication of the device user, the medical device(s) 180 may automatically begin streaming case information via the wireless communication link 2406 for display at the mobile device 105 without any user intervention.

Although described as a particular series of steps, in other embodiments, more or fewer steps may be included. For example, the medical device(s) 180 may only connect to a mobile device 105 that has had a preconfigured pairing established. On the other hand, if it is determined that a predetermined pairing exists between the medical device(s) 180 and a detected mobile device 105, one or more additional steps related to ensuring data security of the wireless connection may be performed. In further embodiments, certain steps may be performed in a different order, or two or more steps may be performed in parallel. For example, a user may be authenticated at the mobile device 105 prior to a connection being established between the medical device(s) 180 and the mobile device 105. Other modifications of the method 2500 are possible while remaining in the scope and purpose of the method 2500.

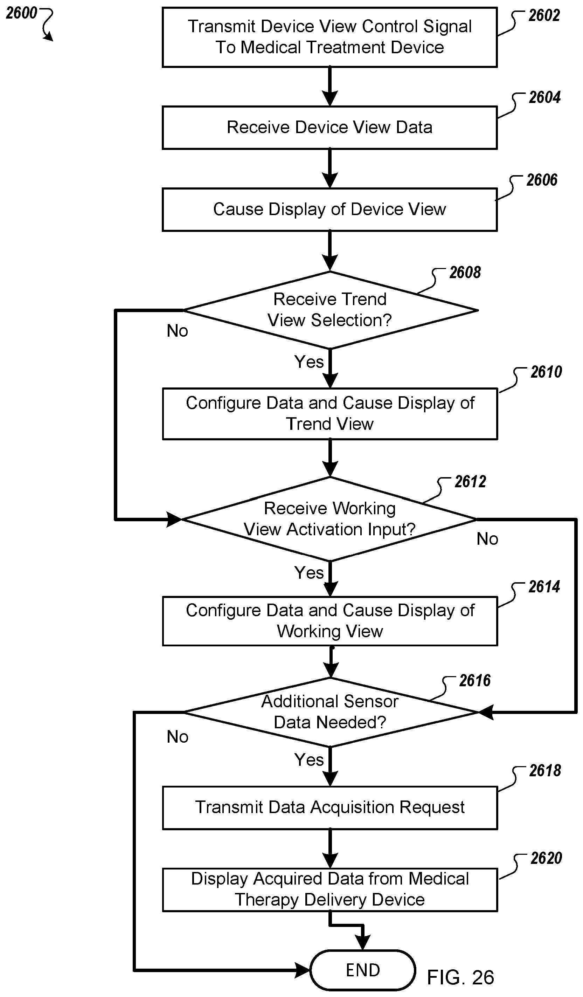

Referring to FIG. 26, an example method for causing display of case information from a medical device at a mobile device during a medical event is illustrated. The method 2600 is, however, an example only and not limiting. The method 2600 can be altered, e.g., by having stages added, removed, rearranged, combined, and/or performed concurrently. In some examples, the method 2600 begins with mobile device 105 transmitting a device view instruction signal to a connected medical device(s) 180 such as a patient monitor/defibrillator and/or a ventilation system (2602). In some implementations, the instruction signal corresponds to a request for case information that is presented within the device display view 1315 and/or 1316 at the mobile device 105. In some examples, the device view is one of multiple display views that can be presented at the mobile device 105 and corresponds to a display view that, in real-time, presents a visual reproduction of the data that is presented at a display interface of the medical device(s) 180. In some embodiments, in response to transmitting the request signal for device view case information, the medical device(s) 180 sends the requested data over the wireless communication link, which is received by the mobile device 105 (2604). In some examples, the mobile device 105 causes display of the received information within the device view at the display interface (2606).

In some implementations, upon detection selection of a trends view input at the mobile device 105 (e.g., selection of trends view selector 1350) (2608), the mobile device may configure the case information to cause display of a trends view display interface at the mobile device (2610). In some implementations, configuring trend data for display at the trends view display interface may include transmitting a signal to the medical device(s) 180 to request transmission of medical trend data for real-time display.

Upon detection of a working view activation input at a display interface (e.g., working view selection tab 1340) (2612), the mobile device may configure the case information to cause display of a working view display interface at the mobile device (2614). In some examples, the working view display interface may be activated when the user configures the mobile device to remove or add any displayed data section (e.g., waveform, data value, or dashboard). For example, the user may select data sections to delete or include in real time at the mobile device 105. In other examples, a saved working view associated with a user account may be automatically presented upon logging in to use the mobile device 105. In some embodiments, the working view window 1415 can also be activated upon addition of any waveform, metric value, or dashboard for viewing. In some embodiments, the working view display interface may correspond to any type of customization of the case information displayed at the mobile device. Stated another way, the working view display interface corresponds to any set of displayed data in any format at the mobile device 105 that differs from the displayed data and format of the device view 1315 and/or 1316.

In some implementations, upon configuration of a working view UI screen at the mobile device, if one or more items of physiological sensor data, treatment data, or caregiver performance data are needed to display within the working view (2616), then in some examples, the mobile device 105 transmits a data acquisition request to the medical device(s) 180 requesting the one or more requested items of data (2618). For example, if a mobile device user selects a chest compression dashboard 1470 for display within a working view at the mobile device 105 and it has not been displayed up to that point, then the mobile device may transmit a data acquisition request to the medical device(s) 180 to obtain CPR case information (e.g., chest compression depth and rate data) for display within the chest compression dashboard 1470. In some examples, upon receiving the requested data, the mobile device 105 configures the requested data for real-time display within a respective data section in the working view interface (2620). In some embodiments, based on the type of data requested, the medical device(s) 180 may transmit the requested data as a streaming data message and/or a REST data message (bulk data transfer).

Referring to FIG. 27A, an exemplary ventilation system is illustrated. The ventilation system 280 may connect an oxygen source 2730 to the patient 101 for respiratory support as an intervention for RD. The ventilation system 280 may include a mechanical ventilation apparatus 2740, a ventilation system controller 2750, and a ventilation system user interface 2760. In an implementation, the medical device(s) 180 may include a patient monitor/defibrillator 285 and the ventilation system 280 with a combined user interface for both devices disposed on the patient monitor/defibrillator.

Referring to FIG. 27B, an example of the mechanical ventilation apparatus for a ventilation system is illustrated. The mechanical ventilation apparatus 2740 may include a gas-mover 2741 configured to move oxygen from the oxygen source 2730 through an inspiratory circuit 2743 to the patient 101 via a patient interface 2744. The ventilation system 280 may use the gas-mover 2741 to provide a range of therapeutic ventilation modalities to provide various interventions. These various interventions may include 1) noninvasive ventilation (NIV) including continuous positive airway pressure (CPAP) and bilevel ventilation (bilevel); 2) high-flow nasal cannula (HFNC); 3) invasive ventilation (IV) using assist/control (AC), synchronized intermittent mandatory ventilation (SIMV) and pressure support (PS) modes; and 4) synchronized ventilation during cardiopulmonary resuscitation (CPR) mode.

The ventilation system 280 may provide ventilation during CPR, and may, for example work in coupling and integrating with one or more critical care monitors, such as one or more patient monitor/defibrillator(s) 285, for example. In some embodiments, for example, during mask ventilation, unprotected airway, the ventilation system 280 may deliver 2 breaths for every 30 compressions as measured by a monitor. In some embodiments, for example, when the airway is protected (e.g., endotracheal tube, Combitube, etc.), the ventilation system 280 may continuously deliver 10 breaths/min independent of the compression rate (asynchronous).

The patient interface 2744 may include an appropriate gas delivery device, such as an intubation tube 277, mask 278, nasal cannula 279, etc. The mechanical ventilation apparatus 2740 further includes an expiratory circuit 2745 and an exhalation valve 2748. Both the inspiratory and expiratory circuits include sensors 2747. The sensors 2747 may include, for example, but not limited to, the pneumotachometer 275, the airway pressure sensor 274, and the spirometer 276. The sensors 2747 enable the ventilation system 280 to measure the patient's respiratory efforts as well as the performance of the ventilation system 280 when providing mechanical respiratory assistance to the patient. The sensors 2747 may generate and provide data to the CSG engine 120, the data including but not limited to flow rate, tidal volume and minute ventilation, respiratory mechanics (e.g., resistance and compliance) and spirometry, and may include, for example, forced vital capacity (FVC), forced vital capacity at 1 second (FEV1) and peak expiratory flow rate (PEF or PEFR). In addition, the patient monitor/defibrillator 285 or another medical device may provide, for example, capnography and/or oximetry data, such as oxyhemoglobin and carboxyhemoglobin saturation and mainstream or other capnographic data such as end tidal CO2 (EtCO2). This data may allow, for example, for calculation of CO2 elimination rate and volumetric capnography, which may include using flow data from the ventilation system 280.

In some embodiments, for patients who require supplemental oxygen (O2), the ventilation system 280 may provide a number of methods to support oxygenation by invasive and non-invasive ventilation. For example, one method may include using a small reservoir bag system that allows entrainment from an O2 flow source. In some embodiments, in a first method, the user may manage the O2 delivery to the patient while monitoring the oxygen saturation (SpO2) measured by the patient monitor/defibrillator 285 or another medical device. A second method may make use of an innovative smart O2 valve (SOV) or module, which may, for example, attach to the inspiratory circuit 2743, a high-pressure O2 cylinder/source and the patient monitor/defibrillator 285 or another medical device This may, for example, provide for automatic control of the patient's oxygenation using physiologic closed loop control (PCLC) where, the SpO2 signal may be used to regulate the output of the SOV. A third method may provide for the functional integration of a portable O2 concentrator (POC), which may, for example, use PCLC to regulate the O2 output of the POC and the O2 entrainment into the ventilation system 280.

Referring to FIG. 27C, an example of user interface for a ventilation system is shown. In an implementation, the ventilation system 280 may include a user interface 2760. The user interface 2760 may display data generated by the ventilation system 280 and/or may include controls for user adjustment of ventilation parameters. Further, as discussed above, in some implementations, the mobile device 105 may be preconfigured to pair with or otherwise establish a connection with the ventilation system 280, so as exchange medical data in accordance with methods described herein.

The data generated by the ventilation system 280 that may be displayed at the user interface 2760 and/or transmitted to the mobile device 105, for visual reproduction thereon, may include, for example, ventilation settings, ventilation parameters, and/or physiological parameters collected by the ventilation system 280. For instance, ventilation settings may include the respiratory rate (breaths per minute (BPM)) 2712, inspiratory:expiratory ratio (I:E, ratio of inspiratory time to expiratory time) 2713, tidal volume (volume of air delivered per breath) (Vt) 2710, positive end-expiratory pressure (PEEP), pressure in the lungs above atmospheric pressure that exists at the end of expiration) 2709, peak inspiratory pressure (PIP) limit 13708, fraction of inspired oxygen (FiO2) 2706, mode setting (e.g., assist/control (AC), synchronized intermittent mandatory ventilation (SIMV), continuous positive airway pressure (CPAP), bilevel (BL)) 2714, etc. In some examples, each of the ventilation settings 2704, 2706, 2708, 2709, 2710, and 2712/2713 may have a corresponding user input 2722a, 2722b, 2722c, 2722d, 2722e, 2722f, and 2722g on the ventilation system 280 for adjusting the respective setting 2704, 2706, 2708, 2709, 2710, and 2712/2713. Ventilation parameters may include, for example, inspiratory pressure data, expiratory pressure data, inspiratory flow data, expiratory flow data, leak detection, or other information measured from the ventilation system 280. Examples of physiological parameters may include non-continuous pulse oximeter (SpO2) measurements 2704, end-tidal CO2 (EtCO2) measurements, continuous SpO2 waveform data 2716, airway waveform data 2718, continuous CO2 waveform data, heart rate 2702, blood pressure, airway pressure data, airway flow data, spirometery data, amongst others.

In some examples, the ventilation system 280 can be configured to generate alarm signals (e.g., visual and/or audible indications) when one or more parameter set points have been exceeded. In one example, when airway pressure exceeds a high airway pressure limit 2720, an alarm can be generated. The user interface 2760 and/or the view windows at the mobile device 105 may provide these alarms.

In some implementations, alarms generated by the ventilation system 280 can include patient safety alarms such as high/low airway pressure, high/low tidal volume, high/low breath rate/apnea, PEEP leak, insufficient flow, spontaneous breath-PIP high/low, spontaneous breath-VT high/low, patient inspiratory demand not met, auto-PEEP, patient disconnect, exhalation system failure/fault, calibration error, suspicious triggers, tubing compliance faults, SPO2 sensor off/low/error, heart rate high/low, etc. The ventilation system 280 can also generate environmental alarms such as low battery, power faults, climatic environment faults, oxygen supply faults, gas intake faults, etc. Self-check alarms can include internal communication errors, pneumatic system failures, power system faults, pulse oximetry module faults, preventive maintenance alerts, etc. In some examples, when an alarm is generated, a pop-up message can be displayed at the ventilation system 280 interface. In addition, one or more alarm set points can be adjustable by a user at the ventilation system 280 interface. For example, alarm set points for airway pressure high/low, tidal volume high/low, breath rate, spontaneous breath, SPO2 % low, and hear rate high/low can be manually adjusted by a device user. When an alarm signal is generated and/or when an alarm pop-up message is displayed at the ventilation system 280 interface 2760, the user may take one or more actions to mute the alarm signal and/or acknowledge the alarm.

In some implementations, when mobile device 105 is communicatively coupled to the ventilation system 280, the ventilation system 280 can transmit case information associated with physiological sensor inputs for display at the mobile device 105 in a similar way as when the medical device(s) 180 is the patient monitor/defibrillator 285. For example, in response to receiving user inputs at the mobile device 105 associated one of the control operations at the ventilation system 280, the mobile device 105, in some implementations, transmits an instruction signal to cause the respective operation to occur at the medical treatment device. In some examples, instruction signals sent from the mobile device 105 to the medical treatment device can instruct the ventilation system 280 to update patient information, treatment information, or diagnostic information for the medical event that is stored in a data repository 2754 of the ventilation system 280. In response to receiving the respective signal, the ventilation system 280 performs the respective operation associated with the instruction signal, which may include storing provided information (e.g., transmitting patient information for updating at the ventilation system 280) or recording an event marker (e.g., transmitting a treatment/event marker for the ventilation system 280 to record in the patient care record) or initiating a snapshot (e.g., transmitting an instruction signal for the medical treatment device to initiate a snapshot of one or more types of case information displayed on a display screen of the ventilation system 280) or activating an analysis feature at the ventilation system 280. In one example, in response to receiving height, gender, and/or weight information for the patient that was input at the mobile device 105, the ventilation system 280 may automatically adjust volume and rate of ventilation being administered to the patient by the ventilation system 280.

The user interface 2760 may include controls for user adjustment of parameter settings and/or alarm set points at the ventilation system 280. For example, the user interface 2760 may include user inputs that correspond to the ventilation system 280 setting inputs 2722a-g so that a user can adjust values for the ventilation system settings 2704, 2706, 2708, 2709, 2710, 2712, and 2713. In some implementations, the user interface 2760 may also include a user input that corresponds to a manual breath button/plateau pressure input 2728 that causes the ventilation system 280 to deliver a manual breath to the patient and/or measure plateau pressure.

In an implementation, the device view 1315 and/or 1316 and/or the working view 1415 at the mobile device 105 may include the user adjustment controls 2722a-g. In some examples, the interfaces at mobile device 105 provide a much more user-friendly interface for efficient use of the ventilation system 280 to provide enhanced patient care without having to directly access the ventilation system 280.

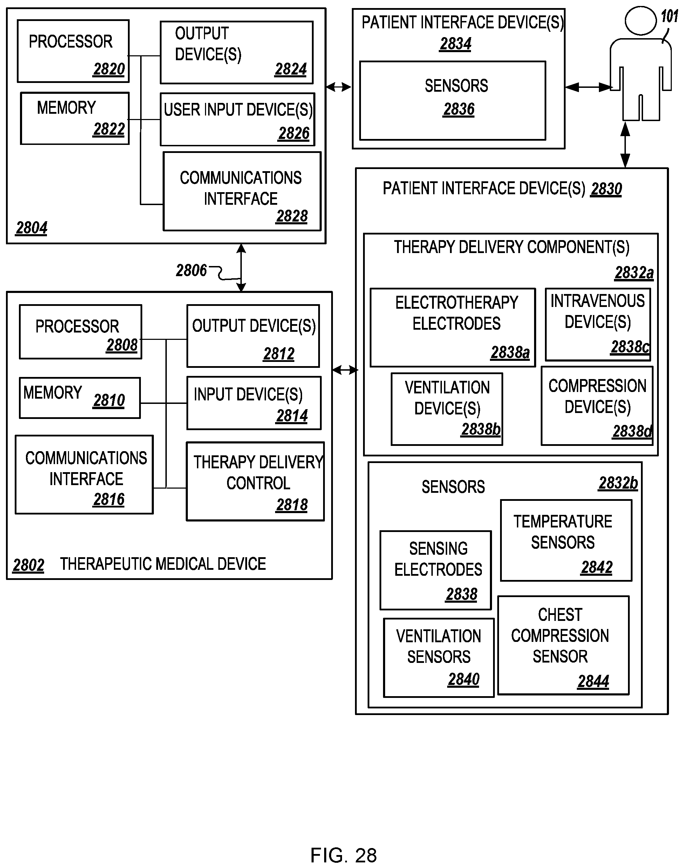

Referring to FIG. 28, examples of components of various devices discussed with regard to FIGS. 1-27C and FIGS. 29-43 are shown schematically. These devices may include a therapeutic medical device 2802 (e.g., medical device(s) 180 in FIG. 2) and one or more mobile devices 2804 (e.g., mobile device 105 in FIG. 2). In an implementation, the therapeutic medical device 2804 may monitor and/or provide diagnostic care for the patient 101 and/or provide medical therapy as an intervention via the patient interface devices 2830. The mobile device 2804 may be a portable computing device such as a tablet, laptop, or smart-phone. The mobile device 2804 may be adapted to function as a medical device or be a display screen of an additional medical device such as when monitoring continuous NIBP measurements. In an implementation, the mobile device 2804 may not be a therapeutic medical device configured to deliver medical therapy to the patient. In such an implementation, the mobile device 2804 may be limited to patient monitoring and/or diagnostic care, such as monitoring a patient status via one or more display screens and/or controlling one or more functional operations at the medical treatment device 2802 as described in the embodiments herein.

The therapeutic medical device 2802 and one or more mobile devices 2804 may be communicatively coupled via communicative coupling 2806 (e.g., the communication link 199 as shown in FIG. 1), which may be a wired and/or a wireless communications link. The wired communications links may include a wired electrically coupling, an optical coupling via an optical cable, etc. The wireless communications link may include coupling via a radio frequency or other transmission media and/or via a network such as a local area network, an ad hoc network, a mesh network, a cellular and/or other communications network, a computer network, etc. The communications link as described herein may utilize protocols such as, for example, 802.11, ZigBee^{®}, Bluetooth^{®}, etc. The communications link may include near field communications which may be implemented via a communications RFID tag. The communications link may include one or more networks such as a local area network, a cellular network, a satellite network, and/or a computer network (e.g., an Internet Protocol (IP) network). In various implementations, the communicative couplings described herein may provide secure and/or authenticated communications channels. In an implementation, the devices described herein may encrypt and/or decrypt the data transmitted and/or received via the communicative couplings.

In some embodiments, the memory 2810 of the medical treatment device 2802, and similarly memory 2822 of mobile device 2804, may include a data store, also referred to as a data repository, and corresponding data storage circuitry including one or more of non-transitory or non-volatile computer readable media, such as flash memory, solid state memory, magnetic memory, optical memory, cache memory, combinations thereof, and others. The data store can be configured to store executable instructions and data used for operation of the medical treatment device 2802 and/or mobile device 2804. In certain implementations, the data storage processing circuitry, in combination with the data store, can include executable instructions that, when executed on the processor 2808 and/or 2820, are configured to cause the at least one processor 2808 and/or 2820 to perform one or more functions of the medical device(s) 180 and the mobile device 105, as described herein.

In FIG. 28, the components 2808, 2810, 2812, 2814, 2816, and 2818 are communicatively coupled (directly and/or indirectly) to each other for bi-directional communication. Similarly, the components 2820, 2822, 2824, 2826, and 2828 are communicatively coupled (directly and/or indirectly) to each other for bi-directional communication.

In some implementations, the components 2808, 2810, 2816, and/or 2818 of the therapeutic medical device 2802 may be combined into one or more discrete components and components 2816 and/or 2818 may be part of the processor 2808. The processor 2808 and the memory 2810 may include and/or be coupled to associated circuitry in order to perform the functions described herein. Additionally, the components 2820, 2822, and 2828 of mobile device 2804 may be combined into one or more discrete components and component 2828 may be part of the processor 2820. The processor 2820 and the memory 2821 may include and/or be coupled to associated circuitry in order to perform the functions described herein.

In some implementations, the therapeutic medical device 2802 may include the therapy delivery control module 2818. For example, the therapy delivery control module 2818 may be an electrotherapy delivery circuit that includes one or more high-voltage capacitors configured to store electrical energy for a pacing pulse or a defibrillating pulse. The electrotherapy delivery circuit may further include resistors, additional capacitors, relays and/or switches, electrical bridges such as an H-bridge (e.g., including a plurality of insulated gate bipolar transistors or IGBTs), voltage measuring components, and/or current measuring components. As another example, the therapy delivery control module 2818 may be a compression device electro-mechanical controller configured to control a mechanical compression device. As a further example, the therapy delivery control module 2818 may be an electro-mechanical controller configured to control drug delivery, temperature management, ventilation, and/or other type of therapy delivery.

The therapeutic medical device 2802 may incorporate and/or be configured to couple to one or more patient interface devices 2830. The patient interface devices 2830 may include one or more therapy delivery component(s) 2832a and one or more sensor(s) 2832b. Similarly, the mobile device 2804 may be adapted for medical use and may incorporate and/or be configured to couple to one or more patient interface device(s) 2834. The patient interface device(s) 2834 may include one or more sensors 2836. The sensor(s) 2836 may be substantially as described herein with regard to the sensor(s) 2832b.

The sensor(s) 2832b and 2836 may include sensing electrodes (e.g., the sensing electrodes 2838), ventilation and/or respiration sensors (e.g., the ventilation and/or respiration sensors 2830), temperature sensors (e.g., the temperature sensor 2842), chest compression sensors (e.g., the chest compression sensor 2844), etc. In some implementations, the information obtained from the sensors 2832b and 2836 can be used to generate information displayed at the therapeutic medical device 2802 and simultaneously at the display views at mobile device 2804 and described above (e.g. in display views 1315, 1415, 1515, 110, and 1316 as shown in FIGS. 13B, 13C, 14A, 14B, 15, 16 and 29-43). In one example, the sensing electrodes 2838 may include cardiac sensing electrodes. The cardiac sensing electrodes may be conductive and/or capacitive electrodes configured to measure changes in a patient's electrophysiology to measure the patient's ECG information. The sensing electrodes 2838 may further measure the transthoracic impedance and/or a heart rate of the patient. The ventilation and/or respiration sensors 2830 may include spirometry sensors, flow sensors, pressure sensors, oxygen and/or carbon dioxide sensors such as, for example, one or more of pulse oximetry sensors, oxygenation sensors (e.g., muscle oxygenation/pH), O2 gas sensors and capnography sensors, impedance sensors, and combinations thereof. The temperature sensors 2842 may include an infrared thermometer, a contact thermometer, a remote thermometer, a liquid crystal thermometer, a thermocouple, a thermistor, etc. and may measure patient temperature internally and/or externally. The chest compression sensor 2844 may include one or more motion sensors including, for example, one or more accelerometers, one or more force sensors, one or more magnetic sensors, one or more velocity sensors, one or more displacement sensors, etc. The chest compression sensor 2844 may provide one or more signals indicative of the chest motion to the therapeutic medical device 2802 via a wired and/or wireless connection. The chest compression sensor 2844 may be, for example, but not limited to, a compression puck, a smart-phone, a hand-held device, a wearable device, etc. The chest compression sensor 2844 may be configured to detect chest motion imparted by a rescuer and/or an automated chest compression device (e.g., a belt system, a piston system, etc.). The chest compression sensor 2844 may provide signals indicative of chest compression data including displacement data, velocity data, release velocity data, acceleration data, force data, compression rate data, dwell time data, hold time data, blood flow data, blood pressure data, etc. In an implementation, the defibrillation and/or pacing electrodes may include or be configured to couple to the chest compression sensor 2844.

In various implementations, the sensors 2832b and 2836 may include one or more sensor devices configured to provide sensor data that includes, for example, but not limited to electrocardiogram (ECG), blood pressure, heart rate, respiration rate, heart sounds, lung sounds, respiration sounds, end tidal CO₂, saturation of muscle oxygen (SMO2), oxygen saturation (e.g., SpO2 and/or PaO2), cerebral blood flow, point of care laboratory measurements (e.g., lactate, glucose, etc.), temperature, electroencephalogram (EEG) signals, brain oxygen level, tissue pH, tissue fluid levels, images and/or videos via ultrasound, laryngoscopy, and/or other medical imaging techniques, near-infrared spectroscopy, pneumography, cardiography, and/or patient movement. Images and/or videos may be two-dimensional or three-dimensional, such a various forms of ultrasound imaging.

The one or more therapy delivery components 2832a may include electrotherapy electrodes (e.g., the electrotherapy electrodes 2838a), ventilation device(s) (e.g., the ventilation devices 2838b), intravenous device(s) (e.g., the intravenous devices 2838c), compression device(s) (e.g., the compression devices 2838d), etc. For example, the electrotherapy electrodes 2838a may include defibrillation electrodes, pacing electrodes, and combinations thereof. The ventilation devices 2838b may include a tube, a mask, an abdominal and/or chest compressor (e.g., a belt, a cuirass, etc.), etc. and combinations thereof. The intravenous devices 2838c may include drug delivery devices, fluid delivery devices, and combinations thereof. The compression devices 2838d may include mechanical compression devices such as abdominal compressors, chest compressors, belts, pistons, and combinations thereof. In various implementation, the therapy delivery component(s) 2832a may be configured to provide sensor data and/or be coupled to and/or incorporate sensors. For example, the electrotherapy electrodes 2838a may provide sensor data such as transthoracic impedance, ECG, heart rate, etc. Further the electrotherapy electrodes 2838a may include and or be coupled to a chest compression sensor. As another example, the ventilation devices 2838b may be coupled to and/or incorporate flow sensors, gas species sensors (e.g., oxygen sensor, carbon dioxide sensor, etc.), etc. As a further example, the intravenous devices 2838c may be coupled to and/or incorporate temperature sensors, flow sensors, blood pressure sensors, etc. As yet another example, the compression devices 2838d may be coupled to and/or incorporate chest compression sensors, patient position sensors, etc. The therapy delivery control modules 2818 may be configured to couple to and control the therapy delivery component(s) 2832a, respectively.

The one or more sensor(s) 2832b and 2836 and/or the therapy delivery component(s) 2832a may provide sensor data. The patient data provided at the display screens of the therapeutic medical device 2802and mobile device 2804 may display the sensor data. For example, the therapeutic medical device 2802 may process signals received from the sensor(s) 2832b and/or the therapy delivery component(s) 2832a to determine the sensor data. Similarly, the mobile device 2804 may process signals received from the sensor(s) 2836 and/or sensor data from the sensors 2832b received via the therapeutic medical device 2802to determine the sensor data.

Referring to FIG. 29, an example of a working view window at the mobile device is shown. As similarly discussed above in regard to FIGS. 14A and 14B, the working view window 1415 may be accessed via the working view tab 1340 on the mobile device 105 and may provide a presentation of the data available on the medical device display in a manner that differs from that of the medical device display. In an implementation, the working view window 1415 may provide physiologic parameters including, but not limited to, an ECG waveform 1320a, an EtCO2 waveform 1320d, an SpO2 waveform 1320c, EtCO2 numeric data 1325a, SpO2 numeric data 1325b, heart rate 13215a, body temperature 1325e, and non-invasive blood pressure 1325d. In an implementation, the CSG engine 120 may detect a change in the medical state of the patient that corresponds to a degradation in that medical state. In response to the detected degradation, the CSG engine 120 may modify the display of parameters at the mobile device 105. For example, the modification may include providing a user notification of the detected degradation in the working view window. For example, the change in the medical state of the patient may automatically trigger the user notification 2910 in the user notification window 1450. The user notification 2910 may include an identification of the detected degradation and/or one or more critical physiologic parameters that correspond to the detected degradation.

For example, the degradation in the medical state may be respiratory distress or acute respiratory failure (ARF) and the CSG engine 120 may detect this degradation due to a change in one or more parameters monitored by the medical device(s) 180. In an implementation, in response to the detection of ARF, the CSG engine 120 may invoke the RD CSG engine 402 to provide CSG for respiratory distress at the CSG UI 110.

For a patient in respiratory distress, the user notification 1450 may include a notification of ARF. Further, the user notification may include one or more parameters displayed at the working view window 1415 and/or one or more parameters that are not displayed at the working view window 1415. The RD CSG engine 402 may provide the critical parameters 2920 for ARF in the user notification window 1450. In the example of ARF, the critical physiologic parameters for ARF may be EtCO2 and SpO2. In an implementation, the RD CSG engine 402 may control the display screen 106 of the mobile device 105 to provide the user notification window 1450 in an unoccupied portion of the working view window 1415 to emphasize and highlight the detected change and the critical physiologic parameters. In this way, the notification grabs the attention of the user without obscuring any other information available at the working view window 1415. Additionally, the RD CSG engine 402 may only populate the user notification window 1450 when there is a critical change in the patient's medical condition so that merely the population of the window 1450 serves to alert the user that some condition of the patient requires additional and immediate attention.

The RD CSG engine 402 identifies the critical physiologic parameters as those that require a caregiver's attention as high priority indicators of a downturn and likely life threatening change in a patient's condition. As such the RD CSG engine 402 curates the display of information at the CSG UI 110 to improve the efficiency and effectiveness of care provided to the patient. The large amount of data that is provided at a medical device display screen, or similarly at the device view screen 1315 or 1316, the trend view 1515, and the working view 1415 screens shows an overall state of a patient.

The RD CSG engine 402 may provide one or more controls for guidance selection options. For example, the RD CSG engine 402 may include the continue control 2930 for an option to continue instructions and/or the cancel control 2940 for an option to end or exit guidance, at the working view window 1415. In an implementation, the RD CSG engine 402 may provide these controls in proximity to the user notification 2910. Activation of the continue control 2930 automatically transitions the display at the mobile device 105 to the CSG UI 110. Activation of the cancel control 2940 disallows an automatic transition to the CSG UI 110. In an implementation, activation of the cancel control 2940 may clear the user notification 2910 and/or the display of critical parameters 2920. The interaction between the caregiver and a user interface of the mobile device 105, for example, a user activation of the continue control 2930, may cause the RD CSG engine 402 to provide instructions for at least one clinical intervention associated with the detected degradation.

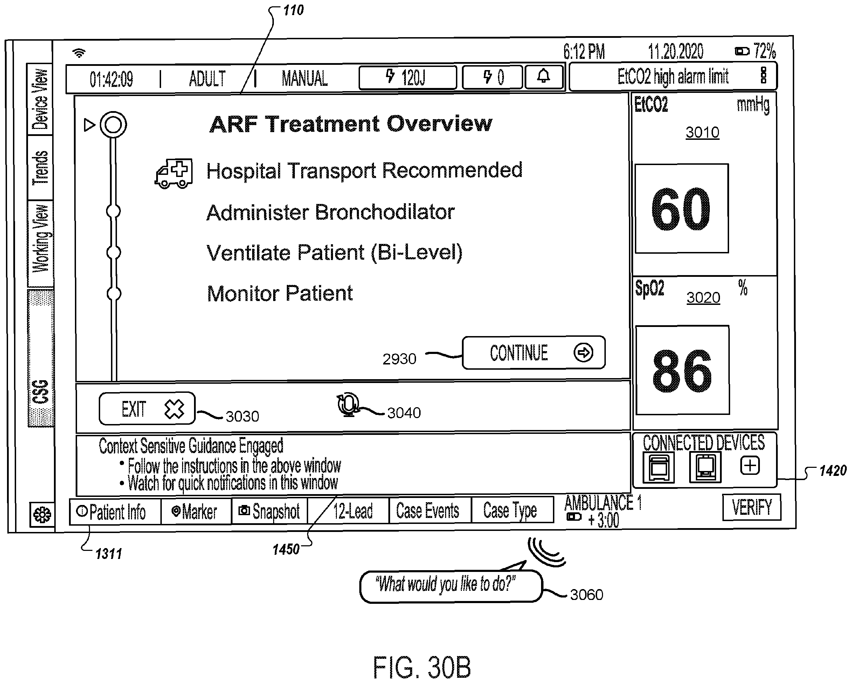

FIGS. 30A and 30B shows examples of a CSG user interface with context sensitive guidance engaged. In response to an activation of the CSG selection tab 1370 and/or an activation of the continue control 2930, the CSG engine 120 may provide the CSG UI 110 at the mobile device 105. In the case of respiratory distress, the RD CSG engine 402 may provide CSG for RD at the CSG UI 110. The RD CSG engine 402 may initiate the CSG with an overview of treatment, for example, the overview 3070 for ARF as shown at least in FIG. 30A. Additionally, the CSG engine 120 provides a CSG engagement notification 3084. The RD CSG engine 402 may limit the number of physiologic parameters provided at the CSG UI 110 in response to the engagement of CSG.

A comparison of the CSG UI 110 in FIG. 30A and the working view window 1415 in FIG. 29 shows a difference in the number of parameters provided in each window. The RD CSG engine 402 replaces the display of multiple physiologic parameters, as seen in the working view window 1415, with a display of only the critical physiologic parameters in the critical parameter windows 3010 and 3020. Thus, the RD CSG engine 402 modifies the display at the mobile device 105 with this replacement. For example, the CSG UI 110 may exclude physiologic parameters other than the critical physiologic parameters to emphasize the critical physiologic parameters. However, the selection tabs 1310, 1340, 1350, and 1370 allow the user to toggle to any other window and view all of the physiologic parameters available at the mobile device 105. Additionally, the exit control 3030 enables the user to activate this control and return to the working view window 1415 should the user prefer not to view or obtain the express guidance from the RD CSG engine 402.

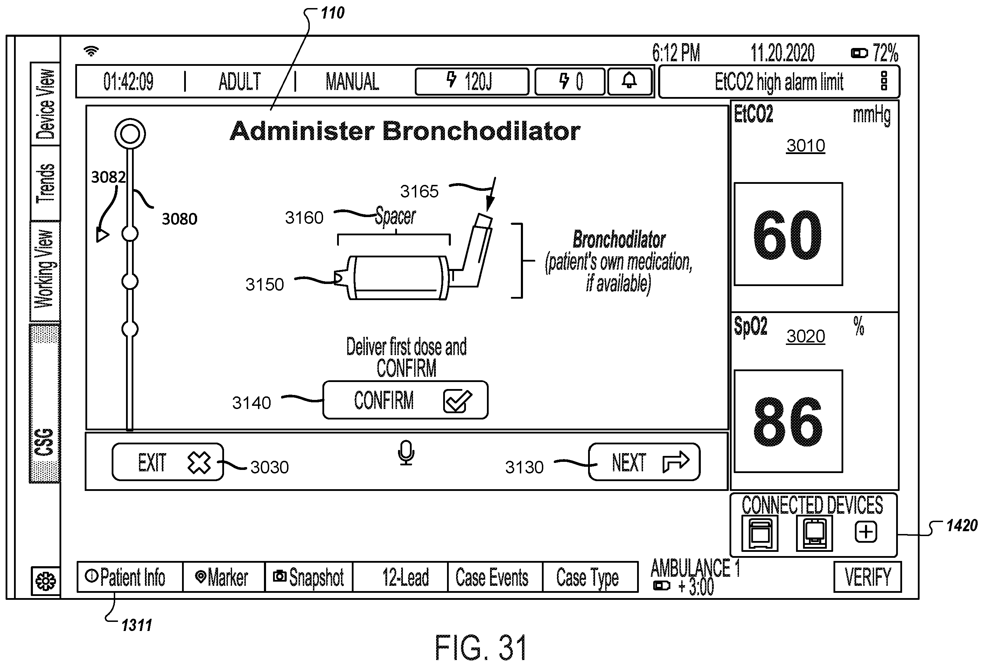

At the overview level, the caregiver has an option to continue with CSG (e.g., via the continue control 2930) or to exit CSG and return to the working view (e.g., via the exit control 3030). If the caregiver selects the continue control, the RD CSG engine 402 provides a sequence of UI screen displays to guide the caregiver through the clinical interventions of the overview 3070. FIGS. 31-40 provide examples of the sequence of UI screen displays for RD CSG, and features of these display screens are described in further detail below.

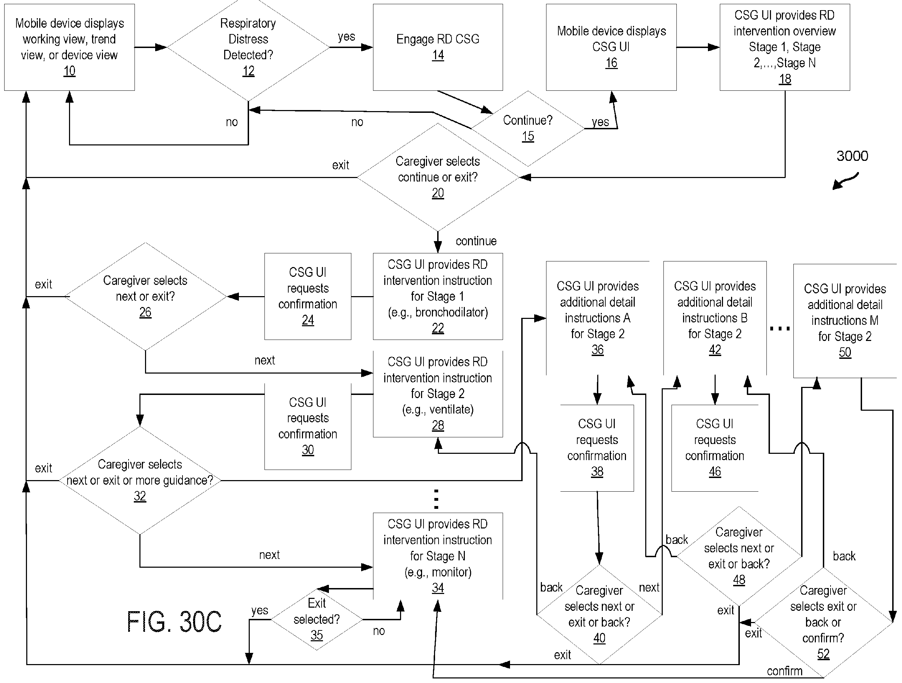

FIG. 30C provides a summary flowchart 3000 for this example of a sequence of display screens for RD CSG. The method 3000 is, however, an example only and not limiting. The method 3000 can be altered, e.g., by having stages added, removed, rearranged, combined, and/or performed concurrently. The CSG engine 120 and/or the RD CSG engine 402 may perform the method 3000. The mobile device displays one of a working view, trend view, or device view at the stage 10. Upon detection 12 of respiratory distress by the CSG engine 120, the CSG engine 120 engages the RD CSG engine 402 and provides a user notification at the CSG UI 110 that CSG has been engaged. The caregiver may select to continue 15 with the CSG or opt out. If the caregiver opts out of CSG, then the mobile device continues to display the working view, trend view, or device view window. If the user selects to continue 15, then the mobile device displays 16 the CSG UI 110. The CSG UI provides 18 an RD intervention overview that includes one or more stages (e.g., stage 1, stage 2,..., stage N). In the example of FIG. 30A, the overview includes three stages, administer bronchodilator, ventilate, monitor. Often, even if a bronchodilator is not absolutely necessary medically, the bronchodilator, particularly if used with a patient's own bronchodilator medication is a relatively low risk intervention that may help and poses a very low risk of harm to the patient, if any at all. The overview shown in FIG. 30A is an example only and other overviews based on the preferences of a particular medical director may be provided. Each medical director may select preferences such as, for example, whether to begin transport to the hospital before or after administration of a bronchodilator, whether to use a bronchodilator with an inhaler or use a nebulizer, the dosage amounts for any medications and the number of doses. The CSG UI overview and the subsequent instructional steps associated with that overview reflect the medical director's preferences and are customizable by a medical director. Thus the screens provided here are only an example of one possible set of CSG screens, instructions, options, etc. In some implementations, the CSG UI may provide guidance according to a standard of care that may be common across multiple caregiver organizations and may or may not provide the same detail with regard to components like drug dosages or an order of operation, for example. Other changes to the overview and associated steps may include one or more of adding more steps, adding more caregiver questions, changing steps, changing the order of the steps, selecting different critical physiologic parameters or data as a trigger for CSG, selecting different data as the critical data, etc.

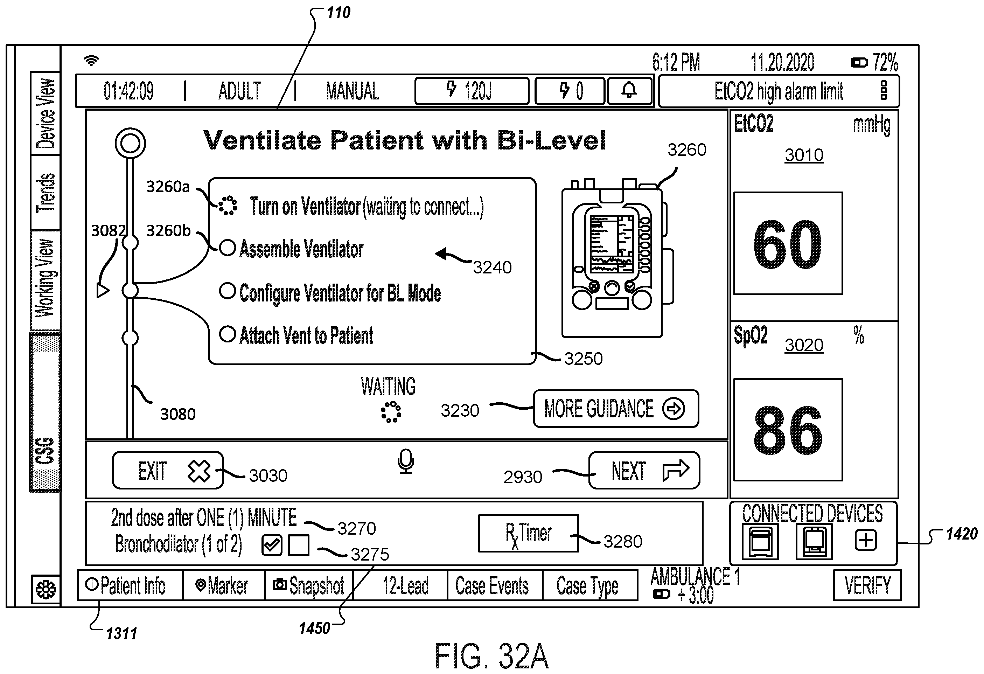

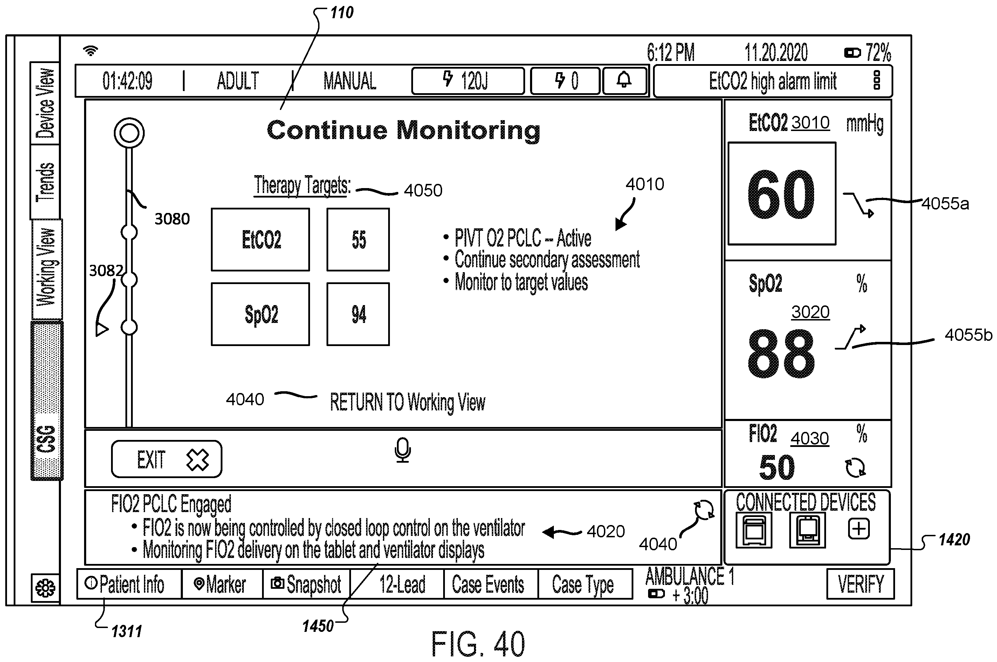

At the overview stage, the caregiver may select 20 to continue with CSG or exit. If the caregiver elects to exit, the mobile device returns to a display of the working view, trend view, or device view window. If the caregiver elects to continue, the RD CSG engine 402 provides a sequence of one or more screens for the one or more stages of the RD intervention. For example, FIG. 31 provides an example of stage 1, administer bronchodilator, FIG. 32A provides an example of stage 2, ventilate, and FIG. 40 provides an example of stage 3, monitor. At each stage, the caregiver has the option (e.g., as shown at steps 26 and 32 in method 3000) to exit CSG and return to a display of the working view, trend view, or device view window. Additionally, at each stage the CSG UI requests a confirmation (e.g., as shown at steps 24 and 30 in method 3000) that an instructed caregiver intervention has been provided to the patient. In an implementation, activation of a confirmation generates a code marker in a case file for the patient encounter. The mobile device 105 may provide an indication of the confirmation to at least one of the medical devices along with an event code indicative of the activity or intervention that is confirmed and the medical device may record the code marker in the case file. Further, once the caregiver confirms administration of an intervention, the caregiver may elect to move to a next step in the CSG guidance (e.g., as shown at steps 26 and 32 in method 3000). The caregiver may exit CSG at any time in order to view a different window at the mobile device 105. However, in order to advance through the CSG screens, the RD CSG engine 402 may require a confirmation that a current step has been completed before moving to the next step.

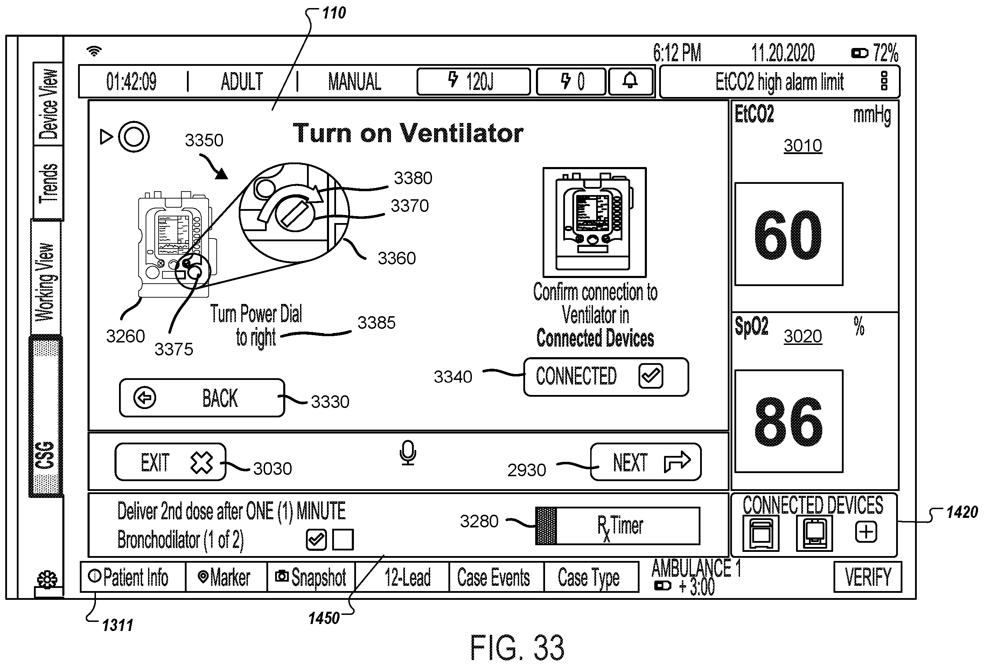

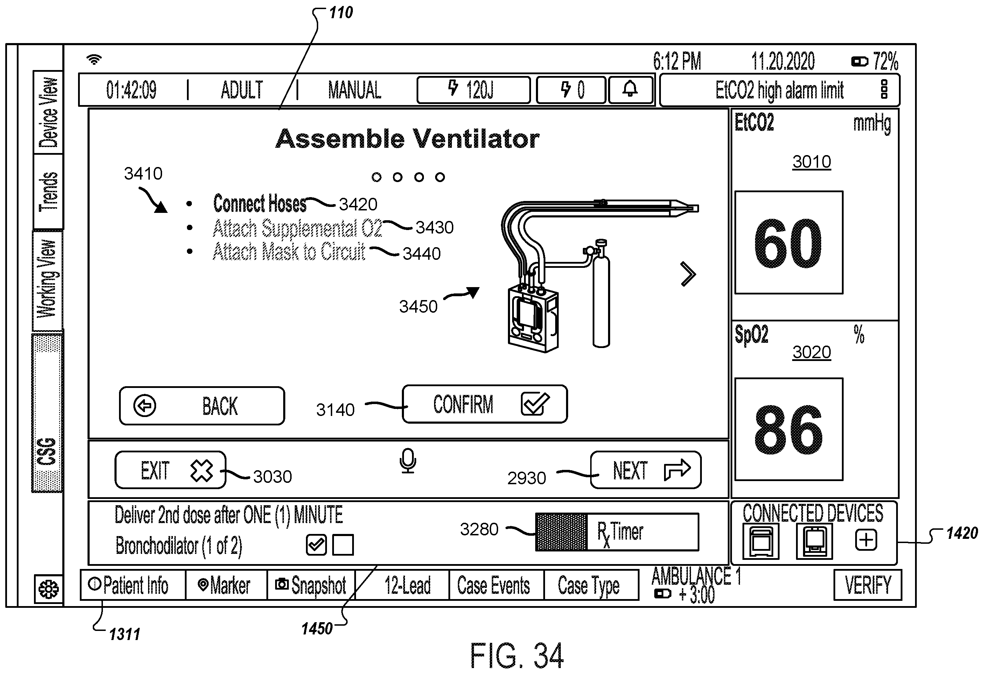

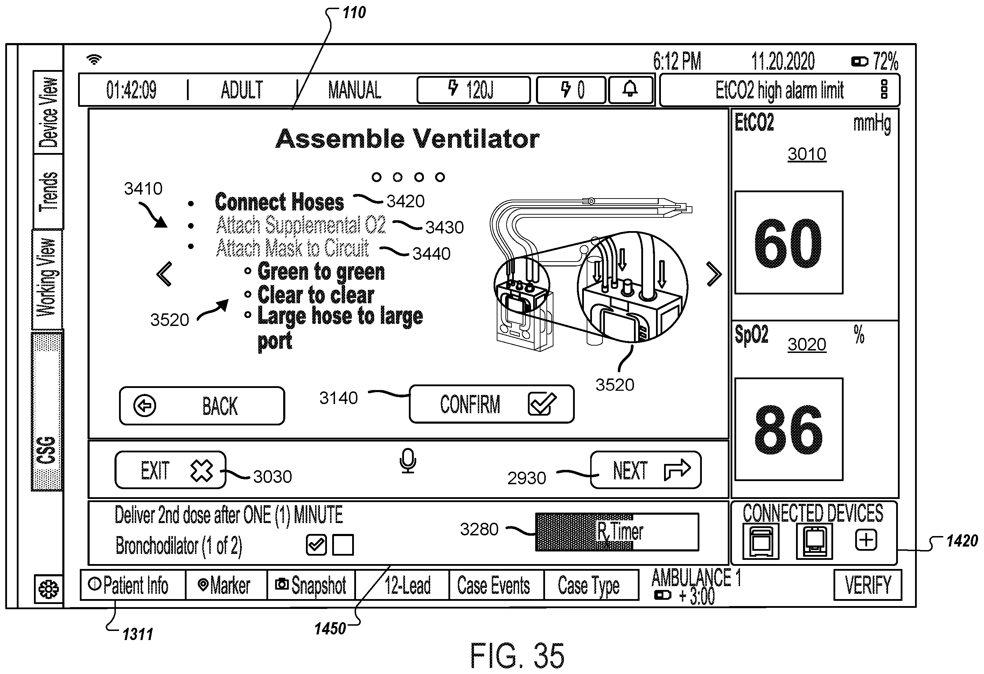

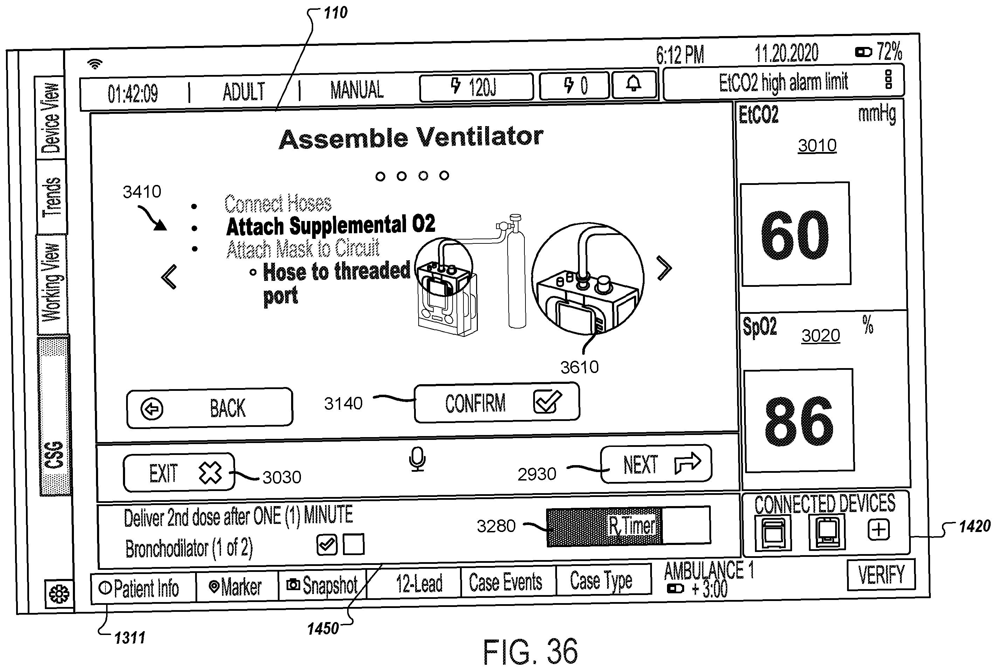

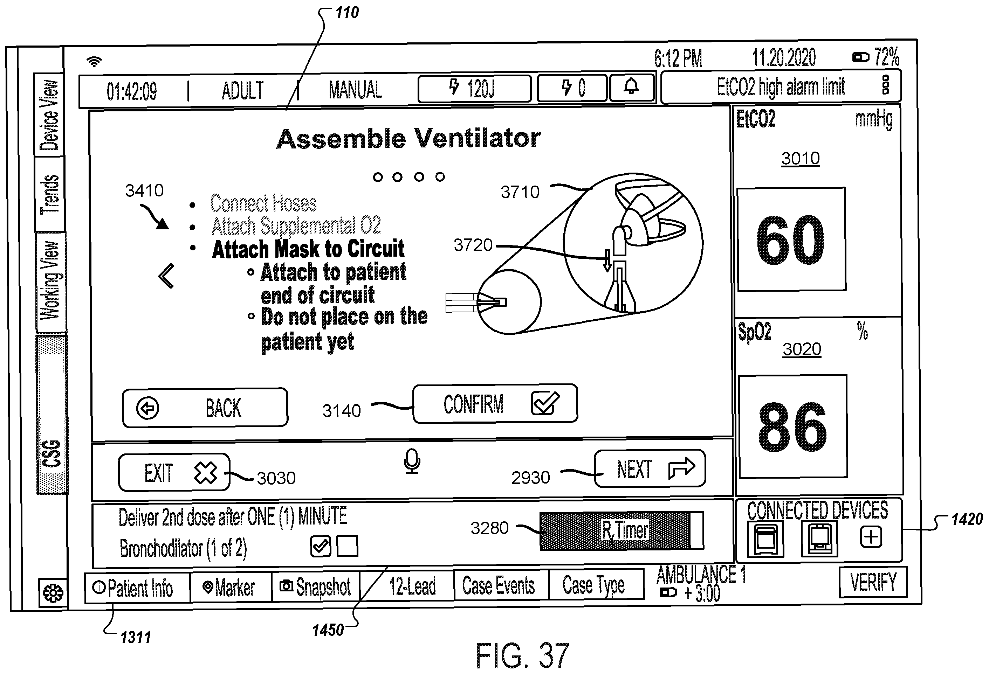

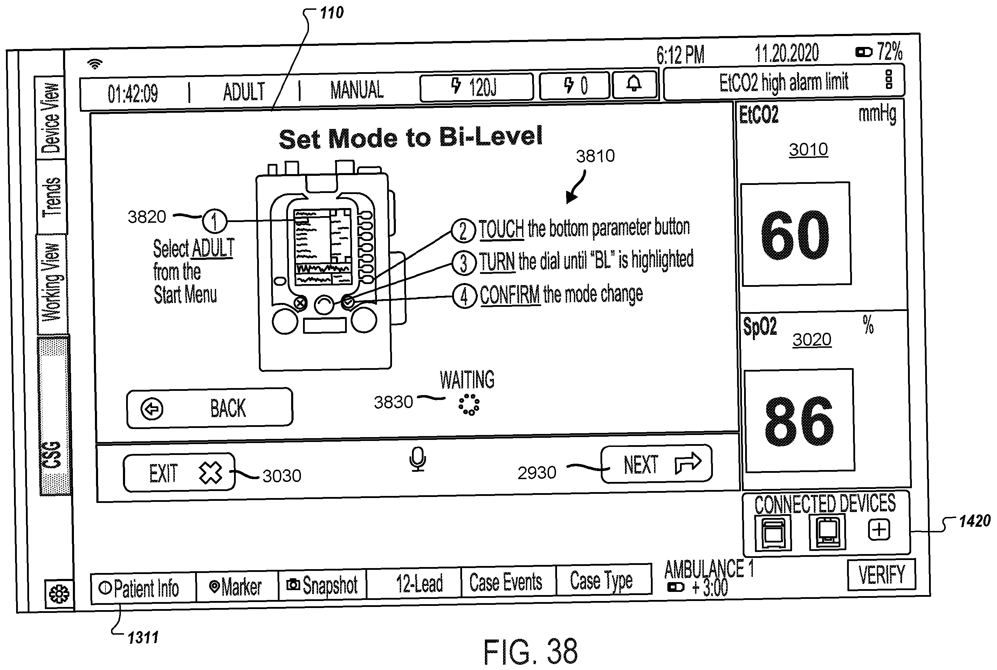

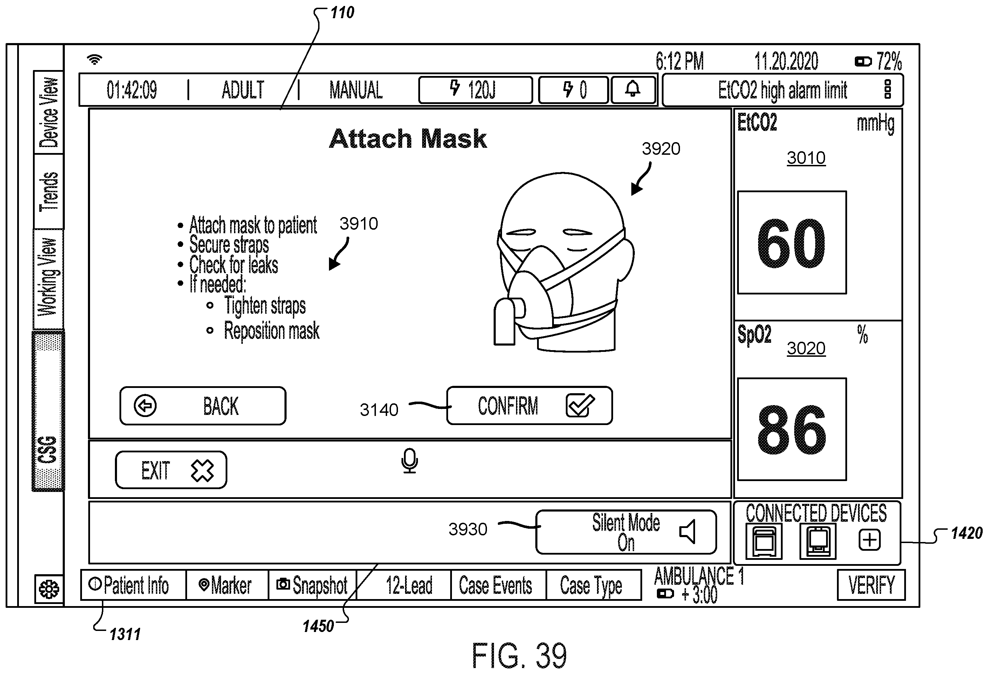

As shown, for example, in conjunction with stage 2 in the method 3000, the CSG UI 110 may provide an option for the caregiver to select additional guidance (e.g., the step 32 in the method 3000). The additional guidance may be more granular instructions. A caregiver of higher skill or longer experience may need less granular information than a lower skilled or less experienced caregiver. If the caregiver selects additional guidance, then the RD CSG engine 402 provides a sequence of one or more screens that provide a breakdown of activities associated with an intervention instruction. The breakdown provided herein is an example only and not limiting of the disclosure. Additional or fewer screens may be provided for more or less detail breakdown than described herein. The RD CSG engine 402 may provide additional detail instructions A, B,...,M (e.g., as shown at the steps 36, 42, and 50 in the method 3000). For example, for the ventilate instruction, the RD CSG engine 402 may provide detailed steps to (A) power on the ventilator (e.g., as shown in FIG. 33), (B) assemble the ventilator (e.g., as shown in FIG. 34), (C) set a mode of the ventilator (e.g., as shown in FIG. 38), (D) attach a mask to the patient to couple the patient to the ventilator (e.g., as shown in FIG. 39). The RD CSG engine 402 may further breakdown one or more of these detailed steps into further detail. For example, RD CSG engine 402 may provide the (B) assemble the ventilator instruction as a series of one or more instructions such as (B1) connect hoses (e.g., as shown in FIG. 35), (B2) attach supplemental oxygen (e.g., as shown in FIG. 36), and (B3) attach mask to circuit (e.g., as shown in FIG. 37). At each of the detailed instruction screens, the user may have an option to select to exit, proceed to a next instruction, or go back to a previous instruction (e.g., as shown at the steps 40 and 48 of the method 3000). In order to proceed to a next instruction, the RD CSG engine 402 may require a confirmation that a current step has been completed (e.g., as shown at the steps 38 and 46 of the method 3000). At the end of a final detailed step (e.g., after step 50 in the method 3000 as exemplified by FIG. 39), the caregiver may exit CSG, go back to a previous step, or confirm 52 the completion of the detailed step in order to move on the a next stage of the overall guidance. For example, as shown in FIG. 39, the RD CSG engine 402 may require confirmation that the mask has been attached to the patient to move on to the monitor stage shown for example in FIG. 40. The RD CSG engine 402 may provide 34 the final intervention instruction, for example, monitor as shown in FIG. 40 along with an option and an instruction for the user to exit 35 and return to a display of the working view, trend view, or device view window.

Referring to FIG. 31, an example of a CSG user interface with caregiver instructions for a clinical intervention is shown. The clinical intervention in this example is administration of a bronchodilator. FIGS. 32A and 33-40 provide other examples of caregiver instructions for clinical interventions, such as, ventilate a patient, attach a mask, and monitor a patient along with caregiver instructions for use and assembly of medical devices supporting or providing the clinical interventions, such as, assemble a ventilator and set a ventilator mode. The instructions for use may include basic instructions for less skilled caregivers or caregivers with limited experience with a particular type of medical device or a particular make and model of a medical device (e.g., a power-on instruction as shown, for example, in FIG. 33 or a mode selection instruction as shown, for example, in FIG. 38).

In an implementation, the CSG UI 110 may provide the critical physiologic parameters along with clinical intervention instructions. For example, as shown in FIGS. 30A-40, the CSG UI 110 shows the critical physiologic parameters 3010 and 3020 in each screen view that includes clinical intervention instructions. In an implementation, the CSG UI 110 may provide only the critical physiologic parameters and exclude other physiologic parameters in each screen view that includes clinical intervention instructions in order to keep the caregiver focused on these parameters without the distraction of other physiologic information and to minimize information interpretation tasks for the caregiver. In an implementation, the CSG UI 110 may provide the critical physiologic parameters in a consistent and same location on the window view in order to minimize confusion and information interpretation tasks for the caregiver.

In an implementation, the RD CSG engine 402 enables the user to customize the amount of guidance provided and the level of detail of that guidance via touch screen controls and/or a voice activated control. In various implementations, in addition to the user notification option to engage CSG as shown at least in FIG. 29, the RD CSG engine 402 further provides the continue control 2930 and the exit control 3030 at the overview level of CSG as shown at least in FIGS. 30A and 30B. These controls enable the user to continue the guidance at a more detailed level than the overview or exit the guidance and return to the working view window 1415.

The RD CSG engine 402 may provide the guidance selection controls at the UI in conjunction with the instructions for the at least one clinical intervention. As shown in FIGS. 29-40, CSG UI 110 provides guidance instructions for clinical interventions along with the controls that enable the user to customize that guidance. For example, in FIGS. 30A and 30B, the CSG UI 110 provides an overview of ARF treatment along with the continue control 2930 and the exit control 3030 that enable the user to either obtain more detailed instructions by continuing with CSG guidance or exit the CSG guidance and automatically return to the working view 1415. Similarly, in FIG. 31, the next control 3130 enables the user to obtain further CSG guidance by moving to another step in the overview provided in FIGS. 30A and 30B (e.g., move from "administer bronchodilator" to "ventilate patient") or exit the CSG guidance and automatically return to the working view 1415 with the exit control 3030. As shown at least in FIGS. 32A and 33-38, the next control 3130 and the exit control 3030 are provided at each detailed step within the overview sequence of clinical interaction instructions (e.g., the overview 3070). The RD CSG system of claim D11, wherein the guidance selection options enable the caregiver to select a level of detail of the provided instructions. The next control 3130 also enables the caregiver to control a pace and timing of provided instructions. The caregiver may need to control the pace and timing because of their skill level and/or because of other emergency activities that may emerge during the guidance.

As shown in FIGS. 29-40, the guidance selection controls include, but are not limited to, a connect devices control 1402, the continue control 2930, the cancel control 2940, the exit control 3030, a next control 3130, a drug administration confirmation control 3140, a device connection confirmation control 3340, a more guidance control 3230, a back control 3330, and a silence mode on/off control 3930. Each of these touch screen controls and/or the voice controls enables a guidance selection option for the user. The user may interact with the CSG UI 110 by selecting a touchscreen control and/or selecting a UI control with a mouse, a keyboard command, a stylus, and/or another UI pointer. For example, the continue control 2930 enables an option to continue CSG instructions, the next control 3130 enables an option to proceed to an next step in a series of CSG instructions for a clinical intervention, the exit control 3030 enables an option to exit CSG instructions, the more guidance control 3230 increase a detail level for CSG, the back control 3330 enables a user to return to a previous instruction in a series of CSG instructions for a clinical intervention, and the silence mode on/off control 3930 enables the user to mute or unmute audible UI output. The silence mode on/off control 3930 may be of critical importance in a military setting where noise could endanger a mission or provoke an assault and may be of critical importance in a noisy emergency environment where background noise may interfere with voice control or understanding of audible CSG instructions.

In an implementation, interactions between a caregiver and the CSG UI 110 may include a voice command or utterance from the caregiver (e.g., the utterance 3050 in FIG. 30A) and/or audible output (e.g., the output 3060 in FIG. 30B) from the CSG UI 110. The CSG UI 110 may provide a digital assistant, as indicated for example by the icon or voice activated control 3040, to allow user control of and input to the RD CSG engine 402 via natural language processing. The voice command or utterance may conform to a pre-determined command list and/or may be an unstructured utterance that is interpreted by the natural language processor. For example, the caregiver may say "continue" or "next" in accordance with a pre-determined command list. Additionally or alternatively, the caregiver may say "I need more help" or "what do I do now" as examples of unstructured text that the natural language processor determines to correspond to structured text of "continue" or "next."

In an implementation, the caregiver 104 may activate the digital assistant with an utterance 3050 (e.g., the user may utter "Digital Assistant" or another command or moniker to activate voice control). In response, the RD CSG engine 402 may provide audible information 3060. For example, the RD CSG engine 402 may audibly query the user for more information or clarification (e.g., audible information may include a question such as "what would you like to do?") and/or may audibly provide any or all of the instructions shown on the CSG UI 110 and/or one or more of the parameters available at the mobile device 105. The voice activated control 3040 may also provide the functions of one or more of the touchscreen controls for the CSG UI 110 as discussed herein. For example, one or more of the audible utterances of "continue," "cancel," "exit," "confirm," "back," "next," "more guidance,", "silence," "connect device," "back," "working view," "trends," "device view," or synonyms or other equivalent function terms and/or combinations thereof may cause the RD CSG engine 402 to perform the same function as the associated touch screen controls 1402, 2930, 2940, 3030, 3130, 3140, 3340, 3230, 3330, and 3920.

As shown in FIGS. 30A-40, the RD CSG engine 402 may provide instructions for at least one clinical intervention as instructions for a caregiver. Examples in these figures include instructions for hospital transport 3072, administration of a bronchodilator 3074, ventilation of a patient 3076, and monitoring a patient 3078. The CSG UI 110 provides a clinical intervention sequence progress bar 3080 and pointer 3082 at each screen to remind the caregiver of the position of a current clinical treatment within the overview. For example, as shown at least in in FIG. 31, the position of the pointer 3082 moves along the progress bar 3080 as the CSG UI 110 progresses from the overview to a second step of administration of a bronchodilator 3074. As shown in FIG. 32A, the position of the pointer 3082 along the progress bar 3080 moves again as the CSG UI 110 progresses to the third step of ventilation of a patient 3076. Finally, as shown in FIG. 40, the pointer 3082 moves further down the progress bar 3080 to indicate that the instructions have reached the final stage of the overview to monitoring a patient 3078.

For each of the clinical intervention instruction stages (e.g., administration of a bronchodilator 3074, ventilation of a patient 3076, and monitoring a patient 3078), the CSG UI 110 provides various care instruction details. As one example, the details may be provided as a list of instructions (e.g., the overview 3070, the ventilation instruction sequence 3240 shown in FIG. 32A, ventilator assembly instructions 3410 shown in FIGS. 34, 35, 36, and 37, the ventilation mode instructions 3810 shown in FIG. 38, the mask attachment instructions 3910, and the monitoring instructions 4010).

In some examples, the CSG UI 110 is configured to a visually distinguish between a current step in an instruction list and one or more of subsequent and previous steps in the instruction list. For example, the pointer 3082 visually indicates a current step in the overview 3070. As another example, the ventilation list 3240 may be provided within an expanded detail window 3250. The window 3250 extends from the progress bar 3080 to indicate a position of this list within the overview and to indicate that the list provides a detailed view of a single step in the overview. Additionally, the ventilation list 3240 may include bullet indicators that may be broken (e.g., bullet indicator 3260a) or solid (e.g., bullet indicator 3260b). The broken bullet indicator indicates a step in progress and the solid bullet indicator indicates an upcoming step.

As a further example, the elements of a list may be provided with combinations of colors, fonts, grey scale, bold type, character size, etc. to distinguish between a current step and upcoming steps. FIGS. 34, 36, and 37 illustrate examples of visual distinct instructions for three stages of ventilator assembly, "connect hoses," "attach supplemental O2," and "attach mask to circuit." As each instruction becomes the current instruction, the CSG UI 110 provides the current instruction in bold face (e.g., current step 3420 of "connect hoses") and provides the other instructions (e.g., subsequent steps 3430 and 3440) in a lighter gray scale face. The contrast in this appearance indicates that "connect hoses" is the current step in FIG. 34, "attach supplemental O2" is the current step in FIG. 36, and "attach mask to circuit" is the current step in FIG. 37. By visually distinguishing between the steps, the CSG UI 110 may focus the caregiver's attention on a current step while still providing information about an entire sequence of steps. Additionally, as shown, for example, at least in FIGS. 35, 36, and 37, additional details associated with a current step may be visual distinct from subsequent steps and may match the visual appearance of the current step. For example, the additional details 3520 are shown at least in FIG. 35 with a visual appearance similar to or identical to the current step 3420 and/or a visual appearance different from the subsequent steps 3430 and 3440).

As yet another example, FIG. 38 shows a list of mode instructions 3810 for a ventilator. In this list, the steps are distinguished from one another by physical placement (e.g., instruction "1" appears to the left of the ventilator and instructions "2," "3," and "4" appear to the right of the ventilator) and by numerical indicators (e.g., the numerical indicator 3820).

At various stages of CSG, the CSG UI may provide an indication that the RD CSG engine 402 is waiting for completion of a task by the caregiver and/or by a medical device. For example, in addition to indicating a current step, the broken bullet indicator 3260a indicates that the system is waiting for a communicative coupling between the ventilation system 280 and the mobile device 105. As another example, in FIG. 38, a waiting icon 3830 indicates that the RD CSG engine 402 is waiting for a caregiver action. The RD CSG engine 402 may receive an indication that a caregiver action is complete by one or more of a confirmation control 3140 and/or an communication from a medical device to the mobile device 105 (e.g., via communicative coupling 199). The waiting indication may be an animated icon in which the elements of the icon change color and/or size in an animated manner.

As another example of presentation of CSG details, the CSG UI 110 may provide these as graphic images. The graphic images may be an illustrated depiction of the medical equipment or a photographic image of the medical equipment. In an implementation, the graphic images may include animated instructions or videos. The graphic images, either illustrated or photographic, may include instructional overlays, such as arrows, words, pointers etc., that indicate a method of use and/or assembly for the medical equipment. In an implementation, the RD CSG engine 402 may access stored graphic images of medical equipment based on the connected devices information. For example, the RD CSG engine 402 may tailor or match the images to a particular make and model of medical device as indicated by the connected devices information. Additionally, although depicted in black and white or grayscale in the figures, the CSG UI 110 may provide the graphic images and/or the instructional overlays in color, either in whole or in part. Further, the instructions corresponding to color images may be rendered in colors that match the images and/or the instructional overlays.

As shown at least in FIG. 32A, the CSG UI 110 may include graphic images of particular items of medical equipment to identify the equipment for a caregiver (e.g., the graphic image 3260 of the ventilator). As another example, as shown at least in FIGS. 31, 32a, 33, 34, 35, 36, 37, 38, and 39, the graphic images (e.g., illustrated depictions, photographic images, or a combination thereof) may include and/or depict instructions for caregiver use of the medical device. The CSG UI 110 may include alphanumeric instructions and/or identification information with the graphic images. For example, in FIG. 31, the graphic image 3150 of a bronchodilator includes an alphanumeric identification 3160 of a part (e.g., the spacer) of the bronchodilator along with a graphical arrow 3165 to indicate an insertion point for the medication.

As another example, in FIG. 33, the graphic image 3350 includes the graphic image 3260 of the ventilator and an expanded view window 3360 that includes an image 3370 of a power dial 3375 on the ventilator 3260 along with a graphic directional arrow 3380 and alphanumeric instructions 3385 to supplement and/or clarify the graphic overlay directional arrow 3380. The image 3370 may be an illustration or a magnification of a photograph of the power dial 3375. FIG. 37 provides another example of an expanded view window 3710 for instructions for connecting a mask to a ventilator circuit. The window 3710 includes graphical depicted instructions (e.g., the directional arrow 3720 for mask attachment).

As shown for example, at least in FIGS. 34, 35, 36, and 37, the graphic images may include expanded view windows that are associated with the listed instructions and that change in association with and according to changes in emphasis on the listed instructions. The graphic images also include varying amounts of detail according to the detail provided in listed instructions. For example, CSG UI 110 provides the highest level (i.e., least detailed overview instructions) of ventilator assembly instructions 3410 in FIG. 34 with an overview image 3450 of a ventilator with hoses and an oxygen tank. In comparison, FIG. 35 provides more detailed instructions 3520 for hose connections and, along with that, a more detailed expanded view window 3530 with graphic overlay directional arrows 3530 and 3531 to indicate specific hose connection instructions. As discussed above, the CSG UI 110 may provide color images. Thus, in the example of FIG. 35, the hoses and connection points referred to as "green" may be shown in the color green. As shown in FIG. 36, the CSG UI 110 adjusts the graphic image and expanded view window to match the listed instructions. For example, the expanded view window 3610 corresponds to the supplemental O2 attachment instructions. Thus as the CSG UI 110 changes the emphasized portion of the clinical intervention instructions to guide a caregiver, the CSG UI 110 also changes the equipment images and expanded views to illustrate the instructions. As another example, the graphic images may include images of a patient where necessary to illustrate proper attachment of a medical sensor or device to the patient (e.g., the image 3920 in FIG. 39 demonstrates a proper attachment of a mask to a patient including a position of the mask on the face relative to facial features and positions of attachment straps).

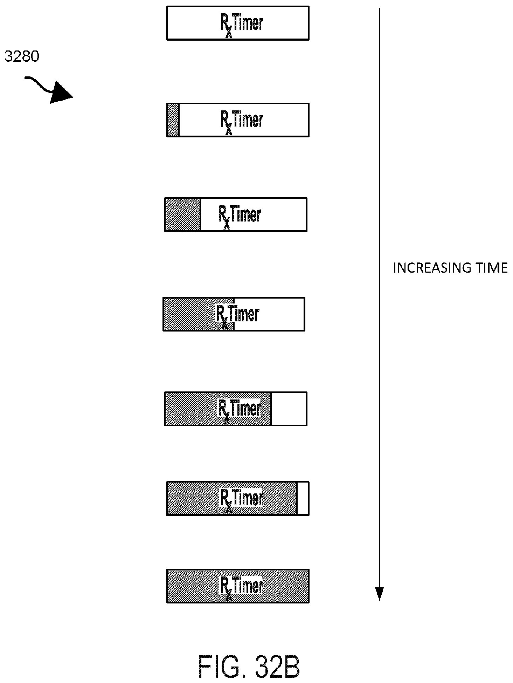

Referring again to FIGS. 32A with further reference to FIG. 32B, an example of a medication dosage timer is shown. In conjunction with an instruction to administer a pharmacologic intervention, the CSG UI may provide dosing instructions and/or reminders 3270, drug delivery confirmation controls 3275, and a dose interval timer 3280. As shown in FIG. 32B, in an implementation, the dosage time 3280 may be a UI feature that fills gradually or changes color gradually to indicate an elapsed time between medication dosages. The RD CSG engine 402 may automatically determine the length of the interval between medication doses. For example, the caregiver may scan a bar code on a bronchodilator or other medication using a camera or scanner coupled to the CSG UI 110. The RD CSG engine 402 may identify the medication based on the bar code scan and may combine that information with demographic patient information (e.g., age, weight, gender, etc.) to determine a medication dosage and dosage timing. The RD CSG engine 402 may automatically set the timer according to the determined dosage and timing. For example, as shown in FIG. 32B, as time elapses, the dosage timer box fills in with a darker color. The fraction of an area of the UI feature that is filled and/or changed color compared to the total area of the UI feature may indicate an amount of time elapsed or remaining. The change in the appearance of the dosage time 3280 is also illustrated in FIGS. 33-37.

At various stages within the CSG, the RD CSG engine 402 may request or require a caregiver confirmation in response to instructions for the at least one clinical intervention. In an implementation, the CSG UI 110 may present a confirmation control as a flashing feature. For example, if a user attempts to exit CSG and/or move to another screen within CSG the RD CSG engine 402 may cause the confirmation control to flash as a prompt for the user to activate the control. FIGS. 31, 34, 35, 36, 37, and 39 provide examples of the confirmation control 3140.

Referring to FIG. 40, an example of a CSG user interface with patient monitoring instructions for a caregiver is shown. By providing the monitoring instructions along with monitoring status, as described below, at the working view window 1415, the RD CSG engine 402 allows the caregiver to proceed with other aspects of patient care while the RD CSG engine 402 continues to monitor the patient's condition after the clinical intervention. The RD CSG engine 402 provides the monitoring information in the notification banner 1450 so that this information does not obstruct any further information on the working view 1415. The RD CSG engine 402 handles the monitoring and provides unobtrusive updates to the caregiver 104 who, in the meantime, is able to attend to other issues for the patient. If the monitored parameters indicate a degradation in the patient's medical condition, the RD CSG engine 402 will again generate an alert as shown in FIG. 29 with an option for further CSG. In an implementation, the RD CSG engine 402 may provide therapy targets 4050 for the critical parameters. Additionally or alternatively, the RD CSG engine 402 may provide indicators (e.g., the arrows 4055a and 4055b) with the critical parameters to that show a direction of change (i.e., increase or decrease) of each parameter. A comparison of the critical parameter values 3010 and 3020 to the therapy targets 4050 along with the indicators 4055a and 4055b gives the caregiver a quick and succinct picture of the patient status with regard to recovery or improvement in the detected degradation. In the case of ARF, the therapy targets for the critical physiologic parameters may be therapy targets for SpO2 and EtCO2

In an implementation, the RD CSG engine 402 may provide status updates for connected medical devices at the CSG UI 110. For example, as shown at least in FIG. 40, the CSG UI 110 provides a status update 4020 for the ventilation system 280. The CSG UI 110 may also include one or more monitored closed loop control parameters 4030. For example, the CSG UI 110 may include an indication of fraction of inspired oxygen (FiO2) for closed loop control of a ventilation system. In an implementation, the CSG UI 110 may include a closed loop control indicator 4040 to indicate that a connected medical device is in a closed loop control mode with the mobile device 105. For example, the status indication for the at least one medical device may be an indication of closed loop control of a ventilator and a FiO2 value updated in real-time.

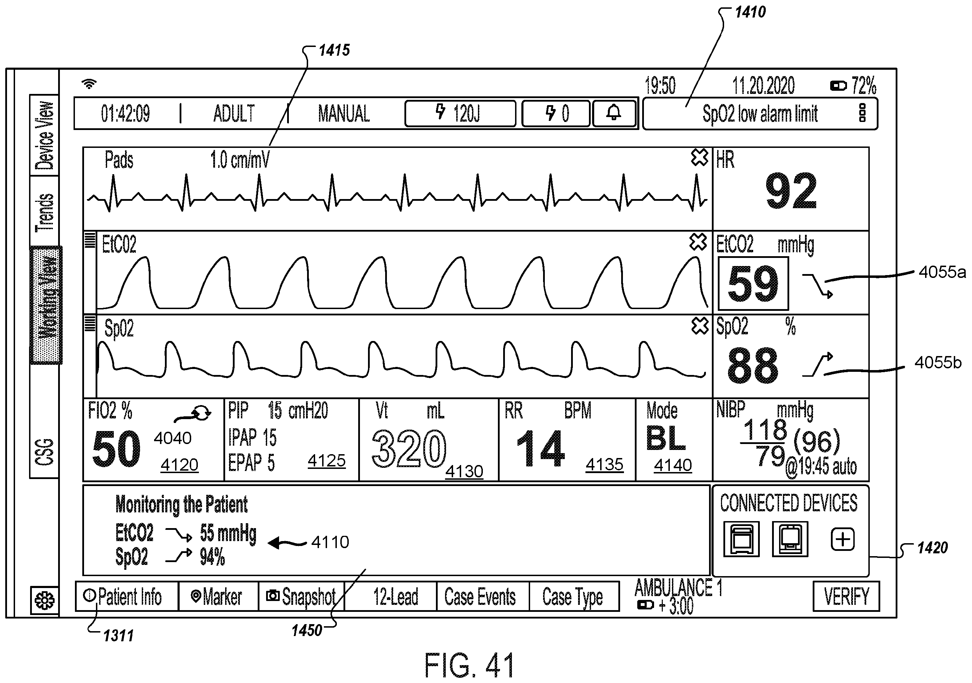

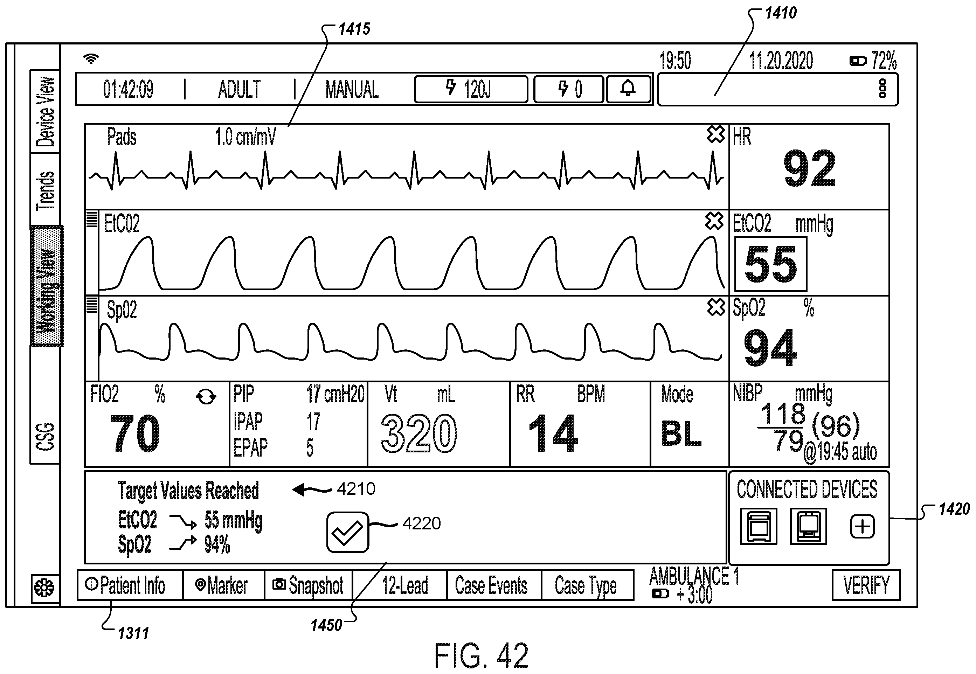

Referring to FIG. 41, an example of a working view window with patient monitoring instructions for a caregiver is shown. In an implementation, the RD CSG engine 402 may return to a working view window following completion of an instructed clinical intervention. The RD CSG engine 402 may provide the user notification banner 1450 at the working view window 1415, where the banner includes one or more of values and trend indicators for the critical physiologic parameters after completion of the at least one clinical intervention,. In order to evaluate the efficacy of a completed intervention for ARF, the RD CSG engine 402 may continue to monitor critical parameters, for example, EtCO2 and SpO2. The RD CSG engine 402 may return to t the working view window 1415, or another window available at the mobile device 105, when a patient has been stabilized in response to ARF. In the example shown in FIG. 41, the patient is connected to the ventilator, the ventilator is in a closed loop control mode, and the user notification window 1450 tracks the progress of the critical parameters towards target values 4110. The working view window may also include the directional indicators 4055a and 4055b that indicate whether the critical parameters are increasing or decreasing. As shown in FIG. 42, the RD CSG engine 402 may include a target achievement notice 4210 in the user notification banner 1450 when the critical physiologic parameters reach their target values. The notice 4210 may include a graphic indication 4220 that monitored critical physiologic parameters have reached a target or threshold and/or are within a target range. In conjunction with the notice 4210, the alarm indicator 1410 may discontinue a display of parameters. For example, the alarm indicator 1410 displays an alarm in FIG. 41, before the target values are reached, and does not display an alarm in FIG. 42 after the target values are reached.

The patient monitoring subsequent to implementation of a clinical intervention may include displaying, at the mobile device, physiologic parameters measured by a medical device used for the clinical intervention. For example, FIG. 29 shows an example of the working view screen 1415 prior to the ARF detection and prior to a connection of the patient to the ventilation system 280. The physiologic parameters shown in FIG. 29 (e.g., ECG, EtCO2, SpO2, HR, NIBP, temperature, etc.) are all available from a patient monitor/defibrillator 285. In comparison, FIG. 41 shows the working view screen 1415 after the ventilation system 280 has been introduced as a connected device, i.e., physically coupled to the patient and communicatively coupled to the mobile device 105. The working view 1415 includes one or more parameters available from the ventilation system 280 (e.g., FiO2 4120, PIP-IPAP-EPAP 4125, Vt 4130, RR 4135, and a ventilation system mode 4140) and an indication 4040 of closed loop control.

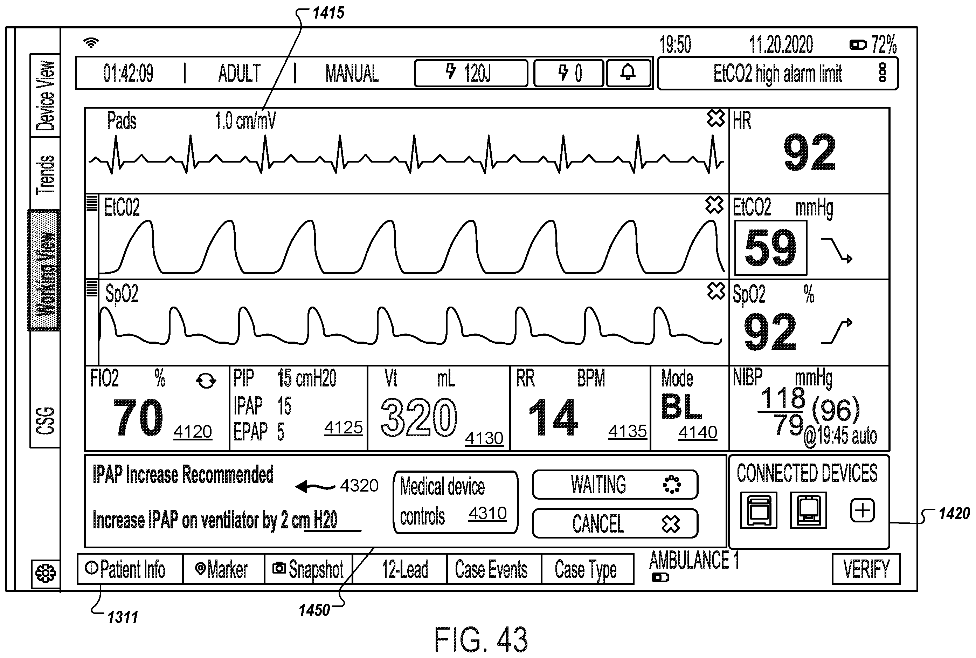

Referring to FIG. 43, an example of a CSG user interface with medical device operation instructions is shown. The medical device operation instructions may be one or more of instructions for the caregiver or remote controls that enable automated instructions to be sent to the medical device(s) 180 from the mobile device 105. In an implementation, the mobile device 105 may include one or more remote medical device controls 4310 at the UI associated with the operation of a communicatively coupled medical device. For example, the medical device controls may control the operation of the ventilation system 280, which may include respiratory aspects or CSG. In an implementation, the mobile device 105 can transmit instruction or command signals to the medical devices in response to a user activation of the medical device control 4310. For example, the RD CSG engine 402 may provide settings recommendation 4320 and the caregiver may implement those recommendations through an input to the mobile device 105. In an implementation, the mobile device 105 may transmit instruction or command signals to the medical devices automatically in response to a determination by the RD CSG engine 402 of particular medical device settings. The transmitted instruction signals from the mobile device 105 to the medical device(s) 180 may provide information, initiate one or more functional operations, or set or change operational parameters or modes of the respective medical treatment device. In a further implementation, the caregiver may utilize controls physically located at the medical device(s) and the working view window 1415 may display those settings (e.g., the PIP-IPAP-EPAP display 4125.

As shown in FIG. 43, and similarly in FIG. 14A, the mobile device 105 may provide patient treatment information associated with multiple medical treatment devices (e.g., both the patient monitor/defibrillator 285 and the ventilation system 280) and/or other data, such as caregiver performance data, respiratory parameter data, or patient respiratory status data. Whereas each medical device provides data specific to that device or able to be collected by that device, the data at each medical device is a subsystem view and not a holistic view of the patient. In contrast, by aggregating the data from multiple medical devices at the mobile device 105 and providing centralized analysis by the CSG engine 102, the CSG system described herein treats the patient as a whole and does not merely respond to a single subsystem. Furthermore, the physical location of the mobile device 105 may not be constrained by a need to couple to the patient as is the case with the medical devices. The medical devices generally require a particular and proximate position relative to the patient in order that sensors and therapy delivery devices may be properly coupled to the patient.

In some examples, the caregiver 104 can monitor patient treatment information received from the all of the medical devices 180 communicatively coupled to the mobile device 105. In addition, in some embodiments, the user interface views at the mobile device 105 may include user inputs that allow the caregiver 104 to transmit instruction signals to provide data or issue commands to control one or more functional operations of the medical device(s) 180. In some embodiments, providing the ability for a single caregiver to view case information from multiple medical treatment devices provides a technical solution to a clinical problem in that a caregiver and/or supervisor using the mobile device 7105 can provide enhanced coaching and support to other rescuers. Further, in certain emergency care situations where patient access or rescue personnel are limited, providing a single caregiver with the ability to monitor case information simultaneously from multiple medical treatment devices and transmit instruction signals to control operation of the medical treatment devices enables patients to receive advanced medical care in remote or non-traditional care environments. However, some embodiments include various roles for various users. In some embodiments, a local wireless communication channel can be established among two or more of the devices at the emergency care scene 98 to enable data to be securely and accurately shared among the devices and systems. For instance, health data about the patient 101, data indicative of treatment delivered to the patient 101, or other types of data can be exchanged over a wireless communication channel, potentially including one or more remote, offsite systems 389. This can help enable treatment by multiple rescuers to be coordinated or integrated in an efficient and accurate manner. In some examples, wired and/or wireless communication channels are established among only some of the devices involved in treatment of the patient 101 (e.g., between two of the devices). In some examples, a wireless communication channel is established among all of the devices involved in treatment of the patient 101.

While certain embodiments have been described, these embodiments have been presented by way of example only and are not intended to limit the scope of the present disclosures. Indeed, the novel methods, apparatuses and systems described herein can be embodied in a variety of other forms; furthermore, various omissions, substitutions and changes in the form of the methods, apparatuses and systems described herein can be made without departing from the spirit of the present disclosures. The accompanying claims are intended to cover such forms or modifications as would fall within the scope of the present disclosures.
We also disclose embodiments in the numbered clauses below.
1. A context sensitive guidance (CSG) system for providing clinical interventions to a patient in an emergency medical event, the CSG system comprising:
   a CSG engine comprising hardware logic and/or software logic;
   a plurality of patient interface devices configured to generate sensor signals indicative of physiologic data for the patient;
   a display device configured to provide a CSG user interface (UI); and
   at least one medical device configured to:
      couple to the plurality of patient interface devices,
      communicatively couple to the CSG engine and the display device,
      receive the sensor signals from the plurality of patient interface devices,
      generate the physiologic data from the sensor signals, and
      send the physiologic data to the CSG engine and the display device,
   wherein the CSG engine is configured to:
      receive an input from the at least one medical device comprising the physiologic data,
      evaluate the physiologic data,
      identify at least one clinical intervention for the patient based on the evaluation, and
      send an output to the at least one medical device based on the identified clinical intervention,
   wherein the at least one medical device is configured to perform at least one operation in response to the output from the CSG engine.
2. The CSG system of clause 1, wherein the at least one clinical intervention comprises a high flow nasal cannula.
3. The CSG system of clause 2, wherein the at least one clinical intervention comprises one of CPAP or bilevel ventilation.
4. The CSG system of clause 3, wherein the at least one clinical intervention comprises a pharmacological intervention.
5. The CSG system of clause 1,
   wherein the output from the CSG engine comprises instructions to perform the identified clinical intervention, and
   wherein the at least one operation performed by the at least one medical device comprises the identified clinical intervention.
6. The CSG system of clause 5,
   wherein the output from the CSG engine comprises at least one CSG event marker associated with the identified clinical intervention, and
   wherein the at least one operation performed by the at least one medical device comprises a recordation of the at least one CSG event marker at a data storage region of the at least one medical device in response to receiving the output from the CSG engine.
7. The CSG system of clause 1,
   wherein the physiologic data is first physiologic data, and
   wherein the CSG engine is configured to:
      receive second physiologic data from the at least one medical device,
      update the at least one clinical intervention for the patient based on the second physiologic data, and
      send instructions to the at least one medical device based on the updated clinical intervention.
8. The CSG system of clause 7,
   wherein the CSG engine is configured to:
   generate an etiology estimate for at least one patient presentation based on the first and second physiologic data,
   request targeted patient data based on the etiology estimate,
   receive the targeted patient data,
   convert the etiology estimate to an etiology determination based on the targeted patient data, and
   provide the etiology determination to one or more of the at least one medical device and the CSG UI.
9. The CSG system of clause 8, wherein the CSG engine is configured to:
   identify at least one pharmacological intervention based on the etiology determination,
   provide a prompt for user confirmation of the at least one pharmacological intervention at the CSG UI, and send instructions to the at least one medical device based on the at least one pharmacological intervention.
10. The CSG system of clause 9, wherein the instructions based on the at least one pharmacological intervention comprise a CSG event marker for the at least one pharmacological intervention.
11. The CSG system of clause 9, wherein the instructions based on the at least one pharmacological intervention comprise instructions to provide the at least one pharmacological intervention.
12. The CSG system of clause 1, comprising a mobile device, wherein the display device is disposed at the mobile device.
13. The CSG system of clause 12, wherein the mobile device comprises the CSG engine .
14. The CSG system of clause 12,
   wherein the mobile device is communicatively coupled to a cloud server comprising the CSG engine , and
   wherein the mobile device comprises a CSG application serviced by the CSG engine .
15. The CSG system of clause 12, wherein the at least one medical device is configured to communicatively couple to the mobile device via a wireless communication link.
16. The CSG system of clause 15, wherein the wireless communication link is at least one of a Wi-Fi link and a Bluetooth link.
17. The CSG system of clause 15, wherein the wireless communication link is a preconfigured pairing between the at least one medical device and the mobile device.
18. The CSG system of clause 1,
   wherein the CSG engine is configured to:
   receive an input from the mobile device comprising an assessment of patient breathing,
   evaluate the assessment of patient breathing for a respiratory distress presentation or a non-respiratory distress presentation, and
   if the assessment of patient breathing indicates the respiratory distress presentation, then implement a respiratory distress (RD) CSG engine, else implement a non-respiratory distress CSG engine.
19. The CSG system of clause 1, wherein the CSG engine comprises a respiratory distress (RD) CSG engine.
20. The CSG system of clause 19, wherein the RD CSG engine comprises a respiration/ventilation (R/V) CSG engine and a hemodynamic CSG engine.
21. The CSG system of clause 20,
   wherein the at least one medical device comprises a patient monitor/defibrillator and a ventilation system, and
   wherein the RD CSG engine is configured to is configured to receive input from the patient monitor/defibrillator and the ventilation system.
22. The CSG system of clause 21,
   wherein the plurality of patient interface devices comprise respiratory gas sensors configured to couple to the patient monitor/defibrillator and lung mechanics sensors configured to couple to the ventilation system, and
   wherein the input from the patient monitor/defibrillator and the ventilation system comprises respiration/ventilation data.
23. The CSG system of clause 22, wherein the respiratory gas sensors comprise a pulse oximetry (SpO2) sensor and an end-tidal carbon dioxide (EtCO2) sensor, the lung mechanics sensors comprise an airway pressure sensor and a pneumotachometer, and the respiration/ventilation data comprises SpO2 data, EtCO2 data, lung resistance data, and lung elastance data.
24. The CSG system of clause 23, wherein the RD CSG engine is configured to:
   receive the SpO2 data and the EtCO2 data,
   evaluate the SpO2 data and the EtCO2 data,
   identify the at least one clinical intervention for the patient based on the evaluation, wherein the at least one clinical intervention comprises oxygen support for the patient from the ventilation system,
   send an output to the ventilation system comprising an instruction for the ventilation system to provide oxygen support.
25. The CSG system of clause 24, wherein the oxygen support comprises supplemental oxygen therapy or supplemental oxygen with one of continuous positive airway pressure (CPAP) therapy or bilevel positive airway pressure.
26. The CSG system of clause 24, wherein the output to the ventilation system comprises an CSG event marker for the oxygen support provided by the ventilation system.
27. The CSG system of clause 24, wherein the R/V CSG engine is configured o:
   evaluate an abnormal life threatening (ALT) hemodynamic condition flag, and
   if the ALT hemodynamic condition flag is set, then divert from the R/V CSG engine to the hemodynamic CSG engine else continue to implement the R/V CSG engine.
28. The CSG system of clause 24, wherein the RD CSG engine is configured to to provide a ventilation instruction for oxygen support at the CSG UI.
29. The CSG system of clause 24, wherein the RD CSG engine is configured to:
   receive the lung resistance data and the lung elastance data,
   update the oxygen support for the patient based on the lung resistance data and the lung elastance data, and send an instruction to the ventilation system for the ventilation system to provide the updated oxygen support.
30. The CSG system of clause 29, wherein the RD CSG engine is configured to to:
   generate an etiology estimate for a respiratory distress presentation of the patient based on the SpO2 data, the EtCO2 data, the lung resistance data, and the lung elastance data,
   request targeted data from the patient monitor/defibrillator and the ventilation system, and
   convert the etiology estimate to an etiology determination based on the targeted data.
31. The CSG system of clause 30, wherein the targeted data comprises one or more of spirometry data, exhaled gas analysis data, and hemodynamic data.
32. The CSG system of clause 30, wherein the RD CSG engine is configured to:
   provide a prompt at the CSG UI for user entry of patient medical history information,
   receive the patient medical history information, and
   convert the etiology estimate to the etiology determination based on the patient medical history information.
33. The CSG system of clause 30, wherein the RD CSG engine is configured to:
   request patient medical history information from a stored medical history file,
   receive the patient medical history information, and
   convert the etiology estimate to the etiology determination based on the patient medical history information.
34. The CSG system of clause 33,
   wherein the stored medical history file comprises a medical records file stored in a remote medical records database, and
   wherein the RD CSG engine is configured to communicatively couple to the remote medical records database via a network.
35. The CSG system of clause 33,
   wherein the stored medical history file comprises a patient charting file.
36. The CSG system of clause 30,
   wherein the etiology determination comprises an obstructive respiratory disease, and
   wherein the RD CSG engine is configured to:
      identify at least one pharmacological intervention for the obstructive respiratory disease,
      provide a prompt for user confirmation of the at least one pharmacological intervention for the obstructive respiratory disease, and
      provide an output to the at least one medical device based on the at least one pharmacological
         intervention.
37. The CSG system of clause 36,
   wherein the at least one pharmacological intervention comprises a bronchodilator, and
   wherein the output to the at least one medical device comprises an instruction to the ventilation system to provide the bronchodilator.
38. The CSG system of clause 37,
   wherein the output to the at least one medical device comprises an CSG event marker comprising a time, date, and dose of the bronchodilator.
39. The CSG system of clause 30,
   wherein the etiology determination comprises a restrictive respiratory disease, and
   wherein the RD CSG engine is configured to:
      identify at least one pharmacological intervention for the restrictive respiratory disease,
      provide a prompt for user confirmation of the at least one pharmacological intervention for the restrictive respiratory disease, and
      provide an output to the at least one medical device based on the at least one pharmacological intervention.
40. The CSG system of clause 39,
   wherein the at least one pharmacological intervention comprises a diuretic, and
   wherein the output to the at least one medical device comprises an CSG event marker comprising a time, date, and dose of the diuretic.
41. The CSG system of clause 21,
   wherein the plurality of patient interface devices comprise hemodynamic sensors configured to couple to the patient monitor/defibrillator, and
   wherein the portion of the input from the patient monitor/defibrillator comprises hemodynamic data.
42. The CSG system of clause 41,
   wherein the hemodynamic sensors comprise a non-invasive blood pressure (NIBP) sensor and an electrode assembly configured to:
   gather electrocardiogram (ECG) data and heart rate data, and
   provide electrotherapy to the patient.
43. The CSG system of clause 41,
   wherein the hemodynamic CSG engine is configured to:
   receive the hemodynamic data,
      identify a presence or an absence of an abnormal life threatening (ALT) patient condition based on the hemodynamic data,
      in the presence of the ALT patient condition,
   set an ALT hemodynamic condition flag,
   provide an alert at the CSG UI indicative of the presence of the ALT patient condition, send a CSG event marker of the presence of the ALT patient condition to the at least one medical device, and
      in the absence of the ALT patient condition,
   provide an update at the CSG UI indicative of the absence of the ALT patient condition, send a CSG event marker of the absence of the ALT patient condition to the at least one medical device.
44. The CSG system of clause 43, wherein the hemodynamic CSG engine is configured to:
   query the patient monitor/defibrillator for a device status, and
   provide the device status at the CSG UI.
45. The CSG system of clause 43, wherein the hemodynamic CSG engine is configured to provide the hemodynamic data at the CSG UI.
46. The CSG system of clause 1, wherein the CSG engine comprises a non-respiratory distress CSG engine comprising at least one of a trauma engine, a sepsis engine, a traumatic brain injury engine, a cardiac arrest engine, and a critical care monitoring engine.
47. The CSG system of clause 1, wherein the display device comprises a touchscreen.
48. The CSG system of clause 1, wherein the display device is disposed at the at least one medical device.
49. The CSG system of clause 48, wherein the at least one medical device is configured to concurrently provide the CSG UI and an operational interface at the display device.
50. The CSG system of clause 1, wherein the CSG engine is disposed at the at least one medical device.
51. The CSG system of clause 1, wherein the at least one medical device comprises a patient monitor/defibrillator, a ventilation system, or a ventilation system coupled to a patient monitor/defibrillator.
52. The CSG system of clause 1, wherein the CSG engine comprises an RD CSG engine configured to:
   receive the physiologic data from the at least one medical device,
   control a display in real-time of the physiologic data at the CSG UI,
   evaluate the physiologic data for a change in at least one physiologic parameter represented in the physiologic data, wherein the change indicates a degradation in a medical state of the patient select at least one clinical intervention based on the degradation,
   provide instructions for the at least one clinical intervention,
   identify a subset of the physiologic data as critical physiologic data based on the degradation and the at least one clinical intervention, and
   modify the display of the physiologic data to emphasize the critical physiologic data.
53. The CSG system of clause 52, wherein the physiologic data comprises ECG, EtCO2, SpO2, heart rate, body temperature, and non-invasive blood pressure,
   the degradation in the medical state comprises acute respiratory failure (ARF), and
   the critical physiologic data comprises EtCO2 and SpO2.
54. The CSG system of clause 52, wherein the RD CSG engine is configured to:
   provide a user notification of the degradation in the medical state of the patient;
   receive a user request for context sensitive guidance; and
   modify the display of the physiologic data by replacing a display of ECG data, EtCO2 data, SpO2 data, heart rate data, body temperature data, and non-invasive blood pressure data at the display device with a display of instructions for the at least one clinical intervention and the EtCO2 and SpO2 data at the CSG UI that excludes the ECG data, the heart rate data, the body temperature data, and the non-invasive blood pressure data.
55. The CSG system of clause 54, wherein the RD CSG engine is configured to provide the user notification of the degradation at at least one of a device view window, a trends view window, or a working view window at the display device.
56. The CSG system of clause 52, wherein the RD CSG engine is configured to:
   establish a communicative coupling with the at least one medical device;
      identify the at least one medical device based on the first communicative coupling; and
   provide a connected devices window at the display device that indicates the identification of the at least one medical device.
57. The CSG system of clause 56, wherein the RD CSG engine is configured to select the at least one clinical intervention based on the identification of the at least one medical device.
58. The CSG system of clause 56, wherein the RD CSG engine is configured to provide the instructions for the at least one clinical intervention to the at least one medical device.
59. The CSG system of clause 56, wherein the RD CSG engine is configured to provide the instructions for the at least one clinical intervention at the CSG UI.
60. The CSG system of clause 59, wherein the instructions comprise instructions for caregiver use of the identified at least one medical device.
61. The CSG system of clause 60,
   wherein the identified at least one medical device is a ventilation system and
   wherein the instructions comprise at least one of ventilation system operation instructions and ventilation system assembly instructions.
62. The CSG system of clause 52, wherein the RD CSG engine is configured to provide the CSG UI window as a selectable window with at least one other selectable window at the display device, the at least one other selectable window comprising a working view window.
63. The CSG system of clause 52, wherein the RD CSG engine is configured to receive caregiver input via one or more of a touch screen entry or a voice command.
64. The RD CSG system of clause 52, wherein the instructions comprise drug delivery instructions.
65. The RD CSG system of clause 64, wherein the drug delivery instructions include a drug delivery confirmation control and a dose interval timer.
66. The RD CSG system of clause 52, wherein the RD CSG engine is configured to provide guidance selection controls at the CSG UI in conjunction with the instructions for the at least one clinical intervention.
67. The RD CSG system of clause 66, wherein the guidance selection controls enable a caregiver to select a level of detail of the instructions.
68. The RD CSG system of clause 66, wherein the guidance selection controls comprise at least one of a continue instructions control, an exit instructions control, a proceed to a next step control, an increase a detail level for guidance control, and a return to a previous instruction control, and a mute or unmute audible UI output control.
69. The RD CSG system of clause 52, wherein the RD CSG engine is configured to receive a caregiver confirmation at the display device in response to the instructions for the at least one clinical intervention.
70. The RD CSG system of clause 69, wherein the caregiver confirmation comprises one or more of a medication administration confirmation, a clinical intervention step confirmation, and a medical device connection confirmation.
71. The RD CSG system of clause 69, wherein the RD CSG engine provides a signal indicative of an event marker to the at least one medical device in response to the caregiver confirmation.
72. A context sensitive guidance (CSG) system for providing resuscitative care to a patient during a medical event, the CSG system comprising:
   a CSG engine comprising hardware logic and/or software logic;
   a medical device system communicatively coupled to the CSG engine and a mobile computing device, the medical device system comprising:
      a plurality of patient interface devices configured to generate sensor signals indicative of physiologic data for the patient during the medical event,
      a medical device screen for presenting the physiologic data, and
      at least one medical device processor operably coupled with the plurality of patient interface devices and the medical device screen, the at least one medical device processor configured to:
         receive the sensor signals corresponding to the patient,
         generate the physiologic data based on the sensor signals,
         display the physiologic data at the medical device screen in a first display format, and
         transmit the physiologic data to the CSG engine and the mobile device; and
         the mobile computing device comprising:
            a mobile device display screen configured to provide a user interface, and
            a mobile device processor operably coupled to the mobile device display screen and configured to:
               receive the physiologic data from the medical device system, and
               cause display of the physiologic data at the user interface as a real time view of the physiologic data displayed at the medical device screen in a second display format that
            provides a visual reproduction of the first display format,
         wherein the CSG engine is configured to:
            receive the physiologic data from the medical device system,
            receive user input at the user interface,
            evaluate the physiologic data and the user input,
            identify a clinical intervention for the patient based on the evaluation,
            generate output for the user interface based on at least one of the clinical intervention and the evaluation, and
            send an output to the at least one medical device based on the identified clinical intervention,
         wherein the at least one medical device is configured to perform at least one operation in response to the output from the CSG engine.
73. The CSG system of clause 72, wherein the visual reproduction of the first display format at the second display format comprises a replication of the first display format.
74. The CSG system of clause 72, wherein the visual reproduction of the first display format at the second display format comprises a rendering in which one or more visual aspects of the physiologic data presented in the second display format are adjusted relative to the first display format.
75. The CSG system of clause 72, wherein the mobile device processor is configured to cause the user interface at the mobile device to:
   provide a plurality of view windows comprising a device view window configured to provide the real time view of the physiologic data in the second display format,
   capture a user selection of a particular view window, and
   provide the particular view window based on the user selection.
76. The CSG system of clause 75, wherein at least one of the plurality of view windows is a scrollable interface configured to provide more information than is displayed in the device view window.
77. The CSG system of clause 75, wherein the device view window includes an ECG waveform, a pulse oximetry waveform, invasive blood pressure (IBP) and/or a CO2 waveform.
78. The CSG system of clause 75, wherein the device view window includes current values for at least one of peripheral capillary oxygen saturation (SpO2¬), carbon monoxide saturation (SpCO), methemoglobin (SpMet), total hemoglobin (SpHB), blood oxygen content (SpOC), pleth variability index (PVI), perfusion index (PI), end-tidal carbon dioxide (ETCO2), non-invasive blood pressure, invasive blood pressure value, heart rate (HR), respiration rate, fraction of inspired oxygen (FiO2), and temperature.
79. The CSG system of clause 75, wherein the plurality of view windows comprises one or more of a trend window and a working window.
80. The CSG system of clause 79, wherein the mobile device processor is configured to:
   generate trend data from the physiologic data, the trend data comprising physiologic data values over time, and
   cause the user interface to provide the trend data in the trend window.
81. The CSG system of clause 80, wherein the trend data comprises at least one of SpO2, ETCO2, systolic blood pressure, diastolic blood pressure, mean arterial pressure, or heart rate values over time.
82. The CSG system of clause 80, wherein the trend window comprises display of the trend data in a graphical format, a tabular format, or a combination thereof.
83. The CSG system of clause 79, wherein the working window comprises caregiver feedback data.
84. The CSG system of clause 83, wherein the caregiver feedback data comprises one or more of chest compression feedback, ventilation feedback, and CPR performance summary data.
85. The CSG system of clause 83, wherein the working window comprises one or more customized display sections for a respective caregiver role and the caregiver feedback data is customized feedback based on the respective caregiver role.
86. The CSG system of clause 75, wherein the plurality of view windows comprises a CSG window configured to capture the user input for the CSG engine and provide the output for the user interface from the CSG engine.
87. The CSG system of clause 86,
   wherein the output for the user interface comprises a prompt for a user entry by a caregiver, and
   wherein the user input comprises information entered by the caregiver at the CSG window in response to the prompt for data entry.
88. The CSG system of clause 87, wherein the prompt for user entry comprises a prompt for patient assessment data.
89. The CSG system of clause 88, wherein the patient assessment data comprises respiratory distress presentation data.
90. The CSG system of clause 89, wherein the prompt for user entry comprises a prompt to confirm the respiratory distress presentation data and/or a prompt to save the respiratory distress presentation data in a case file for the medical event.
91. The CSG system of clause 87, wherein the prompt for user entry comprises a prompt for a CSG event marker.
92. The CSG system of clause 91, wherein the prompt for the CSG event marker comprises a prompt for user entry of the CSG event marker.
93. The CSG system of clause 92, wherein the prompt for the CSG event marker comprises a prompt for user confirmation in response to automatic recordation of the CSG event marker by the CSG engine.
94. The CSG system of clause 91, wherein the prompt for user entry comprises a prompt for a summary of previously entered CSG event markers.
95. The CSG system of clause 91, wherein the CSG event marker comprises a pharmacological intervention marker, a ventilation intervention marker, a hemodynamic intervention marker, an intervention equipment marker, an etiology marker, or a physiologic data evaluation marker.
96. The CSG system of clause 91, wherein the CSG event marker comprises a customizable event marker for manually entering an event marker name.
97. The CSG system of clause 91, wherein the CSG engine is configured to, responsive to receiving the user entry of the CSG event marker, cause display of the CSG event marker at the CSG window.
98. The CSG system of clause 91, wherein the CSG engine is configured to transmit the CSG event marker to the medical device system in response to the user entry with an instruction to record the CSG event marker at the medical device system.
99. The CSG system of clause 87, wherein the prompt for user entry comprises a prompt for patient medical history data.
100. The CSG system of clause 99, wherein the prompt for patient medical history data comprises a prompt to request import medical history information from one or more of an EMS electronic patient care record, a hospital electronic health record, and a health information exchange.
101. The CSG system of clause 100, wherein the CSG engine is configured to import the medical history information in response to the import request.
102. The CSG system of clause 87, wherein the prompt for user entry comprises a prompt for user confirmation of an automatic import of medical history from one or more of an EMS electronic patient care record, a hospital electronic health record, and a health information exchange.
103. The CSG system of clause 87, wherein the prompt for user entry comprises a prompt to perform the clinical intervention.
104. The CSG system of clause 103, wherein the prompt to perform the clinical intervention includes a prompt for user confirmation.
105. The CSG system of clause 103, wherein the clinical intervention comprises a pharmacological intervention, a ventilation intervention or a hemodynamic intervention.
106. The CSG system of clause 103, wherein the prompt for the clinical intervention comprises an intervention metric.
107. The CSG system of clause 87, wherein the prompt for user entry comprises a prompt for user confirmation of an automatic intervention instruction to the medical device system.
108. The CSG system of clause 86, wherein the CSG window is configured to provide the physiologic data from the medical device system and the evaluation of the physiologic data from the CSG engine.
109. The CSG system of clause 108, wherein the physiologic data comprises at least respiratory gas data and lung mechanics data.
110. The CSG system of clause 109, wherein the physiologic data comprises hemodynamics data.
111. The CSG system of clause 108, wherein the CSG window is configured to provide a medical device clinical intervention status.
112. The CSG system of clause 86, wherein the output for the user interface comprises at least one alarm.
113. The CSG system of clause 112, wherein the user input comprises a confirmation of the at least one alarm.
114. The CSG system of clause 86, wherein the output for the user interface comprises a user notification of at least one medical device instruction.
115. The CSG system of clause 114, wherein the at least one medical device instruction is a medical device instruction automatically generated by the CSG engine.
116. The CSG system of clause 115, wherein the user input comprises a confirmation of the medical device instruction automatically generated by the CSG engine.
117. The CSG system of clause 114, wherein the at least one instruction is a medical device instruction manually entered at the CSG window.
118. The CSG system of clause 114, wherein the at least one instruction is an instruction to perform the clinical intervention.
119. The CSG system of clause 114, wherein the at least one instruction is an instruction to record a CSG event marker.
120. The CSG system of clause 114, wherein the at least one instruction is an instruction to monitor the patient.
121. The CSG system of clause 86, wherein the output for the user interface comprises etiology information.
122. The CSG system of clause 121, wherein the etiology information comprises an etiology automatically determined by the CSG engine.
123. The CSG system of clause 122, wherein the user input comprises a confirmation of the automatically determined etiology.
124. The CSG system of clause 121, wherein the etiology information comprises etiology information manually entered at the CSG window.
125. The CSG system of clause 72,
   wherein the output to the at least one medical device comprises instructions to perform the identified clinical intervention, and
   wherein the at least one operation performed by the medical device system comprises the identified clinical intervention.
126. The CSG system of clause 72,
   wherein the physiologic data is first physiologic data, and
   wherein the CSG engine is configured to to:
      receive second physiologic data from the medical device system,
      update the clinical intervention for the patient based on the second physiologic data, and
      send instructions to the medical device system based on the updated clinical intervention.
127. The CSG system of clause 126,
   wherein the CSG engine is configured to:
   generate an etiology estimate for at least one patient presentation based on the first and second physiologic data,
   request targeted patient data based on the etiology estimate,
   receive the targeted patient data,
   convert the etiology estimate to an etiology determination based on the targeted patient data, and
   provide the etiology determination to one or more of the medical device system and the user interface.
128. The CSG system of clause 72, wherein the mobile device comprises the CSG engine .
129. The CSG system of clause 72,
   wherein the mobile device is communicatively coupled to a cloud server comprising the CSG engine , and
   wherein the mobile device comprises a CSG application serviced by the CSG engine .
130. The CSG system of clause 72, wherein the CSG engine comprises a respiratory distress (RD) CSG engine.
131. The CSG system of clause 130, wherein the RD CSG engine comprises respiratory distress CSG engine comprises a respiration/ventilation (R/V) CSG engine and a hemodynamic engine.
132. The CSG system of clause 131,
   wherein the medical device system comprises a patient monitor/defibrillator and a ventilation system, and
   wherein the RD CSG engine is configured to:
      execute the R/V CSG engine based on medical device input from the patient monitor/defibrillator and the ventilation system, and
      execute the hemodynamic CSG engine based on medical device input from the patient monitor/defibrillator.
133. The CSG system of clause 72, wherein the medical device system is communicatively coupled to the mobile computing device via a wireless communication link.
134. The CSG system of clause 133, wherein the wireless communication link is at least one of: a Wi-Fi link, or a Bluetooth link.
135. The CSG system of clause 133, wherein the wireless communication link is a preconfigured pairing between the medical device system and the mobile device.
136. The CSG system of clause 72, wherein the mobile device display screen is a touchscreen.
137. A method of providing context sensitive guidance (CSG) for resuscitative care of a patient during a medical event, the method comprising:
   receiving an input comprising physiologic data from at least one medical device,
   evaluating the physiologic data from the at least one medical device,
   identifying a clinical intervention for the patient based on the evaluation,
   providing a CSG user output based on at least one of the physiologic data and the clinical intervention at a CSG user interface (UI) provided at a mobile device communicatively coupled to the at least one medical device, and
   sending an output to the at least one medical device based on the identified clinical intervention.
138. The method of clause 137,
   wherein the output to the at least one medical device comprises an instruction to perform the identified clinical intervention, and
   wherein the at least one operation performed by the at least one medical device comprises the identified clinical intervention.
139. The method of clause 138, comprising:
   automatically generating the instruction to perform the identified clinical intervention, and
   sending the automatically generated instruction as the output to the at least one medical device.
140. The method of clause 139, comprising:
   providing a prompt to confirm the automatically generated instruction at a CSG user interface, and
   sending the automatically generated instruction in response to the user confirmation.
141. The method of clause 138 comprising:
   providing a prompt to enter the instruction to perform the identified clinical intervention at a CSG user interface provided by the mobile device communicatively coupled to the at least one medical device, and sending the instruction in response to the user entry.
142. The method of clause 137,
   wherein the output to the at least one medical device comprises at least one CSG event marker associated with the identified clinical intervention, and
   wherein the at least one operation performed by the at least one medical device comprises a recordation of the at least one CSG event marker at a data storage region of the at least one medical device in response to receiving the output from the CSG engine.
143. The method of clause 142, comprising:
   automatically generating the at least one CSG event marker based on the identified clinical intervention, and
   sending the automatically generated CSG event marker to the at least one medical device.
144. The method of clause 143, comprising:
   providing a prompt for a user to confirm the automatically generated CSG event marker at a CSG user interface, and
   sending the automatically generated instruction in response to the user confirmation.
145. The method of clause 142, comprising:
   providing a prompt for a user to enter the at least one CSG event marker based on the identified clinical intervention at a CSG user interface provided by the mobile device communicatively coupled to the at least one medical device, and
   sending the at least one CSG event marker in response to the user entry.
146. The method of clause 145, wherein the prompt to enter the at least one CSG event marker comprises a user selection menu of a plurality of CSG event markers.
147. The method of clause 137,
   wherein the physiologic data is first physiologic data, and comprising:
   receiving second physiologic data from the at least one medical device,
   updating the clinical intervention for the patient based on the second physiologic data, and
   sending instructions to the at least one medical device based on the updated clinical intervention.
148. The method of clause 147, comprising:
   generating an etiology estimate for at least one patient presentation based on the first and second physiologic data,
   requesting targeted patient data based on the etiology estimate,
   receiving the targeted patient data,
   converting the etiology estimate to an etiology determination based on the targeted patient data, and
   providing the etiology determination to one or more of the at least one medical device and the CSG UI.
149. The method of clause 148, comprising:
   identifying at least one pharmacological intervention based on the etiology determination,
   providing a prompt for user confirmation of the at least one pharmacological intervention at the CSG UI, and
   sending an output to the at least one medical device based on the at least one pharmacological intervention.
150. The method of clause 149, wherein the output based on the at least one pharmacological intervention comprises an CSG event marker for the at least one pharmacological intervention.
151. The method of clause 149, wherein the output based on the at least one pharmacological intervention comprises an instruction to provide the at least one pharmacological intervention.
152. The method of clause 137,
   wherein the physiologic data comprises SpO2 data and EtCO2 data,
   wherein the clinical intervention comprises oxygen support for the patient from a ventilation system, and
   wherein the output to the at least one medical device comprises an instruction for the ventilation system to provide oxygen support.
153. The method of clause 152, wherein the oxygen support comprises supplemental oxygen therapy or supplemental oxygen with one of continuous positive airway pressure (CPAP) therapy or bilevel positive airway pressure.
154. The method of clause 152, comprising:
   receiving lung resistance data and lung elastance data,
   updating the oxygen support for the patient based on the lung resistance data and the lung elastance data, and
   sending an instruction to the ventilation system for the ventilation system to provide the updated oxygen support.
155. The method of clause 154, comprising:
   generating an etiology estimate for a respiratory distress presentation of the patient based on the SpO2 data, the EtCO2 data, the lung resistance data, and the lung elastance data,
   requesting targeted data from a patient monitor/defibrillator and the ventilation system, and
   convert the etiology estimate to an etiology determination based on the targeted data.
156. The method of clause 155, wherein the targeted data comprises one or more of spirometry data, exhaled gas analysis data, and hemodynamic data.
157. The method of clause 155, comprising:
   providing a prompt at the CSG UI for user entry of patient medical history information,
   receiving the patient medical history information, and
   converting the etiology estimate to the etiology determination based on the patient medical history information.
158. The method of clause 155, comprising:
   requesting patient medical history information from a stored medical history file,
   receiving the patient medical history information, and
   converting the etiology estimate to the etiology determination based on the patient medical history information.
159. The method of clause 155,
   wherein the etiology determination comprises an obstructive respiratory disease, andcomprising:
   identifying at least one pharmacological intervention for the obstructive respiratory disease,
   providing a prompt for user confirmation of the at least one pharmacological intervention for the obstructive respiratory disease, and
   providing an output to the at least one medical device based on the at least one pharmacological intervention.
160. The method of clause 159,
   wherein the at least one pharmacological intervention comprises a bronchodilator, and
   wherein the output to the at least one medical device comprises an instruction to the ventilation system to provide the bronchodilator.
161. The method of clause 160,
   wherein the output to the at least one medical device comprises an CSG event marker comprising a time, date, and dose of the bronchodilator.
162. The method of clause 155,
   wherein the etiology determination comprises a restrictive respiratory disease, and
   comprising:
      identifying at least one pharmacological intervention for the restrictive respiratory disease,
      providing a prompt for user confirmation of the at least one pharmacological intervention for the restrictive respiratory disease, and
      providing an output to the at least one medical device based on the at least one pharmacological intervention.
163. The method of clause 162,
   wherein the at least one pharmacological intervention comprises a diuretic, and
   wherein the output to the at least one medical device comprises an CSG event marker comprising a time, date, and dose of the diuretic.
164. The method of clause 137,
   wherein the physiologic data comprises hemodynamic data, and comprising:
   receiving the hemodynamic data,
   identifying a presence or an absence of an abnormal life threatening (ALT) patient condition based on the hemodynamic data,
   in the presence of the ALT patient condition,
      setting an ALT hemodynamic condition flag,
      providing an alert at the CSG UI indicative of the presence of the ALT patient condition,
      sending a CSG event marker of the presence of the ALT patient condition to the at least one medical device, and
   in the absence of the ALT patient condition,
      providing an update at the CSG UI indicative of the absence of the ALT patient condition, and
      sending a CSG event marker of the presence of the ALT patient condition to the at least one medical device.
165. The method of clause 137, comprising:
   providing a plurality of view windows at a CSG user interface comprising a device view window configured to provide a real time view of the physiologic data as a visual reproduction of a display format for the at least one medical device,
   capturing a user selection of a particular view window, and
   providing the particular view window based on the user selection.
166. The method of clause 165, wherein providing the visual reproduction comprises replicating the display format of the at least one medical device.
167. The method of clause 165, wherein providing the visual reproduction comprises rendering a display of the physiologic data in which one or more visual aspects of the physiologic data are different than the display format of the at least one medical device.
168. The method of clause 165, wherein at least one of the plurality of view windows is a scrollable interface configured to provide more information than is displayed in the device view window.
169. The method of clause 165, wherein the plurality of view windows comprises a CSG window configured to capture CSG user input and provide the CSG user output.
170. The method of clause 169, comprising
   prompting for a user entry by a caregiver, and
   wherein the CSG user input comprises information entered by the caregiver at the CSG window in response to the prompt for data entry.
171. The method of clause 169, comprising prompting for patient assessment data.
172. The method of clause 171, wherein the patient assessment data comprises respiratory distress presentation data.
173. The method of clause 172 comprising one or more of prompting a user to confirm the respiratory distress presentation data and prompting the user to save the respiratory distress presentation data in a case file for the medical event.
174. The method of clause 169, comprising prompting a user for a CSG event marker.
175. The method of clause 174, comprising prompting the user for entry of the CSG event marker.
176. The method of clause 174, comprising prompting the user for confirmation in response to an automatic recordation of the CSG event marker.
177. The method of clause 174, comprising prompting for a summary of previously entered CSG event markers.
178. The method of clause 174, comprising causing display of the CSG event marker at the CSG window.
179. The method of clause 174, comprising transmitting the CSG event marker to the at least one medical device in response to the user entry with an instruction to record the CSG event marker at the at least one medical device.
180. The method of clause 169, comprising prompting for patient medical history data.
181. The method of clause 180 comprising, prompting a user to request import medical history information from one or more of an EMS electronic patient care record, a hospital electronic health record, and a health information exchange.
182. The method of clause 181, comprising importing the medical history information in response to the import request.
183. The method of clause 169, comprising prompting for user confirmation of an automatic import of medical history from one or more of an EMS electronic patient care record, a hospital electronic health record, and a health information exchange.
184. The method of clause 169, comprising prompting a user to perform the clinical intervention.
185. The method of clause 184, comprising prompting for user confirmation of the clinical intervention.
186. The method of clause 184, wherein the clinical intervention comprises a pharmacological intervention, a ventilation intervention or a hemodynamic intervention.
187. The method of clause 185, wherein the prompt for the clinical intervention comprises an intervention metric.
188. The method of clause 169, comprising prompting for user confirmation of an automatic intervention instruction to the at least one medical device.
189. The method of clause 169, comprising:
   providing the physiologic data at the CSG window.
190. The method of clause 169, comprising generating at least one alarm based on the physiologic data.
191. The method of clause 190, comprising prompting a user for a confirmation of the at least one alarm.
192. The method of clause 169, comprising prompting a user for at least one instruction for the at least one medical device.
193. The method of clause 192, wherein the at least one instruction is an automatically generated medical device instruction and comprising prompting the user for confirmation of the automatically generated medical device instruction.
194. The method of clause 192 comprising prompting the user to manually enter at least one medical device instruction at the CSG window.
195. The method of clause 169, comprising providing automatically determined etiology information.
196. The method of clause 195, comprising prompting a user for a confirmation of the automatically determined etiology information.
197. The method of clause 195, comprising prompting a user to manually enter etiology information at the CSG window.
198. The method of clause 137, comprising:
   receiving the input comprising the physiologic data at a CSG engine comprising an RD CSG engine;
   evaluating the physiologic data, by the RD CSG engine, for a change in at least one physiologic parameter represented in the physiologic data, wherein the change indicates a degradation in a medical state of the patient;

   identifying a subset of the physiologic data as critical physiologic data based on the degradation and the identified clinical intervention; and
   modifying a data display provided at the mobile device to emphasize the critical physiologic parameters at the CSG UI.
199. The method of clause 198, wherein the physiologic data comprises ECG, EtCO2, SpO2, heart rate, body temperature, and non-invasive blood pressure,
   the degradation in the medical state comprises acute respiratory failure (ARF), and
   the critical physiologic data comprises EtCO2 and SpO2.
200. The method of clause 199, wherein modifying the CSG UI provided at the mobile device comprises: displaying all of ECG data, EtCO2 data, SpO2 data, heart rate data, body temperature data, and non-invasive blood pressure data at the mobile device;
   providing a user notification of the degradation in the medical state of the patient;
   receiving a user request for context sensitive guidance; and
   providing the CSG UI comprising instructions for the clinical intervention and a display of the EtCO2 and SpO2 data, wherein the CSG UI excludes the ECG data, the heart rate data, the body temperature data, and the non-invasive blood pressure data.
201. The method of clause 200, comprising providing the user notification of the degradation at at least one of a device view window, a trends view window, or a working view window.
202. The method of clause 137, comprising:
   establishing a communicative coupling with the at least one medical device;
   identifying the at least one medical device based on the first communicative coupling; and providing a connected devices window at the mobile device that indicates the identification of the at least one medical device.
203. The method of clause 202, comprising:
   authenticating the at least one medical device, and
   establishing the communicative coupling in response to the authentication.
204. The method of clause 203, comprising enabling patient data encryption configured to prevent access to patient data by unauthenticated devices.
205. The method of clause 203, wherein the at least one medical device is an initial medical device, the method comprising:
   establishing at least one additional communicative coupling with at least one additional medical device subsequent to the initial medical device;
   identifying the at least one additional medical device based on the at least one additional communicative coupling; and
   updating the connected devices window to include the identification of the at least one additional medical device.
206. The method of clause 205, comprising:
   receiving the physiologic data from the initial medical device;
   receiving additional physiologic data from the at least one additional medical device; and
   displaying at least one of the additional physiologic data in real-time at the mobile device.
207. The method of clause 202, comprising:
   providing instructions for the clinical intervention to the at least one medical device.
208. The method of clause 207, comprising:
   providing one or more of operational mode instructions, operational setting instructions, physiologic closed-loop control instructions to the at least one medical device.
209. The method of clause 208, wherein the at least one medical device comprises a ventilation system, the method comprising:
   generating the physiologic closed-loop control instructions based on oxygen concentration of a patient's blood, wherein the physiologic closed-loop control instructions adjust oxygen delivery during a delivery of a mechanical ventilation to the patient to maintain the oxygen concentration of the patient's blood at a desired level or range.
210. The method of clause 202, comprising providing instructions for the clinical intervention at the CSG UI, the instructions comprising instructions for caregiver use of the identified at least one medical device.
211. The method of clause 210,
   wherein the identified at least one medical device is a ventilation system,
   the method comprising providing at least one of ventilation system operation instructions and ventilation system assembly instructions.
212. The method of clause 211, comprising providing the ventilation operation instructions as one or more of power-on instructions and mode selection instructions.
213. The method of clause 212, comprising providing the ventilation system assembly instructions as one or more of hose connection instructions, oxygen tank connection instructions, and mask connection instructions.
214. The method of clause 210, comprising providing the instructions for the clinical intervention as one or more of bronchodilator administration instructions and mask positioning instructions.
215. The method of clause 210, comprising providing the instructions for the clinical intervention as patient monitoring instructions.
216. The method of clause 215, comprising providing an indication of therapy targets for the critical physiologic data and a status indication for the at least one medical device.
217. The method of clause 216,
   wherein the therapy targets for the critical physiologic data comprise therapy targets for SpO2 and EtCO2, and
   comprising providing the status indication for the at least one medical device as an indication of physiologic closed-loop control of a ventilator and a FiO2 value updated in real-time.
218. The method of clause 202, comprising providing the instruction for the clinical intervention as caregiver instructions at the CSG UI window.
219. The method of clause 218, comprising:
   providing a list of instructions, and
   visually distinguishing between a current step in the list of instructions and one or more of subsequent and previous steps in the list of instructions.
220. The method of clause 218, comprising providing the caregiver instructions as alphanumeric instructions, graphic instructions, and a combination thereof.
221. The method of clause 220, comprising providing the graphic instructions as drawings of the at least one medical device configured to guide a caregiver through use of the at least one medical device.
222. The method of clause 218, comprising providing the caregiver instructions as a hospital transport instruction.
223. The method of clause 218, comprising providing the caregiver instructions as drug delivery instructions.
224. The method of clause 223, comprising providing the drug delivery instructions as a drug delivery confirmation control and a dose interval timer.
225. The method of clause 218, comprising providing guidance selection controls at the CSG UI window in conjunction with the instructions for the at least one clinical intervention.
226. The method of clause 225, comprising providing the guidance selection controls that enable a caregiver to select a level of detail of the provided instructions; and
   receiving a selected level of detail of the provided instructions.
227. The method of clause 225, wherein the guidance selection controls comprise at least one of a continue instructions control, an exit instructions control, a proceed to a next step control, an increase a detail level for guidance control, and a return to a previous instruction control, and a mute or unmute audible UI output control.
228. The method of clause 218, comprising receiving a caregiver confirmation at the mobile device in response to the instructions for the at least one clinical intervention.
229. The method of clause 228, wherein the caregiver confirmation comprises one or more of a medication administration confirmation, a clinical intervention step confirmation, and a medical device connection confirmation.
230. The method of clause 228, comprising providing a signal indicative of an event marker to the at least one medical device in response to the caregiver confirmation.
231. The method of clause 198, comprising receiving caregiver input via one or more of a touch screen entry or a voice command.
232. The method of clause 231, comprising controlling the CSG UI based on the voice command from a caregiver.
233. The method of clause 231, comprising providing audible output.
234. The method of clause 233, comprising responding to a caregiver instruction to mute or unmute the audible output by muting or unmuting the audible output.
235. The method of clause 200, providing the CSG UI window as a selectable window with at least one other selectable window at the mobile device, the at least one other selectable window comprising a working view window.
236. The method of clause 235, comprising providing at least a portion of the plurality of physiologic parameters in one or more customized display sections of the working view window.
237. The method of clause 236, wherein modifying the data display based on the degradation comprises providing a user notification of the degradation at the working view window, wherein the user notification includes an identification of the degradation and the critical physiologic data.
238. The method of clause 237, comprising providing the user notification in an unoccupied portion of the working view window to emphasize the critical physiologic parameters.
239. The method of clause 237, comprising excluding from the user notification physiologic data other than the critical physiologic data to emphasize the critical physiologic parameters.
240. The method of clause 237, comprising providing the user notification visually and audibly to emphasize the critical physiologic parameters.
241. The method of clause 235, comprising providing at least one CSG control at the working view window configured to transition the mobile device display screen from the working view window to the CSG UI in response to caregiver activation of the at least one CSG control.
242. The method of clause 235, comprising displaying at the working view window a banner after completion of the at least one clinical intervention, the banner comprising one or more of values and trend indicators for the critical physiologic parameters.
243. The method of clause 235, comprising providing the instructions for the at least one clinical intervention and the critical physiologic data at the CSG UI.
244. The method of clause 243, comprising providing one or more of a medication dosage timer and reminders for intervention steps at the CSG UI.
245. The method of clause 243, comprising providing instructions for caregiver use of the at least one medical device at the CSG UI.
246. The method of clause 243, comprising providing status updates for the at least one medical device at the CSG UI.
247. The method of clause 243, comprising excluding from display physiologic data other than the critical physiologic data to emphasize the critical physiologic data at the CSG UI.
248. The method of clause 243, comprising transitioning from the CSG UI window to the working view window in response to caregiver activation of at least one working view control.
249. The method of clause 235, wherein the at least one other selectable window comprises one or more of a device view window and a trend view window.
250. The method of clause 249, comprising providing a real time display at the device view window of at least a portion of the plurality of physiologic parameters in a visual reproduction of a display format provided on a respective medical device.
251. The method of clause 249, comprising providing user-customizable data trend information at the trend view window for the plurality of physiologic parameters.
252. A context sensitive guidance (CSG) system for providing clinical interventions to a patient in an emergency medical event, the CSG system comprising:
   a CSG engine comprising hardware logic and/or software logic;
   a plurality of patient interface devices comprising a pulse oximetry sensor, a capnography sensor, an airway pressure sensor, and a pneumotachometer; and
      a medical device system comprising a patient monitor/defibrillator coupled to a ventilation system, the medical device system configured to:
      receive signals from the plurality of patient interface devices,
         generate physiologic data comprising SpO2, EtCO2, lung elastance, and lung resistance data from the received signals, and
      send the physiologic data to the CSG engine
      wherein the CSG engine is configured to:
      evaluate the SpO2 and the EtCO2 data,
      identify at least one ventilation intervention for the patient based on the evaluation of the SpO2 and the EtCO2 data,
      send a first instruction to the medical device system based on the at least one ventilation intervention
      evaluate the lung elastance and the lung resistance data,
      update the at least one ventilation intervention based on the evaluation of the lung elastance and the lung resistance data, and
      send a second instruction to the medical device system based on the updated at least one ventilation intervention,
   wherein the medical device system is configured to perform at least one operation in response to the first and second instructions.
253. A method of using a context sensitive guidance (CSG) system to provide clinical interventions to a patient in an emergency medical event, the method comprising:
   receiving signals from a plurality of patient interface devices comprising a pulse oximetry sensor, a capnography sensor, an airway pressure sensor, and a pneumotachometer;
   generating physiologic data comprising SpO2, EtCO2, lung elastance, and lung resistance data from the received signals, and
   evaluating the SpO2 and the EtCO2 data,
   identifying at least one ventilation intervention for the patient based on the evaluation of the SpO2 and the EtCO2 data,
   sending a first instruction to at least one medical device based on the at least one ventilation intervention, the at least one medical device comprising a patient monitor/defibrillator coupled to a ventilation system;
   evaluating the lung elastance and lung resistance data,
   updating the at least one ventilation intervention based on the evaluation of the lung elastance and lung resistance data, and
   sending a second instruction to the at least one medical device based on the updated at least one ventilation intervention;
   wherein the first and second instructions comprise instructions for the at least one medical device to perform at least one operation.
254. A respiratory distress (RD) context sensitive guidance (CSG) system for providing RD CSG during an emergency medical encounter, the RD CSG system comprising:
   at least one medical device configured to couple to a patient via one or more patient interface devices, and
   a mobile computing device configured to communicatively couple to the at least one medical device and
   comprising:
      a mobile device display screen,
      a RD CSG engine comprising hardware logic and/or software logic and configured to:
         receive a plurality of physiologic parameters from the at least one medical device,
         provide a user interface (UI) at the mobile device display screen,
         control a display in real-time of the plurality of physiologic parameters at the UI,
         detect a degradation in a medical state of the patient based on a change in at least one physiologic parameter of the plurality of physiologic parameters,
         select at least one clinical intervention based on the detected degradation,
         provide instructions for the at least one clinical intervention,
         identify a subset of the plurality of physiologic parameters as critical physiologic parameters based on the degradation and the at least one clinical intervention, and modify the display of the plurality of physiologic parameters at the UI to emphasize the critical physiologic parameters.
255. The RD CSG system of clause 254, wherein the at least one medical device comprises two or more medical devices, each medical device configured to deliver a clinical intervention that is different from any other of the two or more medical devices.
256. The RD CSG system of clause 255, wherein the two or more medical devices comprise a patient monitor-defibrillator and a ventilation system.
257. The RD CSG system of clause 256, wherein the one or more patient interface devices comprise a pulse oximeter, a nasal cannula or mask coupled to a capnography sensor, a non-invasive blood pressure (NIBP) sensor, a temperature probe, and electrodes.
258. The RD CSG system of clause 257, wherein
   the plurality of physiologic parameters comprises ECG, EtCO2, SpO2, heart rate, body temperature, and non-invasive blood pressure,
   the degradation in the medical state comprises respiratory distress, and
   the critical physiologic parameters comprise EtCO2 and SpO2.
259. The RD CSG system of clause 255, wherein the two or more medical devices comprise at least one first aid kit.
260. The RD CSG system of clause 255,
   wherein the UI is configured to display the plurality of physiologic parameters for only one patient.
261. The RD CSG system of clause 254, wherein the at least one medical device is configured to provide acute care clinical interventions.
262. The RD CSG system of clause 254, wherein the RD CSG engine is configured to replace a display of the plurality of physiologic parameters at a first data view window at the UI with a display of a CSG UI window inclusive of only the critical physiologic parameters to modify the display.
263. The RD CSG system of clause 262, wherein the first data view window at the UI comprises one of a device view window, a trends view window, or a working view window.
264. The RD CSG system of clause 254, wherein the RD CSG engine is configured to provide a user notification at the UI to modify the display.
265. The RD CSG system of clause 264, wherein the user notification comprises a notification of acute respiratory failure.
266. The RD CSG system of clause 264, wherein the RD CSG engine is configured to provide values and/or trends of the critical physiologic parameters with the user notification.
267. The RD CSG system of clause 254,
   wherein the mobile computing device comprises a communications interface configured to:
      establish a first communicative coupling with the at least one medical device, and
      identify the at least one medical device based on the first communicative coupling, and
   wherein the RD CSG engine is configured to:
      provide a connected devices window at the UI that indicates the identification of the at least one medical device.
268. The RD CSG system of clause 267, wherein the communications interface is configured to: authenticate the at least one medical device, and
   establish the first communicative coupling in response to the authentication.
269. The RD CSG system of clause 268, wherein the RD CSG engine is configured to:
   enable patient data encryption configured to prevent access to patient data by unauthenticated devices.
270. The RD CSG system of clause 267, wherein
   the at least one medical device is an initial medical device,
   the communications interface is configured to:
      establish a second communicative coupling with at least one additional medical device subsequent to the initial medical device, and
      identify the at least one additional medical device based on the second communicative coupling, and
   the RD CSG engine is configured to:
      update the connected devices window to include the identification of the at least one additional medical device.
271. The RD CSG system of clause 270, wherein the RD CSG engine is configured to:
   receive the plurality of physiologic parameters from the initial medical device,
   receive one or more additional physiologic parameters from the at least one additional medical device, and modify the UI in real-time to include at least one of the one or more additional physiologic parameters.
272. The RD CSG system of clause 271, wherein the initial medical device comprises a patient monitor-defibrillator and the plurality of physiologic parameters comprises ECG, EtCO2, SpO2, heart rate, body temperature, and non-invasive blood pressure.
273. The RD CSG system of clause 272,
   wherein the at least one additional medical device comprises a ventilation system, and
   wherein the one or more additional physiologic parameters comprise one or more of airway pressure (Paw), plateau pressure (Pplat), peak inspiratory pressure (PIP), patient oxygen saturation (SpO2), fraction of inspired oxygen (FiO2), positive end-expiratory pressure (PEEP), forced vital capacity (FVC), forced expiratory volume (FEV), peak expiratory flow rate (PEF or PEFR), respiratory resistance (Rrs), respiratory compliance (Crs), inspired and expired tidal volume, minute volume (Ve), end-tidal CO2 (EtCO2), and volume of exhaled carbon dioxide (VCO2).
274. The RD CSG system of clause 267, wherein
   the at least one medical device is a medical device suite associated with a same single patient,
   the mobile computing device is configured to communicatively couple with only one medical device suite, and the plurality of physiologic parameters provided at the mobile device display screen are all associated with the same single patient.
275. The RD CSG system of clause 274,
   wherein the medical device suite, the mobile computing device, and the same single patient are disposed at a point of patient care in a local patient care environment, and
   wherein the communications interface is configured to wirelessly communicatively couple the mobile computing device to the medical device suite via one or more of Bluetooth^{®} and Wi-Fi.
276. The RD CSG system of clause 275, wherein the local patient care environment comprises one of a site of an emergency event, a medical triage area, a military field care site, a medical transport vehicle, or a hospital emergency room.
277. The RD CSG system of clause 275, wherein the communications interface is configured to communicatively couple the mobile computing device to one or more additional computing devices in the local patient care environment.
278. The RD CSG system of clause 275, wherein the communications interface is configured to communicatively couple the mobile computing device to one or more remote computing devices outside of the local patient care environment via a long-range wireless network.
279. The RD CSG system of clause 278, wherein the one or more remote computing devices comprise one or more of an electronic patient care record service, a health information exchange, an electronic medical record service, a cloud server, and a computing device associated with a telemedicine provider.
280. The RD CSG system of clause 267, wherein the RD CSG engine is configured to:
   select the at least one clinical intervention based at least in part on the identified at least one medical device; and provide the instructions for the at least one clinical intervention based on the identified at least one medical device.
281. The RD CSG system of clause 280, wherein the RD CSG engine is configured to:
   provide the instructions for the at least one clinical intervention to the at least one medical device.
282. The RD CSG system of clause 281, wherein the instructions comprise one or more of operational mode instructions, operational setting instructions, physiologic closed-loop control instructions for the at least one medical device.
283. The RD CSG system of clause 282,
   wherein the at least one medical device comprises a ventilator, and
   wherein the RD CSG engine is configured to generate the physiologic closed-loop control instructions based on an oxygenation of a patient's blood, wherein the physiologic closed-loop control instructions adjust oxygen delivery during a delivery of a mechanical ventilation to the patient to maintain the oxygen concentration of the patient's blood at a desired level or range.
284. The RD CSG system of clause 280, wherein the instructions for the at least one clinical intervention include instructions provided at a CSG UI window for caregiver use of the identified at least one medical device.
285. The RD CSG system of clause 284,
   wherein the identified at least one medical device is a ventilation system and
   wherein the instructions comprise at least one of ventilation system operation instructions and ventilation system assembly instructions.
286. The RD CSG system of clause 285, wherein the ventilation system operation instructions comprise one or more of power-on instructions and mode selection instructions.
287. The RD CSG system of clause 285, wherein the ventilation system assembly instructions comprise one or more of hose connection instructions, oxygen tank connection instructions, and mask connection instructions.
288. The RD CSG system of clause 284, wherein the instructions for the at least one clinical intervention comprise one or more of bronchodilator administration instructions and mask positioning instructions.
289. The RD CSG system of clause 284, wherein the instructions for the at least one clinical intervention comprise patient monitoring instructions.
290. The RD CSG system of clause 289, wherein the patient monitoring instructions include an indication of therapy targets for the critical physiologic parameters and a status indication for the at least one medical device.
291. The RD CSG system of clause 290,
   wherein the therapy targets for the critical physiologic parameters comprise therapy targets for SpO2 and EtCO2, and
   wherein the status indication for the at least one medical device comprises indication of physiologic closed loop control of a ventilator and an FiO2 value updated in real-time.
292. The RD CSG system of clause 254, wherein the RD CSG engine is configured to:
   provide the instructions for the at least one clinical intervention as caregiver instructions at a CSG UI window.
293. The RD CSG system of clause 292, wherein the caregiver instructions comprise a list of instructions, and wherein the CSG UI window is configured to a visually distinguish between a current step in the list of instructions and one or more of subsequent and previous steps in the list of instructions.
294. The RD CSG system of clause 292, wherein the CSG UI window is configured to provide the caregiver instructions as alphanumeric instructions, graphic instructions, and a combination thereof.
295. The RD CSG system of clause 294, wherein the graphic instructions comprise drawings of the at least one medical device, where the drawings are configured to guide a caregiver through use of the at least one medical device.
296. The RD CSG system of clause 292, wherein the caregiver instructions comprise a hospital transport instruction.
297. The RD CSG system of clause 292, wherein the caregiver instructions comprise drug delivery instructions.
298. The RD CSG system of clause 297, wherein the drug delivery instructions include a drug delivery confirmation control and a dose interval timer.
299. The RD CSG system of clause 292, wherein the RD CSG engine is configured to provide guidance selection controls at the CSG UI window in conjunction with the instructions for the at least one clinical intervention.
300. The RD CSG system of clause 299, wherein the guidance selection controls enable a caregiver to select a level of detail of the provided instructions.
301. The RD CSG system of clause 299, wherein the guidance selection controls comprise at least one of a continue instructions control, an exit instructions control, a proceed to a next step control, an increase a detail level for guidance control, and a return to a previous instruction control, and a mute or unmute audible UI output control.
302. The RD CSG system of clause 292, wherein the RD CSG engine is configured to receive a caregiver confirmation at the mobile computing device in response to the instructions for the at least one clinical intervention.
303. The RD CSG system of clause 302, wherein the caregiver confirmation comprises one or more of a medication administration confirmation, a clinical intervention step confirmation, and a medical device connection confirmation.
304. The RD CSG system of clause 302, wherein the RD CSG engine provides a signal indicative of an event marker to the at least one medical device in response to the caregiver confirmation.
305. The RD CSG system of clause 254, wherein the RD CSG engine is configured to receive caregiver input via one or more of a touch screen entry or a voice command.
306. The RD CSG system of clause 305, wherein the UI comprises a digital assistant configured to control the UI based on the voice command from a caregiver.
307. The RD CSG system of clause 305, wherein the RD CSG engine is configured to provide audible output.
308. The RD CSG system of clause 307, wherein the RD CSG engine is configured to respond to a caregiver instruction to mute or unmute the audible output.
309. The RD CSG system of clause 254, wherein the RD CSG engine is configured to provide a selectable CSG UI window with at least one other selectable window at the mobile device display screen, the at least one other selectable window comprising a working view window.
310. The RD CSG system of clause 309, wherein the working view window is configured to provide at least a portion of the plurality of physiologic parameters in one or more customized display sections.
311. The RD CSG system of clause 310, wherein to modify the display based on the detected degradation comprises to provide a user notification of the detected degradation at the working view window, wherein the user notification includes an identification of the detected degradation and the critical physiologic parameters.
312. The RD CSG system of clause 311, wherein the RD CSG engine provides the user notification in an unoccupied portion of the working view window to emphasize the critical physiologic parameters.
313. The RD CSG system of clause 311, wherein the user notification excludes one or more physiologic parameters other than the critical physiologic parameters to emphasize the critical physiologic parameters.
314. The RD CSG system of clause 311, wherein the UI provides the user notification visually and audibly to emphasize the critical physiologic parameters.
315. The RD CSG system of clause 309, wherein the working view window comprises at least one CSG control configured to transition the mobile device display screen from the working view window to the CSG UI window in response to caregiver activation of the at least one CSG control.
316. The RD CSG system of clause 309, wherein the working view window is configured to display a banner after completion of the at least one clinical intervention, the banner comprising one or more of values and trend indicators for the critical physiologic parameters.
317. The RD CSG system of clause 309, wherein the CSG UI window is configured to provide the instructions for the at least one clinical intervention and the critical physiologic parameters.
318. The RD CSG system of clause 317, wherein the CSG UI window is configured to provide one or more of a medication dosage timer and reminders for intervention steps.
319. The RD CSG system of clause 317, wherein the CSG UI window is configured to provide instructions for caregiver use of the at least one medical device.
320. The RD CSG system of clause 317, wherein the CSG UI window is configured to provide status updates for the at least one medical device.
321. The RD CSG system of clause 317, wherein the CSG UI window excludes one or more physiologic parameters other than the critical physiologic parameters to emphasize the critical physiologic parameters.
322. The RD CSG system of clause 317, wherein the CSG UI window comprises at least one working view control configured to transition from the CSG UI window to the working view window in response to caregiver activation of the at least one working view control.
323. The RD CSG system of clause 309, wherein the at least one other selectable window comprises one or more of a device view window and a trend view window.
324. The RD CSG system of clause 323, wherein the device view window is configured to provide a real time display of at least a portion of the plurality of physiologic parameters in a visual reproduction of a display format provided on a respective medical device.
325. The RD CSG system of clause 323, wherein the trend view window is configured to provide user-customizable data trend information for the plurality of physiologic parameters.

## Claims

1. A respiratory distress, RD, context sensitive guidance, CSG, system for providing RD CSG during an emergency medical encounter, the RD CSG system comprising:
at least one medical device configured to couple to a patient via one or more patient interface devices, and
a mobile computing device configured to communicatively couple to the at least one medical device and comprising:
a mobile device display screen,
a RD CSG engine comprising hardware logic and/or software logic and configured to:
receive a plurality of physiologic parameters from the at least one medical device,
provide a user interface (UI) at the mobile device display screen,
control a display in real-time of the plurality of physiologic parameters at the UI,
detect a degradation in a medical state of the patient based on a change in at least one physiologic parameter of the plurality of physiologic parameters,
select at least one clinical intervention based on the detected degradation,
provide instructions for the at least one clinical intervention,
identify a subset of the plurality of physiologic parameters as critical physiologic parameters based on the degradation and the at least one clinical intervention, and
modify the display of the plurality of physiologic parameters at the UI to emphasize the critical physiologic parameters.

2. The RD CSG system of claim 1, wherein the at least one medical device comprises two or more medical devices comprising a patient monitor-defibrillator and a ventilation system, and wherein the one or more patient interface devices comprise a pulse oximeter, a nasal cannula or mask coupled to a capnography sensor, a non-invasive blood pressure (NIBP) sensor, a temperature probe, and electrodes.

3. The RD CSG system of any preceding claim, wherein
the plurality of physiologic parameters comprises ECG, EtCO2, SpO2, heart rate, body temperature, and non-invasive blood pressure,
the degradation in the medical state comprises respiratory distress, and
the critical physiologic parameters comprise EtCO2 and SpO2.

4. The RD CSG system of any preceding claim, wherein the RD CSG engine is configured to replace a display of the plurality of physiologic parameters at a first data view window at the UI with a display of a CSG UI window inclusive of only the critical physiologic parameters to modify the display; wherein, optionally,
the first data view window at the UI comprises one of a device view window, a trends view window, or a working view window.

5. The RD CSG system of any preceding claim, wherein the RD CSG engine is configured to provide a user notification at the UI to modify the display, the user notification comprising a notification of acute respiratory failure and values and/or trends of the critical physiologic parameters.

6. The RD CSG system of any preceding claim,
wherein the mobile computing device comprises a communications interface configured to:
establish a first communicative coupling with the at least one medical device,
and
identify the at least one medical device based on the first communicative coupling, and
wherein the RD CSG engine is configured to:
provide a connected devices window at the UI that indicates the identification of the at least one medical device.

7. The RD CSG system of claim 6, wherein
the at least one medical device is an initial medical device,
the communications interface is configured to:
establish a second communicative coupling with at least one additional medical device subsequent to the initial medical device, and
identify the at least one additional medical device based on the second communicative coupling, and
the RD CSG engine is configured to:
update the connected devices window to include the identification of the at least one additional medical device; and wherein, optionally,
the RD CSG engine is configured to:
receive the plurality of physiologic parameters from the initial medical device,
receive one or more additional physiologic parameters from the at least one additional medical device, and
modify the UI in real-time to include at least one of the one or more additional physiologic parameters.

8. The RD CSG system of claim 6, wherein the communications interface is configured to communicatively couple the mobile computing device to one or more remote computing devices outside of the local patient care environment via a long-range wireless network; and, optionally,
wherein the one or more remote computing devices comprise one or more of an electronic patient care record service, a health information exchange, an electronic medical record service, a cloud server, and a computing device associated with a telemedicine provider.

9. The RD CSG system of claim 6, wherein the RD CSG engine is configured to:
select the at least one clinical intervention based at least in part on the identified at least one medical device; and
provide the instructions for the at least one clinical intervention based on the identified at least one medical device.

10. The RD CSG system of claim 9, wherein the RD CSG engine is configured to:
provide the instructions for the at least one clinical intervention to the at least one medical device; and
the instructions comprise one or more of operational mode instructions, operational setting instructions, physiologic closed-loop control instructions for the at least one medical device.

11. The RD CSG system of claim 10,
wherein the at least one medical device comprises a ventilator, and
wherein the RD CSG engine is configured to generate the physiologic closed-loop control instructions based on oxygen concentration of a patient's blood, wherein the physiologic closed-loop control instructions adjust oxygen delivery during a delivery of a mechanical ventilation to the patient to maintain the oxygen concentration of the patient's blood at a desired level or range.

12. The RD CSG system of claim 9, wherein the instructions for the at least one clinical intervention include instructions provided at a CSG UI window for caregiver use of the identified at least one medical device; and, optionally,
wherein the identified at least one medical device is a ventilation system and
wherein the instructions comprise at least one of ventilation system operation instructions and ventilation system assembly instructions.

13. The RD CSG system of any preceding claim, wherein the RD CSG engine is configured to:
provide the instruction for the at least one clinical intervention as caregiver instructions at a CSG UI window.

14. The RD CSG system of claim 13, wherein the caregiver instructions comprise one or more of a hospital transport instruction and drug delivery instructions.

15. The RD CSG system of claim 13, wherein the RD CSG engine is configured to receive a caregiver confirmation at the mobile computing device in response to the instructions for the at least one clinical intervention, wherein the caregiver confirmation comprises one or more of a medication administration confirmation, a clinical intervention step confirmation, and a medical device connection confirmation;
and optionally wherein the RD CSG engine provides a signal indicative of an event marker to the at least one medical device in response to the caregiver confirmation.

16. The RD CSG system of any preceding claim, wherein to modify the display based on the detected degradation comprises to provide a user notification of the detected degradation at a working view window, wherein the user notification includes an identification of the detected degradation and the critical physiologic parameters, and wherein the working view window comprises at least one CSG control configured to transition the mobile device display screen from the working view window to the CSG UI window in response to caregiver activation of the at least one CSG control.

17. The RD CSG system of any preceding claim, wherein the RD CSG engine is configured to provide a selectable CSG UI window with at least one other selectable window at the mobile device display screen, the at least one other selectable window comprising a working view window, and wherein the CSG UI window is configured to provide the instructions for the at least one clinical intervention and the critical physiologic parameters; and, optionally,
wherein the CSG UI window is configured to provide one or more of a medication dosage timer, reminders for intervention steps, instructions for caregiver use of the at least one medical device, and status updates for the at least one medical device.
